# EUROPÄISCHE PATENTSCHRIFT

(11) **EP 1 322 590 B1**
(45) Veröffentlichungstag und Bekanntmachung des Hinweises auf die Patenterteilung: **02.05.2007**
(21) Anmeldenummer: 01986671.4
(22) Anmeldetag: 28.09.2001
(51) Int. Cl.: C07C 215/28, C07C 217/64, A61K 31/137, A61K 31/085, A61P 29/00

(54) **SUBSTITUIERTE 5-AMINO-1-PENTEN-3-OL-DERIVATE**
5-AMINO-1-PENTENE-3-OL SUBSTITUTED DERIVATIVES
DERIVES DE 5-AMINO-1-PENTEN-3-OL SUBSTITUES

(30) Priorität: 30.09.2000 DE 10048714
(43) Veröffentlichungstag der Anmeldung: 02.07.2003
(73) Patentinhaber: Grünenthal GmbH, 52078 Aachen (DE)
(72) Erfinder: BUSCHMANN, Helmut, 08950 Esplugues de Llobregat (ES); MAUL, Corinna, 52066 Aachen (DE); SUNDERMANN, Bernd, 52066 Aachen (DE); JAGUSCH, Utz-Peter, 52066 Aachen (DE); HAURAND, Michael, 52078 Aachen (DE); CHIZH, Boris, Cambridge CB2 4NW (GB)
(86) Internationale Anmeldenummer: PCT/EP2001/011244
(87) Internationale Veröffentlichungsnummer: WO 2002/030869

(56) Entgegenhaltungen:
- WO-A-00/02545
- WO-A-00/15611
- WO-A-99/37296

## Beschreibung

Die vorliegende Erfindung betrifft substituierte 5-Amino-1-penten-3-ol-Derivate, Verfahren zu deren Herstellung, Arzneimittel enthaltend diese Verbindungen und die Verwendung von substituierten 5-Amino-1-penten-3-ol-Derivaten zur Herstellung von Arzneimitteln.

Das cyclische GABA Analoge Gabapentin ist ein klinisch erprobtes Antiepileptikum. Gabapentin zeigt zudem weitere interessante, medizinische relevante Eigenschaften, insbesondere als Analgetikum. Interessant sind deshalb neue Strukturklassen, die Affinität zur Gabapentin-Bindungsstelle aufweisen. Es besteht bei den genannten Indikationen weiterer Bedarf an Substanzen, die in ihren Eigenschaften Übereinstimmungen mit Gabapentin zeigen, beispielsweise in der analgetischen Wirkung. Verbindungen mit dieses Eigenschaft sind bekannt aus WO-A-99/37296, WO-A-00/02545 und WO-A-00/15611.

Die Behandlung chronischer und nichtchronischer Schmerzzustände hat in der Medizin eine große Bedeutung. Es besteht ein weltweiter Bedarf an gut wirksamen Schmerztherapien. Der dringende Handlungsbedarf für eine patientengerechte und zielorientierte Behandlung chronischer und nicht chronischer Schmerzzustände, wobei hierunter die erfolgreiche und zufriedenstellende Schmerzbehandlung für den Patienten zu verstehen ist, dokumentiert sich in der großen Anzahl von wissenschaftlichen Arbeiten, die auf dem Gebiet der angewandten Analgetik bzw. der Grundlagenforschung zur Nociception in letzter Zeit erschienen sind.

Klassische Opioide wie Morphin sind bei der Therapie starker bis stärkster Schmerzen gut wirksam. Ihr Einsatz wird jedoch durch die bekannten Nebenwirkungen z.B. Atemdepression, Erbrechen, Sedierung, Obstipation und Toleranzentwicklung limitiert. Außerdem sind sie bei neuropathischen oder inzidentiellen Schmerzen, unter denen insbesondere Tumorpatienten leiden, weniger wirksam.

Aufgabe der Erfindung war es daher, neue Strukturen, die Affinität zur Gabapentin-Bindungsstelle und/oder entsprechende physiologische Wirksamkeiten, beispielsweise in Hinblick auf Analgesie, aufweisen, aufzufinden.

Gegenstand der Erfindung sind daher substituierte 5-Amino-1-penten-3-ol-Derivate der allgemeinen Formel I, 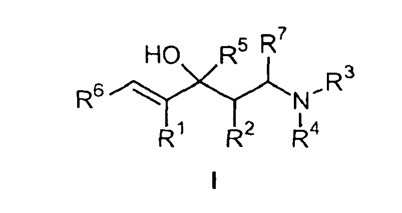 , worin
R¹ und R² jeweils unabhängig voneinander ausgewählt sind aus C₁₋₆-Alkyl, verzweigt oder unverzweigt, gesättigt oder ungesättigt, unsubstituiert oder ein- oder mehrfach substituiert oder
R¹ und R² zusammen einen (CH₂)₂₋₉-Ring bilden, der gegebenfalls mit C₁₋₈-Alkyl, verzweigt oder unverzweigt, gesättigt oder ungesättigt, unsubstituiert oder ein- oder mehrfach substituiert; oder Aryl, unsubstituiert oder ein- oder mehrfach substituiert; substituiert sein kann,
R³ und R⁴ jeweils unabhängig voneinander ausgewählt sind aus C₁₋₆-Alkyl, verzweigt oder unverzweigt, gesättigt oder ungesättigt, unsubstituiert oder ein- oder mehrfach substituiert; C₃₋₆-Cycloalkyl, gesättigt oder ungesättigt, unsubstituiert oder ein- oder mehrfach substituiert; Phenyl, Benzyl oder Phenethyl, unsubstituiert oder ein- oder mehrfach substituiert oder die Reste R³ und R⁴ zusammen einen Ring bilden und CH₂CH₂OCH₂CH₂, CH₂CH₂NR²²CH₂CH₂ oder (CH₂)₃₋₆ bedeuten,
   mit R²² ausgewählt aus H; C₁₋₁₀-Alkyl oder C₃₋₁₀-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über C₁₋₃-Alkyl, gesättigt oder ungesättigt, gebundenem Aryl, C₃₋₁₀-Cycloalkyl oder Heteroaryl, jeweils einfach oder mehrfach substituiert oder unsubstituiert;
R⁵ ausgewählt ist aus C₁₋₁₀-Alkyl, gesättigt oder ungesättigt, verzweigt oder unverzweigt, einfach oder mehrfach substituiert oder unsubstituiert; C₃₋₉-Cycloalkyl, gesättigt oder ungesättigt; Aryl, Heteroaryl, über gesättigtes oder ungesättigtes C₁₋₃-Alkyl gebundenem Aryl, über gesättigtes oder ungesättigtes C₁₋₃-Alkyl gebundenem C₃₋₁₀-Cycloalkyl oder über gesättigtes oder ungesättigtes C₁₋₃-Alkyl gebundenem Heteroaryl, wobei alle Aryl, Heteroaryl und Cycloalkyl-Reste jeweils unabhängig voneinander unsubstituiert oder einfach oder mehrfach substituiert sein können mit Resten unabhängig voneinander ausgewählt aus
   F, Cl, Br, I, OR¹⁸, SR¹⁸, SO₂R¹⁸, SO₂OR¹⁸, CN, COOR¹⁸, NR¹⁹R²⁰; C₁₋₁₀-Alkyl, gesättigt oder ungesättigt, verzweigt oder unverzweigt, einfach oder mehrfach substituiert oder unsubstituiert; C₃₋₉-Cycloalkyl, gesättigt oder ungesättigt, unsubstituiert oder einfach oder mehrfach substituiert; Aryl-, oder Heteroaryl, jeweils unsubstituiert oder einfach oder mehrfach substituiert; oder über gesättigtes oder ungesättigtes C₁₋₃-Alkyl gebundenem Aryl, C₃₋₉-Cycloalkyl oder Heteroaryl, jeweils unsubstituiert oder einfach oder mehrfach substituiert;
   mit R¹⁸ ausgewählt aus H; C₁₋₁₀-Alkyl, gesättigt oder ungesättigt, verzweigt oder unverzweigt, unsubstituiert oder einfach oder mehrfach substituiert; C₃₋₉-Cycloalkyl, gesättigt oder ungesättigt, unsubstituiert oder einfach oder mehrfach substituiert; Aryl-, oder Heteroaryl, unsubstituiert oder einfach oder mehrfach substituiert; oder über gesättigtes oder ungesättigtes C₁₋₃-Alkyl gebundenem Aryl, C₃₋₉-Cycloalkyl oder Heteroaryl, jeweils unsubstituiert oder einfach oder mehrfach substituiert;
   R¹⁹ und R²⁰ unabhängig voneinander ausgewählt aus H; C₁₋₁₀-Alkyl, gesättigt oder ungesättigt, verzweigt oder unverzweigt, unsubstituiert oder einfach oder mehrfach substituiert; C₃₋₉-Cycloalkyl, gesättigt oder ungesättigt, unsubstituiert oder einfach oder mehrfach substituiert; Aryl-, oder Heteroaryl, jeweils unsubstituiert oder einfach oder mehrfach substituiert; oder über gesättigtes oder ungesättigtes C₁₋₃-Alkyl gebundenem Aryl, C₃₋₉-Cycloalkyl oder Heteroaryl, jeweils unsubstituiert oder einfach oder mehrfach substituiert; oder R¹⁹ und R²⁰ zusammen CH₂CH₂OCH₂CH₂, CH₂CH₂NR²¹ CH₂CH₂ oder (CH₂)₃₋₆ bilden,
   mit R²¹ ausgewählt aus H; Phenyl, substituiert oder unsubstituiert; C₁₋₁₀-Alkyl gesättigt oder ungesättigt, verzweigt oder unverzweigt, unsubstituiert oder einfach oder mehrfach substituiert;
R⁶ ausgewählt ist aus C1-10-Alkyl, gesättigt oder ungesättigt, verzweigt oder unverzweigt, einfach oder mehrfach substituiert oder unsubstituiert; C₅₋₇-Cycloalkyl, Aryl oder Heteroaryl, jeweils unsubstituiert oder ein- oder mehrfach substituiert;
R⁷ ausgewählt ist aus H; Aryl oder Heteroaryl; jeweils unsubstituiert oder ein- oder mehrfach substituiert;
gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form ihrer Säuren oder ihrer Basen oder in Form ihrer Salze, insbesondere der physiologisch verträglichen Salze, oder in Form ihrer Solvate, insbesondere der Hydrate.

Die erfindungsgemäßen Substanzen binden an die Gabapentin-Bindungsstelle und zeigen eine ausgeprägte analgetische Wirkung.

Im Sinne dieser Erfindung versteht man unter Alkyl- bzw. Cykloalkyl-Resten gesättigte und ungesättigte (aber nicht aromatische), verzweigte, unverzweigte und cyclische Kohlenwasserstoffe, die unsubstituiert oder ein- oder mehrfach substituiert sein können. Dabei steht C₁₋₂-Alkyl für C1- oder C2-Alkyl, C₁₋₃-Alkyl für C1-, C2- oder C3-Alkyl, C₁₋₄-Alkyl für C1-, C2-, C3- oder C4-Alkyl, C₁₋₅-Alkyl für C1-, C2-, C3-, C4-oder C5-Alkyl, C₁₋₆-Alkyl für C1-, C2-, C3-, C4-, C5- oder C6-Alkyl, C₁₋₇-Alkyl für C1-, C2-, C3-, C4-, C5-, C6- oder C7-Alkyl, C₁₋₈-Alkyl für C1-, C2-, C3-, C4-, C5-, C6-, C7-oder C8-Alkyl, C₁₋₁₀-Alkyl für C1-, C2-, C3-, C4-, C5-, C6-, C7-, C8,- C9- oder C10-Alkyl und C₁₋₁₈-Alkyl für C1-, C2-, C3-, C4-, C5-, C6-, C7-, C8,- C9-, C10-, C11-, C12-, C13-, C14-, C15-, C16-, C17- oder C18-Alkyl. Weiter steht C₃₋₄-Cycloalkyl für C3-oder C4-Cycloalkyl, C₃₋₅-Cycloalkyl für C3-, C4- oder C5-Cycloalkyl, C₃₋₆-Cycloalkyl für C3-, C4-, C5- oder C6-Cycloalkyl, C₃₋₇-Cycloalkyl für C3-, C4-, C5-, C6- oder C7-Cycloalkyl, C₃₋₈-Cycloalkyl für C3-, C4-, C5-, C6-, C7- oder C8-Cycloalkyl, C₄₋₅-Cycloalkyl für C4- oder C5-Cycloalkyl, C₄₋₆-Cycloalkyl für C4-, C5- oder C6-Cycloalkyl, C₄₋₇-Cycloalkyl für C4-, C5-, C6- oder C7-Cycloalkyl, C₅₋₆-Cycloalkyl für C5- oder C6-Cycloalkyl und C₅₋₇-Cycloalkyl für C5-, C6- oder C7-Cycloalkyl, In Bezug auf Cycloalkyl umfaßt der Begriff auch gesättigte Cycloalkyle, in denen ein oder 2 Kohlenstoffatome durch ein Heteroatom, S, N oder O ersetzt sind. Unter den Begriff Cycloalkyl fallen aber insbesondere auch ein- oder mehrfach, vorzugsweise einfach, ungesättigte Cycloalkyle ohne Heteroatom im Ring, solange das Cycloalkyl kein aromatisches System darstellt. Vorzugsweise sind die Alkyl- bzw. Cykloalkyl-Reste Methyl, Ethyl, Vinyl (Ethenyl), Propyl, Allyl (2-Propenyl), 1-Propinyl, Methylethyl, Butyl, 1-Methylpropyl, 2-Methylpropyl, 1,1-Dimethylethyl, Pentyl, 1,1-Dimethylpropyl, 1,2-Dimethylpropyl, 2,2-Dimethylpropyl, Hexyl, 1-Methylpentyl, Cyclopropyl, 2-Methylcyclopropyl, Cyclopropylmethyl, Cyclobutyl, Cyclopentyl, Cyclopentylmethyl, Cyclohexyl, Cycloheptyl, Cyclooctyl, aber auch Adamantyl, CH₂F, CHF₂, CF₃ oder CH₂OH sowie Pyrazolinon, Oxopyrazolinon, [1,4]Dioxan oder Dioxolan.

Dabei versteht man im Zusammenhang mit Alkyl und Cycloalkyl - solange dies nicht ausdrücklich anders definiert ist - unter dem Begriff "substituiert" im Sinne dieser Erfindung die Substitution mindestens eines (gegebenenfalls auch mehrerer) Wasserstoffreste(s) durch F, Cl, Br, I, NH₂, SH oder OH, wobei unter "mehrfach substituiert" bzw. "substituiert" bei mehrfacher Substitution zu verstehen ist, daß die Substitution sowohl an verschiedenen als auch an gleichen Atomen mehrfach mit den gleichen oder verschiedenen Substituenten erfolgt, beispielsweise dreifach am gleichen C-Atom wie im Falle von CF₃ oder an verschiedenen Stellen wie im Falle von -CH(OH)-CH=CH-CHCl₂. Besonders bevorzugte Substituenten sind hier F, Cl und OH. In Bezug auf Cycloalkyl kann der Wasserstoffrest auch durch OC₁₋₃-Alkyl oder C₁₋₃-Alkyl (jeweils ein- oder mehrfach substituiert oder unsubstituiert), insbesondere Methyl, Ethyl, n-Propyl, i-Propyl, CF₃, Methoxy oder Ethoxy, ersetzt sein.

Unter dem Begriff (CH₂)₃₋₆ ist -CH₂-CH₂-CH₂-, -CH₂-CH₂-CH₂-CH₂-, -CH₂-CH₂-CH₂-CH₂-CH₂- und CH₂-CH₂-CH₂-CH₂-CH₂-CH₂- zu verstehen, unter (CH₂)₁₋₄ ist -CH₂-, - CH₂-CH₂-, -CH₂-CH₂-CH₂- und -CH₂-CH₂-CH₂-CH₂- zu verstehen, etc.

Unter einem Aryl-Rest werden Ringsysteme mit mindestens einem armomatischen Ring aber ohne Heteroatome in auch nur einem der Ringe verstanden. Beispiele sind Phenyl-, Naphthyl-, Fluoranthenyl-, Fluorenyl-, Tetralinyl- oder Indanyl, insbesondere 9H-Fluorenyl- oder Anthracenyl-Reste, die unsubstituiert oder einfach oder mehrfach substituiert sein können.

Unter einem Heteroaryl-Rest werden heterocyclische Ringsysteme mit mindestens einem ungesättigten Ring verstanden, die ein oder mehrere Heteroatome aus der Gruppe Stickstoff, Sauerstoff und/oder Schwefel enthalten und auch einfach oder mehrfach substituiert sein können. Beispielhaft seien aus der Gruppe der Heteroaryle Furan, Benzofuran, Thiophen, Benzothiophen, Pyrrol, Pyridin, Pyrimidin, Pyrazin, Chinolin, Isochinolin, Phthalazin, Benzo[1,2,5]thiadiazol, Benzothiazol, Indol, Benzotriazol, Benzodioxolan, Benzodioxan, Carbazol, Indol und Chinazolin aufgeführt.

Dabei versteht man im Zusammenhang mit Aryl und Heteroaryl - wenn nicht ausdrücklich anders definiert - unter substituiert die Substitution des Aryls oder Heteroaryls mit R²³, OR²³ einem Halogen, vorzugsweise F und/oder Cl, einem CF₃, einem CN, einem NO₂, einem NR²⁴R²⁵, einem C₁₋₆-Alkyl (gesättigt), einem C₁₋₆-Alkoxy, einem C₃₋₈-Cycloalkoxy, einem C₃₋₈-Cycloalkyl oder einem C₂₋₆-Alkylen.

Dabei steht der Rest R²³ für H, einen C₁₋₁₀-Alkyl-, vorzugsweise einen C₁₋₆-Alkyl-, einen Aryl- oder Heteroaryl- oder für einen über C₁₋₃-Alkyl, gesättigt oder ungesättigt, oder eine C₁₋₃-Alkylen-Gruppe gebundenen Aryl- oder Heteroaryl-Rest, wobei diese Aryl und Heteroarylreste nicht selbst mit Aryl- oder Heteroaryl-Resten substituiert sein dürfen,
die Reste R²⁴ und R²⁵, gleich oder verschieden, für H, einen C₁₋₁₀-Alkyl-, vorzugsweise einen C₁₋₆-Alkyl-, einen Aryl-, einen Heteroaryl- oder einen über C₁₋₃-Alkyl, gesättigt oder ungesättigt, oder eine C₁₋₃-Alkylen-Gruppe gebundenen Aryl- oder Heteroaryl-Rest bedeuten, wobei diese Aryl und Heteroarylreste nicht selbst mit Aryl- oder Heteroaryl-Resten substituiert sein dürfen,
oder die Reste R²⁴ und R²⁵ bedeuten zusammen CH₂CH₂OCH₂CH₂, CH₂CH₂NR²⁶CH₂CH₂ oder (CH₂)₃₋₆, und
der Rest R²⁶ für H, einen C₁₋₁₀-Alkyl-, vorzugsweise einen C₁₋₆-Alkyl-, einen Aryl-, oder Heteroaryl- Rest oder für einen über C₁₋₃-Alkyl, gesättigt oder ungesättigt, oder eine C₁₋₃-Alkylen-Gruppe gebundenen Aryl- oder Heteroaryl-Rest, wobei diese Aryl und Heteroarylreste nicht selbst mit Aryl- oder Heteroaryl-Resten substituiert sein dürfen.

Unter dem Begriff Salz ist jegliche Form des erfindungsgemäßen Wirkstoffes zu verstehen, in dem dieser eine ionische Form annimmt bzw. geladen ist und mit einem Gegenion (einem Kation oder Anion) gekoppelt ist bzw. sich in Lösung befindet. Darunter sind auch Komplexe des Wirkstoffes mit anderen Molekülen und lonen zu verstehen, insbesondere Komplexe, die über ionische Wechselwirkungen komplexiert sind. Insbesondere versteht man darunter physiologisch verträgliche Salze mit Kationen oder Basen und physiologisch verträgliche Salze mit Anionen oder Säuren.

Unter dem Begriff des physiologisch verträglichen Salzes mit Kationen oder Basen versteht man im Sinne dieser Erfindung Salze mindestens einer der erfindungsgemäßen Verbindungen - meist einer (deprotonierten) Säure - als Anion mit mindestens einem, vorzugsweise anorganischen, Kation, die physiologisch - insbesondere bei Anwendung im Menschen und/oder Säugetier - verträglich sind. Besonders bevorzugt sind die Salze der Alkali- und Erdalkalimetalle aber auch mit NH₄⁺, insbesondere aber (Mono-) oder (Di-) Natrium-, (Mono-) oder (Di-) Kalium-, Magnesium- oder Calzium-Salze.

Unter dem Begriff des physiologisch verträglichen Salzes mit Anionen oder Säuren versteht man im Sinne dieser Erfindung Salze mindestens einer der erfindungsgemäßen Verbindungen - meist, beispielsweise am Stickstoff, protoniert - als Kation mit mindestens einem Anion, die physiologisch - insbesondere bei Anwendung im Menschen und/oder Säugetier - veträglich sind. Insbesondere versteht man darunter im Sinne dieser Erfindung das mit einer physiologisch verträglichen Säure gebildete Salz, nämlich Salze des jeweiligen Wirkstoffes mit anorganischen bzw. organischen Säuren, die physiologisch - insbesondere bei Anwendung im Menschen und/oder Säugetier - verträglich sind. Beispiele für physiologisch verträgliche Salze bestimmter Säuren sind Salze der: Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure, 1,1-Dioxo-1,2-dihydro1λ⁶-benzo[d]isothiazol-3-on (Saccharinsäure), Monomethylsebacinsäure, 5-Oxo-prolin, Hexan-1-sulfonsäure, Nicotinsäure, 2-, 3- oder 4-Aminobenzoesäure, 2,4,6-Trimethyl-benzoesäure, α-Liponsäure, Acetylglycin, Acetylsalicylsäure, Hippursäure und/oder Asparaginsäure. Besonders bevorzugt ist das Hydrochlorid- bzw. Bishydrochlorid-Salz.

In einer bevorzugten Ausführungsform der Erfindung ist bei den erfindungsgemäßen substituierten 5-Amino-1-penten-3-ol-Derivaten gemäß Formel I
R⁷ ausgewählt aus H oder Heteroaryl oder ist R⁷ ein Rest gemäß Formel II, 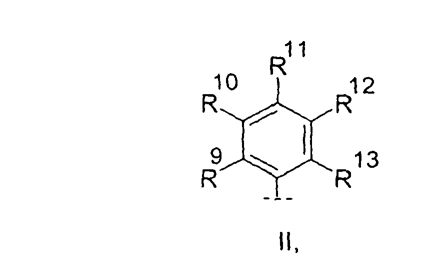 mit R⁹ bis R¹³, jeweils unabhängig voneinander ausgewählt aus H, F, Cl, Br, I, CF₃, CHF₂, CH₂F, OH, OR¹⁴, OCF₃, OCHF₂, OCH₂F, SR¹⁴, SO₂CH₃, SO₂CF_{3;} C₁₋₆-Alkyl, verzweigt oder unverzweigt, gesättigt oder ungesättigt, unsubstituiert oder ein- oder mehrfach substituiert; Phenyl, unsubstituiert oder ein- oder mehrfach substituiert; CN, COOR¹⁴, NO₂ oder
R⁹ und R¹⁰ oder R¹⁰ und R¹¹ zusammen einen OCH₂O- oder OCH₂CH₂O-Ring bilden, und
R¹⁴ ausgewählt ist aus C₁₋₆-Alkyl, verzweigt oder unverzweigt, gesättigt oder ungesättigt, unsubstituiert oder ein- oder mehrfach substituiert; Phenyl, Benzyl, Phenethyl oder Thiophen, jeweils unsubstituiert oder ein- oder mehrfach substituiert, vorzugsweise entspricht R⁷ Wasserstoff.

In einer bevorzugten Ausführungsform der Erfindung bilden bei den erfindungsgemäßen substituierten 5-Amino-1-penten-3-ol-Derivaten gemäß Formel I
R¹ und R² zusammen einen (CH₂)₂₋₅-Ring, der gegebenfalls mit C₁₋₆-Alkyl, verzweigt oder unverzweigt, gesättigt oder ungesättigt, unsubstituiert oder ein- oder mehrfach substituiert; oder Phenyl, unsubstituiert oder ein- oder mehrfach substituiert; substituiert sein kann, der aber vorzugsweise unsubstituiert ist; wobei vorzugsweise R¹ und R² zusammen einen unsubstituierten (CH₂)₂₋₄Ring bilden
oder sind R¹ und R² unabhängig voneinander ausgewählt aus aus C₁₋₃-Alkyl, unverzweigt, gesättigt und unsubstituiert, vorzugsweise CH₃; insbesondere entsprechen R¹ und R² beide CH₃.

In einer weiteren bevorzugten Ausführungsform der Erfindung sind bei den erfindungsgemäßen substituierten 5-Amino-1-penten-3-ol-Derivaten gemäß Formel I
R³ und R⁴ jeweils unabhängig voneinander ausgewählt aus C₁₋₆-Alkyl, verzweigt oder unverzweigt, gesättigt oder ungesättigt, unsubstituiert oder ein- oder mehrfach substituiert; vorzugsweise sind beide CH₃;
oder bilden R³ und R⁴ zusammen einen Ring und bedeuten CH₂CH₂NR²²CH₂CH₂ oder (CH₂)₃₋₆, bedeuten insbesondere zusammen (CH₂)₄₋₅ oder CH₂CH₂NR²²CH₂CH₂, mit R²² ausgewählt aus H oder C₁₋₆-Alkyl, gesättigt, verzweigt oder unverzweigt und unsubstituiert; insbesondere H oder CH₃.

In einer bevorzugten Ausführungsform der Erfindung ist bei den erfindungsgemäßen substituierten 5-Amino-1-penten-3-ol-Derivaten gemäß Formel I
R⁵ ausgewählt aus C₁₋₆-Alkyl, gesättigt oder ungesättigt, verzweigt oder unverzweigt, einfach oder mehrfach substituiert oder unsubstituiert, C₅₋₆-Cycloalkyl, Phenyl, Thiophenyl, Furyl, Benzofuranyl, Benzothiophenyl, Pyrrolyl, Pyridinyl, Pyrimidinyl, Chinolinyl, Isochinolinyl oder Chinazolinyl, vorzugsweise Phenyl, Furyl, Thiophenyl oder C₅₋₆-Cycloalkyl; über gesättigtes oder ungesättigtes C₁₋₃-Alkyl gebundenem Phenyl, C₅₋₆-Cycloalkyl, Thiophenyl, Furyl, Benzofuranyl, Benzothiophenyl, Pyrrolyl, Pyridinyl, Pyrimidinyl, Chinolinyl, Isochinolinyl, Chinazolinyl, vorzugsweise Phenyl, Furyl, Thiophenyl oder C₅₋₆-Cycloalkyl;
wobei alle Aryl, Heteroaryl und Cycloalkyl-Reste jeweils unabhängig voneinander unsubstituiert oder einfach oder mehrfach substituiert sein können, vorzugsweise unsubstituiert oder ein- oder mehrfach mit Substituenten unabhängig voneinander ausgewählt aus F, Cl, Br, I, OH, O-C₁₋₄-Alkyl, C₁₋₆-Alkyl, CF₃, CHF₂, CH₂F, NH₂ und/oder SH substituiert sind,
vorzugsweise
ist R⁵ ausgewählt aus C₁₋₃-Alkyl, gesättigt oder ungesättigt, unsubstituiert und/oder unverzweigt; Naphthyl, Furyl, Cyclohexyl, Cyclopentyl, Phenyl oder Thiophenyl, unsubstituiert oder einfach oder mehrfach, vorzugsweise mit F, Cl, Br, I, CF₃, CHF₂, CH₂F, OH, O-C₁₋₄-Alkyl, C₁₋₆-Alkyl, NH₂ und/oder SH, substituiert; oder über gesättigtes oder ungesättigtes C₁₋₃-Alkyl gebundenem Phenyl, unsubstituiert oder einfach oder mehrfach, vorzugsweise mit F, Cl, Br, I, CF₃, CHF₂, CH₂F, OH, O-C₁₋₄-Alkyl, C₁₋₆-Alkyl, NH₂ und/oder SH, substituiert,
insbesondere
ist R⁵ ausgewählt aus -CH=CH₂, Cyclohexyl, Cyclopentyl; Phenyl, Phenethyl (über CH₂-CH₂ gebundenem Phenyl), Benzyl (über CH₂ gebundenem Phenyl) oder Thiophenyl unsubstituiert oder einfach oder mehrfach, vorzugsweise mit F, Cl, Br, I, OH, OCH₃, OC₂H₅, OC₃H₇, SH, CF₃, CHF₂, CH₂F, CH₃, C₂H₅, C₃H₇ und/oder C₄H₉, bzw. t-Butyl, substituiert.

In einer bevorzugten Ausführungsform der Erfindung ist bei den erfindungsgemäßen substituierten 5-Amino-1-penten-3-ol-Derivaten gemäß Formel I
R⁶ ausgewählt aus Phenyl oder Furyl, jeweils unsubstituiert oder ein- oder mehrfach substituiert, vorzugsweise unsubstituiert oder ein- oder mehrfach mit Substituenten unabhängig voneinander ausgewählt aus Fluor, Chlor, CH₃, OCH₃, CF₃ oder tert.-Butyl substituiert.

In einer bevorzugten Ausführungsform der Erfindung ist bei den erfindungsgemäßen substituierten 5-Amino-1-penten-3-ol-Derivaten gemäß Formel I, wenn R5 ausgewählt ist aus über gesättigtes oder ungesättigtes C1-3-Alkyl gebundenem Aryl, C3-9-Cycloalkyl oder Heteroaryl, das C1-3-Alkyl, über das Aryl, Heteroaryl oder Cycloalkyl gebunden ist, ausgewählt aus:
-CH₂-, -C₂H₄-, C₃H₆-, -C≡C-, -CH=CH-, -CH=CH-CH₂-, -CH₂-CH=CH-, - C≡C-CH₂- oder -CH2-C≡C-, vorzugsweise -CH₂-, -C₂H₄- oder -C≡C-.

In einer bevorzugten Ausführungsform der Erfindung sind die erfindungsgemäßen substituierten 5-Amino-1-penten-3-ol-Derivate ausgewählt aus der folgenden Gruppe:
- 2-Benzyliden-1-(3-chlor-benzyl)-6-dimethylaminomethyl-cyclohexanol
- 2-Benzyliden-6-dimethylaminomethyl-1-(2,5-dimethyl-benzyl)-cyclohexanol
- 2-Benzyliden-1-(2-chlor-6-fluor-benzyl)-6-dimethylaminomethyl-cyclohexanol
- 2-Benzyliden-1-(4-chlor-benzyl)-6-dimethylaminomethyl-cyclohexanol
- 2-Benzyliden-6-dimethylaminomethyl-1-(3-methyl-benzyl)-cyclohexanol
- 2-Benzyliden-6-dimethylaminomethyl-1-(3-trifluormethyl-phenyl)-cyclohexanol
- 2-Benzyliden-1-(3-chlor-4-fluor-phenyl)-6-dimethylaminomethyl-cyclohexanol
- 2-Benzyliden-6-dimethylaminomethyl-1-(2-methyl-benzyl)-cyclohexanol
- 2-Benzyliden-6-dimethylaminomethyl-1-(2-methoxy-phenyl)-cyclohexanol
- 2-Benzyliden-6-dimethylaminomethyl-1-(3-fluor-benzyl)-cyclohexanol
- 2-Benzyliden-1-(4-chlor-3-trifluormethyl-phenyl)-6-dimethylaminomethyl-cyclohexanol
- 2-Benzyliden-6-dimethylaminomethyl-1-(3-methoxy-benzyl)-cyclohexanol
- 2-Benzyliden-6-dimethylaminomethyl-1-(4-fluor-benzyl)-cyclohexanol
- 2-Benzyliden-1-(2-chlor-benzyl)-6-dimethylaminomethyl-cyclohexanol
- 2-Benzyliden-1-(3,5-dichlor-phenyl)-6-dimethylaminomethyl-cyclohexanol
- 2-Benzyliden-1-(3-chlor-phenyl)-6-dimethylaminomethyl-cyclohexanol
- 2-Benzyliden-6-dimethylaminomethyl-1-(3-fluor-phenyl)-cyclohexanol
- 2-Benzyliden-6-dimethylaminomethyl-1-(5-fluor-2-methoxy-phenyl)-cyclohexanol
- 2-Benzyliden-1-cyclohexylmethyl-6-dimethylaminomethyl-cyclohexanol
- 2-Benzyliden-6-dimethylaminomethyl-1-(4-methoxy-phenyl)-cyclohexanol
- 2-Benzyliden-6-dimethylaminomethyl-1-p-tolyl-cyclohexanol
- 2-Benzyliden-6-dimethylaminomethyl-1-(3-phenyl-propyl)-cyclohexanol
- 2-Benzyliden-6-dimethylaminomethyl-1-(3-methoxy-phenyl)-cyclohexanol
- 2-Benzyliden-6-dimethylaminomethyl-1-thiophen-2-yl-cyclohexanol
- 2-Benzyliden-6-dimethylaminomethyl-1-phenylethynyl-cyclohexanol
- 2-Benzyliden-6-dimethylaminomethyl-1-phenethyl-cyclohexanol
- 2-Benzyliden-6-dimethylaminomethyl-1-(4-fluor-phenyl)-cyclohexanol
- 2-Benzyliden-6-dimethylaminomethyl-bicyclohexyl-1-ol
- 2-Benzyliden-6-dimethylaminomethyl-1-m-tolyl-cyclohexanol
- 2-Benzyliden-1-cyclopentyl-6-dimethylaminomethyl-cyclohexanol
- 2-Benzyliden-1-(4-tert-butyl-phenyl)-6-dimethylaminomethyl-cyclohexanol
- 2-Benzyliden-6-dimethylaminomethyl-1-vinyl-cyclohexanol
- 2-Benzyliden-6-dimethylaminomethyl-1-o-tolyl-cyclohexanol
- 2-Benzyliden-6-dimethylaminomethyl-1-(4-fluor-3-methyl-phenyl)-cyclohexanol
- 1-Benzyl-2-benzyliden-6-dimethylaminomethyl-cyclohexanol
- 2-Benzyliden-1-(4-chlor-phenyl)-6-dimethylaminomethyl-cyclohexanol
- 2-Benzyliden-6-dimethylaminomethyl-1-phenyl-cyclohexanol
- 1-(3-Chlor-benzyl)-2-(4-chlor-benzyliden)-6-dimethylaminomethyl-cyclohexanol
- 2-(4-Chlor-benzyliden)-6-dimethylaminomethyl-1-(2,5-dimethyl-benzyl)-cyclohexanol
- 2-(4-Chlor-benzyliden)-1-(2-chlor-6-fluor-benzyl)-6-dimethylaminomethyl-cyclohexanol
- 1-(4-Chlor-benzyl)-2-(4-chlor-benzyliden)-6-dimethylaminomethyl-cyclohexanol
- 2-(4-Chlor-benzyliden)-6-dimethylaminomethyl-1-(3-trifluormethyl-phenyl)-cyclohexanol
- 2-(4-Chlor-benzyliden)-1-(3-chlor-4-fluor-phenyl)-6-dimethylaminomethyl-cyclohexanol
- 2-(4-Chlor-benzyliden)-6-dimethylaminomethyl-1-(2-methyl-benzyl)-cyclohexanol
- 2-(4-Chlor-benzyliden)-6-dimethylaminomethyl-1-(2-methoxy-phenyl)-cyclohexanol
- 2-(4-Chlor-benzyliden)-6-dimethylaminomethyl-1-(3-fluor-benzyl)-cyclohexanol
- 2-(4-Chlor-benzyliden)-1-(4-chlor-3-trifluormethyl-phenyl)-6-dimethylaminomethyl-cyclohexanol
- 2-(4-Chlor-benzyliden)-6-dimethylaminomethyl-1-(3-methoxy-benzyl)-cyclohexanol
- 2-(4-Chlor-benzyliden)-6-dimethylaminomethyl-1-(4-fluor-benzyl)-cyclohexanol
- 1-(2-Chlor-benzyl)-2-(4-chlor-benzyliden)-6-dimethylaminomethyl-cyclohexanol
- 2-(4-Chlor-benzyliden)-1-(3,5-dichlor-phenyl)-6-dimethylaminomethyl-cyclohexanol
- 2-(4-Chlor-benzyliden)-1-(3-chlor-phenyl)-6-dimethylaminomethyl-cyclohexanol
- 2-(4-Chlor-benzyliden)-6-dimethylaminomethyl-1-(3-fluor-phenyl)-cyclohexanol
- 2-(4-Chlor-benzyliden)-6-dimethylaminomethyl-1-(5-fluor-2-methoxy-phenyl)-cyclohexanol
- 2-(4-Chlor-benzyliden)-1-cyclohexylmethyl-6-dimethylaminomethyl-cyclohexanol
- 2-(4-Chlor-benzyliden)-6-dimethylaminomethyl-1-(4-methoxy-phenyl)-cyclohexanol
- 2-(4-Chlor-benzyliden)-6-dimethylaminomethyl-1-p-tolyl-cyclohexanol
- 2-(4-Chlor-benzyliden)-6-dimethylaminomethyl-1-(3-phenyl-propyl)-cyclohexanol
- 2-(4-Chlor-benzyliden)-6-dimethylaminomethyl-1-(3-methoxy-phenyl)-cyclohexanol
- 2-(4-Chlor-benzyliden)-6-dimethylaminomethyl-1-thiophen-2-yl-cyclohexanol
- 2-(4-Chlor-benzyliden)-6-dimethylaminomethyl-1-phenylethynyl-cyclohexanol
- 2-(4-Chlor-benzyliden)-6-d imethylaminomethyl-1-phenethyl-cyclohexanol
- 2-(4-Chlor-benzyliden)-6-dimethylaminomethyl-1-(4-fluor-phenyl)-cyclohexanol
- 2-(4-Chlor-benzyliden)-6-dimethylaminomethyl-bicyclohexyl-1-ol
- 2-(4-Chlor-benzyliden)-6-dimethylaminomethyl-1-m-tolyl-cyclohexanol
- 2-(4-Chlor-benzyliden)-1-cyclopentyl-6-dimethylaminomethyl-cyclohexanol
- 1-(4-tert-Butyl-phenyl)-2-(4-chlor-benzyliden)-6-dimethylaminomethyl-cyclohexanol
- 2-(4-Chlor-benzyliden)-6-dimethylaminomethyl-1-vinyl-cyclohexanol
- 2-(4-Chlor-benzyliden)-6-dimethylaminomethyl-1-o-tolyl-cyclohexanol
- 2-(4-Chlor-benzyliden)-6-dimethylaminomethyl-1-(4-fluor-3-methyl-phenyl)-cyclohexanol
- 1-Benzyl-2-(4-chlor-benzyliden)-6-dimethylaminomethyl-cyclohexanol
- 2-(4-Chlor-benzyliden)-1-(4-chlor-phenyl)-6-dimethylaminomethyt-cyclohexanol
- 2-(4-Chlor-benzyliden)-6-dimethylaminomethyl-1-phenyl-cyclohexanol
- 1-(3-Chlor-benzyl)-2-dimethylaminomethyl-6-(4-methoxy-benzyliden)-cyclohexanol
- 2-Dimethylaminomethyl-1-(2,5-dimethyl-benzyl)-6-(4-methoxy-benzyliden)-cyclohexanol
- 1-(2-Chlor-6-fluor-benzyl)-2-dimethylaminomethyl-6-(4-methoxy-benzyliden)-cyclohexanol
- 2-Dimethylaminomethyl-6-(4-methoxy-benzyliden)-1 -(3-methyl-benzyl)-cyclohexanol
- 2-Dimethylaminomethyl-6-(4-methoxy-benzyliden)-1-(3-trifluormethyl-phenyl)-cyclohexanol
- 1-(3-Chlor-4-fluor-phenyl)-2-dimethylaminomethyl-6-(4-methoxy-benzyliden)-cyclohexanol
- 2-Dimethylaminomethyl-6-(4-methoxy-benzyliden)-1-(2-methoxy-phenyl)-cyclohexanol
- 1-(4-Chlor-3-trifluormethyl-phenyl)-2-dimethylaminomethyl-6-(4-methoxy-benzyliden)-cyclohexanol
- 2-Dimethylaminomethyl-1-(4-fluor-benzyl)-6-(4-methoxy-benzyliden)-cyclohexanol
- 1-(2-Chlor-benzyl)-2-dimethylaminomethyl-6-(4-methoxy-benzyliden)-cyclohexanol
- 1-(3,5-Dichlor-phenyl)-2-dimethylaminomethyl-6-(4-methoxy-benzyliden)-cyclohexanol
- 1-(3-Chlor-phenyl)-2-dimethylaminomethyl-6-(4-methoxy-benzyliden)-cyclohexanol
- 2-Dimethylaminomethyl-1-(5-fluor-2-methoxy-phenyl)-6-(4-methoxy-benzyliden)-cyclohexanol
- 1-Cyclohexylmethyl-2-dimethylaminomethyl-6-(4-methoxy-benzyliden)-cyclohexanol
- 2-Dimethylaminomethyl-6-(4-methoxy-benzyliden)-1-(4-methoxy-phenyl)-cyclohexanol
- 2-Dimethylaminomethyl-6-(4-methoxy-benzyliden)-1-p-tolyl-cyclohexanol
- 1-(2,3-Dichlor-phenyl)-2-dimethylaminomethyl-6-(4-methoxy-benzyliden)-cyclohexanol
- 2-Dimethylaminomethyl-6-(4-methoxy-benzyliden)-1-(3-phenyl-propyl)-cyclohexanol
- 2-Dimethylaminomethyl-6-(4-methoxy-benzyliden)-1-(3-methoxy-phenyl)-cyclohexanol
- 2-Dimethylaminomethyl-6-(4-methoxy-benzyliden)-1-thiophen-2-yl-cyclohexanol
- 2-Dimethylaminomethyl-6-(4-methoxy-benzyliden)-1-phenylethynyl-cyclohexanol
- 2-Dimethylaminomethyl-6-(4-methoxy-benzyliden)-1-phenethyl-cyclohexanol
- 2-Dimethylaminomethyl-1-(4-fluor-phenyl)-6-(4-methoxy-benzyliden)-cyclohexanol
- 6-Dimethylaminomethyl-2-(4-methoxy-benzyliden)-bicyclohexyl-1-ol
- 2-Dimethylaminomethyl-6-(4-methoxy-benzyliden)-1-m-tolyl-cyclohexanol
- 1-Cyclopentyl-2-dimethylaminomethyl-6-(4-methoxy-benzyliden)-cyclohexanol
- 1-(4-tert-Butyl-phenyl)-2-dimethylaminomethyl-6-(4-methoxy-benzyliden)-cyclohexanol
- 2-Dimethylaminomethyl-6-(4-methoxy-benzyliden)-1-vinyl-cyclohexanol
- 2-Dimethylaminomethyl-6-(4-methoxy-benzyliden)-1-o-tolyl-cyclohexanol
- 2-Dimethylaminomethyl-1-(4-fluor-3-methyl-phenyl)-6-(4-methoxy-benzyliden)-cyclohexanol
- 1-Benzyl-2-dimethylaminomethyl-6-(4-methoxy-benzyliden)-cyclohexanol
- 1-(4-Chlor-phenyl)-2-dimethylaminomethyl-6-(4-methoxy-benzyliden)-cyclohexanol
- 2-Dimethylaminomethyl-6-(4-methoxy-benzyliden)-1-phenyl-cyclohexanol
- 1-(3-Chlor-benzyl)-2-dimethylaminomethyl-6-(3-methoxy-benzyliden)-cyclohexanol
- 2-Dimethylaminomethyl-1-(2,5-dimethyl-benzyl)-6-(3-methoxy-benzyliden)-cyclohexanol
- 2-Dimethylaminomethyl-6-(3-methoxy-benzyliden)-1-(2-methyl-benzyl)-cyclohexanol
- 2-Dimethylaminomethyl-6-(3-methoxy-benzyliden)-1-(2-methoxy-phenyl)-cyclohexanol
- 1-(4-Chlor-3-trifluormethyl-phenyl)-2-dimethylaminomethyl-6-(3-methoxy-benzyliden)-cyclohexanol
- 1-(2-Chlor-benzyl)-2-dimethylaminomethyl-6-(3-methoxy-benzyliden)-cyclohexanol
- 6-Dimethylaminomethyl-2-(3-methoxy-benzyliden)-bicyclohexyl-1-ol
- 1-Cyclopentyl-2-dimethylaminomethyl-6-(3-methoxy-benzyliden)-cyclohexanol
- 2-Dimethylaminomethyl-6-(3-methoxy-benzyliden)-1-vinyl-cyclohexanol
- 2-Dimethylaminomethyl-1-(4-fluor-3-methyl-phenyl)-6-(3-methoxy-benzyliden)-cyclohexanol
- 1-Benzyl-2-dimethylaminomethyl-6-(3-methoxy-benzyliden)-cyclohexanol
- 2-Dimethylaminomethyl-6-(3-methoxy-benzyliden)-1-phenyl-cyclohexanol
- 1-Benzyl-2-(2-chlor-benzyliden)-6-dimethylaminomethyl-cyclohexanol
- 5-Dimethylamino-2,4-dimethyl-1,3-diphenyl-pent-1-en-3-ol
- 3-(4-Chlor-phenyl)-5-dimethylamino-2,4-dimethyl-1-phenyl-pent-1-en-3-ol
- 3-Benzyl-5-dimethylamino-2,4-dimethyl-1-phenyl-pent-1-en-3-ol
- 5-Dimethylamino-3-(4-fluor-3-methyl-phenyl)-2,4-dimethyl-1-phenyl-pent-1-en-3-ol
- 5-Dimethylamino-2,4-dimethyl-1-phenyl-3-o-tolyl-pent-1-en-3-ol
- 3-(2-Dimethylamino-1-methyl-ethyl)-2-methyl-1-phenyl-penta-1,4-dien-3-ol
- 3-(4-tert-Butyl-phenyl)-5-dimethylamino-2,4-dimethyl-1-phenyl-pent-1-en-3-ol
- 3-Cyclopentyl-5-dimethylamino-2,4-dimethyl-1-phenyl-pent-1-en-3-ol
- 5-Dimethylamino-2,4-dimethyl-1-phenyl-3-m-tolyl-pent-1-en-3-ol
- 3-Cyclohexyl-5-dimethylamino-2,4-dimethyl-1-phenyl-pent-1-en-3-ol
- 5-Dimethylamino-3-(4-fluor-phenyl)-2,4-dimethyl-1-phenyl-pent-1-en-3-ol
- 5-Dimethylamino-2,4-dimethyl-3-phenethyl-1-phenyl-pent-1-en-3-ol
- 3-(2-Dimethylamino-1-methyl-ethyl)-2-methyl-1,5-diphenyl-pent-1-en-4-yn-3-ol
- 5-Dimethylamino-2,4-dimethyl-1-phenyl-3-thiophen-2-yl-pent-1-en-3-ol
- 3-(2,4-Dichlor-phenyl)-5-dimethylamino-2,4-dimethyl-1-phenyl-pent-1-en-3-ol
- 5-Dimethylamino-3-(3-methoxy-phenyl)-2,4-dimethyl-1-phenyl-pent-1-en-3-ol
- 3-(2-Dimethylamino-1-methyl-ethyl)-2-methyl-1,6-diphenyl-hex-1-en-3-ol
- 3-(2,3-Dichlor-phenyl)-5-dimethylamino-2,4-dimethyl-1-phenyl-pent-1-en-3-ol
- 5-Dimethylamino-2,4-dimethyl-1-phenyl-3-p-tolyl-pent-1-en-3-ol
- 5-Dimethylamino-3-(4-methoxy-phenyl)-2,4-dimethyl-1-phenyl-pent-1-en-3-ol
- 3-Cyclohexylmethyl-5-dimethylamino-2,4-dimethyl-1-phenyl-pent-1-en-3-ol
- 5-Dimethylamino-3-(5-fluor-2-methoxy-phenyl)-2,4-dimethyl-1-phenyl-pent-1-en-3-ol
- 5-Dimethylamino-3-(3-fluor-phenyl)-2,4-dimethyl-1-phenyl-pent-1-en-3-ol
- 3-(3-Chlor-phenyl)-5-dimethylamino-2,4-dimethyl-1-phenyl-pent-1-en-3-ol
- 3-(3,5-Dichlor-phenyl)-5-dimethylamino-2,4-dimethyl-1-phenyl-pent-1-en-3-ol
- 3-(2-Chlor-benzyl)-5-dimethylamino-2,4-dimethyl-1-phenyl-pent-1-en-3-ol
- 5-Dimethylamino-3-(4-fluor-benzyl)-2,4-dimethyl-1-phenyl-pent-1-en-3-ol
- 3-(4-Chlor-3-trifluormethyl-phenyl)-5-dimethylamino-2,4-dimethyl-1-phenyl-pent-1-en-3-ol
- 5-Dimethylamino-3-(2-methoxy-phenyl)-2,4-dimethyl-1-phenyl-pent-1-en-3-ol
- 5-Dimethylamino-2,4-dimethyl-3-(2-methyl-benzyl)-1-phenyl-pent-1-en-3-ol
- 3-(3-Chlor-4-fluor-phenyl)-5-dimethylamino-2,4-dimethyl-1-phenyl-pent-1-en-3-ol
- 5-Dimethylamino-2,4-dimethyl-1-phenyl-3-(3-trifluoromethyl-phenyl)-pent-1-en-3-ol
- 5-Dimethylamino-2,4-dimethyl-3-(3-methyl-benzyl)-1-phenyl-pent-1-en-3-ol
- 3-(4-Chlor-benzyl)-5-dimethylamino-2,4-dimethyl-1-phenyl-pent-1-en-3-ol
- 3-(2-Chlor-6-fluor-benzyl)-5-dimethylamino-2,4-dimethyl-1-phenyl-pent-1-en-3-ol
- 5-Dimethylamino-3-(2,5-dimethyl-benzyl)-2,4-dimethyl-1-phenyl-pent-1-en-3-ol
- 3-(3-Chlor-benzyl)-5-dimethylamino-2,4-dimethyl-1-phenyl-pent-1-en-3-ol
- 3-(2,4-Dichlor-benzyl)-5-dimethylamino-2,4-dimethyl-1-phenyl-pent-1-en-3-ol
- 5-Dimethylamino-1-(4-fluor-phenyl)-2,4-dimethyl-3-phenyl-pent-1-en-3-ol
- 3-Benzyl-5-dimethylamino-1-(4-fluor-phenyl)-2,4-dimethyl-pent-1-en-3-ol
- 5-Dimethylamino-3-(4-fluor-3-methyl-phenyl)-1-(4-fluor-phenyl)-2,4-dimethyl-pent-1-en-3-ol
- 5-Dimethylamino-1-(4-fluor-phenyl)-2,4-dimethyl-3-o-tolyl-pent-1-en-3-ol
- 3-(2-Dimethylamino-1-methyl-ethyl)-1-(4-fluor-phenyl)-2-methyl-penta-1,4-dien-3-ol
- 3-(4-tert-Butyl-phenyl)-5-dimethylamino-1-(4-fluor-phenyl)-2,4-dimethyl-pent-1-en-3-ol
- 3-Cyclopentyl-5-dimethylamino-1-(4-fluor-phenyl)-2,4-dimethyl-pent-1-en-3-ol
- 5-Dimethylamino-1-(4-fluor-phenyl)-2,4-dimethyl-3-m-tolyl-pent-1-en-3-ol
- 3-Cyclohexyl-5-dimethylamino-1-(4-fluor-phenyl)-2,4-dimethyl-pent-1-en-3-ol
- 5-Dimethylamino-1,3-bis-(4-fluor-phenyl)-2,4-dimethyl-pent-1-en-3-ol
- 5-Dimethylamino-1-(4-fluor-phenyl)-2,4-dimethyl-3-phenethyl-pent-1-en-3-ol
- 3-(2-Dimethylamino-1-methyl-ethyl)-1-(4-fluor-phenyl)-2-methyl-5-phenyl-pent-1-en-4-yn-3-ol
- 5-Dimethylamino-1-(4-fluor-phenyl)-2,4-dimethyl-3-thiophen-2-yl-pent-1-en-3-ol
- 3-(2,4-Dichlor-phenyl)-5-dimethylamino-1-(4-fluor-phenyl)-2,4-dimethyl-pent-1-en-3-ol
- 5-Dimethylamino-1-(4-fluor-phenyl)-3-(3-methoxy-phenyl)-2,4-dimethyl-pent-1-en-3-ol
- 3-(2-Dimethylamino-1-methyl-ethyl)-1-(4-fluor-phenyl)-2-methyl-6-phenyl-hex-1-en-3-ol
- 3-(2,3-Dichlor-phenyl)-5-dimethylamino-1-(4-fluor-phenyl)-2,4-dimethyl-pent-1-en-3-ol
- 5-Dimethylamino-1-(4-fluor-phenyl)-2,4-dimethyl-3-p-tolyl-pent-1-en-3-ol
- 5-Dimethylamino-1-(4-fluor-phenyl)-3-(4-methoxy-phenyl)-2,4-dimethyl-pent-1-en-3-ol
- 3-Cyclohexylmethyl-5-dimethylamino-1-(4-fluor-phenyl)-2,4-dimethyl-pent-1-en-3-ol
- 5-Dimethylamino-3-(5-fluor-2-methoxy-phenyl)-1-(4-fluor-phenyl)-2,4-dimethyl-pent-1-en-3-ol
- 5-Dimethylamino-3-(3-fluor-phenyl)-1-(4-fluor-phenyl)-2,4-dimethyl-pent-1-en-3-ol
- 3-(3-Chlor-phenyl)-5-dimethylamino-1-(4-fluor-phenyl)-2,4-dimethyl-pent-1-en-3-ol
- 3-(3,5-Dichlor-phenyl)-5-dimethylamino-1-(4-fluor-phenyl)-2,4-dimethyl-pent-1-en-3-ol
- 3-(2-Chlor-benzyl)-5-dimethylamino-1-(4-fluor-phenyl)-2,4-dimethyl-pent-1-en-3-ol
- 5-Dimethylamino-3-(4-fluor-benzyl)-1-(4-fluor-phenyl)-2,4-dimethyl-pent-1-en-3-ol
- 5-Dimethylamino-1-(4-fluor-phenyl)-3-(3-methoxy-benzyl)-2,4-dimethyl-pent-1-en-3-ol
- 3-(4-Chlor-3-trifluormethyl-phenyl)-5-dimethylamino-1-(4-fluor-phenyl)-2,4-dimethyl-pent-1-en-3-ol
- 5-Dimethylamino-3-(3-fluor-benzyl)-1-(4-fluor-phenyl)-2,4-dimethyl-pent-1-en-3-ol
- 5-Dimethylamino-1-(4-fluor-phenyl)-3-(2-methoxy-phenyl)-2,4-dimethyl-pent-1-en-3-ol
- 5-Dimethylamino-1-(4-fluor-phenyl)-2,4-dimethyl-3-(2-methyl-benzyl)-pent-1-en-3-ol
- 3-(3-Chlor-4-fluor-phenyl)-5-dimethylamino-1-(4-fluor-phenyl)-2,4-dimethyl-pent-1-en-3-ol
- 5-Dimethylamino-1-(4-fluor-phenyl)-2,4-dimethyl-3-(3-trifluormethyl-phenyl)-pent-1-en-3-ol
- 5-Dimethylamino-1-(4-fluor-phenyl)-2,4-dimethyl-3-(3-methyl-benzyl)-pent-1-en-3-ol
- 3-(4-Chlor-benzyl)-5-dimethylamino-1-(4-fluor-phenyl)-2,4-dimethyl-pent-1-en-3-ol
- 3-(2-Chlor-6-fluor-benzyl)-5-dimethylamino-1-(4-fluor-phenyl)-2,4-dimethyl-pent-1-en-3-ol
- 5-Dimethylamino-3-(2,5-dimethyl-benzyl)-1-(4-fluor-phenyl)-2,4-dimethyl-pent-1-en-3-ol
- 3-(3-Chlor-benzyl)-5-dimethylamino-1-(4-fluor-phenyl)-2,4-dimethyl-pent-1-en-3-ol
- 3-(2,4-Dichlor-benzyl)-5-dimethylamino-1-(4-fluor-phenyl)-2,4-dimethyl-pent-1-en-3-ol
- 1-(4-Chlor-phenyl)-5-dimethylamino-2,4-dimethyl-3-phenyl-pent-1-en-3-ol
- 1,3-Bis-(4-chlor-phenyl)-5-dimethylamino-2,4-dimethyl-pent-1-en-3-ol
- 3-Benzyl-1-(4-chlor-phenyl)-5-dimethylamino-2,4-dimethyl-pent-1-en-3-ol
- 1-(4-Chlor-phenyl)-5-dimethylamino-3-(4-fluor-3-methyl-phenyl)-2,4-dimethyl-pent-1-en-3-ol
- 1-(4-Chlor-phenyl)-5-dimethylamino-2,4-dimethyl-3-o-tolyl-pent-1-en-3-ol
- 1-(4-Chlor-phenyl)-3-(2-dimethylamino-1-methyl-ethyl)-2-methyl-penta-1,4-dien-3-ol
- 3-(4-tert-Butyl-phenyl)-1-(4-chlor-phenyl)-5-dimethylamino-2,4-dimethyl-pent-1-en-3-ol
- 1-(4-Chlor-phenyl)-3-cyclopentyl-5-dimethylamino-2,4-dimethyl-pent-1-en-3-ol
- 1-(4-Chlor-phenyl)-5-dimethylamino-2,4-dimethyl-3-m-tolyl-pent-1-en-3-ol
- 1-(4-Chlor-phenyl)-3-cyclohexyl-5-dimethylamino-2,4-dimethyl-pent-1-en-3-ol
- 1-(4-Chlor-phenyl)-5-dimethylamino-3-(4-fluor-phenyl)-2,4-dimethyl-pent-1-en-3-ol
- 1-(4-Chlor-phenyl)-5-dimethylamino-2,4-dimethyl-3-phenethyl-pent-1-en-3-ol
- 1-(4-Chlor-phenyl)-5-dimethylamino-2,4-dimethyl-3-thiophen-2-yl-pent-1-en-3-ol
- 1-(4-Chlor-phenyl)-3-(2,4-dichlor-phenyl)-5-dimethylamino-2,4-dimethyl-pent-1-en-3-ol
- 1-(4-Chlor-phenyl)-5-dimethylamino-3-(3-methoxy-phenyl)-2,4-dimethyl-pent-1-en-3-ol
- 1-(4-Chlor-phenyl)-3-(2-dimethylamino-1-methyl-ethyl)-2-methyl-6-phenyl-hex-1-en-3-ol
- 1-(4-Chlor-phenyl)-3-(2,3-dichlor-phenyl)-5-dimethylamino-2,4-dimethyl-pent-1-en-3-ol
- 1-(4-Chlor-phenyl)-5-dimethylamino-2,4-dimethyl-3-p-tolyl-pent-1-en-3-ol
- 1-(4-Chlor-phenyl)-5-dimethylamino-3-(4-methoxy-phenyl)-2,4-dimethyl-pent-1-en-3-ol
- 1-(4-Chlor-phenyl)-3-cyclohexylmethyl-5-dimethylamino-2,4-dimethyl-pent-1-en-3-ol
- 1-(4-Chlor-phenyl)-5-dimethylamino-3-(5-fluor-2-methoxy-phenyl)-2,4-dimethyl-pent-1-en-3-ol
- 1-(4-Chlor-phenyl)-5-dimethylamino-3-(3-fluor-phenyl)-2,4-dimethyl-pent-1-en-3-ol
- 3-(3-Chlor-phenyl)-1-(4-chlor-phenyl)-5-dimethylamino-2,4-dimethyl-pent-1-en-3-ol
- 1-(4-Chlor-phenyl)-3-(3,5-dichlor-phenyl)-5-dimethylamino-2,4-dimethyl-pent-1-en-3-ol
- 3-(2-Chlor-benzyl)-1-(4-chlor-phenyl)-5-dimethylamino-2,4-dimethyl-pent-1-en-3-ol
- 1-(4-Chlor-phenyl)-5-dimethylamino-3-(4-fluor-benzyl)-2,4-dimethyl-pent-1-en-3-ol
- 1-(4-Chlor-phenyl)-5-dimethylamino-3-(3-methoxy-benzyl)-2,4-dimethyl-pent-1-en-3-ol
- 1-(4-Chlor-phenyl)-3-(4-chlor-3-trifluormethyl-phenyl)-5-dimethylamino-2,4-dimethyl-pent-1-en-3-ol
- 1-(4-Chlor-phenyl)-5-dimethylamino-3-[2-(3-fluor-phenyl)-ethyl]-2,4-dimethyl-pent-1-en-3-ol
- 1-(4-Chlor-phenyl)-5-dimethylamino-3-(2-methoxy-phenyl)-2,4-dimethyl-pent-1-en-3-ol
- 1-(4-Chlor-phenyl)-5-dimethylamino-2,4-dimethyl-3-(2-methyl-benzyl)-pent-1-en-3-ol
- 3-(3-Chlor-4-fluor-phenyl)-1-(4-chlor-phenyl)-5-dimethylamino-2,4-dimethyl-pent-1-en-3-ol
- 1-(4-Chlor-phenyl)-5-dimethylamino-2,4-dimethyl-3-(3-trifluormethyl-phenyl)-pent-1-en-3-ol
- 1-(4-Chlor-phenyl)-5-dimethylamino-2,4-dimethyl-3-(3-methyl-benzyl)-pent-1-en-3-ol
- 3-(4-Chlor-benzyl)-1-(4-chlor-phenyl)-5-dimethylamino-2,4-dimethyl-pent-1-en-3-ol
- 3-(2-Chlor-6-fluor-benzyl)-1-(4-chlor-phenyl)-5-dimethylamino-2,4-dimethyl-pent-1-en-3-ol
- 1-(4-Chlor-phenyl)-5-dimethylamino-3-(2,5-dimethyl-benzyl)-2,4-dimethyl-pent-1-en-3-ol
- 3-(3-Chlor-benzyl)-1-(4-chlor-phenyl)-5-dimethylamino-2,4-dimethyl-pent-1-en-3-ol
- 1-(4-Chlor-phenyl)-3-(2,4-dichlor-benzyl)-5-dimethylamino-2,4-dimethyl-pent-1-en-3-ol
- 5-Dimethylamino-1-(4-methoxy-phenyl)-2,4-dimethyl-3-phenyl-pent-1-en-3-ol
- 3-(4-Chlor-phenyl)-5-dimethylamino-1-(4-methoxy-phenyl)-2,4-dimethyl-pent-1-en-3-ol
- 3-Benzyl-5-dimethylamino-1-(4-methoxy-phenyl)-2,4-dimethyl-pent-1-en-3-ol
- 5-Dimethylamino-3-(4-fluor-3-methyl-phenyl)-1-(4-methoxy-phenyl)-2,4-dimethyl-pent-1-en-3-ol
- 5-Dimethylamino-1-(4-methoxy-phenyl)-2,4-dimethyl-3-o-tolyl-pent-1-en-3-ol
- 3-(2-Dimethylamino-1-methyl-ethyl)-1-(4-methoxy-phenyl)-2-methyl-penta-1,4-dien-3-ol
- 3-(4-tert-Butyl-phenyl)-5-dimethylamino-1-(4-methoxy-phenyl)-2,4-dimethyl-pent-1-en-3-ol
- 3-Cyclopentyl-5-dimethylamino-1-(4-methoxy-phenyl)-2,4-dimethyl-pent-1-en-3-ol
- 5-Dimethylamino-1-(4-methoxy-phenyl)-2,4-dimethyl-3-m-tolyl-pent-1-en-3-ol
- 3-Cyclohexyl-5-dimethylamino-1-(4-methoxy-phenyl)-2,4-dimethyl-pent-1-en-3-ol
- 5-Dimethylamino-3-(4-fluor-phenyl)-1-(4-methoxy-phenyl)-2,4-dimethyl-pent-1-en-3-ol
- 5-Dimethylamino-1-(4-methoxy-phenyl)-2,4-dimethyl-3-phenethyl-pent-1-en-3-ol
- 3-(2-Dimethylamino-1-methyl-ethyl)-1-(4-methoxy-phenyl)-2-methyl-5-phenyl-pent-1-en-4-yn-3-ol
- 5-Dimethylamino-1-(4-methoxy-phenyl)-2,4-dimethyl-3-thiophen-2-yl-pent-1-en-3-ol
- 3-(2,4-Dichlor-phenyl)-5-dimethylamino-1-(4-methoxy-phenyl)-2,4-dimethyl-pent-1-en-3-ol
- 5-Dimethylamino-3-(3-methoxy-phenyl)-1-(4-methoxy-phenyl)-2,4-dimethyl-pent-1-en-3-ol
- 3-(2-Dimethylamino-1-methyl-ethyl)-1-(4-methoxy-phenyl)-2-methyl-6-phenyl-hex-1-en-3-ol
- 3-(2,3-Dichlor-phenyl)-5-dimethylamino-1-(4-methoxy-phenyl)-2,4-dimethyl-pent-1-en-3-ol
- 5-Dimethylamino-1-(4-methoxy-phenyl)-2,4-dimethyl-3-p-tolyl-pent-1-en-3-ol
- 5-Dimethylamino-1,3-bis-(4-methoxy-phenyl)-2,4-dimethyl-pent-1-en-3-ol
- 3-Cyclohexylmethyl-5-dimethylam ino-1-(4-methoxy-phenyl)-2,4-dimethyl-pent-1-en-3-ol
- 5-Dimethylamino-3-(5-fluor-2-methoxy-phenyl)-1-(4-methoxy-phenyl)-2,4-d imethyl-pent-1-en-3-ol
- 5-Dimethylamino-3-(3-fluor-phenyl)-1-(4-methoxy-phenyl)-2,4-dimethyl-pent-1-en-3-ol
- 3-(3-Chlor-phenyl)-5-dimethylamino-1-(4-methoxy-phenyl)-2,4-dimethyl-pent-1-en-3-ol
- 3-(3,5-Dichlor-phenyl)-5-dimethylamino-1-(4-methoxy-phenyl)-2,4-dimethyl-pent-1-en-3-ol
- 3-(2-Chlor-benzyl)-5-dimethylamino-1-(4-methoxy-phenyl)-2,4-dimethyl-pent-1-en-3-ol
- 5-Dimethylamino-3-(4-fluor-benzyl)-1-(4-methoxy-phenyl)-2,4-dimethyl-pent-1-en-3-ol
- 5-Dimethylamino-3-(3-fluor-benzyl)-1-(4-methoxy-phenyl)-2,4-dimethyl-pent-1-en-3-ol
- 5-Dimethylamino-3-(2-methoxy-phenyl)-1-(4-methoxy-phenyl)-2,4-dimethyl-pent-1-en-3-ol
- 5-Dimethylamino-1-(4-methoxy-phenyl)-2,4-dimethyl-3-(2-methyl-benzyl)-pent-1-en-3-ol
- 3-(3-Chlor-4-fluor-phenyl)-5-dimethylamino-1-(4-methoxy-phenyl)-2,4-dimethyl-pent-1-en-3-ol
- 5-Dimethylamino-1-(4-methoxy-phenyl)-2,4-dimethyl-3-(3-trifluormethyl-phenyl)-pent-1-en-3-ol
- 3-(4-Chlor-benzyl)-5-dimethylamino-1-(4-methoxy-phenyl)-2,4-dimethyl-pent-1-en-3-ol
- 3-(2-Chlor-6-fluor-benzyl)-5-dimethylamino-1-(4-methoxy-phenyl)-2,4-dimethyl-pent-1-en-3-ol
- 3-(3-Chlor-benzyl)-5-dimethylamino-1-(4-methoxy-phenyl)-2,4-dimethyl-pent-1-en-3-ol
- 2-Benzyliden-1-(4-tert-butyl-phenyl)-5-dimethylaminomethyl-cyclopentanol
- 2-Benzyliden-1-cyclohexyl-5-dimethylaminomethyl-cyclopentanol
- 2-Benzyliden-5-dimethylaminomethyl-1-phenethyl-cyclopentanol
- 2-Benzyliden-5-dimethylaminomethyl-1-(4-fluor-benzyl)-cyclopentanol
- 2-Benzyliden-5-dimethylaminomethyl-1-(3-fluor-benzyl)-cyclopentanol
- 2-Benzyliden-5-dimethylaminomethyl-1-(2-methoxy-phenyl)-cyclopentanol
- 2-Benzyliden-5-dimethylaminomethyl-1-(2-methyl-benzyl)-cyclopentanol
- 2-Benzyliden-5-dimethylaminomethyl-1-(3-methyl-benzyl)-cyclopentanol
- 2-Benzyliden-1-(4-chlor-benzyl)-5-dimethylaminomethyl-cyclopentanol
- 2-Benzyliden-1-(2-chlor-6-fluor-benzyl)-5-dimethylaminomethyl-cyclopentanol
- 2-Benzyliden-5-dimethylaminomethyl-1-(2,5-dimethyl-benzyl)-cyclopentanol
- 2-Benzyliden-1-(3-chlor-benzyl)-5-dimethylaminomethyl-cyclopentanol
- 2-Dimethylaminomethyl-5-(3-methoxy-benzyl)-bicyclopentyl-1-ol
- 2-Dimethylaminomethyl-5-(3-methoxy-benzyl)-1-m-tolyl-cyclopentanol
- 1-Cyclohexyl-2-dimethylaminomethyl-5-(3-methoxy-benzyl)-cyclopentanol
- 2-Dimethylaminomethyl-5-(3-methoxy-benzyl)-1-phenethyl-cyclopentanol
- 2-Dimethylaminomethyl-5-(3-methoxy-benzyl)-1-phenylethynyl-cyclopentanol
- 2-Dimethylaminomethyl-5-(3-methoxy-benzyl)-1-thiophen-2-yl-cyclopentanol
- 2-Dimethylaminomethyl-5-(3-methoxy-benzyl)-1-(3-methoxy-phenyl)-cyclopentanol
- 2-Dimethylaminomethyl-5-(3-methoxy-benzyl)-1-(3-phenyl-propyl)-cyclopentanol
- 2-Dimethylaminomethyl-5-(3-methoxy-benzyl)-1-(4-methoxy-phenyl)-cyclopentanol
- 2-Dimethylaminomethyl-1-(3-fluor-benzyl)-5-(3-methoxy-benzyliden)-cyclopentanol
- 2-Dimethylaminomethyl-5-(3-methoxy-benzyliden)-1-(2-methoxy-phenyl)-cyclopentanol
- 2-Dimethylaminomethyl-5-(3-methoxy-benzyliden)-1-(2-methyl-benzyl)-cyclopentanol
- 2-Dimethylaminomethyl-5-(3-methoxy-benzyliden)-1-(3-methyl-benzyl)-cyclopentanol
- 2-Dimethylaminomethyl-1-(2,5-dimethyl-benzyl)-5-(3-methoxy-benzyliden)-cyclopentanol
- 1-(3-Chlor-benzyl)-2-dimethylaminomethyl-5-(3-methoxy-benzyliden)-cyclopentanol
- 1-Benzyl-2-dimethylaminomethyl-5-(3-methoxy-benzyliden)-cyclopentanol
- 2-Dimethylaminomethyl-5-(3-methoxy-benzyliden)-1-o-tolyl-cyclopentanol
- 2-Dimethylaminomethyl-5-(3-methoxy-benzyliden)-1-vinyl-cyclopentanol
- 1-(4-tert-Butyl-phenyl)-2-dimethylaminomethyl-5-(3-methoxy-benzyliden)-cyclopentanol
- 2-Dimethylaminomethyl-5-(3-methoxy-benzyliden)-bicyclopentyl-1-ol
- 1-Cyclohexyl-2-dimethylaminomethyl-5-(3-methoxy-benzyliden)-cyclopentanol
- 2-Dimethylaminomethyl-1-(4-fluor-phenyl)-5-(3-methoxy-benzyliden)-cyclopentanol
- 2-Dimethylaminomethyl-5-(3-methoxy-benzyliden)-1-phenethyl-cyclopentanol
- 2-Dimethylaminomethyl-5-(3-methoxy-benzyliden)-1-phenylethynyl-cyclopentanol
- 2-Dimethylaminomethyl-5-(3-methoxy-benzyliden)-1-thiophen-2-yl-cyclopentanol
- 2-Dimethylaminomethyl-5-(3-methoxy-benzyliden)-1-(3-methoxy-phenyl)-cyclopentanol
- 2-Dimethylaminomethyl-5-(3-methoxy-benzyliden)-1-(3-phenyl-propyl)-cyclopentanol
- 2-Dimethylaminomethyl-5-(3-methoxy-benzyliden)-1-p-tolyl-cyclopentanol
- 2-Dimethylaminomethyl-5-(3-methoxy-benzyliden)-1-(4-methoxy-phenyl)-cyclopentanol
- 1-(2-Chlor-benzyl)-2-dimethylaminomethyl-5-(4-methoxy-benzyliden)-cyclopentanol
- 2-Dimethylaminomethyl-1-(3-methoxy-benzyl)-5-(4-methoxy-benzyliden)-cyclopentanol
- 2-Dimethylaminomethyl-1-(3-fluor-benzyl)-5-(4-methoxy-benzyliden)-cyclopentanol
- 2-Dimethylaminomethyl-5-(4-methoxy-benzyliden)-1-(2-methoxy-phenyl)-cyclopentanol
- 2-Dimethylaminomethyl-5-(4-methoxy-benzyliden)-1-(3-methyl-benzyl)-cyclopentanol
- 1-(4-Chlor-benzyl)-2-dimethylaminomethyl-5-(4-methoxy-benzyliden)-cyclopentanol
- 1-(2-Chlor-6-fluor-benzyl)-2-dimethylaminomethyl-5-(4-methoxy-benzyliden)-cyclopentanol
- 1-(3-Chlor-benzyl)-2-dimethylaminomethyl-5-(4-methoxy-benzyliden)-cyclopentanol
- 1-(2,4-Dichlor-benzyl)-2-dimethylaminomethyl-5-(4-methoxy-benzyliden)-cyclopentanol
- 2-Benzyliden-7-dimethylaminomethyl-1-phenyl-cycloheptanol
- 2-Benzyliden-1-(4-chlor-phenyl)-7-dimethylaminomethyl-cycloheptanol
- 1-Benzyl-2-benzyliden-7-dimethylaminomethyl-cycloheptanol
- 2-Benzyliden-7-dimethylaminomethyl-1-(4-fluor-3-methyl-phenyl)-cycloheptanol
- 2-Benzyliden-7-dimethylaminomethyl-1-o-tolyl-cycloheptanol
- 2-Benzyliden-7-dimethylaminomethyl-1-vinyl-cycloheptanol
- 2-Benzyliden-1-(4-tert-butyl-phenyl)-7-dimethylaminomethyl-cycloheptanol
- 2-Benzyliden-1-cyclopentyl-7-dimethylaminomethyl-cycloheptanol
- 2-Benzyliden-7-dimethylaminomethyl-1-m-tolyl-cycloheptanol
- 2-Benzyliden-1-cyclohexyl-7-dimethylaminomethyl-cycloheptanol
- 2-Benzyliden-7-dimethylaminomethyl-1-(4-fluor-phenyl)-cycloheptanol
- 2-Benzyliden-7-dimethylaminomethyl-1-phenylethynyl-cycloheptanol
- 2-Benzyliden-7-dimethylaminomethyl-1-thiophen-2-yl-cycloheptanol
- 2-Benzyliden-7-dimethylaminomethyl-1-(3-methoxy-phenyl)-cycloheptanol
- 2-Benzyliden-1-cyclohexylmethyl-7-dimethylaminomethyl-cycloheptanol
- 2-Benzyliden-7-dimethylaminomethyl-1-(3-fluor-4-methoxy-phenyl)-cycloheptanol
- 2-Benzyliden-7-dimethylaminomethyl-1-(3-fluor-phenyl)-cycloheptanol
- 2-Benzyliden-1-(3-chlor-phenyl)-7-dimethylaminomethyl-cycloheptanol
- 2-Benzyliden-1-(3,5-dichlor-phenyl)-7-dimethylaminomethyl-cycloheptanol
- 2-Benzyliden-7-dimethylaminomethyl-1-(4-fluor-benzyl)-cycloheptanol
- 2-Benzyliden-7-dimethylaminomethyl-1-(4-methoxy-benzyl)-cycloheptanol
- 2-Benzyliden-7-dimethylaminomethyl-1-(3-fluor-benzyl)-cycloheptanol
- 2-Benzyliden-7-dimethylaminomethyl-1-(2-methoxy-phenyl)-cycloheptanol
- 2-Benzyliden-7-dimethylaminomethyl-1-(2-methyl-benzyl)-cycloheptanol
- 2-Benzyliden-1-(3-chlor-4-fluor-phenyl)-7-dimethylaminomethyl-cycloheptanol
- 2-Benzyliden-7-dimethylaminomethyl-1-(3-trifluormethyl-phenyl)-cycloheptanol
- 2-Benzyliden-7-dimethylaminomethyl-1-(3-methyl-benzyl)-cycloheptanol
- 2-Benzyliden-1-(4-chlor-benzyl)-7-dimethylaminomethyl-cycloheptanol
- 2-Benzyliden-1-(2-chlor-6-fluor-benzyl)-7-dimethylaminomethyl-cycloheptanol
- 2-Benzyliden-7-dimethylaminomethyl-1-(2,5-dimethyl-benzyl)-cycloheptanol
- 2-Benzyliden-1-(3-chlor-benzyl)-7-dimethylaminomethyl-cycloheptanol
- 2-Dimethylaminomethyl-7-(3-methoxy-benzyliden)-1-phenyl-cycloheptanol
- 1-(4-Chlor-phenyl)-2-dimethylaminomethyl-7-(3-methoxy-benzyliden)-cycloheptanol
- 1-Benzyl-2-dimethylaminomethyl-7-(3-methoxy-benzyliden)-cycloheptanol
- 2-Dimethylaminomethyl-1-(4-fluor-3-methyl-phenyl)-7-(3-methoxy-benzyliden)-cycloheptanol
- 2-Dimethylaminomethyl-7-(3-methoxy-benzyliden)-1-o-tolyl-cycloheptanol
- 2-Dimethylaminomethyl-7-(3-methoxy-benzyliden)-1-vinyl-cycloheptanol
- 1-(4-tert-Butyl-phenyl)-2-dimethylaminomethyl-7-(3-miathoxy-beniyliden)-cycloheptanol
- 1-Cyclopentyl-2-dimethylaminomethyl-7-(3-methoxy-benzyliden)-cycloheptanol
- 2-Dimethylaminomethyl-7-(3-methoxy-benzyliden)-1-m-tolyl-cycloheptanol
- 1-Cyclohexyl-2-dimethylaminomethyl-7-(3-methoxy-benzyliden)-cycloheptanol
- 2-Dimethylaminomethyl-1-(4-fiuor-phenyl)-7-(3-methoxy-benzyliden)-cyclohepta nol
- 2-Dimethylaminomethyl-7-(3-methoxy-benzyliden)-1-phenethyl-cycloheptanol
- 2-Dimethylaminomethyl-7-(3-methoxy-benzyliden)-1-phenylethynyl-cycloheptanol
- 2-Dimethylaminomethyl-7-(3-methoxy-benzyliden)-1-thiophen-2-yl-cycloheptanol
- 2-Dimethylaminomethyl-7-(3-methoxy-benzyliden)-1-(3-methoxy-phenyl)-cycloheptanol
- 2-Dimethylaminomethyl-7-(3-methoxy-benzyliden)-1-(3-phenyl-propyl)-cycloheptanol
- 2-Dimethylaminomethyl-7-(3-methoxy-benzyliden)-1-p-tolyl-cycloheptanol
- 2-Dimethylaminomethyl-7-(3-methoxy-benzyliden)-1-(4-methoxy-phenyl)-cycloheptanol
- 1-Cyclohexylmethyl-2-dimethylaminomethyl-7-(3-methoxy-benzyliden)-cycloheptanol
- 1-(3-Chlor-phenyl)-2-dimethylaminomethyl-7-(3-methoxy-benzyliden)-cycloheptanol
- 1-(3,5-Dichlor-phenyl)-2-dimethylaminomethyl-7-(3-methoxy-benzyliden)-cycloheptanol
- 1-(2-Chlor-benzyl)-2-dimethylaminomethyl-7-(3-methoxy-benzyliden)-cycloheptanol
- 1-(4-Chlor-3-trifluormethyl-phenyl)-2-dimethylaminomethyl-7-(3-methoxy-benzyliden)-cycloheptanol
- 2-Dimethylaminomethyl-1-(3-fluor-phenyl)-7-(3-methoxy-benzyliden)-cycloheptanol
- 2-Dimethylaminomethyl-7-(3-methoxy-benzyliden)-1-(2-methoxy-phenyl)-cycloheptanol
- 2-Dimethylaminomethyl-7-(3-methoxy-benzyliden)-1-(2-methyl-benzyl)-cycloheptanol
- 2-Dimethylaminomethyl-7-(3-methoxy-benzyliden)-1-(3-trifluormethyl-phenyl)-cycloheptanol
- 2-Dimethylaminomethyl-7-(3-methoxy-benzyliden)-1-(3-methyl-benzyl)-cycloheptanol
- 2-Dimethylaminomethyl-1-(2,5-dimethyl-benzyl)-7-(3-methoxy-benzyliden)-cycloheptanol
- 1-(3-Chlor-benzyl)-2-dimethylaminomethyl-7-(3-methoxy-benzyliden)-cycloheptanol
- 1-Benzyl-2-dimethylaminomethyl-7-(4-methoxy-benzyliden)-cycloheptanol
- 2-Dimethylaminomethyl-1-(4-fluor-3-methyl-phenyl)-7-(4-methoxy-benzyliden)-cycloheptanol
- 2-Dimethylaminomethyl-7-(4-methoxy-benzyliden)-1-o-tolyl-cycloheptanol
- 2-Dimethylaminomethyl-7-(4-methoxy-benzyliden)-1-vinyl-cycloheptanol
- 1-(4-tert-Butyl-phenyl)-2-dimethylaminomethyl-7-(4-methoxy-benzyliden)-cycloheptanol
- 1-Cyclopentyl-2-dimethylaminomethyl-7-(4-methoxy-benzyliden)-cycloheptanol
- 2-Dimethylaminomethyl-7-(4-methoxy-benzyliden)-1-m-tolyl-cycloheptanol
- 1-Cyclohexyl-2-dimethylaminomethyl-7-(4-methoxy-benzyliden)-cycloheptanol
- 2-Dimethylaminomethyl-1-(4-fluor-phenyl)-7-(4-methoxy-benzyliden)-cycloheptanol
- 2-Dimethylaminomethyl-7-(4-methoxy-benzyliden)-1-phenethyl-cycloheptanol
- 2-Dimethylaminomethyl-7-(4-methoxy-benzyliden)-1-phenylethynyl-cycloheptanol
- 2-Dimethylaminomethyl-7-(4-methoxy-benzyliden)-1-thiophen-2-yl-cycloheptanol
- 2-Dimethylaminomethyl-7-(4-methoxy-benzyliden)-1-(3-methoxy-phenyl)-cycloheptanol
- 2-Dimethylaminomethyl-7-(4-methoxy-benzyliden)-1-(3-phenyl-propyl)-cycloheptanol
- 2-Dimethylaminomethyl-7-(4-methoxy-benzyliden)-1-p-tolyl-cycloheptanol
- 2-Dimethylaminomethyl-7-(4-methoxy-benzyliden)-1-(4-methoxy-phenyl)-cycloheptanol
- 1-Cyclohexylmethyl-2-dimethylaminomethyl-7-(4-methoxy-benzyliden)-cycloheptanol
- 1-(3-Chlor-phenyl)-2-dimethylaminomethyl-7-(4-methoxy-benzyliden)-cycloheptanol
- 1-(2-Chlor-benzyl)-2-dimethylaminomethyl-7-(4-methoxy-benzyliden)-cycloheptanol
- 2-Dimethylaminomethyl-7-(4-methoxy-benzyliden)-1-(2-methoxy-phenyl)-cycloheptanol
- 2-Dimethylaminomethyl-7-(4-methoxy-benzyliden)-1-(2-methyl-benzyl)-cycloheptanol
- 2-Dimethylaminomethyl-7-(4-methoxy-benzyliden)-1-(3-trifluormethyl-phenyl)-cycloheptanol
- 2-Dimethylaminomethyl-7-(4-methoxy-benzyliden)-1-(3-methyl-benzyl)-cycloheptanol
- 1-(3-Chlor-benzyl)-2-dimethylaminomethyl-7-(4-methoxy-benzyliden)-cycloheptanol
- 1-(3,5-Dichlor-phenyl)-2-dimethylaminomethyl-7-(4-methoxy-benzyliden)-cycloheptanol
- 2-Benzyliden-7-dimethylaminomethyl-1-(3-hydroxy-phenyl)-cycloheptanol;
- 2-Dimethylaminomethyl-1-(3-hydroxy-phenyl)-7-(3-methoxy-benzyliden)-cycloheptanol;
- 2-Benzyliden-7-dimethylaminomethyl-1-(3-methoxy-phenyl)-cycloheptanol;
- 3-[1-(2-Dimethylamino-1-methyl-ethyl)-1-hydroxy-2-methyl-3-phenyl-allyl]-phenol;
- 3-(4-Chlor-benzyl)-5-dimethylamino-2,4-dimethyl-1-phenyl-pent-1-en-3-ol;
- 5-Dimethylamino-3-(3-methoxy-phenyl)-2,4-dimethyl-1-phenyl-pent-1-en-3-ol;
- 3-(2-Benzyliden-6-dimethylaminomethyl-1-hydroxy-cyclohexyl)-phenol;
- 1-Benzyl-2-benzyliden-7-(4-methyl-piperazin-1-ylmethyl)-cycloheptanol;
- 2-Benzyliden-1-(2-chlor-benzyl)-7-(4-methyl-piperazin-1-ylmethyl)-cycloheptanol;
- 2-Benzyliden-1-(3-chlor-benzyl)-7-(4-methyl-piperazin-1-ylmethyl)-cycloheptanol;
- 2-Benzyliden-1-(4-chlor-benzyl)-7-(4-methyl-piperazin-1-ylmethyl)-cycloheptanol;
- 2-Benzyliden-1-(2-fluor-benzyl)-7-(4-methyl-piperazin-1-ylmethyl)-cycloheptanol;
- 2-Benzyliden-1-(3-fluor-benzyl)-7-(4-methyl-piperazin-1-ylmethyl)-cycloheptanol;
- 2-Benzyliden-1-(4-fluor-benzyl)-7-(4-methyl-piperazin-1-ylmethyl)-cycloheptanol;
- 2-Benzyliden-1-(2-methyl-benzyl)-7-(4-methyl-piperazin-1-ylmethyl)-cycloheptanol;
- 2-Benzyliden-1-(3-methyl-benzyl)-7-(4-methyl-piperazin-1-ylmethyl)-cycloheptanol;
- 2-Benzyliden-1-(4-methyl-benzyl)-7-(4-methyl-piperazin-1-ylmethyl)-cycloheptanol;
- 2-Benzyliden-1-(2-methoxy-benzyl)-7-(4-methyl-piperazin-1-ylmethyl)-cycloheptanol;
- 2-Benzyliden-1-(3-methoxy-benzyl)-7-(4-methyl-piperazin-1-ylmethyl)-cycloheptanol;
- 2-Benzyliden-1-(4-methoxy-benzyl)-7-(4-methyl-piperazin-1-ylmethyl)-cycloheptanol;
- 2-Benzyliden-1-(2,6-dichlor-benzyl)-7-(4-methyl-piperazin-1-ylmethyl)-cycloheptanol;
- 2-Benzyliden-1-(2-chlor-6-fluor-benzyl)-7-(4-methyl-piperazin-1-ylmethyl)-cycloheptanol;
- 2-Benzyliden-1-(2,6-difluor-benzyl)-7-(4-methyl-piperazin-1-ylmethyl)-cycloheptanol;
- 1-Benzyl-2-(4-chlor-benzyliden)-7-(4-methyl-piperazin-1-ylmethyl)-cycloheptanol;
- 1-(2-Chlor-benzyl)-2-(4-chlor-benzyliden)-7-(4-methyl-piperazin-1-ylmethyl)-cycloheptanol;
- 1-(3-Chlor-benzyl)-2-(4-chlor-benzyliden)-7-(4-methyl-piperazin-1-ylmethyl)-cycloheptanol;
- 1-(4-Chlor-benzyl)-2-(4-chlor-benzyliden)-7-(4-methyl-piperazin-1-ylmethyl)-cycloheptanol;
- 2-(4-Chlor-benzyliden)-1-(2-fluor-benzyl)-7-(4-methyl-piperazin-1-ylmethyl)-cycloheptanol;
- 2-(4-Chlor-benzyliden)-1-(3-fluor-benzyl)-7-(4-methyl-piperazin-1-ylmethyl)-cycloheptanol;
- 2-(4-Chlor-benzyliden)-1-(4-fluor-benzyl)-7-(4-methyl-piperazin-1-ylmethyl)-cycloheptanol;
- 2-(4-Chlor-benzyliden)-1-(2-methyl-benzyl)-7-(4-methyl-piperazin-1-ylmethyl)-cycloheptanol;
- 2-(4-Chlor-benzyliden)-1-(3-methyl-benzyl)-7-(4-methyl-piperazin-1-ylmethyl)-cycloheptanol;
- 2-(4-Chlor-benzyliden)-1-(4-methyl-benzyl)-7-(4-methyl-piperazin-1-ylmethyl)-cycloheptanol;
- 2-(4-Chlor-benzyliden)-1-(2-methoxy-benzyl)-7-(4-methyl-piperazin-1-ylmethyl)-cycloheptanol;
- 2-(4-Chlor-benzyliden)-1-(3-methoxy-benzyl)-7-(4-methyl-piperazin-1-ylmethyl)-cycloheptanol;
- 2-(4-Chlor-benzyliden)-1-(4-methoxy-benzyl)-7-(4-methyl-piperazin-1-ylmethyl)-cycloheptanol;
- 2-(4-Chlor-benzyliden)-1-(2,6-dichlor-benzyl)-7-(4-methyl-piperazin-1-ylmethyl)-cycloheptanol;
- 2-(4-Chlor-benzyliden)-1-(2,6-difluor-benzyl)-7-(4-methyl-piperazin-1-ylmethyl)-cycloheptanol;
- 2-(4-Chlor-benzyliden)-1-(2-chlor-6-fluor-benzyl)-7-(4-methyl-piperazin-1-ylmethyl)-cycloheptanol;
- 1-Benzyl-2-(4-fluor-benzyliden)-7-(4-methyl-piperazin-1-ylmethyl)-cycloheptanol;
- 1-(2-Chlor-benzyl)-2-(4-fluor-benzyliden)-7-(4-methyl-piperazin-1-ylmethyl)-cycloheptanol;
- 1-(3-Chlor-benzyl)-2-(4-fluor-benzyliden)-7-(4-methyl-piperazin-1-ylmethyl)-cycloheptanol;
- 1-(4-Chlor-benzyl)-2-(4-fluor-benzyliden)-7-(4-methyl-piperazin-1-ylmethyl)-cycloheptanol;
- 1-(2-Fluor-benzyl)-2-(4-fluor-benzyliden)-7-(4-methyl-piperazin-1-ylmethyl)-cycloheptanol;
- 1-(3-Fluor-benzyl)-2-(4-fluor-benzyliden)-7-(4-methyl-piperazin-1-ylmethyl)-cycloheptanol;
- 1-(4-Fluor-benzyl)-2-(4-fluor-benzyliden)-7-(4-methyl-piperazin-1-ylmethyl)-cycloheptanol;
- 2-(4-Fluor-benzyliden)-1-(2-methyl-benzyl)-7-(4-methyl-piperazin-1-ylmethyl)-cycloheptanol;
- 2-(4-Fluor-benzyliden)-1-(3-methyl-benzyl)-7-(4-methyl-piperazin-1-ylmethyl)-cycloheptanol;
- 2-(4-Fluor-benzyliden)-1-(4-methyl-benzyl)-7-(4-methyl-piperazin-1-ylmethyl)-cycloheptanol;
- 2-(4-Fluor-benzyliden)-1-(2-methoxy-benzyl)-7-(4-methyl-piperazin-1-ylmethyl)-cycloheptanol;
- 2-(4-Fluor-benzyliden)-1-(3-methoxy-benzyl)-7-(4-methyl-piperazin-1-ylmethyl)-cycloheptanol;
- 2-(4-Fluor-benzyliden)-1-(4-methoxy-benzyl)-7-(4-methyl-piperazin-1-ylmethyl)-cycloheptanol;
- 1-(2,6-Dichlor-benzyl)-2-(4-fluor-benzyliden)-7-(4-methyl-piperazin-1-ylmethyl)-cycloheptanol;
- 1-(2,6-Difluor-benzyl)-2-(4-fluor-benzyliden)-7-(4-methyl-piperazin-1-ylmethyl)-cycloheptanol;
- 1-(2-Chlor-6-fluorbenzyl)-2-(4-fluor-benzyliden)-7-(4-methyl-piperazin-1-ylmethyl)-cycloheptanol;
- 1-Benzyl-2-furan-2-ylmethylen-7-(4-methyl-piperazin-1-ylmethyl)-cycloheptanol;
- 1-(2-Chlor-benzyl)-2-furan-2-ylmethylen-7-(4-methyl-piperazin-1-ylmethyl)-cycloheptanol;
- 1-(3-Chlor-benzyl)-2-furan-2-ylmethylen-7-(4-methyl-piperazin-1-ylmethyl)-cycloheptanol;
- 1-(4-Chlor-benzyl)-2-furan-2-ylmethylen-7-(4-methyl-piperazin-1-ylmethyl)-cycloheptanol;
- 1-(2-Fluor-benzyl)-2-furan-2-ylmethylen-7-(4-methyl-piperazin-1-ylmethyl)-cycloheptanol;
- 1-(3-Fluor-benzyl)-2-furan-2-ylmethylen-7-(4-methyl-piperazin-1-ylmethyl)-cycloheptanol;
- 1-(4-Fluor-benzyl)-2-furan-2-ylmethylen-7-(4-methyl-piperazin-1-ylmethyl)-cycloheptanol;
- 2-Furan-2-ylmethylen-1-(2-methyl-benzyl)-7-(4-methyl-piperazin-1-ylmethyl)-cycloheptanol;
- 2-Furan-2-ylmethylen-1-(3-methyl-benzyl)-7-(4-methyl-piperazin-1-ylmethyl)-cycloheptanol;
- 2-Furan-2-ylmethylen-1-(4-methyl-benzyl)-7-(4-methyl-piperazin-1-ylmethyl)-cycloheptanol;
- 2-Furan-2-ylmethylen-1-(2-methoxy-benzyl)-7-(4-methyl-piperazin-1-ylmethyl)-cycloheptanol;
- 2-Furan-2-ylmethylen-1-(3-methoxy-benzyl)-7-(4-methyl-piperazin-1-ylmethyl)-cycloheptanol;
- 2-Furan-2-ylmethylen-1-(4-methoxy-benzyl)-7-(4-methyl-piperazin-1-ylmethyl)-cycloheptanol;
- 1-(2,6-Dichlor-benzyl)-2-furan-2-ylmethylen-7-(4-methyl-piperazin-1-ylmethyl)-cycloheptanol;
- 1-(2,6-Difluor-benzyl)-2-furan-2-ylmethylen-7-(4-methyl-piperazin-1-ylmethyl)-cycloheptanol;
- 1-(2-Chlor-6-fluor-benzyl)-2-furan-2-ylmethylen-7-(4-methyl-piperazin-1-ylmethyl)-cycloheptanol;
- 1-Benzyl-3-furan-2-ylmethylen-7-(4-methyl-piperazin-1-ylmethyl)-cycloheptanol;
- 1-(2-Chlor-benzyl)-3-furan-2-ylmethylen-7-(4-methyl-piperazin-1-ylmethyl)-cycloheptanol;
- 1-(3-Chlor-benzyl)-3-furan-2-ylmethylen-7-(4-methyl-piperazin-1-ylmethyl)-cycloheptanol;
- 1-(4-Chlor-benzyl)-3-furan-2-ylmethylen-7-(4-methyl-piperazin-1-ylmethyl)-cycloheptanol;
- 1-(2-Fluor-benzyl)-3-furan-2-ylmethylen-7-(4-methyl-piperazin-1-ylmethyl)-cycloheptanol;
- 1-(3-Fluor-benzyl)-3-furan-2-ylmethylen-7-(4-methyl-piperazin-1-ylmethyl)-cycloheptanol;
- 1-(4-Fluor-benzyl)-3-furan-2-ylmethylen-7-(4-methyl-piperazin-1-ylmethyl)-cycloheptanol;
- 3-Furan-2-ylmethylen-1-(2-methyl-benzyl)-7-(4-methyl-piperazin-1-ylmethyl)-cycloheptanol;
- 3-Furan-2-ylmethylen-1-(3-methyl-benzyl)-7-(4-methyl-piperazin-1-ylmethyl)-cycloheptanol;
- 3-Furan-2-ylmethylen-1-(4-methyl-benzyl)-7-(4-methyt-piperazin-1-ylmethyl)-cycloheptanol;
- 3-Fura n-2-ylmethylen-1-(2-methoxy-benzyl)-7-(4-methyl-piperazin-1-ylmethyl)-cycloheptanol;
- 3-Furan-2-ylmethylen-1-(3-methoxy-benzyl)-7-(4-methyl-piperazin-1-ylmethyl)-cycloheptanol;
- 3-Furan-2-ylmethylen-1-(4-methoxy-benzyl)-7-(4-methyl-piperazin-1-ylmethyl)-cycloheptanol;
- 1-(2,6-Dichlor-benzyl)-3-furan-2-ylmethylen-7-(4-methyl-piperazin-1-ylmethyl)-cycloheptanol;
- 1-(2,6-Difluor-benzyl)-3-furan-2-ylmethylen-7-(4-methyl-piperazin-1-ylmethyl)-cycloheptanol;
- 1-(2-Chlor-6-fluor-benzyl)-3-furan-2-ylmethylen-7-(4-methyl-piperazin-1-ylmethyl)-cycloheptanol
gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form ihrer Säuren oder ihrer Basen oder in Form ihrer Salze, insbesondere der physiologisch verträglichen Salze, oder in Form ihrer Solvate, insbesondere der Hydrate, vorzugsweise des Hydrochlorids oder Bishydrochlorids.

In einer bevorzugten Ausführungsform der Erfindung sind bei den erfindungsgemäßen substituierten 5-Amino-1-penten-3-ol-Derivaten gemäß Formel I die E-Isomeren der Derivate gemäß Formel I bevorzugt wie in Formel I' gezeigt:

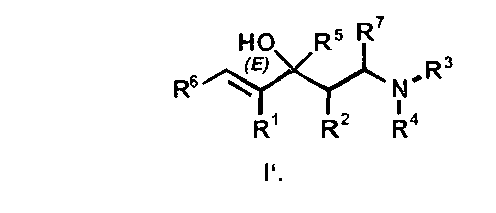

In einer weiteren bevorzugten Ausführungsform der Erfindung stehen bei den erfindungsgemäßen substituierten 5-Amino-1-penten-3-ol-Derivaten gemäß Formel I, wenn R7 Wasserstoff ist und R1 und R2 zusammen einen Ring bilden, die OH-Gruppe und die Aminomethylengruppe CHR⁷-NR³R⁴ gemäß Formel I cis zueinander stehen, wie in Formel I" gezeigt:

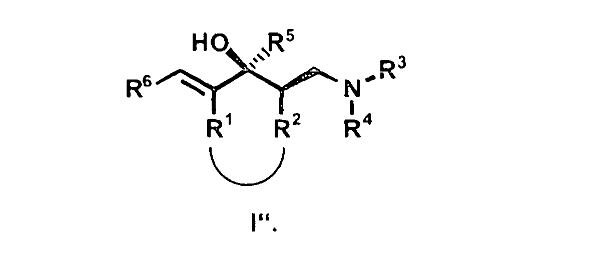

Die erfindungsgemäßen Substanzen sind toxikologisch unbedenklich, so daß sie sich als pharmazeutischer Wirkstoff in Arzneimitteln eignen. Ein weiterer Gegenstand der Erfindung sind daher Arzneimittel enthaltend wenigstens ein erfindungsgemäßes substituiertes 5-Amino-1-penten-3-ol-Derivat, sowie gegebenenfalls geeignete Zusatz- und/oder Hilfsstoffe und/oder gegebenenfalls weitere Wirkstoffe.

Die erfindungsgemäßen Arzneimittel enthalten neben mindestens einem erfindungsgemäßen substituierten 5-Amino-1-penten-3-ol-Derivat gegebenenfalls geeignete Zusatz- und/oder Hilfsstoffe, so auch auch Trägermaterialien, Füllstoffe, Lösungsmittel, Verdünnungsmittel, Farbstoffe und/oder Bindemittel und können als flüssige Arzneiformen in Form von Injektionslösungen, Tropfen oder Säfte, als halbfeste Arzneiformen in Form von Granulaten, Tabletten, Pellets, Patches, Kapseln, Pflaster oder Aerosolen verabreicht werden. Die Auswahl der Hilfsstoffe etc. sowie die einzusetzenden Mengen derselben hängen davon ab, ob das Arzneimittel oral, peroral, parenteral, intravenös, intraperitoneal, intradermal, intramuskulär, intranasal, buccal, rektal oder örtlich, zum Beispiel auf die Haut, die Schleimhäute oder in die Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße substituierte 5-Amino-1-penten-3-ol-Derivate in einem Depot, in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die erfindungsgemäßen substituierten 5-Amino-1-penten-3-ol-Derivate verzögert freisetzen. Prinzipiell können den erfindungsgemäßen Arzneimitteln andere dem Fachmann bekannte weitere Wirkstoffe zugesetzt werden.

Die an den Patienten zu verabreichende Wirkstoffmenge variiert in Abhängigkeit vom Gewicht des Patienten, von der Applikationsart, der Indikation und dem Schweregrad der Erkrankung. Üblicherweise werden 0,005 bis 1000 mg/kg, bevorzugt 0,05 bis 5 mg/kg wenigstens eines erfindungsgemäßen substituierten 5-Amino-1-penten-3-ol-Derivats appliziert.

In einer bevorzugten Form des Arzneimittel liegt ein enthaltenes erfindungsgemäßes substituiertes 5-Amino-1-penten-3-ol-Derivat als reines Diastereomer und/oder Enantiomer, als Razemat oder als nicht-äquimolare oder äquimolare Mischung der Diastereomere und/oder Enantiomere vor.

Gabapentin ist ein bekanntes Antiepileptikum mit antikonvulsiver Wirkung. Neben dieser wird Gabapentin von auch in verschiedenen anderen Indikation eingesetzt, unter anderem von behandelnden Ärzten bei Migräne und bipolaren Störungen sowie Hitzewallungen (z.B. in der Postmenopause) verschrieben (M. Schrope, Modern Drug Discovery, September 2000, S. 11). Andere Indikationenen, in denen Gabapentin ein therapeutisches Potential zeigt, wurden während der Humanstudien und im klinischen Gebrauch identifiziert (J.S. Bryans, D.J. Wustrow; "3-Substituted GABA Analogs with Central Nervous System Activity: A Review" in Med. Res. Rev. (1999), S. 149-177). In diesem Übersichtsartikel wird detailliert die Wirkung von Gabapentin aufgelistet. So ist Gabapentin wirksam in der Behandlung chronischer Schmerzen und Verhaltensstörungen. Insbesondere sind aufgeführt: Antikonvulsive und antiepileptische Wirkungen, der Einsatz gegen chronischen, neuropathischen Schmerz, insbesondere thermische Hyperalgesie, mechanische Allodynie, Kälte-Allodynie. Weiter wirkt es gegen durch Nervenschädigungen ausgelöste Neuropathie, insbesondere eben neuropathischen Schmerz, wie auch inflammatorischen und postoperativen Schmerz erfolgreich. Gabapentin ist auch erfolgreich bei antipsychotischen Effekten insbesondere als Anxiolytikum. Weitere überprüfte Indikationen umfassen: Amyotropische Laterale Sklerose (ALS), Reflex Sympasthetic Dystrophy (RSD), Spastische Lähmung, Restless Leg Syndrom, Behandlung von Symptomen und Schmerz aufgrund von Multipler Sklerose, erworbener Nystagmus, Behandlung der Symptome der Parkinsonschen Krankheit, der schmerzvollen diabetischen Neuropathie und psychatrischer Störungen, z.B. bipolare Störungen, Stimmungsschwankungen, manisches Verhalten. Weiter erfolgreich war der Einsatz von Gabapentin bei erythromelalgischem Schmerz, postpoliomyelitisem Schmerz, trigeminaler Neuralgie und postherpetischer Neuralgie (Bryans und Wustrow (1999), a.a.O.). Allgemein bekannt und auch dem genannten Übersichtsartikel anhand der Beispiele zu entnehmen ist auch die allgemeine Wirksamkeit in neurodegenerativen Erkrankungen. Solche Neurodegenerativen Erkrankungen sind z.B. Morbus Alzheimer, Morbus Huntington, Morbus Parkinson und Epilepsie. Bekannt ist auch die Wirksamkeit von Gabapentin bei gastrointestinalen Schädigungen.

Alle erfindungsgemäßen Substanzen verdrängen Gabapentin von seiner - auch in der Wissenschaft bisher noch unbekannten - Bindungsstelle. Das impliziert aber, daß die erfindungsgemäßen Substanzen an der gleichen Bindungsstelle binden und über sie physiologische wirken werden, vermutlich mit dem gleichen Wirkungsprofil wie Gabapentin. Daß diese Annahme der gleichen Wirkung bei gleicher Bindungsstelle auch zutrifft, wird durch die analgetische Wirkung bewiesen. So verdrängen die erfindungsgemäßen Verbindungen nicht nur Gabapentin von seiner Bindungsstelle sondern wirken auch - wie Gabapentin - deutlich analgetisch.

Daher ist ein weiterer Gegenstand der Erfindung die Verwendung eines erfindungsgemäßen substituierten 5-Amino-1-penten-3-ol-Derivats zur Herstellung eines Arzneimittels zur Behandlung von Schmerz, insbesondere von neuropathischem, chronischem oder akutem Schmerz.

Die erfindungsgemäßen Substanzen sind auch zur Behandlung insbesondere mit neuropathischem Schmerz verbundener Symptome aber auch anderen verwandten Indikationen einsetzbar. Ein weiterer Gegenstand der Erfindung ist daher die Verwendung eines erfindungsgemäßen substituierten 5-Amino-1-penten-3-ol-Derivats zur Herstellung eines Arzneimittels zur Behandlung von Migräne, Hyperalgesie und Allodynie, insbesondere thermischer Hyperalgesie, mechanischer Hyperalgesie und Allodynie und Kälte-Allodynie, oder von inflammatorischem oder postoperativem Schmerz.

Auch in anderen Indikationen sind die erfindungsgemäßen Verbindungen einsetzbar. Daher ist ein weiterer Gegenstand der Erfindung die Verwendung eines erfindungsgemäßen substituierten 5-Amino-1-penten-3-ol-Derivats zur Herstellung eines Arzneimittels zur Behandlung von Epilepsie, Hitzewallungen, Beschwerden in der Postmenopause, Amyotropischer Lateraler Sklerose (ALS), Reflex Sympasthetic Dystrophy (RSD), Spastischer Lähmung, Restless Leg Syndrom, erworbenem Nystagmus; psychatrischen bzw. neuropathologischen Störungen, wie bipolaren Störungen, Anxiety, Panikanfällen, Stimmungsschwankungen, manischem Verhalten, Depressionen, manisch-depressivem Verhalten; schmerzvoller diabetischer Neuropathie, Symptomen und Schmerzen aufgrund von Multipler Sklerose oder der Parkinsonschen Krankheit, neurodegenerativen Erkrankungen, wie Morbus Alzheimer, Morbus Huntington, Morbus Parkinson und Epilepsie; gastrointestinaler Schädigung; von erythromelalgischem oder postpoliomyelitischem Schmerz, trigeminaler oder postherpetischer Neuralgie; oder als Antikonvulsivum, Analgetikum oder Anxiolytikum.

Dabei kann es bevorzugt sein, wenn ein verwendetes substituiertes 5-Amino-1-penten-3-ol-Derivat gemäß einem der Ansprüche 1 bis 11, als reines Diastereomer und/oder Enantiomer, als Razemat oder als nicht-äquimolare oder äquimolare Mischung der Diastereomere und/oder Enantiomere vorliegt.

Ein weiterer Gegenstand der Erfindung ist ein Verfahren zur Behandlung eines nichthumanen Säugetieres oder Menschen, das oder der eine Behandlung medizinisch relevanter Symptome benötigt, durch Verabreichung einer therapeutisch wiksamen Dosis eines erfindungsgemäßen substituierten 5-Amino-1-penten-3-ol-Derivats, oder eines erfindungsgemäßen Arzneimittels. Die Erfindung betrifft insbesondere entsprechende Verfahren zur Behandlung von Schmerz, insbesondere von neuropathischem, chronischem oder akutem Schmerz; Migräne, Hyperalgesie und Allodynie, insbesondere thermischer Hyperalgesie, mechanischer Hyperalgesie und Allodynie und Kälte-Allodynie, oder von inflammatorischem oder postoperativem Schmerz; Epilepsie, Hitzewallungen, Beschwerden in der Postmenopause, Amyotropischer Lateraler Sklerose (ALS), Reflex Sympasthetic Dystrophy (RSD), Spastischer Lähmung, Restless Leg Syndrom, erworbenem Nystagmus; psychatrischen bzw. neuropathologischen Störungen, wie bipolaren Störungen, Anxiety, Panikanfällen, Stimmungsschwankungen, manischem Verhalten, Depressionen, manisch-depressivem Verhalten; schmerzvoller diabetischer Neuropathie, Symptomen und Schmerzen aufgrund von Multipler Sklerose oder der Parkinsonschen Krankheit, neurodegenerativen Erkrankungen, wie Alzheimer Disease, Huntington's Disease, Parkinson Disease und Epilepsie; von erythromelalgischem oder postpoliomyelitischem Schmerz, trigeminaler oder postherpetischer Neuralgie

Ein weiterer Gegenstand der Erfindung ist ein Verfahren zur Herstellung eines erfindungsgemäßen substituierten 5-Amino-1-penten-3-ol-Derivats wie in der folgenden Beschreibung und Beispielen ausgeführt.

### Allgemeine Herstellvorschrift

Für die synthetischen Arbeiten sind in der Literatur beschriebene Reaktionen angewandt (z.B. R.C. Larock, Comprehensive Organic Transformations, 2^{nd} edition, Wiley, New York 1999 und dort zitierte Literatur) worden.
5-Amino-1-penten-3-ol-Derivate der allgemeinen Formel **I** 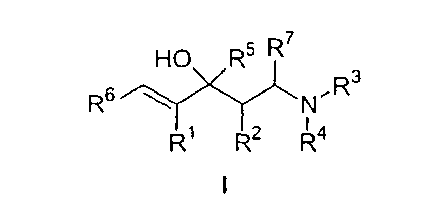 lassen sich durch ein Verfahren herstellen, welches dadurch gekennzeichnet ist, daß man ein β-Aminoketon (im weiteren auch als Mannich-Basen bezeichnet) der Formel **IA**, in der die Reste R¹ bis R⁴, R⁶ und R⁷ eine der oben für Formel **I** beschriebenen Bedeutungen haben 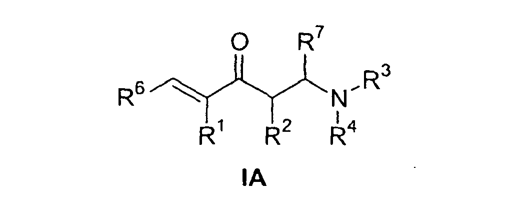 mit einer metallorganischen Verbindung der Formel **III**

R⁵-Z **III**

in der Z MgCl, MgBr, MgI oder Li bedeutet und R⁵ eine der oben für Formel **I** beschriebenen Bedeutungen hat, zu einer Verbindung der Formel **I** umsetzt.

Die Umsetzung eines β-Aminoketons IA mit einer Grignardverbindung der Formel **III**, in der Z MgCl, MgBr oder Mgl bedeutet, oder mit einer lithiumorganischen Verbindung der Formel III kann in einem aliphatischen Ether, beispielsweise Diethylether und/oder Tetrahydrofuran, bei Temperaturen zwischen -70 °C und +60 °C durchgeführt werden. Lithiumorganische Verbindungen der Formel **III**, in der Z Cl, Br oder I bedeutet, lassen sich durch Umsetzung mit beispielsweise einer n-Butyllithium/Hexan-Lösung durch Halogen-Lithiumaustausch erhalten.
β-Aminoketone der allgemeinen Formel **IA** lassen sich nach literaturbekannten Verfahren (Houben-Weyl - Methoden der Organischen Chemie, E21 b, 1995, S. 1925-1929; M. Tramontini, L. Angiolini, Mannich Bases, Chemistry and Uses,CRS Press, 1994 und dort zitierte Literatur) herstellen.
β-Aminoketone der allgemeinen Formel **IA**, in der R⁷ ausgewählt ist aus Aryl oder Heteroaryl; jeweils unsubstituiert oder ein- oder mehrfach substituiert, lassen sich durch Reaktion der Mannichbasen der Formel **IV** durch Aldolkondensation mit aromatischen oder heteroaromatischen Aldehyden der allgemeinen Formel **V** herstellen.

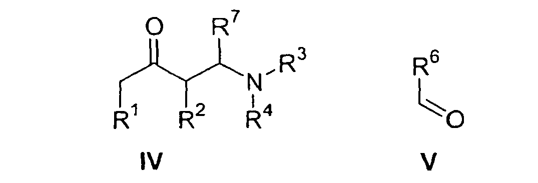

Bevorzugt lassen sich Verbindungen der Formel **IA** durch Reaktion der Enamine der Struktur **IVa** durch Aldolkondensation mit aromatischen oder heteroaromatischen Aldehyden der allgemeinen Formel **V** herstellen, wobei in Formel **IVa** R^{a} und R^{b} bevorzugt gemeinsam -(CH₂)₂-O-(CH₂)₂- darstellen (Hünig, Märkl, Sauer, Integriertes Organisches Praktikum, Verlag Chemie, Weinheim, 1979).

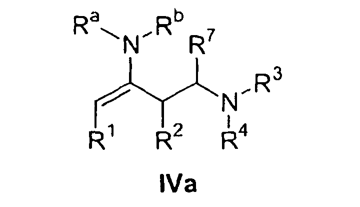

Bevorzugt lassen sich β-Aminoketone der allgemeinen Formel **IV** durch Umsetzung von Enaminen der allgemeinen Formel **VI,** wobei diese Reaktion besonders bevorzugt ist für Verbindungen, in denen R⁷ ≠ H ist, 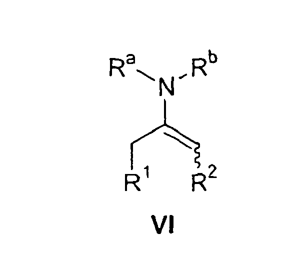 mit einem Iminiumsalz der allgemeinen Formel **VII**, 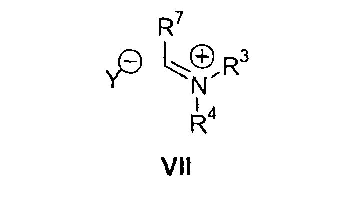 worin Y vorzugsweise Cl⁻, AlCl₄⁻, Br⁻ oder I⁻ bedeutet, erhalten.

Verbindungen, in denen R⁶ H entspricht, können analog mit den literaturbekannten Verfahren der klassischen Mannich-Reaktion über das Eschnmoser-Salz oder BuLi hergestellt werden.

Die Enamine der allgemeinen Formel **VI** werden nach literaturbekannten Verfahren durch die Umsetzung von Ketonen der allgemeinen Formel **VIII** 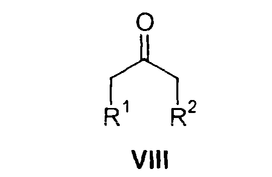 mit sekundären Amine, vorzugsweise Dimethylamin, Pyrrolidin, Piperidin oder Morpholin, erhalten (Acta Chem. Scand. B 38, 1984, S. 49-53).

Ketone der Formel **VIII** wurden entweder kommerziell erworben oder nach Literaturverfahren synthetisiert.

Die Iminiumsalze der allgemeinen Formel **VII** werden nach literaturbekannten Verfahren durch Umsetzung von Aminalen der allgemeinen Formel **IX** 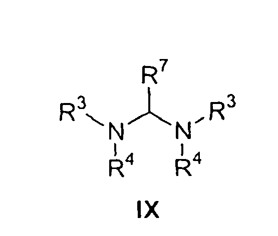 mit Säurechloriden, beispielsweise Acetylchlorid oder Thionylchlorid, hergestellt (Houben-Weyl -Methoden der Organischen Chemie, E21 b, 1995, S. 1925-1929).

Die Imminiumsalze der allgemeinen Formel **VII** müssen dabei nicht isoliert werden, sondern können in situ erzeugt und mit Enaminen der allgemeinen Formel **VI** zu Mannichbasen der allgemeinen Formel **IV** umgesetzt werden (Angew. Chem. 106, 1994, S. 2531-2533). Aufgrund der Keto-Enol-Tautomerie analogen Enamin-Imin-Tautomerie sind statt der Enamine der allgemeinen Formel **VI** auch Imine der allgemeinen Formel **X** 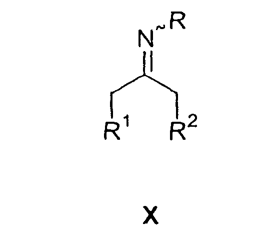 einsetzbar. Alternativ dazu können Ketone der allgemeinen Formel **IV** auch direkt mit Imminiumsalzen der allgemeinen Formel **VII** umgesetzt werden.

Mannichbasen der allgemeinen Formel **IV** können aber auch durch Umsetzung von Enaminen der allgemeinen Formel **VI** mit einem aromatischen oder heteroaromatischen Aldehyd der allgemeinen Formel **XI** 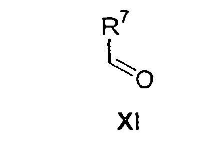 und einem sekundären Amin der allgemeinen Formel HNR³R⁴ **XII** auch in Form des korrespondierenden Hydrochlorids HNR³R⁴·HCl, vorzugsweise in Gegenwart von Triethylamin, Chlortrimethylsilan und Natriumiodid direkt hergestellt werden (Synlett 1997, S. 974-976).

Die Mannichbasen der allgemeinen Formel **IV** werden mit den oben beschriebenen Verfahren in Abhängigkeit von den Reaktionsbedingungen bevorzugt mit der relativen Konfiguration der allgemeine Formel **IVa** erhalten, 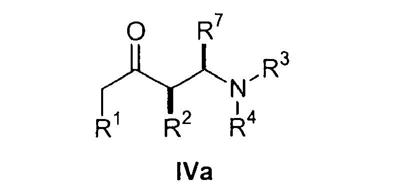 in denen die Aminogruppe *anti* zu R² angeordnet ist. Diese Verbindungen der allgemeinen Formel **IVa** lassen sich durch Kristallisation, auch ihrer Salze, beispielsweise der Hydrochloride, oder durch chromatographische Trennung diastereomerenrein erhalten.

Die Darstellung von Mannichbasen der allgemeinen Formel **IV** durch 1,4-Addition sekundärer Amine der allgemeinen Formel **XII** an Enone der allgemeinen Formel **XIII,** 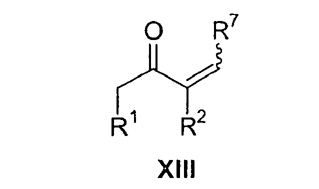 die aus der Aldolkondensation von Ketonen der allgemeinen Formel **IV** mit aromatischen oder heteroaromatischen Aldehyden der allgemeinen Formel **XI** erhalten werden, verläuft dagegen weniger stereoselektiv (US-Patent 4,017,637). Diese Vorgehensweise eignet sich daher zur Darstellung der anderen möglichen Stereosiomeren.

Werden chirale Amine zur Darstellung von Enaminen der allgemeinen Formel **VI** oder Imine der allgemeinen Formel **X** eingesetzt, so können in der nachfolgenden Mannichreaktion enantiomeren-angereicherte bis enantiomerenreine Mannichbasen der allgemeinen Formel **IV** erhalten werden (Houben-Weyl - Methoden der Organischen Chemie, E21b, 1995, S. 1925-1929).

Alternativ zur oben beschriebenen Synthesesequenz können Verbindungen der Formel **IA**, in der R⁷ Wasserstoff ist, auch dadurch erhalten werden, daß Ketone der Formel **VIII** mit aromatischen oder heteroaromatischen Aldehyden der allgemeinen Formel **V** in einer Aldolkondensation zu Verbindungen der Formel **XIV** umgesetzt werden.

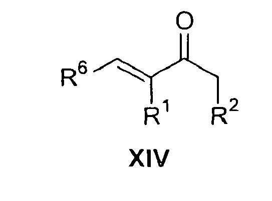

Bevorzugt lassen sich Verbindungen der Formel **XIV** durch Reaktion der Enamine der Struktur **VIa** durch Aldolkondensation mit aromatischen oder heteroaromatischen Aldehyden der allgemeinen Formel **V** herstellen, wobei in Formel **VIa** R^{a} und R^{b} bevorzugt gemeinsam -(CH₂)₂-O-(CH₂)₂- darstellen (Hünig, Märkl, Sauer, Integriertes Organisches Praktikum, Verlag Chemie, Weinheim, 1979).

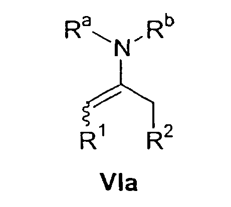

Verbindungen der Formel **IVX** können direkt mit Iminiumsalzen der Formel **VII** zu den Mannichbasen der Formel **IA** umgesetzt werden.

Die bei der Aminomethylierungsreaktion entstehenden diastereomeren Mannichbasen der Formel **IA** und **IV** lassen sich entweder durch säulenchromatographische Trennung oder durch fraktionierte Kristallisation ihrer Hydrochloride aus einem organischem Lösungsmittel wie z.B. 2-Butanon oder Aceton diastereomerenrein erhalten.

Enantiomerenreine Mannichbasen der Formel **IA** und **IV** können auch durch eine Aminomethylierung unter Verwendung enantiomerenreiner Ketone der Formel **IA** und **VI** erhalten werden oder durch Racematspaltung über die Kristallisation diastereomerer Salze unter Verwendung chiraler Säuren, bevorzugt Weinsäure, Weinsäure-Derivate oder Mandelsäure (J. Gawroński, K. Gawrońska, Tartaric and Malic Acids in Synthesis, Wiley, New York 1999 und dort zitierte Literatur), hergestellt werden.

### Salzbildung

Die Verbindungen der Formel I lassen sich mit physiologisch verträglichen Säuren, beispielsweise Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure, 1,1-Dioxo-1,2-dihydro1λ⁶-benzo[*d*]isothiazol-3-on (Saccharinsäure), Monomethylsebacinsäure, 5-Oxo-prolin, Hexan-1-sulfonsäure, Nicotinsäure, 2-, 3-oder 4-Aminobenzoesäure, 2,4,6-Trimethyl-benzoesäure, α-Liponsäure, Acetylglycin, Acetylsalicylsäure, Hippursäure und/oder Asparaginsäure, in der sich bekannter Weise in ihre Salze überführen. Vorzugsweise wird die Salzbildung in einem Lösungsmittel, beispielsweise Diethylether, Diisopropylether, Essigsäurealkylester, Aceton und/oder 2-Butanon oder auch Wasser durchgeführt. Zur Herstellung der Hydrochloride eignet sich darüber hinaus Trimethylchlorsilan in wäßriger Lösung.

Im folgenden wird die Erfindung weiter durch Beispiele erläutert, ohne sie darauf zu beschränken.

### Beispiele

Die folgenden Beispiele zeigen erfindungsgemäße Verbindungen sowie deren Darstellung und mit diesen durchgeführte Wirksamkeitsuntersuchungen.

Dabei gelten generell folgende Angaben:

Die eingesetzten Chemikalien und Lösungsmittel wurden kommerziell bei den herkömmlichen Anbietern erworben (Acros, Avocado, Aldrich, Fluka, Lancaster, Maybridge, Merck, Sigma, TCI etc.) oder synthetisiert.

Die Analytik erfolgte über ESI-Massenspektrometrie und/oder HPLC und/oder NMR-Spektroskopie.

### Darstellung der Mannichbasen

### Beispiele:

### 2-Benzyliden-6-dimethylaminomethyl-cyclohexanon

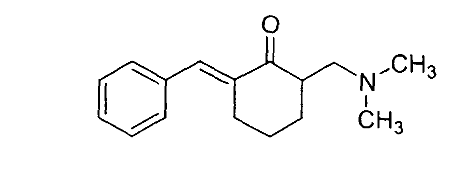

### 1. Stufe: 4-Cyclohex-1-enyl-morpholin

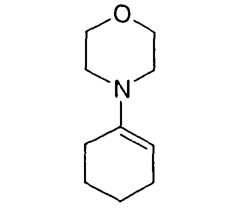

98,2 g (1 mol) Cyclohexanon, 87,7 g (1,2 mol) Morpholin wurden in 400 ml getrocknetem Toluol unter Zusatz von 1 g p-Toluolsulfonsäure unter Rückfluß an einem Wasserabscheider erhitzt. Nach Abscheidung von 18,7 ml Wasser (nach ca. vier Stunden) wurden 4 g gepulvertes Calciumhydrid zugesetzt und weitere 30 Minuten unter Rückfluß erhitzt. Nach destillativer Entfernung des Lösungsmittels wurde der Rückstand destilliert. Die bei 70 - 73 °C bei 0,1 bar übergehende Fraktion wurde ohne weiter Reinigung weiter umgesetzt (Ausbeute 108 g, 65 % der Theorie).

### 2. Stufe: 2-Benzyliden-cyclohexanon

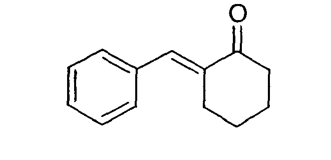

108 g (0,65 mol) 4-Cyclohex-1-enyl-morpholin aus Stufe 1 und 58,0 g (0,55 mol) Benzaldehyd wurden in 110 ml getrocknetem Toluol gelöst und 48 Stunden unter Rückfluß an einem Wasserabscheider erhitzt. Es schieden sich 9 ml Wasser ab. Nach dem Abkühlen auf Raumtemperatur wurden unter Eisbadkühlung 170 ml 18 %ige Salzsäure und 60 ml Toluol unter Rühren zugetropft. Es wurde 1 Stunde bei Raumtemperatur nachgerührt. Nach Phasentrennung wurde die wäßrige Phase zweimal mit je 100 ml Toluol extrahiert. Die vereinigten organischen Phasen wurden jeweils einmal mit 150 ml Wasser und anschließend mit 100 ml gesättigter Natriumcarbonatlösung gewaschen. Nach Trocknung über Natriumsulfat wurde das Lösungsmittel destillativ entfernt. Der Rückstand wurde destilliert und die Fraktion, die bei 120 - 125 °C und 0.2 bar überging, wurde aus n-Hexan umkristallisiert. Auf diese Weise wurden 48,1 g (47 % der Theorie) hellgelbe, wachsartige Kristalle mit einem Schmelzpunkt von 53 - 55 °C erhalten.

### 3. Stufe: 2-Benzyliden-6-dimethylaminomethyl-cyclohexanon

43 g (0,23 mol) 2-Benzyliden-cyclohexanon, welches aus Stufe 2 erhalten wurde, und 21,6 g (0,23 mol) Dimethylammoniummethylenchlorid wurden in 200 ml trockenem Acetonitril bei Raumtemperatur gerührt. Nach Zugabe von 0,2 ml Acetylchlorid wurde noch drei Stunden bei Raumtemperatur weitergerührt, wobei eine farblose, klare Lösung entstand. Anschließend wurden 200 ml getrockneter Ether zum Reaktionsgemisch getropft, wobei das Hydrochlorid auskristallisierte. Es wurden 56,1g (87 % der Theorie) farblose Kristalle erhalten. Aus dem Hydrochlorid wurde mit Dichlormethan/Natronlauge die Base freigesetzt und nach dem Trocknen der Lösung das Dichlormethan destillativ entfernt.

Wie im obigen Beispiel beschrieben wurden in analoger Weise folgende Mannichbasen der Formel **II** hergestellt:
- 2-Dimethylaminomethyl-6-(2-methoxy-benzyliden)-cyclohexanon
- 2-Dimethylaminomethyl-6-(3-methoxy-benzyliden)-cyclohexanon
- 2-Dimethylaminomethyl-6-(4-methoxy-benzyliden)-cyclohexanon
- 2-(2-Chlor-benzyliden)-6-dimethylaminomethyl-cyclohexanon
- 2-(3-Chlor-benzyliden)-6-dimethylaminomethyl-cyclohexanon
- 2-(4-Chlor-benzyliden)-6-dimethylaminomethyl-cyclohexanon
- 2-Dimethylaminomethyl-6-(2-fluor-benzyliden)-cyclohexanon
- 2-Dimethylaminomethyl-6-(3-fluor-benzyliden)-cyclohexanon
- 2-Dimethylaminomethyl-6-(4-fluor- benzyliden)-cyclohexanon
- 2-Dimethylaminomethyl-6-(2-methyl-benzyliden)-cyclohexanon
- 2-Dimethylaminomethyl-6-(3-methyl-benzyliden)-cyclohexanon
- 2-Dimethylaminomethyl-6-(4-methyl-benzyliden)-cyclohexanon
- 2-(3,4-Dichlor-benzyliden)-6-dimethylaminomethyl-cyclohexanon
- 2-Biphenyl-4-ylmethylen-6-dimethylaminomethyl-cyclohexanon
- 2-(4-tert-Butyl-benzyliden)-6-dimethylaminomethyl-cyclohexanon
- 2-Dimethylaminomethyl-6-naphthalin-1-ylmethylen-cyclohexanon
- 2-Dimethylaminomethyl-6-naphthalin-2-ylmethylen-cyclohexanon
- 2-Benzyliden-6-dimethylaminomethyl-4-phenyl-cyclohexanon
- 2-(2-Chlor-benzyliden)-6-dimethylaminomethyl-4-phenyl-cyclohexanon
- 2-(3-Chlor-benzyliden)-6-dimethylaminomethyl-4-phenyl-cyclohexanon
- 2-(4-Chlor-benzyliden)-6-dimethylaminomethyl-4-phenyl-cyclohexanon
- 2-Dimethylaminomethyl-6-(2-fluor-benzyliden)-4-phenyl-cyclohexanon
- 2-Dimethylaminomethyl-6-(3-fluor-benzyliden)-4-phenyl-cyclohexanon
- 2-Dimethylaminomethyl-6-(4-fluor-benzyliden)-4-phenyl-cyclohexanon
- 2-Dimethylarninomethyl-6-(2-methoxy-benzyliden)-4-phenyl-cyclohexanon
- 2-Dimethylaminomethyl-6-(3-methoxy-benzyliden)-4-phenyl-cyclohexanon
- 2-Dimethylaminomethyl-6-(4-methoxy-benzyliden)-4-phenyl-cyclohexanon
- 2-Dimethylaminomethyl-5-(2-methoxy-benzyliden)-cyclopentanon
- 2-Dimethylaminomethyl-5-(3-methoxy-benzyliden)-cyclopentanon
- 2-Dimethylaminomethyl-5-(4-methoxy-benzyliden)-cyclopentanon
- 2-(2-Chlor-benzyliden)-5-dimethylaminomethyl-cyclopentanon
- 2-(3-Chlor-benzyliden)-5-dimethylaminomethyl-cyclopentanon
- 2-(4-Chlor-benzyliden)-5-dimethylaminomethyl-cyclopentanon
- 2-Dimethylaminomethyl-5-(2-fluor-benzyliden)-cyclopentanon
- 2-Dimethylaminomethyl-5-(3-fluor-benzyliden)-cyclopentanon
- 2-Dimethylaminomethyl-5-(4-fluor-benzyliden)-cyclopentanon
- 2-Dimethylaminomethyl-5-(2-methyl-benzyliden)-cyclopentanon
- 2-Dimethylaminomethyl-5-(3-methyl-benzyliden)-cyclopentanon
- 2-Dimethylaminomethyl-5-(4-methyl-benzyliden)-cyclopentanon
- 2-(3,4-Dichlor-benzyliden)-5-dimethylaminomethyl-cyclopentanon
- 2-Biphenyl-4-ylmethylen-5-dimethylaminomethyl-cyclopentanon
- 2-(4-tert-Butyl-benzyliden)-5-dimethylaminomethyl-cyclopentanon
- 2-Dimethylaminomethyl-5-naphthalin-1-ylmethylen-cyclopentanon
- 2-Dimethylaminomethyl-5-naphthalin-2-ylmethylen-cyclopentanon
- 2-Dimethylaminomethyl-7-(2-methoxy-benzyliden)-cycloheptanon
- 2-Dimethylaminomethyl-7-(3-methoxy-benzyliden)-cycloheptanon
- 2-Dimethylaminomethyl-7-(4-methoxy-benzyliden)-cycloheptanon
- 2-(2-Chlor-benzyliden)-7-dimethylaminomethyl-cycloheptanon
- 2-(3-Chlor-benzyliden)-7-dimethylaminomethyl-cycloheptanon
- 2-(4-Chlor-benzyliden)-7-dimethylaminomethyl-cycloheptanon
- 2-Dimethylaminomethyl-7-(2-fluor-benzyliden)-cycloheptanon
- 2-Dimethylaminomethyl-7-(3-fluor-benzyliden)-cycloheptanon
- 2-Dimethylaminomethyl-7-(4-fluor-benzyliden)-cycloheptanon
- 2-Dimethylaminomethyl-7-(2-methyl-benzyliden)-cycloheptanon
- 2-Dimethylaminomethyl-7-(3-methyl-benzyliden)-cycloheptanon
- 2-Dimethylaminomethyl-7-(4-methyl-benzyliden)-cycloheptanon
- 2-(3,4-Dichlor-benzyliden)-7-dimethylaminomethyl-cydoheptanon
- 2-Biphenyl-4-ylmethylen-7-dimethylaminomethyl-cycloheptanon
- 2-(4-tert-Butyl-benzyliden)-7-dimethylaminomethyl-cycloheptanon
- 2-Dimethylaminomethyl-7-naphthalin-1-ylmethylen-cycloheptanon
- 2-Dimethylaminomethyl-7-naphthalin-2-ylmethylen-cycloheptanon
- 5-Dimethylamino-2,4-dimethyl-1-phenyl-pent-1-en-3-on
- 5-Dimethylamino-1-(2-methoxy-phenyl)-2,4-dimethyl-pent-1-en-3-on
- 5-Dimethylamino-1-(3-methoxy-phenyl)-2,4-dimethyl-pent-1-en-3-on
- 5-Dimethylamino-1-(4-methoxy-phenyl)-2,4-dimethyl-pent-1-en-3-on
- 1-(2-Chlor-phenyl)-5-dimethylamino-2,4-dimethyl-pent-1-en-3-on
- 1-(3-Chlor-phenyl)-5-dimethylamino-2,4-dimethyl-pent-1-en-3-on
- 1-(4-Chlor-phenyl)-5-dimethylamino-2,4-dimethyl-pent-1-en-3-on
- 5-Dimethylamino-1-(2-fluor-phenyl)-2,4-dimethyl-pent-1-en-3-on
- 5-Dimethylamino-1-(3-fluor-phenyl)-2,4-dimethyl-pent-1-en-3-on
- 5-Dimethylamino-1-(4-fluor-phenyl)-2,4-dimethyl-pent-1-en-3-on
- 5-Dimethylamino-2,4-dimethyl-1-o-tolyl-pent-1-en-3-on
- 5-Dimethylamino-2,4-dimethyl-1-m-tolyl-pent-1-en-3-on
- 5-Dimethylamino-2,4-dimethyl-1-p-tolyl-pent-1-en-3-on
- 1-(3,4-Dichlor-phenyl)-5-dimethylamino-2,4-dimethyl-pent-1-en-3-on
- 1-Biphenyl-4-yl-5-dimethylamino-2,4-dimethyl-pent-1-en-3-on

### Allgemeine Arbeitsvorschrift 1

In einem ausgeheizten und unter Inertgas auf - 10°C abgekühlten Reaktionsgefäß wurde die in THF gelöste Mannichbase (400µl, 0,5 mol/l) vorgelegt. Unter Rühren wurden daraufhin 2 Äquivalente des vorbereiteten Grignard- oder Organolithium-Reagenzes zugegeben (0,5 mol/l in THF oder Diethylether, 800 µl). Das Reaktionsgemisch wurde bei Raumtemperatur gerührt. Nach drei Stunden wurde erneut auf -10°C gekühlt und mit Ammoniumchlorid-Lösung hydrolisiert.
Das Reaktionsgemisch wurde zweimal mit Ethylacetat extrahiert und bei 40°C im Vakuum eingeengt.

Zur Charakterisierung meist der chlorierter Verbindungen wurde jeweils ein ESI-MS aufgenommen. Zur Charakterisierung wurde ansonsten jeweils ein NMR-Spektrum aufgenommen.

### Beispielsubstanzen:

| | |
|---|---|
| | |
| Beispiel 1 | 2-Benzyliden-1-(3-chlor-benzyl)-6-dimethylaminomethyl-cyclohexanol |
| Beispiel 2 | 2-Benzyliden-6-dimethylaminomethyl-1-(2,5-dimethyl-benzyl)-cyclohexanol |
| Beispiel 3 | 2-Benzyliden-1-(2-chlor-6-fluor-benzyl)-6-dimethylaminomethyl-cyclohexanol |
| Beispiel 4 | 2-Benzyliden-1-(4-chlor-benzyl)-6-dimethylaminomethyl-cyclohexanol |
| Beispiel 5 | 2-Benzyliden-6-dimethylaminomethyl-1-(3-methyl-benzyl)-cyclohexanol |
| Beispiel 6 | 2-Benzyliden-6-dimethylaminomethyl-1-(3-trifluormethyl-phenyl)-cyclohexanol |
| Beispiel 7 | 2-Benzyliden-1-(3-chlor-4-fluor-phenyl)-6-dimethylaminomethyl-cyclohexanol |
| Beispiel 8 | 2-Benzyliden-6-dimethylaminomethyl-1-(2-methyl-benzyl)-cyclohexanol |
| Beispiel 9 | 2-Benzyliden-6-dimethylaminomethyl-1-(2-methoxy-phenyl)-cyclohexanol |
| Beispiel 10 | 2-Benzyliden-6-dimethylaminomethyl-1-(3-fluor-benzyl)-cyclohexanol |
| Beispiel 11 | 2-Benzyliden-1-(4-chlor-3-trifluormethyl-phenyl)-6-dimethylaminomethyl-cyclohexanol |
| Beispiel 12 | 2-Benzyliden-6-dimethylaminomethyl-1-(3-methoxy-benzyl)-cyclohexanol |
| Beispiel 13 | 2-Benzyliden-6-dimethylaminomethyl-1-(4-fluor-benzyl)-cyclohexanol |
| Beispiel 14 | 2-Benzyliden-1-(2-chlor-benzyl)-6-dimethylaminomethyl-cyclohexanol |
| Beispiel 15 | 2-Benzyliden-1-(3,5-dichlor-phenyl)-6-dimethylaminomethyl-cyclohexanol |
| Beispiel 16 | 2-Benzyliden-1-(3-chlor-phenyl)-6-dimethylaminomethyl-cyclohexanol |
| Beispiel 17 | 2-Benzyliden-6-dimethylaminomethyl-1-(3-fluor-phenyl)-cyclohexanol |
| Beispiel 18 | 2-Benzyliden-6-dimethylaminomethyl-1-(5-fluor-2-methoxy-phenyl)-cyclohexanol |
| Beispiel 19 | 2-Benzyliden-1-cyclohexylmethyl-6-dimethylaminomethyl-cyclohexanol |
| Beispiel 20 | 2-Benzyliden-6-dimethylaminomethyl-1-(4-methoxy-phenyl)-cyclohexanol |
| Beispiel 21 | 2-Benzyliden-6-dimethylaminomethyl-1-p-tolyl-cyclohexanol |
| Beispiel 22 | 2-Benzyliden-6-dimethylaminomethyl-1-(3-phenyl-propyl)-cyclohexanol |
| Beispiel 23 | 2-Benzyliden-6-dimethylaminomethyl-1-(3-methoxy-phenyl) -cyclohexanol |
| Beispiel 24 | 2-Benzyliden-6-dimethylaminomethyl-1-thiophen-2-yl-cyclohexanol |
| Beispiel 25 | 2-Benzyliden-6-dimethylaminomethyl-1-phenylethynyl-cyclohexanol |
| Beispiel 26 | 2-Benzyliden-6-dimethylaminomethyl-1-phenethyl-cyclohexanol |
| Beispiel 27 | 2-Benzyliden-6-dimethylaminomethyl-1-(4-fluor-phenyl)-cyclohexanol |
| Beispiel 28 | 2-Benzyliden-6-dimethylaminomethyl-bicyclohexyl-1-ol |
| Beispiel 29 | 2-Benzyliden-6-dimethylaminomethyl-1-m-tolyl-cyclohexanol |
| Beispiel 30 | 2-Benzyliden-1-cyclopentyl-6-dimethylaminomethyl-cyclohexanol |
| Beispiel 31 | 2-Benzyliden-1-(4-tert-butyl-phenyl)-6-dimethylaminomethyl-cyclohexanol |
| Beispiel 32 | 2-Benzyliden-6-dimethylaminomethyl-1-vinyl-cyclohexanol |
| Beispiel 33 | 2-Benzyliden-6-dimethylaminomethyl-1-o-tolyl-cyclohexanol |
| Beispiel 34 | 2-Benzyliden-6-dimethylaminomethyl-1-(4-fluor-3-methyl-phenyl)-cyclohexanol |
| Beispiel 35 | 1-Benzyl-2-benzyliden-6-dimethylaminomethyl-cyclohexanol |
| Beispiel 36 | 2-Benzyliden-1-(4-chlor-phenyl)-6-dimethylaminomethyl-cyclohexanol |
| Beispiel 37 | 2-Benzyliden-6-dimethylaminomethyl-1-phenyl-cyclohexanol |
| Beispiel 38 | 1-(3-Chlor-benzyl)-2-(4-chlor-benzyliden)-6-dimethylaminomethyl-cyclohexanol |
| Beispiel 39 | 2-(4-Chlor-benzyliden)-6-dimethylaminomethyl-1-(2,5-dimethyl-benzyl)-cyclohexanol |
| Beispiel 40 | 2-(4-Chlor-benzyliden)-1-(2-chlor-6-fluor-benzyl)-6-dimethylaminomethyl-cyclohexanol |
| Beispiel 41 | 1-(4-Chlor-benzyl)-2-(4-chlor-benzyliden)-6-dimethylaminomethyl-cyclohexanol |
| Beispiel 42 | 2-(4-Chlor-benzyliden)-6-dimethylaminomethyl-1-(3-trifluormethyl-phenyl)-cyclohexanol |
| Beispiel 43 | 2-(4-Chlor-benzyliden)-1-(3-chlor-4-fluor-phenyl)-6-dimethylaminomethyl-cyclohexanol |
| Beispiel 44 | 2-(4-Chlor-benzyliden)-6-dimethylaminomethyl-1-(2-methyl-benzyl)-cyclohexanol |
| Beispiel 45 | 2-(4-Chlor-benzyliden)-6-dimethylaminomethyl-1-(2-methoxy-phenyl)-cyclohexanol |
| Beispiel 46 | 2-(4-Chlor-benzyliden)-6-dimethylaminomethyl-1-(3-fluor-benzyl)-cyclohexanol |
| Beispiel 47 | 2-(4-Chlor-benzyliden)-1-(4-chlor-3-trifluormethyl-phenyl)-6-dimethylaminomethyl-cyclohexanol |
| Beispiel 48 | 2-(4-Chlor-benzyliden)-6-dimethylaminomethyl-1-(3-methoxy-benzyl)-cyclohexanol |
| Beispiel 49 | 2-(4-Chlor-benzyliden)-6-dimethylaminomethyl-1-(4-fluor-benzyl)-cyclohexanol |
| Beispiel 50 | 1-(2-Chlor-benzyl)-2-(4-chlor-benzyliden)-6-dimethylaminomethyl-cyclohexanol |
| Beispiel 51 | 2-(4-Chlor-benzyliden)-1-(3,5-dichlor-phenyl)-6-dimethylaminomethyl-cyclohexanol |
| Beispiel 52 | 2-(4-Chlor-benzyliden)-1-(3-chlor-phenyl)-6-dimethylaminomethyl-cyclohexanol |
| Beispiel 53 | 2-(4-Chlor-benzyliden)-6-dimethylaminomethyl-1-(3-fluor-phenyl)-cyclohexanol |
| Beispiel 54 | 2-(4-Chlor-benzyliden)-6-dimethylaminomethyl-1-(5-fluor-2-methoxy-phenyl)-cyclohexanol |
| Beispiel 55 | 2-(4-Chlor-benzyliden)-1-cyclohexylmethyl-6-dimethylaminomethyl-cyclohexanol |
| Beispiel 56 | 2-(4-Chlor-benzyliden)-6-dimethylaminomethyl-1-(4-methoxy-phenyl)-cyclohexanol |
| Beispiel 57 | 2-(4-Chlor-benzyliden)-6-dimethylaminomethyl-1-p-tolyl-cyclohexanol |
| Beispiel 58 | 2-(4-Chlor-benzyliden)-6-dimethylaminomethyl-1-(3-phenyl-propyl)-cyclohexanol |
| Beispiel 59 | 2-(4-Chlor-benzyliden)-6-dimethylaminomethyl-1-(3-methoxy-phenyl)-cyclohexanol |
| Beispiel 60 | 2-(4-Chlor-benzyliden)-6-dimethylaminomethyl-1-thiophen-2-yl-cyclohexanol |
| Beispiel 61 | 2-(4-Chlor-benzyliden)-6-dimethylaminomethyl-1-phenylethynyl-cyclohexanol |
| Beispiel 62 | 2-(4-Chlor-benzyliden)-6-dimethylaminomethyl-1-phenethyl-cyclohexanol |
| Beispiel 63 | 2-(4-Chlor-benzyliden)-6-dimethylaminomethyl-1-(4-fluor-phenyl)-cyclohexanol |
| Beispiel 64 | 2-(4-Chlor-benzyliden)-6-dimethylaminomethyl-bicyclohexyl-1-ol |
| Beispiel 65 | 2-(4-Chlor-benzyliden)-6-dimethylaminomethyl-1-m-tolyl-cyclohexanol |
| Beispiel 66 | 2-(4-Chlor-benzyliden)-1-cyclopentyl-6-dimethylaminomethyl-cyclohexanol |
| Beispiel 67 | 1-(4-tert-Butyl-phenyl)-2-(4-chlor-benzyliden)-6-dimethylaminomethyl-cyclohexanol |
| Beispiel 68 | 2-(4-Chlor-benzyliden)-6-dimethylaminomethyl-1-vinyl-cyclohexanol |
| Beispiel 69 | 2-(4-Chlor-benzyliden)-6-dimethylaminomethyl-1-o-tolyl-cyclohexanol |
| Beispiel 70 | 2-(4-Chlor-benzyliden)-6-dimethylaminomethyl-1-(4-fluor-3-methyl-phenyl)-cyclohexanol |
| Beispiel 71 | 1-Benzyl-2-(4-chlor-benzyliden)-6-dimethylaminomethyl-cyclohexanol |
| Beispiel 72 | 2-(4-Chlor-benzyliden)-1-(4-chlor-phenyl)-6-dimethylaminomethyl-cyclohexanol |
| Beispiel 73 | 2-(4-Chlor-benzyliden)-6-dimethylaminomethyl-1-phenyl-cyclohexanol |
| Beispiel 74 | 1-(3-Chlor-benzyl)-2-dimethylaminomethyl-6-(4-methoxy-benzyliden)-cyclohexanol |
| Beispiel 75 | 2-Dimethylaminomethyl-1-(2,5-dimethyl-benzyl)-6-(4-methoxy-benzyliden)-cyclohexanol |
| Beispiel 76 | 1-(2-Chlor-6-fluor-benzyl)-2-dimethylaminomethyl-6-(4-methoxy-benzyliden)-cyclohexanol |
| Beispiel 77 | 2-Dimethylaminomethyl-6-(4-methoxy-benzyliden)-1-(3-methyl-benzyl)-cyclohexanol |
| Beispiel 78 | 2-Dimethylaminomethyl-6-(4-methoxy-benzyliden)-1-(3-trifluormethyl-phenyl)-cyclohexanol |
| Beispiel 79 | 1-(3-Chlor-4-fluor-phenyl)-2-dimethylaminomethyl-6-(4-methoxy-benzyliden)-cyclohexanol |
| Beispiel 80 | 2-Dimethylaminomethyl-6- (4-methoxy-benzyliden)-1-(2-methoxy-phenyl)-cyclohexanol |
| Beispiel 81 | 1-(4-Chlor-3-trifluormethyl-phenyl)-2-dimethylaminomethyl-6-(4-methoxy-benzyliden)-cyclohexanol |
| Beispiel 82 | 2-Dimethylaminomethyl-1-(4-fluor-benzyl)-6-(4-methoxy-benzyliden)-cyclohexanol |
| Beispiel 83 | 1-(2-Chlor-benzyl)-2-dimethylaminomethyl-6-(4-methoxy-benzyliden)-cyclohexanol |
| Beispiel 84 | 1-(3,5-Dichlor-phenyl)-2-dimethylaminomethyl-6-(4-methoxy-benzyliden)-cyclohexanol |
| Beispiel 85 | 1-(3-Chlor-phenyl)-2-dimethylaminomethyl-6-(4-methoxy-benzyliden)-cyclohexanol |
| Beispiel 86 | 2-Dimethylaminomethyl-1-(5-fluor-2-methoxy-phenyl)-6-(4-methoxy-benzyliden)-cyclohexanol |
| Beispiel 87 | 1-Cyclohexylmethyl-2-dimethylaminomethyl-6-(4-methoxy-benzyliden)-cyclohexanol |
| Beispiel 88 | 2-Dimethylaminomethyl-6-(4-methoxy-benzyliden)-1-(4-methoxy-phenyl)-cyclohexanol |
| Beispiel 89 | 2-Dimethylaminomethyl-6-(4-methoxy-benzyliden)-1-p-tolyl-cyclohexanol |
| Beispiel 90 | 1-(2,3-Dichlor-phenyl)-2-dimethylaminomethyl-6-(4-methoxy-benzyliden)-cyclohexanol |
| Beispiel 91 | 2-Dimethylaminomethyl-6-(4-methoxy-benzyliden)-1-(3-phenyl-propyl)-cyclohexanol |
| Beispiel 92 | 2-Dimethylaminomethyl-6-(4-methoxy-benzyliden)-1-(3-methoxy-phenyl)-cyclohexanol |
| Beispiel 93 | 2-Dimethylaminomethyl-6-(4-methoxy-benzyliden)-1-thiophen-2-yl-cyclohexanol |
| Beispiel 94 | 2-Dimethylaminomethyl-6-(4-methoxy-benzyliden)-1-phenylethynyl-cyclohexanol |
| Beispiel 95 | 2-Dimethylaminomethyl-6-(4-methoxy-benzyliden)-1-phenethyl-cyclohexanol |
| Beispiel 96 | 2-Dimethylaminomethyl-1-(4-fluor-phenyl)-6-(4-methoxy-benzyliden)-cyclohexanol |
| Beispiel 97 | 6-Dimethylaminomethyl-2-(4-methoxy-benzyliden)-bicyclohexyl-1-ol |
| Beispiel 98 | 2-Dimethylaminomethyl-6-(4-methoxy-benzyliden)-1-m-tolyl-cyclohexanol |
| Beispiel 99 | 1-Cyclopentyl-2-dimethylaminomethyl-6-(4-methoxy-benzyliden)-cyclohexanol |
| Beispiel 100 | 1-(4-tert-Butyl-phenyl)-2-dimethylaminomethyl-6-(4-methoxy-benzyliden)-cyclohexanol |
| Beispiel 101 | 2-Dimethylaminomethyl-6-(4-methoxy-benzyliden)-1-vinyl-cyclohexanol |
| Beispiel 102 | 2-Dimethylaminomethyl-6-(4-methoxy-benzyliden)-1-o-tolyl-cyclohexanol |
| Beispiel 103 | 2-Dimethylaminomethyl-1-(4-fluor-3-methyl-phenyl)-6-(4-methoxy-benzyliden)-cyclohexanol |
| Beispiel 104 | 1-Benzyl-2-dimethylaminomethyl-6-(4-methoxy-benzyliden)-cyclohexanol |
| Beispiel 105 | 1-(4-Chlor-phenyl)-2-dimethylaminomethyl-6-(4-methoxy-benzyliden)-cyclohexanol |
| Beispiel 106 | 2-Dimethylaminomethyl-6-(4-methoxy-benzyliden)-1-phenyl-cyclohexanol |
| Beispiel 107 | 1-(3-Chlor-benzyl)-2-dimethylaminomethyl-6-(3-methoxy-benzyliden)-cyclohexanol |
| Beispiel 108 | 2-Dimethylaminomethyl-1-(2,5-dimethyl-benzyl)-6-(3-methoxy-benzyliden)-cyclohexanol |
| Beispiel 109 | 2-Dimethylaminomethyl-6-(3-methoxy-benzyliden)-1-(2-methyl-benzyl)-cyclohexanol |
| Beispiel 110 | 2-Dimethylaminomethyl-6-(3-methoxy-benzyliden)-1-(2-methoxy-phenyl)-cyclohexanol |
| Beispiel 111 | 1-(4-Chlor-3-trifluormethyl-phenyl)-2-dimethylaminomethyl-6-(3-methoxy-benzyliden)-cyclohexanol |
| Beispiel 112 | 1-(2-Chlor-benzyl)-2-dimethylaminomethyl-6-(3-methoxy-benzyliden)-cyclohexanol |
| Beispiel 113 | 6-Dimethylaminomethyl-2-(3-methoxy-benzyliden)-bicyclohexyl-1-ol |
| Beispiel 114 | 1-Cyclopentyl-2-dimethylaminomethyl-6-(3-methoxy-benzyliden)-cyclohexanol |
| Beispiel 115 | 2-Dimethylaminomethyl-6-(3-methoxy-benzyliden)-1-vinyl-cyclohexanol |
| Beispiel 116 | 2-Dimethylaminomethyl-1-(4-fluor-3-methyl-phenyl)-6-(3-methoxy-benzyliden)-cyclohexanol |
| Beispiel 117 | 1-Benzyl-2-dimethylaminomethyl-6-(3-methoxy-benzyliden)-cyclohexanol |
| Beispiel 118 | 2-Dimethylaminomethyl-6-(3-methoxy-benzyliden)-1-phenyl-cyclohexanol |
| Beispiel 119 | 1-Benzyl-2-(2-chlor-benzyliden)-6-dimethylaminomethyl-cyclohexanol |
| Beispiel 120 | 5-Dimethylamino-2,4-dimethyl-1,3-diphenyl-pent-1-en-3-ol |
| Beispiel 121 | 3-(4-Chlor-phenyl)-5-dimethylamino-2,4-dimethyl-1-phenyl-pent-1-en-3-ol |
| Beispiel 122 | 3-Benzyl-5-dimethylamino-2,4-dimethyl-1-phenyl-pent-1-en-3-ol |
| Beispiel 123 | 5-Dimethylamino-3-(4-fluor-3-methyl-phenyl)-2,4-dimethyl-1-phenyl-pent-1-en-3-ol |
| Beispiel 124 | 5-Dimethylamino-2,4-dimethyl-1-phenyl-3-o-tolyl-pent-1-en-3-ol |
| Beispiel 125 | 3-(2-Dimethylamino-1-methyl-ethyl)-2-methyl-1-phenyl-penta-1,4-dien-3-ol |
| Beispiel 126 | 3-(4-tert-Butyl-phenyl)-5-dimethylamino-2,4-dimethyl-1-phenyl-pent-1-en-3-ol |
| Beispiel 127 | 3-Cyclopentyl-5-dimethylamino-2,4-dimethyl-1-phenyl-pent-1-en-3-ol |
| Beispiel 128 | 5-Dimethylamino-2,4-dimethyl-1-phenyl-3-m-tolyl-pent-1-en-3-ol |
| Beispiel 129 | 3-Cyclohexyl-5-dimethylamino-2,4-dimethyl-1-phenyl-pent-1-en-3-ol |
| Beispiel 130 | 5-Dimethylamino-3-(4-fluor-phenyl)-2,4-dimethyl-1-phenyl-pent-1-en-3-ol |
| Beispiel 131 | 5-Dimethylamino-2,4-dimethyl-3-phenethyl-1-phenyl-pent-1-en-3-ol |
| Beispiel 132 | 3-(2-Dimethylamino-1-methyl-ethyl)-2-methyl-1,5-diphenyl-pent-1-en-4-yn-3-ol |
| Beispiel 133 | 5-Dimethylamino-2,4-dimethyl-1-phenyl-3-thiophen-2-yl-pent-1-en-3-ol |
| Beispiel 134 | 3-(2,4-Dichlor-phenyl)-5-dimethylamino-2,4-dimethyl-1-phenyl-pent-1-en-3-ol |
| Beispiel 135 | 5-Dimethylamino-3-(3-methoxy-phenyl)-2,4-dimethyl-1-phenyl-pent-1-en-3-ol |
| Beispiel 136 | 3-(2-Dimethylamino-1-methyl-ethyl)-2-methyl-1,6-diphenyl-hex-1-en-3-ol |
| Beispiel 137 | 3-(2,3-Dichlor-phenyl)-5-dimethylamino-2,4-dimethyl-1-phenyl-pent-1-en-3-ol |
| Beispiel 138 | 5-Dimethylamino-2,4-dimethyl-1-phenyl-3-p-tolyl-pent-1-en-3-ol |
| Beispiel 139 | 5-Dimethylamino-3-(4-methoxy-phenyl)-2,4-dimethyl-1-phenyl-pent-1-en-3-ol |
| Beispiel 140 | 3-Cyclohexylmethyl-5-dimethylamino-2,4-dimethyl-1-phenyl-pent-1-en-3-ol |
| Beispiel 141 | 5-Dimethylamino-3-(5-fluor-2-methoxy-phenyl)-2,4-dimethyl-1-phenyl-pent-1-en-3-ol |
| Beispiel 142 | 5-Dimethylamino-3-(3-fluor-phenyl)-2,4-dimethyl-1-phenyl-pent-1-en-3-ol |
| Beispiel 143 | 3-(3-Chlor-phenyl)-5-dimethylamino-2,4-dimethyl-1-phenyl-pent-1-en-3-ol |
| Beispiel 144 | 3-(3,5-Dichlor-phenyl)-5-dimethylamino-2,4-dimethyl-1-phenyl-pent-1-en-3-ol |
| Beispiel 145 | 3-(2-Chlor-benzyl)-5-dimethylamino-2,4-dimethyl-1-phenyl-pent-1-en-3-ol |
| Beispiel 146 | 5-Dimethylamino-3-(4-fluor-benzyl)-2,4-dimethyl-1-phenyl-pent-1-en-3-ol |
| Beispiel 147 | 3-(4-Chlor-3-trifluormethyl-phenyl)-5-dimethylamino-2,4-dimethyl-1-phenyl-pent-1-en-3-ol |
| Beispiel 148 | 5-Dimethylamino-3-(2-methoxy-phenyl)-2,4-dimethyl-1-phenyl-pent-1-en-3-ol |
| Beispiel 149 | 5-Dimethylamino-2,4-dimethyl-3-(2-methyl-benzyl)-1-phenyl-pent-1-en-3-ol |
| Beispiel 150 | 3-(3-Chlor-4-fluor-phenyl)-5-dimethylamino-2,4-dimethyl-1-phenyl-pent-1-en-3-ol |
| Beispiel 151 | 5-Dimethylamino-2,4-dimethyl-1-phenyl-3-(3-trifluormethyl-phenyl)-pent-1-en-3-ol |
| Beispiel 152 | 5-Dimethylamino-2,4-dimethyl-3-(3-methyl-benzyl)-1-phenyl-pent-1-en-3-ol |
| Beispiel 153 | 3-(4-Chlor-benzyl)-5-dimethylamino-2,4-dimethyl-1-phenyl-pent-1-en-3-ol |
| Beispiel 154 | 3-(2-Chlor-6-fluor-benzyl)-5-dimethylamino-2,4-dimethyl-1-phenyl-pent-1-en-3-ol |
| Beispiel 155 | 5-Dimethylamino-3-(2,5-dimethyl-benzyl)-2,4-dimethyl-1-phenyl-pent-1-en-3-ol |
| Beispiel 156 | 3-(3-Chlor-benzyl)-5-dimethylamino-2,4-dimethyl-1-phenyl-pent-1-en-3-ol |
| Beispiel 157 | 3-(2,4-Dichlor-benzyl)-5-dimethylamino-2,4-dimethyl-1-phenyl-pent-1-en-3-ol |
| Beispiel 158 | 5-Dimethylamino-1-(4-fluor-phenyl)-2,4-dimethyl-3-phenyl-pent-l-en-3-ol |
| Beispiel 159 | 3-Benzyl-5-dimethylamino-1-(4-fluor-phenyl)-2,4-dimethyl-pent-1-en-3-ol |
| Beispiel 160 | 5-Dimethylamino-3-(4-fluor-3-methyl-phenyl)-1-(4-fluor-phenyl)-2,4-dimethyl-pent-1-en-3-ol |
| Beispiel 161 | 5-Dimethylamino-1-(4-fluor-phenyl)-2,4-dimethyl-3-o-tolyl-pent-1-en-3-ol |
| Beispiel 162 | 3-(2-Dimethylamino-1-methyl-ethyl)-1-(4-fluor-phenyl)-2-methyl-penta-1,4-dien-3-ol |
| Beispiel 163 | 3-(4-tert-Butyl-phenyl)-5-dimethylamino-1-(4-fluor-phenyl)-2,4-dimethyl-pent-1-en-3-ol |
| Beispiel 164 | 3-Cyclopentyl-5-dimethylamino-1-(4-fluor-phenyl)-2,4-dimethyl-pent-1-en-3-ol |
| Beispiel 165 | 5-Dimethylamino-1-(4-fluor-phenyl)-2,4-dimethyl-3-m-tolyl-pent-1-en-3-ol |
| Beispiel 166 | 3-Cyclohexyl-5-dimethylamino-1-(4-fluor-phenyl)-2,4-dimethyl-pent-1-en-3-ol |
| Beispiel 167 | 5-Dimethylamino-1,3-bis-(4-fluor-phenyl)-2,4-dimethyl-pent-1-en-3-ol |
| Beispiel 168 | 5-Dimethylamino-1-(4-fluor-phenyl)-2,4-dimethyl-3-phenethyl-pent-1-en-3-ol |
| Beispiel 169 | 3-(2-Dimethylamino-1-methyl-ethyl)-1-(4-fluor-phenyl)-2-methyl-5-phenyl-pent-1-en-4-yn-3-ol |
| Beispiel 170 | 5-Dimethylamino-1-(4-fluor-phenyl)-2,4-dimethyl-3-thiophen-2-yl-pent-1-en-3-ol |
| Beispiel 171 | 3-(2,4-Dichlor-phenyl)-5-dimethylamino-1-(4-fluor-phenyl)-2,4-dimethyl-pent-1-en-3-ol |
| Beispiel 172 | 5-Dimethylamino-1-(4-fluor-phenyl)-3-(3-methoxy-phenyl)-2,4-dimethyl-pent-1-en-3-ol |
| Beispiel 173 | 3-(2-Dimethylamino-1-methyl-ethyl)-1-(4-fluor-phenyl)-2-methyl-6-phenyl-hex-1-en-3-ol |
| Beispiel 174 | 3-(2,3-Dichlor-phenyl)-5-dimethylamino-1-(4-fluor-phenyl)-2,4-dimethyl-pent-1-en-3-ol |
| Beispiel 175 | 5-Dimethylamino-1-(4-fluor-phenyl)-2,4-dimethyl-3-p-tolyl-pent-1-en-3-ol |
| Beispiel 176 | 5-Dimethylamino-1-(4-fluor-phenyl)-3-(4-methoxy-phenyl)-2,4-dimethyl-pent-1-en-3-ol |
| Beispiel 177 | 3-Cyclohexylmethyl-5-dimethylamino-1-(4-fluor-phenyl)-2,4-dimethyl-pent-1-en-3-ol |
| Beispiel 178 | 5-Dimethylamino-3-(5-fluor-2-methoxy-phenyl)-1-(4-fluor-phenyl)-2,4-dimethyl-pent-1-en-3-ol |
| Beispiel 179 | 5-Dimethylamino-3-(3-fluor-phenyl)-1-(4-fluor-phenyl)-2,4-dimethyl-pent-1-en-3-ol |
| Beispiel 180 | 3-(3-Chlor-phenyl)-5-dimethylamino-1-(4-fluor-phenyl)-2,4-dimethyl-pent-1-en-3-ol |
| Beispiel 181 | 3-(3,5-Dichlor-phenyl)-5-dimethylamino-1-(4-fluor-phenyl)-2,4-dimethyl-pent-1-en-3-ol |
| Beispiel 182 | 3-(2-Chlor-benzyl)-5-dimethylamino-1-(4-fluor-phenyl)-2,4-dimethyl-pent-1-en-3-ol |
| Beispiel 183 | 5-Dimethylamino-3-(4-fluor-benzyl)-1-(4-fluor-phenyl)-2,4-dimethyl-pent-1-en-3-ol |
| Beispiel 184 | 5-Dimethylamino-1-(4-fluor-phenyl)-3-(3-methoxy-benzyl)-2,4-dimethyl-pent-1-en-3-ol |
| Beispiel 185 | 3-(4-Chlor-3-trifluormethyl-phenyl)-5-dimethylamino-1-(4-fluor-phenyl)-2,4-dimethyl-pent-1-en-3-ol |
| Beispiel 186 | 5-Dimethylamino-3-(3-fluor-benzyl)-1-(4-fluor-phenyl)-2,4-dimethyl-pent-1-en-3-ol |
| Beispiel 187 | 5-Dimethylamino-1-(4-fluor-phenyl)-3-(2-methoxy-phenyl) -2,4-dimethyl-pent-1-en-3-ol |
| Beispiel 188 | 5-Dimethylamino-1-(4-fluor-phenyl)-2,4-dimethyl-3-(2-methyl-benzyl)-pent-1-en-3-ol |
| Beispiel 189 | 3-(3-Chlor-4-fluor-phenyl)-5-dimethylamino-1-(4-fluor-phenyl)-2,4-dimethyl-pent-1-en-3-ol |
| Beispiel 190 | 5-Dimethylamino-1-(4-fluor-phenyl)-2,4-dimethyl-3-(3-trifluormethyl-phenyl) -pent-1-en-3-ol |
| Beispiel 191 | 5-Dimethylamino-1-(4-fluor-phenyl)-2,4-dimethyl-3-(3-methyl-benzyl)-pent-1-en-3-ol |
| Beispiel 192 | 3-(4-Chlor-benzyl)-5-dimethylamino-1-(4-fluor-phenyl)-2,4-dimethyl-pent-1-en-3-ol |
| Beispiel 193 | 3-(2-Chlor-6-fluor-benzyl)-5-dimethylamino-1-(4-fluor-phenyl)-2,4-dimethyl-pent-1-en-3-ol |
| Beispiel 194 | 5-Dimethylamino-3-(2,5-dimethyl-benzyl)-1-(4-fluor-phenyl)-2,4-dimethyl-pent-1-en-3-ol |
| Beispiel 195 | 3-(3-Chlor-benzyl)-5-dimethylamino-1-(4-fluor-phenyl)-2,4-dimethyl-pent-1-en-3-ol |
| Beispiel 196 | 3-(2,4-Dichlor-benzyl)-5-dimethylamino-1-(4-fluor-phenyl)-2,4-dimethyl-pent-1-en-3-ol |
| Beispiel 197 | 1-(4-Chlor-phenyl)-5-dimethylamino-2,4-dimethyl-3-phenyl-pent-1-en-3-ol |
| Beispiel 198 | 1,3-Bis-(4-chlor-phenyl)-5-dimethylamino-2,4-dimethyl-pent-1-en-3-ol |
| Beispiel 199 | 3-Benzyl-1-(4-chlor-phenyl)-5-dimethylamino-2,4-dimethyl-pent-1-en-3-ol |
| Beispiel 200 | 1-(4-Chlor-phenyl)-5-dimethylamino-3-(4-fluor-3-methyl-phenyl)-2,4-dimethyl-pent-1-en-3-ol |
| Beispiel 201 | 1-(4-Chlor-phenyl)-5-dimethylamino-2,4-dimethyl-3-o-tolyl-pent-1-en-3-ol |
| Beispiel 202 | 1-(4-Chlor-phenyl)-3-(2-dimethylamino-1-methyl-ethyl)-2-methyl-penta-1,4-dien-3-ol |
| Beispiel 203 | 3-(4-tert-Butyl-phenyl)-1-(4-chlor-phenyl)-5-dimethylamino-2,4-dimethyl-pent-1-en-3-ol |
| Beispiel 204 | 1-(4-Chlor-phenyl)-3-cyclopentyl-5-dimethylamino-2,4-dimethyl-pent-1-en-3-ol |
| Beispiel 205 | 1-(4-Chlor-phenyl)-5-dimethylamino-2,4-dimethyl-3-m-tolyl-pent-1-en-3-ol |
| Beispiel 206 | 1-(4-Chlor-phenyl)-3-cyclohexyl-5-dimethylamino-2,4-dimethyl-pent-1-en-3-ol |
| Beispiel 207 | 1-(4-Chlor-phenyl)-5-dimethylamino-3-(4-fluor-phenyl)-2,4-dimethyl-pent-1-en-3-ol |
| Beispiel 208 | 1-(4-Chlor-phenyl)-5-dimethylamino-2,4-dimethyl-3-phenethyl-pent-1-en-3-ol |
| Beispiel 209 | 1-(4-Chlor-phenyl)-5-dimethylamino-2,4-dimethyl-3-thiophen-2-yl-pent-1-en-3-ol |
| Beispiel 210 | 1-(4-Chlor-phenyl)-3-(2,4-dichlor-phenyl)-5-dimethylamino-2,4-dimethyl-pent-1-en-3-ol |
| Beispiel 211 | 1-(4-Chlor-phenyl)-5-dimethylamino-3-(3-methoxy-phenyl)-2,4-dimethyl-pent-1-en-3-ol |
| Beispiel 212 | 1-(4-Chlor-phenyl)-3-(2-dimethylamino-1-methyl-ethyl)-2-methyl-6-phenyl-hex-1-en-3-ol |
| Beispiel 213 | 1-(4-Chlor-phenyl)-3-(2,3-dichlor-phenyl)-5-dimethylamino-2,4-dimethyl-pent-1-en-3-ol |
| Beispiel 214 | 1-(4-Chlor-phenyl)-5-dimethylamino-2,4-dimethyl-3-p-tolyl-pent-1-en-3-ol |
| Beispiel 215 | 1-(4-Chlor-phenyl)-5-dimethylamino-3-(4-methoxy-phenyl)-2,4-dimethyl-pent-1-en-3-ol |
| Beispiel 216 | 1-(4-Chlor-phenyl)-3-cyclohexylmethyl-5-dimethylamino-2,4-dimethyl-pent-1-en-3-ol |
| Beispiel 217 | 1-(4-Chlor-phenyl)-5-dimethylamino-3-(5-fluor-2-methoxy-phenyl)-2,4-dimethyl-pent-1-en-3-ol |
| Beispiel 218 | 1-(4-Chlor-phenyl)-5-dimethylamino-3-(3-fluor-phenyl)-2,4-dimethyl-pent-1-en-3-ol |
| Beispiel 219 | 3-(3-Chlor-phenyl)-1-(4-chlor-phenyl)-5-dimethylamino-2,4-dimethyl-pent-1-en-3-ol |
| Beispiel 220 | 1-(4-Chlor-phenyl)-3-(3,5-dichlor-phenyl)-5-dimethylamino-2,4-dimethyl-pent-1-en-3-ol |
| Beispiel 221 | 3-(2-Chlor-benzyl)-1-(4-chlor-phenyl)-5-dimethylamino-2,4-dimethyl-pent-1-en-3-ol |
| Beispiel 222 | 1-(4-Chlor-phenyl)-5-dimethylamino-3-(4-fluor-benzyl)-2,4-dimethyl-pent-1-en-3-ol |
| Beispiel 223 | 1-(4-Chlor-phenyl)-5-dimethylamino-3-(3-methoxy-benzyl)-2,4-dimethyl-pent-1-en-3-ol |
| Beispiel 224 | 1-(4-Chlor-phenyl)-3-(4-chlor-3-trifluormethyl-phenyl)-5-dimethylamino-2,4-dimethyl-pent-1-en-3-ol |
| Beispiel 225 | 1-(4-Chlor-phenyl)-5-dimethylamino-3-[2-(3-fluor-phenyl)-ethyl]-2,4-dimethyl-pent-1-en-3-ol |
| Beispiel 226 | 1-(4-Chlor-phenyl)-5-dimethylamino-3-(2-methoxy-phenyl)-2,4-dimethyl-pent-1-en-3-ol |
| Beispiel 227 | 1-(4-Chlor-phenyl)-5-dimethylamino-2,4-dimethyl-3-(2-methyl-benzyl)-pent-1-en-3-ol |
| Beispiel 228 | 3- (3-Chlor-4-fluor-phenyl)-1-(4-chlor-phenyl)-5-dimethylamino-2,4-dimethyl-pent-1-en-3-ol |
| Beispiel 229 | 1-(4-Chlor-phenyl)-5-dimethylamino-2,4-dimethyl-3-(3-trifluormethyl-phenyl)-pent-1-en-3-ol |
| Beispiel 230 | 1-(4-Chlor-phenyl)-5-dimethylamino-2,4-dimethyl-3-(3-methyl-benzyl)-pent-1-en-3-ol |
| Beispiel 231 | 3-(4-Chlor-benzyl)-1-(4-chlor-phenyl)-5-dimethylamino-2,4-dimethyl-pent-1-en-3-ol |
| Beispiel 232 | 3-(2-Chlor-6-fluor-benzyl)-1-(4-chlor-phenyl)-5-dimethylamino-2,4-dimethyl-pent-1-en-3-ol |
| Beispiel 233 | 1-(4-Chlor-phenyl)-5-dimethylamino-3-(2,5-dimethyl-benzyl)-2,4-dimethyl-pent-1-en-3-ol |
| Beispiel 234 | 3-(3-Chlor-benzyl)-1-(4-chlor-phenyl)-5-dimethylamino-2,4-dimethyl-pent-1-en-3-ol |
| Beispiel 235 | 1-(4-Chlor-phenyl)-3-(2,4-dichlor-benzyl)-5-dimethylamino-2,4-dimethyl-pent-1-en-3-ol |
| Beispiel 236 | 5-Dimethylamino-1-(4-methoxy-phenyl)-2,4-dimethyl-3-phenyl-pent-1-en-3-ol |
| Beispiel 237 | 3-(4-Chlor-phenyl)-5-dimethylamino-1-(4-methoxy-phenyl)-2,4-dimethyl-pent-1-en-3-ol |
| Beispiel 238 | 3-Benzyl-5-dimethylamino-1-(4-methoxy-phenyl)-2,4-dimethyl-pent-1-en-3-ol |
| Beispiel 239 | 5-Dimethylamino-3-(4-fluor-3-methyl-phenyl)-1-(4-methoxy-phenyl)-2,4-dimethyl-pent-1-en-3-ol |
| Beispiel 240 | 5-Dimethylamino-1-(4-methoxy-phenyl)-2,4-dimethyl-3-o-tolyl-pent-1-en-3-ol |
| Beispiel 241 | 3-(2-Dimethylamino-1-methyl-ethyl)-1-(4-methoxy-phenyl)-2-methyl-penta-1,4-dien-3-ol |
| Beispiel 242 | 3-(4-tert-Butyl-phenyl)-5-dimethylamino-1-(4-methoxy-phenyl)-2,4-dimethyl-pent-1-en-3-ol |
| Beispiel 243 | 3-Cyclopentyl-5-dimethylamino-1-(4-methoxy-phenyl)-2,4-dimethyl-pent-1-en-3-ol |
| Beispiel 244 | 5-Dimethylamino-1-(4-methoxy-phenyl)-2,4-dimethyl-3-m-tolyl-pent-1-en-3-ol |
| Beispiel 245 | 3-Cyclohexyl-5-dimethylamino-1-(4-methoxy-phenyl)-2,4-dimethyl-pent-1-en-3-ol |
| Beispiel 246 | 5-Dimethylamino-3-(4-fluor-phenyl)-1-(4-methoxy-phenyl)-2,4-dimethyl-pent-1-en-3-ol |
| Beispiel 247 | 5-Dimethylamino-1-(4-methoxy-phenyl)-2,4-dimethyl-3-phenethyl-pent-1-en-3-ol |
| Beispiel 248 | 3-(2-Dimethylamino-1-methyl-ethyl)-1-(4-methoxy-phenyl)-2-methyl-5-phenyl-pent-1-en-4-yn-3-ol |
| Beispiel 249 | 5-Dimethylamino-1-(4-methoxy-phenyl)-2,4-dimethyl-3-thiophen-2-yl-pent-1-en-3-ol |
| Beispiel 250 | 3-(2,4-Dichlor-phenyl)-5-dimethylamino-1-(4-methoxy-phenyl)-2,4-dimethyl-pent-1-en-3-ol |
| Beispiel 251 | 5-Dimethylamino-3-(3-methoxy-phenyl)-1-(4-methoxy-phenyl)-2,4-dimethyl-pent-1-en-3-ol |
| Beispiel 252 | 3-(2-Dimethylamino-1-methyl-ethyl)-1-(4-methoxy-phenyl)-2-methyl-6-phenyl-hex-1-en-3-ol |
| Beispiel 253 | 3-(2,3-Dichlor-phenyl)-5-dimethylamino-1-(4-methoxy-phenyl)-2,4-dimethyl-pent-1-en-3-ol |
| Beispiel 254 | 5-Dimethylamino-1-(4-methoxy-phenyl)-2,4-dimethyl-3-p-tolyl-pent-1-en-3-ol |
| Beispiel 255 | 5-Dimethylamino-1,3-bis-(4-methoxy-phenyl)-2,4-dimethyl-pent-1-en-3-ol |
| Beispiel 256 | 3-Cyclohexylmethyl-5-dimethylamino-1-(4-methoxy-phenyl)-2,4-dimethyl-pent-1-en-3-ol |
| Beispiel 257 | 5-Dimethylamino-3-(5-fluor-2-methoxy-phenyl)-1-(4-methoxy-phenyl)-2,4-dimethyl-pent-1-en-3-ol |
| Beispiel 258 | 5-Dimethylamino-3-(3-fluor-phenyl)-1-(4-methoxy-phenyl)-2,4-dimethyl-pent-1-en-3-ol |
| Beispiel 259 | 3-(3-Chlor-phenyl)-5-dimethylamino-1-(4-methoxy-phenyl)-2,4-dimethyl-pent-1-en-3-ol |
| Beispiel 260 | 3-(3,5-Dichlor-phenyl)-5-dimethylamino-1-(4-methoxy-phenyl)-2,4-dimethyl-pent-1-en-3-ol |
| Beispiel 261 | 3-(2-Chlor-benzyl)-5-dimethylamino-1-(4-methoxy-phenyl)-2,4-dimethyl-pent-1-en-3-ol |
| Beispiel 262 | 5-Dimethylamino-3-(4-fluor-benzyl)-1-(4-methoxy-phenyl)-2,4-dimethyl-pent-1-en-3-ol |
| Beispiel 263 | 5-Dimethylamino-3-(3-fluor-benzyl)-1-(4-methoxy-phenyl)-2,4-dimethyl-pent-1-en-3-ol |
| Beispiel 264 | 5-Dimethylamino-3-(2-methoxy-phenyl)-1-(4-methoxy-phenyl)-2,4-dimethyl-pent-1-en-3-ol |
| Beispiel 265 | 5-Dimethylamino-1-(4-methoxy-phenyl)-2,4-dimethyl-3-(2-methyl-benzyl)-pent-1-en-3-ol |
| Beispiel 266 | 3-(3-Chlor-4-fluor-phenyl)-5-dimethylamino-1-(4-methoxy-phenyl)-2,4-dimethyl-pent-1-en-3-ol |
| Beispiel 267 | 5-Dimethylamino-1-(4-methoxy-phenyl)-2,4-dimethyl-3-(3-trifluormethyl-phenyl)-pent-1-en-3-ol |
| Beispiel 268 | 3-(4-Chlor-benzyl)-5-dimethylamino-1-(4-methoxy-phenyl)-2,4-dimethyl-pent-1-en-3-ol |
| Beispiel 269 | 3-(2-Chlor-6-fluor-benzyl)-5-dimethylamino-1-(4-methoxy-phenyl)-2,4-dimethyl-pent-1-en-3-ol |
| Beispiel 270 | 3-(3-Chlor-benzyl)-5-dimethylamino-1-(4-methoxy-phenyl)-2,4-dimethyl-pent-1-en-3-ol |
| Beispiel 271 | 2-Benzyliden-1-(4-tert-butyl-phenyl)-5-dimethylaminomethyl-cyclopentanol |
| Beispiel 272 | 2-Benzyliden-1-cyclohexyl-5-dimethylaminomethyl-cyclopentanol |
| Beispiel 273 | 2-Benzyliden-5-dimethylaminomethyl-1-phenethyl-cyclopentanol |
| Beispiel 274 | 2-Benzyliden-5-dimethylaminomethyl-1-(4-fluor-benzyl)-cyclopentanol |
| Beispiel 275 | 2-Benzyliden-5-dimethylaminomethyl-1-(3-fluor-benzyl)-cyclopentanol |
| Beispiel 276 | 2-Benzyliden-5-dimethylaminomethyl-1-(2-methoxy-phenyl)-cyclopentanol |
| Beispiel 277 | 2-Benzyliden-5-dimethylaminomethyl-1-(2-methyl-benzyl)-cyclopentanol |
| Beispiel 278 | 2-Benzyliden-5-dimethylaminomethyl-1-(3-methyl-benzyl)-cyclopentanol |
| Beispiel 279 | 2-Benzyliden-1-(4-chlor-benzyl)-5-dimethylaminomethyl-cyclopentanol |
| Beispiel 280 | 2-Benzyliden-1-(2-chlor-6-fluor-benzyl)-5-dimethylaminomethyl-cyclopentanol |
| Beispiel 281 | 2-Benzyliden-5-dimethylaminomethyl-1-(2,5-dimethyl-benzyl)-cyclopentanol |
| Beispiel 282 | 2-Benzyliden-1-(3-chlor-benzyl)-5-dimethylaminomethyl-cyclopentanol |
| Beispiel 283 | 2-Dimethylaminomethyl-5-(3-methoxy-benzyl)-bicyclopentyl-1-ol |
| Beispiel 284 | 2-Dimethylaminomethyl-5- O-methoxy-benzyl)-1-m-tolyl-cyclopentanol |
| Beispiel 285 | 1-Cyclohexyl-2-dimethylaminomethyl-5-(3-methoxy-benzyl)-cyclopentanol |
| Beispiel 286 | 2-Dimethylaminomethyl-5-(3-methoxy-benzyl)-1-phenethyl-cyclopentanol |
| Beispiel 287 | 2-Dimethylaminomethyl-5-(3-methoxy-benzyl)-1-phenylethynyl-cyclopentanol |
| Beispiel 288 | 2-Dimethylaminomethyl-5-(3-methoxy-benzyl)-1-thiophen-2-yl-cyclopentanol |
| Beispiel 289 | 2-Dimethylaminomethyl-5-(3-methoxy-benzyl)-1-(3-methoxy-phenyl)-cyclopentanol |
| Beispiel 290 | 2-Dimethylaminomethyl-5-(3-methoxy-benzyl)-1-(3-phenyl-propyl)-cyclopentanol |
| Beispiel 291 | 2-Dimethylaminomethyl-5-(3-methoxy-benzyl)-1-(4-methoxy-phenyl)-cyclopentanol |
| Beispiel 292 | 2-Dimethylaminomethyl-1-(3-fluor-benzyl)-5-(3-methoxy-benzyliden)-cyclopentanol |
| Beispiel 293 | 2-Dimethylaminomethyl-5-(3-methoxy-benzyliden)-1-(2-methoxy-phenyl)-cyclopentanol |
| Beispiel 294 | 2-Dimethylaminomethyl-5-(3-methoxy-benzyliden)-1-(2-methyl-benzyl)-cyclopentanol |
| Beispiel 295 | 2-Dimethylaminomethyl-5-(3-methoxy-benzyliden)-1-(3-methyl-benzyl)-cyclopentanol |
| Beispiel 296 | 2-Dimethylaminomethyl-1-(2,5-dimethyl-benzyl)-5-(3-methoxy-benzyliden)-cyclopentanol |
| Beispiel 297 | 1-(3-Chlor-benzyl)-2-dimethylaminomethyl-5-(3-methoxy-benzyliden)-cyclopentanol |
| Beispiel 298 | 1-Benzyl-2-dimethylaminomethyl-5-(3-methoxy-benzyliden)-cyclopentanol |
| Beispiel 299 | 2-Dimethylaminomethyl-5-(3-methoxy-benzyliden)-1-o-tolyl-cyclopentanol |
| Beispiel 300 | 2-Dimethylaminomethyl-5-(3-methoxy-benzyliden)-1-vinyl-cyclopentanol |
| Beispiel 301 | 1-(4-tert-Butyl-phenyl)-2-dimethylaminomethyl-5-(3-methoxy-benzyliden)-cyclopentanol |
| Beispiel 302 | 2-Dimethylaminomethyl-5-(3-methoxy-benzyliden)-bicyclopentyl-1-ol |
| Beispiel 303 | 1-Cyclohexyl-2-dimethylaminomethyl-5-(3-methoxy-benzyliden)-cyclopentanol |
| Beispiel 304 | 2-Dimethylaminomethyl-1-(4-fluor-phenyl)-5-(3-methoxy-benzyliden)-cyclopentanol |
| Beispiel 305 | 2-Dimethylaminomethyl-5-(3-methoxy-benzyliden)-1-phenethyl-cyclopentanol |
| Beispiel 306 | 2-Dimethylaminomethyl-5-(3-methoxy-benzyliden)-1-phenylethynyl-cyclopentanol |
| Beispiel 307 | 2-Dimethylaminomethyl-5-(3-methoxy-benzyliden)-1-thiophen-2-yl-cyclopentanol |
| Beispiel 308 | 2-Dimethylaminomethyl-5-(3-methoxy-benzyliden)-1-(3-methoxy-phenyl)-cyclopentanol |
| Beispiel 309 | 2-Dimethylaminomethyl-5-(3-methoxy-benzyliden)-1-(3-phenyl-propyl)-cyclopentanol |
| Beispiel 310 | 2-Dimethylaminomethyl-5-(3-methoxy-benzyliden)-1-p-tolyl-cyclopentanol |
| Beispiel 311 | 2-Dimethylaminomethyl-5-(3-methoxy-benzyliden)-1-(4-methoxy-phenyl)-cyclopentanol |
| Beispiel 312 | 1-(2-Chlor-benzyl)-2-dimethylaminomethyl-5-(4-methoxy-benzyliden)-cyclopentanol |
| Beispiel 313 | 2-Dimethylaminomethyl-1-(3-methoxy-benzyl)-5-(4-methoxy-benzyliden)-cyclopentanol |
| Beispiel 314 | 2-Dimethylami.nomethyl-1-(3-fluor-benzyl)-5-(4-methoxy-benzyliden)-cyclopentanol |
| Beispiel 315 | 2-Dimethylaminomethyl-5-(4-methoxy-benzyliden)-1-(2-methoxy-phenyl)-cyclopentanol |
| Beispiel 316 | 2-Dimethylaminomethyl-5-(4-methoxy-benzyliden)-1-(3-methyl-benzyl)-cyclopentanol |
| Beispiel 317 | 1-(4-Chlor-benzyl)-2-dimethylaminomethyl-5-(4-methoxy-benzyliden)-cyclopentanol |
| Beispiel 318 | 1-(2-Chlor-6-fluor-benzyl)-2-dimethylaminomethyl-5-(4-methoxy-benzyliden)-cyclopentanol |
| Beispiel 319 | 1-(3-Chlor-benzyl)-2-dimethylaminomethyl-5-(4-methoxy-benzyliden)-cyclopentanol |
| Beispiel 320 | 1-(2,4-Dichlor-benzyl)-2-dimethylaminomethyl-5-(4-methoxy-benzyliden)-cyclopentanol |
| Beispiel 321 | 2-Benzyliden-7-dimethylaminomethyl-1-phenyl-cycloheptanol |
| Beispiel 322 | 2-Benzyliden-1-(4-chlor-phenyl)-7-dimethylaminomethyl-cycloheptanol |
| Beispiel 323 | 1-Benzyl-2-benzyliden-7-dimethylaminomethyl-cycloheptanol |
| Beispiel 324 | 2-Benzyliden-7-dimethylaminomethyl-1-(4-fluor-3-methyl-phenyl)-cycloheptanol |
| Beispiel 325 | 2-Benzyliden-7-dimethylaminomethyl-1-o-tolyl-cycloheptanol |
| Beispiel 326 | 2-Benzyliden-7-dimethylaminomethyl-1-vinyl-cycloheptanol |
| Beispiel 327 | 2-Benzyliden-1-(4-tert-butyl-phenyl)-7-dimethylaminomethyl-cycloheptanol |
| Beispiel 328 | 2-Benzyliden-1-cyclopentyl-7-dimethylaminomethyl-cycloheptanol |
| Beispiel 329 | 2-Benzyliden-7-dimethylaminomethyl-1-m-tolyl-cycloheptanol |
| Beispiel 330 | 2-Benzyliden-1-cyclohexyl-7-dimethylaminomethyl-cycloheptanol |
| Beispiel 331 | 2-Benzyliden-7-dimethylaminomethyl-1-(4-fluor-phenyl)-cycloheptanol |
| Beispiel 332 | 2-Benzyliden-7-dimethylaminomethyl-1-phenylethynyl-cycloheptanol |
| Beispiel 333 | 2-Benzyliden-7-dimethylaminomethyl-1-thiophen-2-yl-cycloheptanol |
| Beispiel 334 | 2-Benzyliden-7-dimethylaminomethyl-1-(3-methoxy-phenyl)-cycloheptanol |
| Beispiel 335 | 2-Benzyliden-1-cyclohexylmethyl-7-dimethylaminomethyl-cycloheptanol |
| Beispiel 336 | 2-Benzyliden-7-dimethylaminomethyl-1-(3-fluor-4-methoxy-phenyl)-cycloheptanol |
| Beispiel 337 | 2-Benzyliden-7-dimethylaminomethyl-1-(3-fluor-phenyl)-cycloheptanol |
| Beispiel 338 | 2-Benzyliden-1-(3-chlor-phenyl)-7-dimethylaminomethyl-cycloheptanol |
| Beispiel 339 | 2-Benzyliden-1-(3,5-dichlor-phenyl)-7-dimethylaminomethyl-cycloheptanol |
| Beispiel 340 | 2-Benzyliden-7-dimethylaminomethyl-1-(4-fluor-benzyl)-cycloheptanol |
| Beispiel 341 | 2-Benzyliden-7-dimethylaminomethyl-1-(4-methoxy-benzyl)-cycloheptanol |
| Beispiel 342 | 2-Benzyliden-7-dimethylaminomethyl-1-(3-fluor-benzyl)-cycloheptanol |
| Beispiel 343 | 2-Benzyliden-7-dimethylaminomethyl-1-(2-methoxy-phenyl)-cycloheptanol |
| Beispiel 344 | 2-Benzyliden-7-dimethylaminomethyl-1-(2-methyl-benzyl)-cycloheptanol |
| Beispiel 345 | 2-Benzyliden-1-(3-chlor-4-fluor-phenyl)-7-dimethylaminomethyl-cycloheptanol |
| Beispiel 346 | 2-Benzyliden-7-dimethylaminomethyl-1-(3-trifluormethyl-phenyl)-cycloheptanol |
| Beispiel 347 | 2-Benzyliden-7-dimethylaminomethyl-1-(3-methyl-benzyl)-cycloheptanol |
| Beispiel 348 | 2-Benzyliden-1-(4-chlor-benzyl)-7-dimethylaminomethyl-cycloheptanol |
| Beispiel 349 | 2-Benzyliden-1-(2-chlor-6-fluor-benzyl)-7-dimethylaminomethyl-cycloheptanol |
| Beispiel 350 | 2-Benzyliden-7-dimethylaminomethyl-1-(2,5-dimethyl-benzyl)-cycloheptanol |
| Beispiel 351 | 2-Benzyliden-1-(3-chlor-benzyl)-7-dimethylaminomethyl-cycloheptanol |
| Beispiel 352 | 2-Dimethylaminomethyl-7-(3-methoxy-benzyliden)-1-phenyl-cycloheptanol |
| Beispiel 353 | 1-(4-Chlor-phenyl)-2-dimethylaminomethyl-7-(3-methoxy-benzyliden)-cycloheptanol |
| Beispiel 354 | 1-Benzyl-2-dimethylaminomethyl-7-(3-methoxy-benzyliden)-cycloheptanol |
| Beispiel 355 | 2-Dimethylaminomethyl-1-(4-fluor-3-methyl-phenyl)-7-(3-methoxy-benzyliden)-cycloheptanol |
| Beispiel 356 | 2-Dimethylaminomethyl-7-(3-methoxy-benzyliden)-1-o-tolyl-cycloheptanol |
| Beispiel 357 | 2-Dimethylaminomethyl-7-(3-methoxy-benzyliden)-1-vinyl-cycloheptanol |
| Beispiel 358 | 1-(4-tert-Butyl-phenyl)-2-dimethylaminomethyl-7-(3-methoxy-benzyliden)-cycloheptanol |
| Beispiel 359 | 1-Cyclopentyl-2-dimethylaminomethyl-7-(3-methoxy-benzyliden)-cycloheptanol |
| Beispiel 360 | 2-Dimethylaminomethyl-7-(3-methoxy-benzyliden)-1-m-tolyl-cycloheptanol |
| Beispiel 361 | 1-Cyclohexyl-2-dimethylaminomethyl-7-(3-methoxy-benzyliden)-cycloheptanol |
| Beispiel 362 | 2-Dimethylaminomethyl-1-(4-fluor-phenyl)-7-(3-methoxy-benzyliden)-cycloheptanol |
| Beispiel 363 | 2-Dimethylaminomethyl-7-(3-methoxy-benzyliden)-1-phenethyl-cycloheptanol |
| Beispiel 364 | 2-Dimethylaminomethyl-7-(3-methoxy-benzyliden)-1-phenylethynyl-cycloheptanol |
| Beispiel 365 | 2-Dimethylaminomethyl-7-(3-methoxy-benzyliden)-1-thiophen-2-yl-cycloheptanol |
| Beispiel 366 | 2-Dimethylaminomethyl-7-(3-methoxy-benzyliden)-1-(3-methoxy-phenyl)-cycloheptanol |
| Beispiel 367 | 2-Dimethylaminomethyl-7-(3-methoxy-benzyliden)-1-(3-phenyl-propyl)-cycloheptanol |
| Beispiel 368 | 2-Dimethylaminomethyl-7-(3-methoxy-benzyliden)-1-p-tolyl-cycloheptanol |
| Beispiel 369 | 2-Dimethylaminomethyl-7-(3-methoxy-benzyliden)-1-(4-methoxy-phenyl)-cycloheptanol |
| Beispiel 370 | 1-Cyclohexylmethyl-2-dimethylaminomethyl-7-(3-methoxy-benzyliden)-cycloheptanol |
| Beispiel 371 | 1-(3-Chlor-phenyl)-2-dimethylaminomethyl-7-(3-methoxy-benzyliden)-cycloheptanol |
| Beispiel 372 | 1-(3,5-Dichlor-phenyl)-2-dimethylaminomethyl-7-(3-methoxy-benzyliden)-cycloheptanol |
| Beispiel 373 | 1-(2-Chlor-benzyl)-2-dimethylaminomethyl-7-(3-methoxy-benzyliden)-cycloheptanol |
| Beispiel 374 | 1-(4-Chlor-3-trifluormethyl-phenyl)-2-dimethylaminomethyl-7-(3-methoxy-benzyliden)-cycloheptanol |
| Beispiel 375 | 2-Dimethylaminomethyl-1-(3-fluor-phenyl)-7-(3-methoxy-benzyliden)-cycloheptanol |
| Beispiel 376 | 2-Dimethylaminomethyl-7-(3-methoxy-benzyliden)-1-(2-methoxy-phenyl)-cycloheptanol |
| Beispiel 377 | 2-Dimethylaminomethyl-7-(3-methoxy-benzyliden)-1-(2-methyl-benzyl)-cycloheptanol |
| Beispiel 378 | 2-Dimethylaminomethyl-7-(3-methoxy-benzyliden)-1-(3-trifluormethyl-phenyl)-cycloheptanol |
| Beispiel 379 | 2-Dimethylaminomethyl-7-(3-methoxy-benzyliden)-1-(3-methyl-benzyl)-cycloheptanol |
| Beispiel 380 | 2-Dimethylaminomethyl-1-(2,5-dimethyl-benzyl)-7-(3-methoxy-benzyliden)-cycloheptanol |
| Beispiel 381 | 1-(3-Chlor-benzyl)-2-dimethylaminomethyl-7-(3-methoxy-benzyliden)-cycloheptanol |
| Beispiel 382 | 1-Benzyl-2-dimethylaminomethyl-7-(4-methoxy-benzyliden)-cycloheptanol |
| Beispiel 383 | 2-Dimethylaminomethyl-1-(4-fluor-3-methyl-phenyl)-7-(4-methoxy-benzyliden)-cycloheptanol |
| Beispiel 384 | 2-Dimethylaminomethyl-7-(4-methoxy-benzyliden)-1-o-tolyl-cycloheptanol |
| Beispiel 385 | 2-Dimethylaminomethyl-7-(4-methoxy-benzyliden)-1-vinyl-cycloheptanol |
| Beispiel 386 | 1-(4-tert-Butyl-phenyl)-2-dimethylaminomethyl-7-(4-methoxy-benzyliden)-cycloheptanol |
| Beispiel 387 | 1-Cyclopentyl-2-dimethylaminomethyl-7-(4-methoxy-benzyliden)-cycloheptanol |
| Beispiel 388 | 2-Dimethylaminomethyl-7-(4-methoxy-benzyliden)-1-m-tolyl-cycloheptanol |
| Beispiel 389 | 1-Cyclohexyl-2-dimethylaminomethyl-7-(4-methoxy-benzyliden)-cycloheptanol |
| Beispiel 390 | 2-Dimethylaminomethyl-1-(4-fluor-phenyl)-7-(4-methoxy-benzyliden)-cycloheptanol |
| Beispiel 391 | 2-Dimethylaminomethyl-7-(4-methoxy-benzyliden)-1-phenethyl-cycloheptanol |
| Beispiel 392 | 2-Dimethylaminomethyl-7-(4-methoxy-benzyliden)-1-phenylethynyl-cycloheptanol |
| Beispiel 393 | 2-Dimethylaminomethyl-7-(4-methoxy-benzyliden)-1-thiophen-2-yl-cycloheptanol |
| Beispiel 394 | 2-Dimethylaminomethyl-7-(4-methoxy-benzyliden)-1-(3-methoxy-phenyl)-cycloheptanol |
| Beispiel 395 | 2-Dimethylaminomethyl-7-(4-methoxy-benzyliden)-1-(3-phenyl-propyl)-cycloheptanol |
| Beispiel 396 | 2-Dimethylaminomethyl-7-(4-methoxy-benzyliden)-1-p-tolyl-cycloheptanol |
| Beispiel 397 | 2-Dimethylaminomethyl-7-(4-methoxy-benzyliden)-1-(4-methoxy-phenyl)-cycloheptanol |
| Beispiel 398 | 1-Cyclohexylmethyl-2-dimethylaminomethyl-7-(4-methoxy-benzyliden)-cycloheptanol |
| Beispiel 399 | 1-(3-Chlor-phenyl)-2-dimethylaminomethyl-7-(4-methoxy-benzyliden)-cycloheptanol |
| Beispiel 400 | 1-(2-Chlor-benzyl)-2-dimethylaminomethyl-7-(4-methoxy-benzyliden)-cycloheptanol |
| Beispiel 401 | 2-Dimethylaminomethyl-7-(4-methoxy-benzyliden)-1-(2-methoxy-phenyl)-cycloheptanol |
| Beispiel 402 | 2-Dimethylaminomethyl-7-(4-methoxy-benzyliden)-1-(2-methyl-benzyl)-cycloheptanol |
| Beispiel 403 | 2-Dimethylaminomethyl-7-(4-methoxy-benzyliden)-1-(3-trifluormethyl-phenyl)-cycloheptanol |
| Beispiel 404 | 2-Dimethylaminomethyl-7-(4-methoxy-benzyliden)-1-(3-methyl-benzyl)-cycloheptanol |
| Beispiel 405 | 1-(3-Chlor-benzyl)-2-dimethylaminomethyl-7-(4-methoxy-benzyliden)-cycloheptanol |
| Beispiel 406 | 1-(3,5-Dichlor-phenyl)-2-dimethylaminomethyl-7-(4-methoxy-benzyliden)-cycloheptanol |
| Beispiel 407 | 2-Benzyliden-7-dimethylaminomethyl-1-(3-hydroxy-phenyl)-cycloheptanol; Hydrochlorid |
| Beispiel 408 | 2-Dimethylaminomethyl-1-(3-hydroxy-phenyl)-7-(3-methoxy-benzyliden)-cycloheptanol; Hydrochlorid |
| Beispiel 409 | 2-Benzyliden-7-dimethylaminomethyl-1-(3-methoxy-phenyl)-cycloheptanol; Hydrochlorid |
| Beispiel 410 | 3-[1-(2-Dimethylamino-1-methyl-ethyl)-1-hydroxy-2-methyl-3-phenyl-allyl]-phenol; Hydrochlorid |
| Beispiel 411 | 3-(4-Chlor-benzyl)-5-dimethylamino-2,4-dimethyl-1-phenyl-pent-1-en-3-ol; Hydrochlorid |
| Beispiel 412 | 5-Dimethylamino-3-(3-methoxy-phenyl)-2,4-dimethyl-1-phenyl-pent-1-en-3-ol; Hydrochlorid |
| Beispiel 413 | 3-(2-Benzyliden-6-dimethylaminomethyl-1-hydroxy-cyclohexyl)-phenol; Hydrochlorid |
| 414. Beispiel | 1-Benzyl-2-benzyliden-7-(4-methyl-piperazin-1-ylmethyl)-cycloheptanol; bishydrochloride |
| 415. Beispiel | 2-Benzyliden-1-(2-chlor-benzyl)-7-(4-methyl-piperazin-1-ylmethyl)-cycloheptanol; bishydrochloride |
| 416. Beispiel | 2-Benzyliden-1-(3-chlor-benzyl)-7-(4-methyl-piperazin-1-ylmethyl)-cycloheptanol; bishydrochloride |
| 417. Beispiel | 2-Benzyliden-1-(4-chlor-benzyl)-7-(4-methyl-piperazin-1-ylmethyl)-cycloheptanol; bishydrochloride |
| 418. Beispiel | 2-Benzyliden-1-(2-fluor-benzyl)-7-(4-methyl-piperazin-1-ylmethyl)-cycloheptanol; bishydrochloride |
| 419. Beispiel | 2-Benzyliden-1-(3-fluor-benzyl)-7-(4-methyl-piperazin-1-ylmethyl)-cycloheptanol; bishydrochloride |
| 420. Beispiel | 2-Benzyliden-1-(4-fluor-benzyl)-7-(4-methyl-piperazin-1-ylmethyl)-cycloheptanol; bishydrochloride |
| 421. Beispiel | 2-Benzyliden-1-(2-methyl-benzyl)-7-(4-methyl-piperazin-1-ylmethyl)-cycloheptanol; bishydrochloride |
| 422. Beispiel | 2-Benzyliden-1-(3-methyl-benzyl)-7-(4-methyl-piperazin-1-ylmethyl)-cycloheptanol; bishydrochloride |
| 423. Beispiel | 2-Benzyliden-1-(4-methyl-benzyl)-7-(4-methyl-piperazin-1-ylmethyl)-cycloheptanol; bishydrochloride |
| 424. Beispiel | 2-Benzyliden-1-(2-methoxy-benzyl)-7-(4-methyl-piperazin-1-ylmethyl)-cycloheptanol; bishydrochloride |
| 425. Beispiel | 2-Benzyliden-1-(3-methoxy-benzyl)-7-(4-methyl-piperazin-1-ylmethyl)-cycloheptanol; bishydrochloride |
| 426. Beispiel | 2-Benzyliden-1-(4-methoxy-benzyl)-7-(4-methyl-piperazin-1-ylmethyl)-cycloheptanol; bishydrochloride |
| 427. Beispiel | 2-Benzyliden-1-(2,6-dichlor-benzyl)-7-(4-methyl-piperazin-1-ylmethyl)-cycloheptanol; bishydrochloride |
| 428. Beispiel | 2-Benzyliden-1-(2-chlor-6-fluor-benzyl)-7-(4-methyl-piperazin-1-ylmethyl)-cycloheptanol; bishydrochloride |
| 429. Beispiel | 2-Benzyliden-1-(2,6-difluor-benzyl)-7-(4-methyl-piperazin-1-ylmethyl)-cycloheptanol; bishydrochloride |
| 430. Beispiel | 1-Benzyl-2-(4-chlor-benzyliden)-7-(4-methyl-piperazin-1-ylmethyl)-cycloheptanol; bishydrochloride |
| 431. Beispiel | 1-(2-Chlor-benzyl)-2-(4-chlor-benzyliden)-7-(4-methyl-piperazin-1-ylmethyl)-cycloheptanol; bishydrochloride |
| 432. Beispiel | 1-(3-Chlor-benzyl)-2-(4-chlor-benzyliden)-7-(4-methyl-piperazin-1-ylmethyl)-cycloheptanol; bishydrochloride |
| 433. Beispiel | 1-(4-Chlor-benzyl)-2-(4-chlor-benzyliden)-7-(4-methyl-piperazin-1-ylmethyl)-cycloheptanol; bishydrochloride |
| 434. Beispiel | 2-(4-Chlor-benzyliden)-1-(2-fluor-benzyl)-7-(4-methyl-piperazin-1-ylmethyl)-cycloheptanol; bishydrochloride |
| 435. Beispiel | 2-(4-Chlor-benzyliden)-1-(3-fluor-benzyl)-7-(4-methyl-piperazin-1-ylmethyl)-cycloheptanol; bishydrochloride |
| 436. Beispiel | 2-(4-Chlor-benzyliden)-1-(4-fluor-benzyl)-7-(4-methyl-piperazin-1-ylmethyl)-cycloheptanol; bishydrochloride |
| 437. Beispiel | 2-(4-Chlor-benzyliden)-1-(2-methyl-benzyl)-7-(4-methyl-piperazin-1-ylmethyl)-cycloheptanol; bishydrochloride |
| 438. Beispiel | 2-(4-Chlor-benzyliden)-1-(3-methyl-benzyl)-7-(4-methyl-piperazin-1-ylmethyl)-cycloheptanol; bishydrochloride |
| 439. Beispiel | 2-(4-Chlor-benzyliden)-1-(4-methyl-benzyl)-7-(4-methyl-piperazin-1-ylmethyl)-cycloheptanol; bishydrochloride |
| 440. Beispiel | 2-(4-Chlor-benzyliden)-1-(2-methoxy-benzyl)-7-(4-methyl-piperazin-1-ylmethyl)-cycloheptanol; bishydrochloride |
| 441. Beispiel | 2-(4-Chlor-benzyliden)-1-(3-methoxy-benzyl)-7-(4-methyl-piperazin-1-ylmethyl)-cycloheptanol; bishydrochloride |
| 442. Beispiel | 2-(4-Chlor-benzyliden)-1-(4-methoxy-benzyl)-7-(4-methyl-piperazin-1-ylmethyl)-cycloheptanol; bishydrochloride |
| 443. Beispiel | 2-(4-Chlor-benzyliden)-1-(2,6-dichlor-benzyl)-7-(4-methyl-piperazin-1-ylmethyl)-cycloheptanol; bishydrochloride |
| 444. Beispiel | 2-(4-Chlor-benzyliden)-1-(2,6-difluor-benzyl)-7-(4-methyl-piperazin-1-ylmethyl)-cycloheptanol; bishydrochloride |
| 445. Beispiel | 2-(4-Chlor-benzyliden)-1-(2-chlor-6-fluor-benzyl)-7-(4-methyl-piperazin-1-ylmethyl)-cycloheptanol; bishydrochloride |
| 446. Beispiel | 1-Benzyl-2-(4-fluor-benzyliden)-7-(4-methyl-piperazin-1-ylmethyl)-cycloheptanol; bishydrochloride |
| 447. Beispiel | 1-(2-Chlor-benzyl)-2-(4-fluor-benzyliden)-7-(4-methyl-piperazin-1-ylmethyl)-cycloheptanol; bishydrochloride |
| 448. Beispiel | 1-(3-Chlor-benzyl)-2-(4-fluor-benzyliden)-7-(4-methyl-piperazin-1-ylmethyl)-cycloheptanol; bishydrochloride |
| 449. Beispiel | 1-(4-Chlor-benzyl)-2-(4-fluor-benzyliden)-7-(4-methyl-piperazin-1-ylmethyl)-cycloheptanol; bishydrochloride |
| 450. Beispiel | 1-(2-Fluor-benzyl)-2-(4-fluor-benzyliden)-7-(4-methyl-piperazin-1-ylmethyl)-cycloheptanol; bishydrochloride |
| 451. Beispiel | 1-(3-Fluor-benzyl)-2-(4-fluor-benzyliden)-7-(4-methyl-piperazin-1-ylmethyl)-cycloheptanol; bishydrochloride |
| 452. Beispiel | 1-(4-Fluor-benzyl)-2-(4-fluor-benzyliden)-7-(4-methyl-piperazin-1-ylmethyl)-cycloheptanol; bishydrochloride |
| 453. Beispiel | 2-(4-Fluor-benzyliden)-1-(2-methyl-benzyl)-7-(4-methyl-piperazin-1-ylmethyl)-cycloheptanol; bishydrochloride |
| 454. Beispiel | 2-(4-Fluor-benzyliden)-1-(3-methyl-benzyl)-7-(4-methyl-piperazin-1-ylmethyl)-cycloheptanol; bishydrochloride |
| 455. Beispiel | 2-(4-Fluor-benzyliden)-1-(4-methyl-benzyl)-7-(4-methyl-piperazin-1-ylmethyl)-cycloheptanol; bishydrochloride |
| 456. Beispiel | 2-(4-Fluor-benzyliden)-1-(2-methoxy-benzyl)-7-(4-methyl-piperazin-1-ylmethyl)-cycloheptanol; bishydrochloride |
| 457. Beispiel | 2-(4-Fluor-benzyliden)-1-(3-methoxy-benzyl)-7-(4-methyl-piperazin-1-ylmethyl)-cycloheptanol; bishydrochloride |
| 458. Beispiel | 2-(4-Fluor-benzyliden)-1-(4-methoxy-benzyl)-7-(4-methyl-piperazin-1-ylmethyl)-cycloheptanol; bishydrochloride |
| 459. Beispiel | 1-(2,6-Dichlor-benzyl)-2-(4-fluor-benzyliden)-7-(4-methyl-piperazin-1-ylmethyl)-cycloheptanol; bishydrochloride |
| 460. Beispiel | 1-(2,6-Difluor-benzyl)-2-(4-fluor-benzyliden)-7-(4-methyl-piperazin-1-ylmethyl)-cycloheptanol; bishydrochloride |
| 461. Beispiel | 1-(2-Chlor-6-fluorbenzyl)-2-(4-fluor-benzyliden)-7-(4-methyl-piperazin-1-ylmethyl)-cycloheptanol; bishydrochloride |
| 462. Beispiel | 1-Benzyl-2-furan-2-ylmethylen-7-(4-methyl-piperazin-1-ylmethyl)-cycloheptanol; bishydrochloride |
| 463. Beispiel | 1-(2-Chlor-benzyl)-2-furan-2-ylmethylen-7-(4-methyl-piperazin-1-ylmethyl)-cycloheptanol; bishydrochloride |
| 464. Beispiel | 1-(3-Chlor-benzyl)-2-furan-2-ylmethylen-7-(4-methyl-piperazin-1-ylmethyl)-cycloheptanol; bishydrochloride |
| 465. Beispiel | 1-(4-Chlor-benzyl)-2-furan-2-ylmethylen-7-(4-methyl-piperazin-1-ylmethyl)-cycloheptanol; bishydrochloride |
| 466. Beispiel | 1-(2-Fluor-benzyl)-2-furan-2-ylmethylen-7-(4-methyl-piperazin-1-ylmethyl)-cycloheptanol; bishydrochloride |
| 467. Beispiel | 1-(3-Fluor-benzyl)-2-furan-2-ylmethylen-7-(4-methyl-piperazin-1-ylmethyl)-cycloheptanol; bishydrochloride |
| 468. Beispiel | 1-(4-Fluor-benzyl)-2-furan-2-ylmethylen-7-(4-methyl-piperazin-1-ylmethyl)-cycloheptanol; bishydrochloride |
| 469. Beispiel | 2-Furan-2-ylmethylen-1-(2-methyl-benzyl)-7-(4-methyl-piperazin-1-ylmethyl)-cycloheptanol; bishydrochloride |
| 470. Beispiel | 2-Furan-2-ylmethylen-1-(3-methyl-benzyl) -7- (4-methyl-piperazin-1-ylmethyl)-cycloheptanol; bishydrochloride |
| 471. Beispiel | 2-Furan-2-ylmethylen-1-(4-methyl-benzyl)-7-(4-methyl-piperazin-1-ylmethyl)-cycloheptanol; bishydrochloride |
| 472. Beispiel | 2-Furan-2-ylmethylen-1-(2-methoxy-benzyl)-7-(4-methyl-piperazin-1-ylmethyl)-cycloheptanol; bishydrochloride |
| 473. Beispiel | 2-Furan-2-ylmethylen-1-(3-methoxy-benzyl)-7-(4-methyl-piperazin-1-ylmethyl)-cycloheptanol; bishydrochloride |
| 474. Beispiel | 2-Furan-2-ylmethylen-1-(4-methoxy-benzyl)-7-(4-methyl-piperazin-1-ylmethyl)-cycloheptanol; bishydrochloride |
| 475. Beispiel | 1-(2,6-Dichlor-benzyl)-2-furan-2-ylmethylen-7-(4-methyl-piperazin-1-ylmethyl)-cycloheptanol; bishydrochloride |
| 476. Beispiel | 1-(2,6-Difluor-benzyl)-2-furan-2-ylmethylen-7-(4-methyl-piperazin-1-ylmethyl)-cycloheptanol; bishydrochloride |
| 477. Beispiel | 1-(2-Chlor-6-fluor-benzyl)-2-furan-2-ylmethylen-7-(4-methyl-piperazin-1-ylmethyl)-cycloheptanol; bishydrochloride |
| 478. Beispiel | 1-Benzyl-3-furan-2-ylmethylen-7-(4-methyl-piperazin-1-ylmethyl)-cycloheptanol; bishydrochloride |
| 479. Beispiel | 1-(2-Chlor-benzyl)-3-furan-2-ylmethylen-7-(4-methyl-piperazin-1-ylmethyl)-cycloheptanol; bishydrochloride |
| 480. Beispiel | 1-(3-Chlor-benzyl)-3-furan-2-ylmethylen-7-(4-methyl-piperazin-1-ylmethyl)-cycloheptanol; bishydrochloride |
| 481. Beispiel | 1-(4-Chlor-benzyl)-3-furan-2-ylmethylen-7-(4-methyl-piperazin-1-ylmethyl)-cycloheptanol; bishydrochloride |
| 482. Beispiel | 1-(2-Fluor-benzyl)-3-furan-2-ylmethylen-7-(4-methyl-piperazin-1-ylmethyl)-cycloheptanol; bishydrochloride |
| 483. Beispiel | 1-(3-Fluor-benzyl)-3-furan-2-ylmethylen-7-(4-methyl-piperazin-1-ylmethyl)-cycloheptanol; bishydrochloride |
| 484. Beispiel | 1-(4-Fluor-benzyl)-3-furan-2-ylmethylen-7-(4-methyl-piperazin-1-ylmethyl)-cycloheptanol; bishydrochloride |
| 485. Beispiel | 3-Furan-2-ylmethylen-1-(2-methyl-benzyl)-7-(4-methyl-piperazin-1-ylmethyl)-cycloheptanol; bishydrochloride |
| 486. Beispiel | 3-Furan-2-ylmethylen-1-(3-methyl-benzyl)-7-(4-methyl-piperazin-1-ylmethyl)-cycloheptanol; bishydrochloride |
| 487. Beispiel | 3-Furan-2-ylmethylen-1-(4-methyl-benzyl)-7-(4-methyl-piperazin-1-ylmethyl)-cycloheptanol; bishydrochloride |
| 488. Beispiel | 3-Furan-2-ylmethylen-1-(2-methoxy-benzyl)-7-(4-methyl-piperazin-1-ylmethyl)-cycloheptanol; bishydrochloride |
| 489. Beispiel | 3-Furan-2-ylmethylen-1-(3-methoxy-benzyl)-7-(4-methyl-piperazin-1-ylmethyl)-cycloheptanol; bishydrochloride |
| 490. Beispiel | 3-Furan-2-ylmethylen-1-(4-methoxy-benzyl)-7-(4-methyl-piperazin-1-ylmethyl)-cycloheptanol; bishydrochloride |
| 491. Beispiel | 1-(2,6-Dichlor-benzyl)-3-furan-2-ylmethylen-7-(4-methyl-piperazin-1-ylmethyl)-cycloheptanol; bishydrochloride |
| 492. Beispiel | 1-(2,6-Difluor-benzyl)-3-furan-2-ylmethylen-7-(4-methyl-piperazin-1-ylmethyl)-cycloheptanol; bishydrochloride |
| 493. Beispiel | 1-(2-Chlor-6-fluor-benzyl)-3-furan-2-ylmethylen-7-(4-methyl-piperazin-1-ylmethyl)-cycloheptanol; bishydrochloride |

### Pharmakologische Untersuchungen

Beim Bindungsassay wird Gabapentin benutzt, um die Bindung und Affinitäten der ausgewählten Verbindungen zu überprüfen. Die Affinität der erfindungsgemäßen Verbindungen wird über die Verdrängung von Gabapentin von seiner Bindungsstelle gemessen. Wenn die ausgewählten Verbindungen Gabapentin von seiner Bindungsstelle verdrängen können, so kann man erwarten, daß sie dem Gabapentin vergleichbare pharmakologische Eigenschaften entfalten z.B. als Agenz gegen Schmerz oder Epilepsie. Die erfindungsgemäßen Verbindungen zeigen eine gute HemmungNerdrängung von Gabapentin in diesem Assay. Die untersuchten Verbindungen weisen daher in diesem biochemischen Assay eine Affinität zur bislang unbekannten Gabapentin-Bindungsstelle auf.

| **Beispiel** | **%Hemmung Gabapentin, 10µMol** |
|---|---|
| 120 | 37 |
| 152 | 30 |
| 156 | 46 |
| 184 | 47 |
| 223 | 40 |
| 369 | 43 |
| 353 | 44 |

### Analgesieprüfung im Writhing-Test an der Maus

Die Untersuchung auf analgetische Wirksamkeit wurde im Phenylchinon-induzierten Writhing an der Maus (modifiziert nach I.C. Hendershot und J. Forsaith J. Pharmacol. Exp. Ther. **1959**, 125, 237-240) durchgeführt. Dazu wurden männliche NMRI-Mäuse im Gewicht von 25 bis 30 g verwendet. Gruppen von 10 Tieren pro Substanzdosis erhielten 10 Minuten nach intravenöser Gabe der Prüfsubstanzen 0,3 ml/Maus einer 0,02%igen wäßrigen Lösung von Phenylchinon (Phenylbenzochinon, Fa. Sigma, Deisenhofen; Herstellung der Lösung unter Zusatz von 5 % Äthanol und Aufbewahrung im Wasserbad bei 45°C) intraperitoneal appliziert. Die Tiere wurden einzeln in Beobachtungskäfige gesetzt. Mittels eines Drucktastenzähler wurde die Anzahl der schmerzinduzierten Streckbewegungen (sogenannte Writhingreaktionen = Durchdrücken des Körpers mit Abstrecken der Hinterextremitäten) 5 bis 20 Minuten nach der Phenylchinon-Gabe ausgezählt. Als Kontrolle wurden Tiere mitgeführt, die nur physiologische Kochsalzlösung erhalten. Für einige Beispiele wurde die Zahl der reagierenden Tiere bestimmt:

| **Beispiel** | **Reagierende Tiere/Kontrolltiere (Writhing iv)** |
|---|---|
| 407 | 6/10 (2,15 mg/kg) |
| 408 | 9/10 (10 mg/kg) |
| 409 | 5/10 (10 mg/kg) |
| 410 | 5/10 (10 mg/kg) |
| 411 | 3/10 (10 mg/kg) |
| 412 | 9/10 (10 mg/kg) |
| 413 | 4/10 (10 mg/kg) |
| | |
| | |
| | |

## Patentansprüche

1. Substituierte 5-Amino-1-penten-3-ol-Derivate der allgemeinen Formel I, 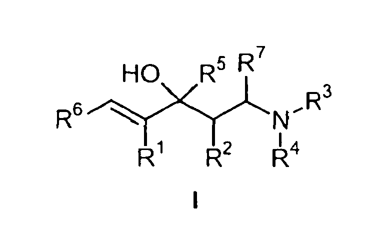 , worin
R¹ und R² jeweils unabhängig voneinander ausgewählt sind aus C₁₋₆-Alkyl, verzweigt oder unverzweigt, gesättigt oder ungesättigt, unsubstituiert oder ein- oder mehrfach substituiert oder
R¹ und R² zusammen einen (CH₂)₂₋₉-Ring bilden, der gegebenfalls mit C₁₋₈-Alkyl, verzweigt oder unverzweigt, gesättigt oder ungesättigt, unsubstituiert oder ein- oder mehrfach substituiert; oder Aryl, unsubstituiert oder ein- oder mehrfach substituiert; substituiert sein kann,
R³ und R⁴ jeweils unabhängig voneinander ausgewählt sind aus C₁₋₆-Alkyl, verzweigt oder unverzweigt, gesättigt oder ungesättigt, unsubstituiert oder ein- oder mehrfach substituiert; C₃₋₆-Cycloalkyl, gesättigt oder ungesättigt, unsubstituiert oder ein- oder mehrfach substituiert; Phenyl, Benzyl oder Phenethyl, unsubstituiert oder ein- oder mehrfach substituiert
oder
die Reste R³ und R⁴ zusammen einen Ring bilden und CH₂CH₂OCH₂CH₂, CH₂CH₂NR²²CH₂CH₂ oder (CH₂)₃₋₆ bedeuten,
mit R²² ausgewählt aus H; C₁₋₁₀-Alkyl oder C₃₋₁₀-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über C₁₋₃-Alkyl, gesättigt oder ungesättigt, gebundenem Aryl, C₃₋₁₀-Cycloalkyl oder Heteroaryl, jeweils einfach oder mehrfach substituiert oder unsubstituiert;
R5 ausgewählt ist aus C1-10-Alkyl, gesättigt oder ungesättigt, verzweigt oder unverzweigt, einfach oder mehrfach substituiert oder unsubstituiert; C3-9-Cycloalkyl, gesättigt oder ungesättigt; Aryl, Heteroaryl, über gesättigtes oder ungesättigtes C1-3-Alkyl gebundenem Aryl, über gesättigtes oder ungesättigtes C1-3-Alkyl gebundenem C3-10-Cycloalkyl oder über gesättigtes oder ungesättigtes C1-3-Alkyl gebundenem Heteroaryl, wobei alle Aryl, Heteroaryl und Cycloalkyl-Reste jeweils unabhängig voneinander unsubstituiert oder einfach oder mehrfach substituiert sein können mit Resten unabhängig voneinander ausgewählt aus
F, Cl, Br, I, OR¹⁸, SR¹⁸, SO₂R¹⁸, SO₂OR¹⁸, CN, COOR¹⁸, NR¹⁹R²⁰; C₁₋₁₀-Alkyl, gesättigt oder ungesättigt, verzweigt oder unverzweigt, einfach oder mehrfach substituiert oder unsubstituiert; C₃₋₉-Cycloalkyl, gesättigt oder ungesättigt, unsubstituiert oder einfach oder mehrfach substituiert; Aryl-, oder Heteroaryl, jeweils unsubstituiert oder einfach oder mehrfach substituiert; oder über gesättigtes oder ungesättigtes C₁₋₃-Alkyl gebundenem Aryl, C₃₋₉-Cycloalkyl oder Heteroaryl, jeweils unsubstituiert oder einfach oder mehrfach substituiert;
mit R¹⁸ ausgewählt aus H; C₁₋₁₀-Alkyl, gesättigt oder ungesättigt, verzweigt oder unverzweigt, unsubstituiert oder einfach oder mehrfach substituiert; C₃₋₉-Cycloalkyl, gesättigt oder ungesättigt, unsubstituiert oder einfach oder mehrfach substituiert; Aryl-, oder Heteroaryl, unsubstituiert oder einfach oder mehrfach substituiert; oder über gesättigtes oder ungesättigtes C₁₋₃-Alkyl gebundenem Aryl, C₃₋₉-Cycloalkyl oder Heteroaryl, jeweils unsubstituiert oder einfach oder mehrfach substituiert;
R¹⁹ und R²⁰ unabhängig voneinander ausgewählt aus H; C₁₋₁₀-Alkyl, gesättigt oder ungesättigt, verzweigt oder unverzweigt, unsubstituiert oder einfach oder mehrfach substituiert; C₃₋₉-Cycloalkyl, gesättigt oder ungesättigt, unsubstituiert oder einfach oder mehrfach substituiert; Aryl-, oder Heteroaryl, jeweils unsubstituiert oder einfach oder mehrfach substituiert; oder über gesättigtes oder ungesättigtes C₁₋₃-Alkyl gebundenem Aryl, C₃₋₉-Cycloalkyl oder Heteroaryl, jeweils unsubstituiert oder einfach oder mehrfach substituiert;
oder R¹⁹ und R²⁰ zusammen CH₂CH₂OCH₂CH₂, CH₂CH₂NR²¹ CH₂CH₂ oder (CH₂)₃₋₆ bilden,
mit R²¹ ausgewählt aus H; Phenyl, substituiert oder unsubstituiert; C₁₋₁₀-Alkyl gesättigt oder ungesättigt, verzweigt oder unverzweigt, unsubstituiert oder einfach oder mehrfach substituiert;
R⁶ ausgewählt ist aus C1-10-Alkyl, gesättigt oder ungesättigt, verzweigt oder unverzweigt, einfach oder mehrfach substituiert oder unsubstituiert; C₅₋₇-Cycloalkyl, Aryl oder Heteroaryl, jeweils unsubstituiert oder ein- oder mehrfach substituiert;
R⁷ ausgewählt ist aus H; Aryl oder Heteroaryl; jeweils unsubstituiert oder ein- oder mehrfach substituiert;
gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form ihrer Säuren oder ihrer Basen oder in Form ihrer Salze, insbesondere der physiologisch verträglichen Salze, oder in Form ihrer Solvate, insbesondere der Hydrate.

2. Substituierte 5-Amino-1-penten-3-ol-Derivate gemäß Anspruch 1, **dadurch gekennzeichnet, daß**
R⁷ ausgewählt ist aus H oder Heteroaryl oder ein Rest gemäß Formel II ist, 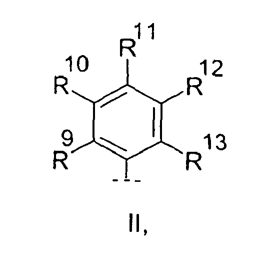 mit R⁹ bis R¹³, jeweils unabhängig voneinander ausgewählt aus H, F, Cl, Br, I, CF₃, CHF₂, CH₂F, OH, OR¹⁴, OCF₃, OCHF₂, OCH₂F, SR¹⁴, SO₂CH₃, SO₂CF₃; C₁₋₆-Alkyl, verzweigt oder unverzweigt, gesättigt oder ungesättigt, unsubstituiert oder ein- oder mehrfach substituiert; Phenyl, unsubstituiert oder ein- oder mehrfach substituiert; CN, COOR¹⁴, NO₂ oder
R⁹ und R¹⁰ oder R¹⁰ und R¹¹ zusammen einen OCH₂O- oder OCH₂CH₂O-Ring bilden, und
R¹⁴ ausgewählt ist aus C₁₋₆-Alkyl, verzweigt oder unverzweigt, gesättigt oder ungesättigt, unsubstituiert oder ein- oder mehrfach substituiert; Phenyl, Benzyl, Phenethyl oder Thiophen, jeweils unsubstituiert oder ein- oder mehrfach substituiert,
vorzugsweise
R⁷ Wasserstoff entspricht.

3. Substituierte 5-Amino-1-penten-3-ol-Derivate gemäß einem der Ansprüche 1 oder 2, **dadurch gekennzeichnet, daß**
R¹ und R² zusammen einen (CH₂)₂₋₅-Ring bilden, der gegebenfalls mit C₁₋₆-Alkyl, verzweigt oder unverzweigt, gesättigt oder ungesättigt, unsubstituiert oder ein- oder mehrfach substituiert; oder Phenyl, unsubstituiert oder ein- oder mehrfach substituiert; substituiert sein kann, der aber vorzugsweise unsubstituiert ist; vorzugsweise R¹ und R² zusammen einen unsubstituierten (CH₂)₂₋₄Ring bilden
oder
R¹ und R² unabhängig voneinander ausgewählt sind aus aus C₁₋₃-Alkyl, unverzweigt, gesättigt und unsubstituiert, vorzugsweise CH₃, insbesondere R¹ und R² beide CH₃ entsprechen.

4. Substituierte 5-Amino-1-penten-3-ol-Derivate gemäß einem der Ansprüche 1 bis 3, **dadurch gekennzeichnet, daß**
R³ und R⁴ jeweils unabhängig voneinander ausgewählt sind aus C₁₋₆-Alkyl, verzweigt oder unverzweigt, gesättigt oder ungesättigt, unsubstituiert oder ein- oder mehrfach substituiert; vorzugsweise beide CH₃ sind,
oder
R³ und R⁴ zusammen einen Ring bilden und CH₂CH₂NR²²CH₂CH₂ oder (CH₂)₃₋₆ bedeuten, insbesondere zusammen (CH₂)₄₋₅ oder CH₂CH₂NR²²CH₂CH₂ bedeuten, mit R²² ausgewählt aus H oder C₁₋₆-Alkyl, gesättigt, verzweigt oder unverzweigt und unsubstituiert; insbesondere H oder CH₃.

5. Substituierte 5-Amino-1-penten-3-ol-Derivate gemäß einem der Ansprüche 1 bis 4, **dadurch gekennzeichnet, daß**
R⁵ ausgewählt ist aus C₁₋₆-Alkyl, gesättigt oder ungesättigt, verzweigt oder unverzweigt, einfach oder mehrfach substituiert oder unsubstituiert, C₅₋₆-Cycloalkyl, Phenyl, Thiophenyl, Furyl, Benzofuranyl, Benzothiophenyl, Pyrrolyl, Pyridinyl, Pyrimidinyl, Chinolinyl, Isochinolinyl oder Chinazolinyl, vorzugsweise Phenyl, Furyl, Thiophenyl oder C₅₋₆-Cycloalkyl; über gesättigtes oder ungesättigtes C₁₋₃-Alkyl gebundenem Phenyl, C₅₋₆-Cycloalkyl, Thiophenyl, Furyl, Benzofuranyl, Benzothiophenyl, Pyrrolyl, Pyridinyl, Pyrimidinyl, Chinolinyl, Isochinolinyl, Chinazolinyl, vorzugsweise Phenyl, Furyl, Thiophenyl oder C₅₋₆-Cycloalkyl;
wobei alle Aryl, Heteroaryl und Cycloalkyl-Reste jeweils unabhängig voneinander unsubstituiert oder einfach oder mehrfach substituiert sein können, vorzugsweise unsubstituiert oder ein- oder mehrfach mit Substituenten unabhängig voneinander ausgewählt aus F, Cl, Br, I, OH, O-C₁₋₄-Alkyl, C₁₋₆-Alkyl, CF₃, CHF₂, CH₂F, NH₂ und/oder SH substituiert sind,
vorzugsweise
R⁵ ausgewählt ist aus C₁₋₃-Alkyl, gesättigt oder ungesättigt, unsubstituiert und/oder unverzweigt; Naphthyl, Furyl, Cyclohexyl, Cyclopentyl, Phenyl oder Thiophenyl, unsubstituiert oder einfach oder mehrfach, vorzugsweise mit F, Cl, Br, I, CF₃, CHF₂, CH₂F, OH, O-C₁₋₄-Alkyl, C₁₋₆-Alkyl, NH₂ und/oder SH, substituiert; oder über gesättigtes oder ungesättigtes C₁₋₃-Alkyl gebundenem Phenyl, unsubstituiert oder einfach oder mehrfach, vorzugsweise mit F, Cl, Br, I, CF₃, CHF₂, CH₂F, OH, O-C₁₋₄-Alkyl, C₁₋₆-Alkyl, NH₂ und/oder SH, substituiert,
insbesondere
R⁵ ausgewählt ist aus -CH=CH₂, Cyclohexyl, Cyclopentyl; Phenyl, Phenethyl (über CH₂-CH₂ gebundenem Phenyl), Benzyl (über CH₂ gebundenem Phenyl) oder Thiophenyl unsubstituiert oder einfach oder mehrfach, vorzugsweise mit F, Cl, Br, I, OH, OCH₃, OC₂H₅, OC₃H₇, SH, CF₃, CHF₂, CH₂F, CH₃, C₂H₅, C₃H₇ und/oder C₄H₉, bzw. t-Butyl, substituiert.

6. Substituierte 5-Amino-1-penten-3-ol-Derivate gemäß einem der Ansprüche 1 bis 5, **dadurch gekennzeichnet, daß**
R⁶ ausgewählt ist aus Phenyl oder Furyl, jeweils unsubstituiert oder ein- oder mehrfach substituiert, vorzugsweise unsubstituiert oder ein- oder mehrfach mit Substituenten unabhängig voneinander ausgewählt aus Fluor, Chlor, CH₃, OCH₃, CF₃ oder tert.-Butyl substituiert.

7. Substituierte 5-Amino-1-penten-3-ol-Derivate gemäß einem der Ansprüche 1 bis 6, **dadurch gekennzeichnet, daß**, wenn R5 ausgewählt ist aus über gesättigtes oder ungesättigtes C₁₋₃-Alkyl gebundenem Aryl, C₃₋₉-Cycloalkyl oder Heteroaryl, das C₁₋₃-Alkyl über das Aryl, Heteroaryl oder Cycloalkyl gebunden ist, ausgewählt ist aus:
-CH₂-, -C₂H₄-, C₃H₆-, -C≡C-, -CH=CH-, -CH=CH-CH₂-, -CH₂-CH=CH-,-C≡C-CH₂- oder -CH2-C≡C-, vorzugsweise -CH₂-, -C₂H₄- oder -C≡C-.

8. Substituierte 5-Amino-1-penten-3-ol-Derivate gemäß einem der Ansprüche 1 bis 7, **dadurch gekennzeichnet, daß** sie ausgewählt sind aus der folgenden Gruppe:
• 2-Benzyliden-1-(3-chlor-benzyl)-6-dimethylaminomethyl-cyclohexanol
• 2-Benzyliden-6-dimethylaminomethyl-1-(2,5-dimethyl-benzyl)-cyclohexanol
• 2-Benzyliden-1-(2-chlor-6-fluor-benzyl)-6-dimethylaminomethyl-cyclohexanol
• 2-Benzyliden-1-(4-chlor-benzyl)-6-dimethylaminomethyl-cyclohexanol
• 2-Benzyliden-6-dimethylaminomethyl-1-(3-methyl-benzyl)-cyclohexanol
• 2-Benzyliden-6-dimethylaminomethyl-1-(3-trifluormethyl-phenyl)-cyclohexanol
• 2-Benzyliden-1-(3-chlor-4-fluor-phenyl)-6-dimethylaminomethyl-cyclohexanol
• 2-Benzyliden-6-dimethylaminomethyl-1-(2-methyl-benzyl)-cyclohexanol
• 2-Benzyliden-6-dimethylaminomethyl-1-(2-methoxy-phenyl)-cyclohexanol
• 2-Benzyliden-6-dimethylaminomethyl-1-(3-fluor-benzyl)-cyclohexanol
• 2-Benzyliden-1-(4-chlor-3-trifluormethyl-phenyl)-6-dimethylaminomethyl-cyclohexanol
• 2-Benzyliden-6-dimethylaminomethyl-1-(3-methoxy-benzyl)-cyclohexanol
• 2-Benzyliden-6-dimethylaminomethyl-1-(4-fluor-benzyl)-cyclohexanol
• 2-Benzyliden-1-(2-chlor-benzyl)-6-dimethylaminomethyl-cyclohexanol
• 2-Benzyliden-1-(3,5-dichlor-phenyl)-6-dimethylaminomethyl-cyclohexanol
• 2-Benzyliden-1-(3-chlor-phenyl)-6-dimethylaminomethyl-cyclohexanol
• 2-Benzyliden-6-dimethylaminomethyl-1-(3-fluor-phenyl)-cyclohexanol
• 2-Benzyliden-6-dimethylaminomethyl-1-(5-fluor-2-methoxy-phenyl)-cyclohexanol
• 2-Benzyliden-1-cyclohexylmethyl-6-dimethylaminomethyl-cyclohexanol
• 2-Benzyliden-6-dimethylaminomethyl-1-(4-methoxy-phenyl)-cyclohexanol
• 2-Benzyliden-6-dimethylaminomethyl-1-p-tolyl-cyclohexanol
• 2-Benzyliden-6-dimethylaminomethyl-1-(3-phenyl-propyl)-cyclohexanol
• 2-Benzyliden-6-dimethylaminomethyl-1-(3-methoxy-phenyl)-cyclohexanol
• 2-Benzyliden-6-dimethylaminomethyl-1-thiophen-2-yl-cyclohexanol
• 2-Benzyliden-6-dimethylaminomethyl-1-phenylethynyl-cyclohexanol
• 2-Benzyliden-6-dimethylaminomethyl-1-phenethyl-cyclohexanol
• 2-Benzyliden-6-dimethylaminomethyl-1-(4-fluor-phenyl)-cyclohexanol
• 2-Benzyliden-6-dimethylaminomethyl-bicyclohexyl-1-ol
• 2-Benzyliden-6-dimethylaminomethyl-1-m-tolyl-cyclohexanol
• 2-Benzyliden-1-cyclopentyl-6-dimethylaminomethyl-cyclohexanol
• 2-Benzyliden-1-(4-tert-butyl-phenyl)-6-dimethylaminomethyl-cyclohexanol
• 2-Benzyliden-6-dimethylaminomethyl-1-vinyl-cyclohexanol
• 2-Benzyliden-6-dimethylaminomethyl-1-o-tolyl-cyclohexanol
• 2-Benzyliden-6-dimethylaminomethyl-1-(4-fluor-3-methyl-phenyl)-cyclohexanol
• 1-Benzyl-2-benzyliden-6-dimethylaminomethyl-cyclohexanol
• 2-Benzyliden-1-(4-chlor-phenyl)-6-dimethylaminomethyl-cyclohexanol
• 2-Benzyliden-6-dimethylaminomethyl-1-phenyl-cyclohexanol
• 1-(3-Chlor-benzyl)-2-(4-chlor-benzyliden)-6-dimethylaminomethyl-cyclohexanol
• 2-(4-Chlor-benzyliden)-6-dimethylaminomethyl-1-(2,5-dimethyl-benzyl)-cyclohexanol
• 2-(4-Chlor-benzyliden)-1-(2-chlor-6-fluor-benzyl)-6-dimethylaminomethyl-cyclohexanol
• 1-(4-Chlor-benzyl)-2-(4-chlor-benzyliden)-6-dimethylaminomethyl-cyclohexanol
• 2-(4-Chlor-benzyliden)-6-dimethylaminomethyl-1-(3-trifluormethyl-phenyl)-cyclohexanol
• 2-(4-Chlor-benzyliden)-1-(3-chlor-4-fluor-phenyl)-6-dimethylaminomethyl-cyclohexanol
• 2-(4-Chlor-benzyliden)-6-dimethylaminomethyl-1-(2-methyl-benzyl)-cyclohexanol
• 2-(4-Chlor-benzyliden)-6-dimethylaminomethyl-1-(2-methoxy-phenyl)-cyclohexanol
• 2-(4-Chlor-benzyliden)-6-dimethylaminomethyl-1-(3-fluor-benzyl)-cyclohexanol
• 2-(4-Chlor-benzyliden)-1-(4-chlor-3-trifluormethyl-phenyl)-6-dimethylaminomethyl-cyclohexanol
• 2-(4-Chlor-benzyliden)-6-dimethylaminomethyl-1-(3-methoxy-benzyl)-cyclohexanol
• 2-(4-Chlor-benzyliden)-6-dimethylaminomethyl-1-(4-fluor-benzyl)-cyclohexanol
• 1-(2-Chlor-benzyl)-2-(4-chlor-benzyliden)-6-dimethylaminomethyl-cyclohexanol
• 2-(4-Chlor-benzyliden)-1-(3,5-dichlor-phenyl)-6-dimethylaminomethyl-cyclohexanol
• 2-(4-Chlor-benzyliden)-1-(3-chlor-phenyl)-6-dimethylaminomethyl-cyclohexanol
• 2-(4-Chlor-benzyliden)-6-dimethylaminomethyl-1-(3-fluor-phenyl)-cyclohexanol
• 2-(4-Chlor-benzyliden)-6-dimethylaminomethyl-1-(5-fluor-2-methoxy-phenyl)-cyclohexanol
• 2-(4-Chlor-benzyliden)-1-cyclohexylmethyl-6-dimethylaminomethyl-cyclohexanol
• 2-(4-Chlor-benzyliden)-6-dimethylaminomethyl-1-(4-methoxy-phenyl)-cyclohexanol
• 2-(4-Chlor-benzyliden)-6-dimethylaminomethyl-1-p-tolyl-cyclohexanol
• 2-(4-Chlor-benzyliden)-6-dimethylaminomethyl-1-(3-phenyl-propyl)-cyclohexanol
• 2-(4-Chlor-benzyliden)-6-dimethylaminomethyl-1-(3-methoxy-phenyl)-cyclohexanol
• 2-(4-Chlor-benzyliden)-6-dimethylaminomethyl-1-thiophen-2-yl-cyclohexanol
• 2-(4-Chlor-benzyliden)-6-dimethylaminomethyl-1-phenylethynyl-cyclohexanol
• 2-(4-Chlor-benzyliden)-6-dimethylaminomethyl-1-phenethyl-cyclohexanol
• 2-(4-Chlor-benzyliden)-6-dimethylaminomethyl-1-(4-fluor-phenyl)-cyclohexanol
• 2-(4-Chlor-benzyliden)-6-dimethylaminomethyl-bicyclohexyl-1-ol
• 2-(4-Chlor-benzyliden)-6-dimethylaminomethyl-1-m-tolyl-cyclohexanol
• 2-(4-Chlor-benzyliden)-1-cyclopentyl-6-dimethylaminomethyl-cyclohexanol
• 1-(4-tert-Butyl-phenyl)-2-(4-chlor-benzyliden)-6-dimethylaminomethyl-cyclohexanol
• 2-(4-Chlor-benzyliden)-6-dimethylaminomethyl-1-vinyl-cyclohexanol
• 2-(4-Chlor-benzyliden)-6-dimethylaminomethyl-1-o-tolyl-cyclohexanol
• 2-(4-Chlor-benzyliden)-6-dimethylaminomethyl-1-(4-fluor-3-methyl-phenyl)-cyclohexanol
• 1-Benzyl-2-(4-chlor-benzyliden)-6-dimethylaminomethyl-cyclohexanol
• 2-(4-Chlor-benzyliden)-1-(4-chlor-phenyl)-6-dimethylaminomethyl-cyclohexanol
• 2-(4-Chlor-benzyliden)-6-dimethylaminomethyl-1-phenyl-cyclohexanol
• 1-(3-Chlor-benzyl)-2-dimethylaminomethyl-6-(4-methoxy-benzyliden)-cyclohexanol
• 2-Dimethylaminomethyl-1-(2,5-dimethyl-benzyl)-6-(4-methoxy-benzyliden)-cyclohexanol
• 1-(2-Chlor-6-fluor-benzyl)-2-dimethylaminomethyl-6-(4-methoxy-benzyliden)-cyclohexanol
• 2-Dimethylaminomethyl-6-(4-methoxy-benzyliden)-1-(3-methyl-benzyl)-cyclohexanol
• 2-Dimethylaminomethyl-6-(4-methoxy-benzyliden)-1-(3-trifluormethyl-phenyl)-cyclohexanol
• 1-(3-Chlor-4-fluor-phenyl)-2-dimethylaminomethyl-6-(4-methoxy-benzyliden)-cyclohexanol
• 2-Dimethylaminomethyl-6-(4-methoxy-benzyliden)-1-(2-methoxy-phenyl)-cyclohexanol
• 1-(4-Chlor-3-trifluormethyl-phenyl)-2-dimethylaminomethyl-6-(4-methoxy-benzyliden)-cyclohexanol
• 2-Dimethylaminomethyl-1-(4-fluor-benzyl)-6-(4-methoxy-benzyliden)-cyclohexanol
• 1-(2-Chlor-benzyl)-2-dimethylaminomethyl-6-(4-methoxy-benzyliden)-cyclohexanol
• 1-(3,5-Dichlor-phenyl)-2-dimethylaminomethyl-6-(4-methoxy-benzyliden)-cyclohexanol
• 1-(3-Chlor-phenyl)-2-dimethylaminomethyl-6-(4-methoxy-benzyliden)-cyclohexanol
• 2-Dimethylaminomethyl-1-(5-fluor-2-methoxy-phenyl)-6-(4-methoxy-benzyliden)-cyclohexanol
• 1-Cyclohexylmethyl-2-dimethylaminomethyl-6-(4-methoxy-benzyliden)-cyclohexanol
• 2-Dimethylaminomethyl-6-(4-methoxy-benzyliden)-1-(4-methoxy-phenyl)-cyclohexanol
• 2-Dimethylaminomethyl-6-(4-methoxy-benzyliden)-1-p-tolyl-cyclohexanol
• 1-(2,3-Dichlor-phenyl)-2-dimethylaminomethyl-6-(4-methoxy-benzyliden)-cyclohexanol
• 2-Dimethylaminomethyl-6-(4-methoxy-benzyliden)-1-(3-phenyl-propyl)-cyclohexanol
• 2-Dimethylaminomethyl-6-(4-methoxy-benzyliden)-1-(3-methoxy-phenyl)-cyclohexanol
• 2-Dimethylaminomethyl-6-(4-methoxy-benzyliden)-1-thiophen-2-yl-cyclohexanol
• 2-Dimethylaminomethyl-6-(4-methoxy-benzyliden)-1-phenylethynyl-cyclohexanol
• 2-Dimethylaminomethyl-6-(4-methoxy-benzyliden)-1-phenethyl-cyclohexanol
• 2-Dimethylaminomethyl-1-(4-fluor-phenyl)-6-(4-methoxy-benzyliden)-cyclohexanol
• 6-Dimethylaminomethyl-2-(4-methoxy-benzyliden)-bicyclohexyl-1-ol
• 2-Dimethylaminomethyl-6-(4-methoxy-benzyliden)-1-m-tolyl-cyclohexanol
• 1-Cyclopentyl-2-dimethylaminomethyl-6-(4-methoxy-benzyliden)-cyclohexanol
• 1-(4-tert-Butyl-phenyl)-2-dimethylaminomethyl-6-(4-methoxy-benzyliden)-cyclohexanol
• 2-Dimethylaminomethyl-6-(4-methoxy-benzyliden)-1-vinyl-cyclohexanol
• 2-Dimethylaminomethyl-6-(4-methoxy-benzyliden)-1-o-tolyl-cyclohexanol
• 2-Dimethylaminomethyl-1-(4-fluor-3-methyl-phenyl)-6-(4-methoxy-benzyliden)-cyclohexanol
• 1-Benzyl-2-dimethylaminomethyl-6-(4-methoxy-benzyliden)-cyclohexanol
• 1-(4-Chlor-phenyl)-2-dimethylaminomethyl-6-(4-methoxy-benzyliden)-cyclohexanol
• 2-Dimethylaminomethyl-6-(4-methoxy-benzyliden)-1-phenyl-cyclohexanol
• 1-(3-Chlor-benzyl)-2-dimethylaminomethyl-6-(3-methoxy-benzyliden)-cyclohexanol
• 2-Dimethylaminomethyl-1-(2,5-dimethyl-benzyl)-6-(3-methoxy-benzyliden)-cyclohexanol
• 2-Dimethylaminomethyl-6-(3-methoxy-benzyliden)-1-(2-methyl-benzyl)-cyclohexanol
• 2-Dimethylaminomethyl-6-(3-methoxy-benzyliden)-1-(2-methoxy-phenyl)-cyclohexanol
• 1-(4-Chlor-3-trifluormethyl-phenyl)-2-dimethylaminomethyl-6-(3-methoxy-benzyliden)-cyclohexanol
• 1-(2-Chlor-benzyl)-2-dimethylaminomethyl-6-(3-methoxy-benzyliden)-cyclohexanol
• 6-Dimethylaminomethyl-2-(3-methoxy-benzyliden)-bicyclohexyl-1-ol
• 1-Cyclopentyl-2-dimethylaminomethyl-6-(3-methoxy-benzyliden)-cyclohexanol
• 2-Dimethylaminomethyl-6-(3-methoxy-benzyliden)-1-vinyl-cyclohexanol
• 2-Dimethylaminomethyl-1-(4-fluor-3-methyl-phenyl)-6-(3-methoxy-benzyliden)-cyclohexanol
• 1-Benzyl-2-dimethylaminomethyl-6-(3-methoxy-benzyliden)-cyclohexanol
• 2-Dimethylaminomethyl-6-(3-methoxy-benzyliden)-1-phenyl-cyclohexanol
• 1-Benzyl-2-(2-chlor-benzyliden)-6-dimethylaminomethyl-cyclohexanol
• 5-Dimethylamino-2,4-dimethyl-1,3-diphenyl-pent-1-en-3-ol
• 3-(4-Chlor-phenyl)-5-dimethylamino-2,4-dimethyl-1-phenyl-pent-1-en-3-ol
• 3-Benzyl-5-dimethylamino-2,4-dimethyl-1-phenyl-pent-1-en-3-ol
• 5-Dimethylamino-3-(4-fluor-3-methyl-phenyl)-2,4-dimethyl-1-phenyl-pent-1-en-3-ol
• 5-Dimethylamino-2,4-dimethyl-1-phenyl-3-o-tolyl-pent-1-en-3-ol
• 3-(2-Dimethylamino-1-methyl-ethyl)-2-methyl-1-phenyl-penta-1,4-dien-3-ol
• 3-(4-tert-Butyl-phenyl)-5-dimethylamino-2,4-dimethyl-1-phenyl-pent-1-en-3-ol
• 3-Cyclopentyl-5-dimethylamino-2,4-dimethyl-1-phenyl-pent-1-en-3-ol
• 5-Dimethylamino-2,4-dimethyl-1-phenyl-3-m-tolyl-pent-1-en-3-ol
• 3-Cyclohexyl-5-dimethylamino-2,4-dimethyl-1-phenyl-pent-1-en-3-ol
• 5-Dimethylamino-3-(4-fluor-phenyl)-2,4-dimethyl-1-phenyl-pent-1-en-3-ol
• 5-Dimethylamino-2,4-dimethyl-3-phenethyl-1-phenyl-pent-1-en-3-ol
• 3-(2-Dimethylamino-1-methyl-ethyl)-2-methyl-1,5-diphenyl-pent-1-en-4-yn-3-ol
• 5-Dimethylamino-2,4-dimethyl-1-phenyl-3-thiophen-2-yl-pent-1-en-3-ol
• 3-(2,4-Dichlor-phenyl)-5-dimethylamino-2,4-dimethyl-1-phenyl-pent-1-en-3-ol
• 5-Dimethylamino-3-(3-methoxy-phenyl)-2,4-dimethyl-1-phenyl-pent-1-en-3-ol
• 3-(2-Dimethylamino-1-methyl-ethyl)-2-methyl-1,6-diphenyl-hex-1-en-3-ol
• 3-(2,3-Dichlor-phenyl)-5-dimethylamino-2,4-dimethyl-1-phenyl-pent-1-en-3-ol
• 5-Dimethylamino-2,4-dimethyl-1-phenyl-3-p-tolyl-pent-1-en-3-ol
• 5-Dimethylamino-3-(4-methoxy-phenyl)-2,4-dimethyl-1-phenyl-pent-1-en-3-ol
• 3-Cyclohexylmethyl-5-dimethylamino-2,4-dimethyl-1-phenyl-pent-1-en-3-ol
• 5-Dimethylamino-3-(5-fluor-2-methoxy-phenyl)-2,4-dimethyl-1-phenyl-pent-1-en-3-ol
• 5-Dimethylamino-3-(3-fluor-phenyl)-2,4-dimethyl-1-phenyl-pent-1-en-3-ol
• 3-(3-Chlor-phenyl)-5-dimethylamino-2,4-dimethyl-1-phenyl-pent-1-en-3-ol
• 3-(3,5-Dichlor-phenyl)-5-dimethylamino-2,4-dimethyl-1-phenyl-pent-1-en-3-ol
• 3-(2-Chlor-benzyl)-5-dimethylamino-2,4-dimethyl-1-phenyl-pent-1-en-3-ol
• 5-Dimethylamino-3-(4-fluor-benzyl)-2,4-dimethyl-1-phenyl-pent-1-en-3-ol
• 3-(4-Chlor-3-trifluormethyl-phenyl)-5-dimethylamino-2,4-dimethyl-1-phenyl-pent-1-en-3-ol
• 5-Dimethylamino-3-(2-methoxy-phenyl)-2,4-dimethyl-1-phenyl-pent-1-en-3-ol
• 5-Dimethylamino-2,4-dimethyl-3-(2-methyl-benzyl)-1-phenyl-pent-1-en-3-ol
• 3-(3-Chlor-4-fluor-phenyl)-5-dimethylamino-2,4-dimethyl-1-phenyl-pent-1-en-3-ol
• 5-Dimethylamino-2,4-dimethyl-1-phenyl-3-(3-trifluormethyl-phenyl)-pent-1-en-3-ol
• 5-Dimethylamino-2,4-dimethyl-3-(3-methyl-benzyl)-1-phenyl-pent-1-en-3-ol
• 3-(4-Chlor-benzyl)-5-dimethylamino-2,4-dimethyl-1-phenyl-pent-1-en-3-ol
• 3-(2-Chlor-6-fluor-benzyl)-5-dimethylamino-2,4-dimethyl-1-phenyl-pent-1-en-3-ol
• 5-Dimethylamino-3-(2,5-dimethyl-benzyl)-2,4-dimethyl-1-phenyl-pent-1-en-3-ol
• 3-(3-Chlor-benzyl)-5-dimethylamino-2,4-dimethyl-1-phenyl-pent-1-en-3-ol
• 3-(2,4-Dichlor-benzyl)-5-dimethylamino-2,4-dimethyl-1-phenyl-pent-1-en-3-ol
• 5-Dimethylamino-1-(4-fluor-phenyl)-2,4-dimethyl-3-phenyl-pent-1-en-3-ol
• 3-Benzyl-5-dimethylamino-1-(4-fluor-phenyl)-2,4-dimethyl-pent-1-en-3-ol
• 5-Dimethylamino-3-(4-fluor-3-methyl-phenyl)-1-(4-fluor-phenyl)-2,4-dimethyl-pent-1-en-3-ol
• 5-Dimethylamino-1-(4-fluor-phenyl)-2,4-dimethyl-3-o-tolyl-pent-1-en-3-ol
• 3-(2-Dimethylamino-1-methyl-ethyl)-1-(4-fluor-phenyl)-2-methyl-penta-1,4-dien-3-ol
• 3-(4-tert-Butyl-phenyl)-5-dimethylamino-1-(4-fluor-phenyl)-2,4-dimethyl-pent-1-en-3-ol
• 3-Cyclopentyl-5-dimethylamino-1-(4-fluor-phenyl)-2,4-dimethyl-pent-1-en-3-ol
• 5-Dimethylamino-1-(4-fluor-phenyl)-2,4-dimethyl-3-m-tolyl-pent-1-en-3-ol
• 3-Cyclohexyl-5-dimethylamino-1-(4-fluor-phenyl)-2,4-dimethyl-pent-1-en-3-ol
• 5-Dimethylamino-1,3-bis-(4-fluor-phenyl)-2,4-dimethyl-pent-1-en-3-ol
• 5-Dimethylamino-1-(4-fluor-phenyl)-2,4-dimethyl-3-phenethyl-pent-1-en-3-ol
• 3-(2-Dimethylamino-1-methyl-ethyl)-1-(4-fluor-phenyl)-2-methyl-5-phenyl-pent-1-en-4-yn-3-ol
• 5-Dimethylamino-1-(4-fluor-phenyl)-2,4-dimethyl-3-thiophen-2-yl-pent-1-en-3-ol
• 3-(2,4-Dichlor-phenyl)-5-dimethylamino-1-(4-fluor-phenyl)-2,4-dimethyl-pent-1-en-3-ol
• 5-Dimethylamino-1-(4-fluor-phenyl)-3-(3-methoxy-phenyl)-2,4-dimethyl-pent-1-en-3-ol
• 3-(2-Dimethylamino-1-methyl-ethyl)-1-(4-fluor-phenyl)-2-methyl-6-phenyl-hex-1-en-3-ol
• 3-(2,3-Dichlor-phenyl)-5-dimethylamino-1-(4-fluor-phenyl)-2,4-dimethyl-pent-1-en-3-ol
• 5-Dimethylamino-1-(4-fluor-phenyl)-2,4-dimethyl-3-p-tolyl-pent-1-en-3-ol
• 5-Dimethylamino-1-(4-fluor-phenyl)-3-(4-methoxy-phenyl)-2,4-dimethyl-pent-1-en-3-ol
• 3-Cyclohexylmethyl-5-dimethylamino-1-(4-fluor-phenyl)-2,4-dimethyl-pent-1-en-3-ol
• 5-Dimethylamino-3-(5-fluor-2-methoxy-phenyl)-1-(4-fluor-phenyl)-2,4-dimethyl-pent-1-en-3-ol
• 5-Dimethylamino-3-(3-fluor-phenyl)-1-(4-fluor-phenyl)-2,4-dimethyl-pent-1-en-3-ol
• 3-(3-Chlor-phenyl)-5-dimethylamino-1-(4-fluor-phenyl)-2,4-dimethyl-pent-1-en-3-ol
• 3-(3,5-Dichlor-phenyl)-5-dimethylamino-1-(4-fluor-phenyl)-2,4-dimethyl-pent-1-en-3-ol
• 3-(2-Chlor-benzyl)-5-dimethylamino-1-(4-fluor-phenyl)-2,4-dimethyl-pent-1-en-3-ol
• 5-Dimethylamino-3-(4-fluor-benzyl)-1-(4-fluor-phenyl)-2,4-dimethyl-pent-1-en-3-ol
• 5-Dimethylamino-1-(4-fluor-phenyl)-3-(3-methoxy-benzyl)-2,4-dimethyl-pent-1-en-3-ol
• 3-(4-Chlor-3-trifluormethyl-phenyl)-5-dimethylamino-1-(4-fluor-phenyl)-2,4-dimethyl-pent-1-en-3-ol
• 5-Dimethylamino-3-(3-fluor-benzyl)-1-(4-fluor-phenyl)-2,4-dimethyl-pent-1-en-3-ol
• 5-Dimethylamino-1-(4-fluor-phenyl)-3-(2-methoxy-phenyl)-2,4-dimethyl-pent-1-en-3-ol
• 5-Dimethylamino-1-(4-fluor-phenyl)-2,4-dimethyl-3-(2-methyl-benzyl)-pent-1-en-3-ol
• 3-(3-Chlor-4-fluor-phenyl)-5-dimethylamino-1-(4-fluor-phenyl)-2,4-dimethyl-pent-1-en-3-ol
• 5-Dimethylamino-1-(4-fluor-phenyl)-2,4-dimethyl-3-(3-trifluormethyl-phenyl)-pent-1-en-3-ol
• 5-Dimethylamino-1-(4-fluor-phenyl)-2,4-dimethyl-3-(3-methyl-benzyl)-pent-1-en-3-ol
• 3-(4-Chlor-benzyl)-5-dimethylamino-1-(4-fluor-phenyl)-2,4-dimethyl-pent-1-en-3-ol
• 3-(2-Chlor-6-fluor-benzyl)-5-dimethylamino-1-(4-fluor-phenyl)-2,4-dimethyl-pent-1-en-3-ol
• 5-Dimethylamino-3-(2,5-dimethyl-benzyl)-1-(4-fluor-phenyl)-2,4-dimethyl-pent-1-en-3-ol
• 3-(3-Chlor-benzyl)-5-dimethylamino-1-(4-fluor-phenyl)-2,4-dimethyl-pent-1-en-3-ol
• 3-(2,4-Dichlor-benzyl)-5-dimethylamino-1-(4-fluor-phenyl)-2,4-dimethyl-pent-1-en-3-ol
• 1-(4-Chlor-phenyl)-5-dimethylamino-2,4-dimethyl-3-phenyl-pent-1-en-3-ol
• 1,3-Bis-(4-chlor-phenyl)-5-dimethylamino-2,4-dimethyl-pent-1-en-3-ol
• 3-Benzyl-1-(4-chlor-phenyl)-5-dimethylamino-2,4-dimethyl-pent-1-en-3-ol
• 1-(4-Chlor-phenyl)-5-dimethylamino-3-(4-fluor-3-methyl-phenyl)-2,4-dimethyl-pent-1-en-3-ol
• 1-(4-Chlor-phenyl)-5-dimethylamino-2,4-dimethyl-3-o-tolyl-pent-1-en-3-ol
• 1-(4-Chlor-phenyl)-3-(2-dimethylamino-1-methyl-ethyl)-2-methyl-penta-1,4-dien-3-ol
• 3-(4-tert-Butyl-phenyl)-1-(4-chlor-phenyl)-5-dimethylamino-2,4-dimethyl-pent-1-en-3-ol
• 1-(4-Chlor-phenyl)-3-cyclopentyl-5-dimethylamino-2,4-dimethyl-pent-1-en-3-ol
• 1-(4-Chlor-phenyl)-5-dimethylamino-2,4-dimethyl-3-m-tolyl-pent-1-en-3-ol
• 1-(4-Chlor-phenyl)-3-cyclohexyl-5-dimethylamino-2,4-dimethyl-pent-1-en-3-ol
• 1-(4-Chlor-phenyl)-5-dimethylamino-3-(4-fluor-phenyl)-2,4-dimethyl-pent-1-en-3-ol
• 1-(4-Chlor-phenyl)-5-dimethylamino-2,4-dimethyl-3-phenethyl-pent-1-en-3-ol
• 1-(4-Chlor-phenyl)-5-dimethylamino-2,4-dimethyl-3-thiophen-2-yl-pent-1-en-3-ol
• 1-(4-Chlor-phenyl)-3-(2,4-dichlor-phenyl)-5-dimethylamino-2,4-dimethyl-pent-1-en-3-ol
• 1-(4-Chlor-phenyl)-5-dimethylamino-3-(3-methoxy-phenyl)-2,4-dimethyl-pent-1-en-3-ol
• 1-(4-Chlor-phenyl)-3-(2-dimethylamino-1-methyl-ethyl)-2-methyl-6-phenyl-hex-1-en-3-ol
• 1-(4-Chlor-phenyl)-3-(2,3-dichlor-phenyl)-5-dimethylamino-2,4-dimethyl-pent-1-en-3-ol
• 1-(4-Chlor-phenyl)-5-dimethylamino-2,4-dimethyl-3-p-tolyl-pent-1-en-3-ol
• 1-(4-Chlor-phenyl)-5-dimethylamino-3-(4-methoxy-phenyl)-2,4-dimethyl-pent-1-en-3-ol
• 1-(4-Chlor-phenyl)-3-cyclohexylmethyl-5-dimethylamino-2,4-dimethyl-pent-1-en-3-ol
• 1-(4-Chlor-phenyl)-5-dimethylamino-3-(5-fluor-2-methoxy-phenyl)-2,4-dimethyl-pent-1-en-3-ol
• 1-(4-Chlor-phenyl)-5-dimethylamino-3-(3-fluor-phenyl)-2,4-dimethyl-pent-1-en-3-ol
• 3-(3-Chlor-phenyl)-1-(4-chlor-phenyl)-5-dimethylamino-2,4-dimethyl-pent-1-en-3-ol
• 1-(4-Chlor-phenyl)-3-(3,5-dichlor-phenyl)-5-dimethylamino-2,4-dimethyl-pent-1-en-3-ol
• 3-(2-Chlor-benzyl)-1-(4-chlor-phenyl)-5-dimethylamino-2,4-dimethyl-pent-1-en-3-ol
• 1-(4-Chlor-phenyl)-5-dimethylamino-3-(4-fluor-benzyl)-2,4-dimethyl-pent-1-en-3-ol
• 1-(4-Chlor-phenyl)-5-dimethylamino-3-(3-methoxy-benzyl)-2,4-dimethyl-pent-1-en-3-ol
• 1-(4-Chlor-phenyl)-3-(4-chlor-3-trifluormethyl-phenyl)-5-dimethylamino-2,4-dimethyl-pent-1-en-3-ol
• 1-(4-Chlor-phenyl)-5-dimethylamino-3-[2-(3-fluor-phenyl)-ethyl]-2,4-dimethyl-pent-1-en-3-ol
• 1-(4-Chlor-phenyl)-5-dimethylamino-3-(2-methoxy-phenyl)-2,4-dimethyl-pent-1-en-3-ol
• 1-(4-Chlor-phenyl)-5-dimethylamino-2,4-dimethyl-3-(2-methyl-benzyl)-pent-1-en-3-ol
• 3-(3-Chlor-4-fluor-phenyl)-1-(4-chlor-phenyl)-5-dimethylamino-2,4-dimethyl-pent-1-en-3-ol
• 1-(4-Chlor-phenyl)-5-dimethylamino-2,4-dimethyl-3-(3-trifluormethyl-phenyl)-pent-1-en-3-ol
• 1-(4-Chlor-phenyl)-5-dimethylamino-2,4-dimethyl-3-(3-methyl-benzyl)-pent-1-en-3-ol
• 3-(4-Chlor-benzyl)-1-(4-chlor-phenyl)-5-dimethylamino-2,4-dimethyl-pent-1-en-3-ol
• 3-(2-Chlor-6-fluor-benzyl)-1-(4-chlor-phenyl)-5-dimethylamino-2,4-dimethyl-pent-1-en-3-ol
• 1-(4-Chlor-phenyl)-5-dimethylamino-3-(2,5-dimethyl-benzyl)-2,4-dimethyl-pent-1-en-3-ol
• 3-(3-Chlor-benzyl)-1-(4-chlor-phenyl)-5-dimethylamino-2,4-dimethyl-pent-1-en-3-ol
• 1-(4-Chlor-phenyl)-3-(2,4-dichlor-benzyl)-5-dimethylamino-2,4-dimethyl-pent-1-en-3-ol
• 5-Dimethylamino-1-(4-methoxy-phenyl)-2,4-dimethyl-3-phenyl-pent-1-en-3-ol
• 3-(4-Chlor-phenyl)-5-dimethylamino-1-(4-methoxy-phenyl)-2,4-dimethyl-pent-1-en-3-ol
• 3-Benzyl-5-dimethylamino-1-(4-methoxy-phenyl)-2,4-dimethyl-pent-1-en-3-ol
• 5-Dimethylamino-3-(4-fluor-3-methyl-phenyl)-1-(4-methoxy-phenyl)-2,4-d imethyl-pent-1-en-3-ol
• 5-Dimethylamino-1-(4-methoxy-phenyl)-2,4-dimethyl-3-o-tolyl-pent-1-en-3-ol
• 3-(2-Dimethylamino-1-methyl-ethyl)-1-(4-methoxy-phenyl)-2-methyl-penta-1,4-dien-3-ol
• 3-(4-tert-Butyl-phenyl)-5-dimethylamino-1-(4-methoxy-phenyl)-2,4-dimethyl-pent-1-en-3-ol
• 3-Cyclopentyl-5-dimethylamino-1-(4-methoxy-phenyl)-2,4-dimethyl-pent-1-en-3-ol
• 5-Dimethylamino-1-(4-methoxy-phenyl)-2,4-dimethyl-3-m-tolyl-pent-1-en-3-ol
• 3-Cyclohexyl-5-dimethylamino-1-(4-methoxy-phenyl)-2,4-dimethyl-pent-1-en-3-ol
• 5-Dimethylamino-3-(4-fluor-phenyl)-1-(4-methoxy-phenyl)-2,4-dimethyl-pent-1-en-3-ol
• 5-Dimethylamino-1-(4-methoxy-phenyl)-2,4-dimethyl-3-phenethyl-pent-1-en-3-ol
• 3-(2-Dimethylamino-1-methyl-ethyl)-1-(4-methoxy-phenyl)-2-methyl-5-phenyl-pent-1-en-4-yn-3-ol
• 5-Dimethylamino-1-(4-methoxy-phenyl)-2,4-dimethyl-3-thiophen-2-yl-pent-1-en-3-ol
• 3-(2,4-Dichlor-phenyl)-5-dimethylamino-1-(4-methoxy-phenyl)-2,4-dimethyl-pent-1-en-3-ol
• 5-Dimethylamino-3-(3-methoxy-phenyl)-1-(4-methoxy-phenyl)-2,4-dimethyl-pent-1-en-3-ol
• 3-(2-Dimethylamino-1-methyl-ethyl)-1-(4-methoxy-phenyl)-2-methyl-6-phenyl-hex-1-en-3-ol
• 3-(2,3-Dichlor-phenyl)-5-dimethylamino-1-(4-methoxy-phenyl)-2,4-dimethyl-pent-1-en-3-ol
• 5-Dimethylamino-1-(4-methoxy-phenyl)-2,4-dimethyl-3-p-tolyl-pent-1-en-3-ol
• 5-Dimethylamino-1,3-bis-(4-methoxy-phenyl)-2,4-dimethyl-pent-1-en-3-ol
• 3-Cyclohexylmethyl-5-dimethylamino-1-(4-methoxy-phenyl)-2,4-dimethyl-pent-1-en-3-ol
• 5-Dimethylamino-3-(5-fluor-2-methoxy-phenyl)-1-(4-methoxy-phenyl)-2,4-dimethyl-pent-1-en-3-ol
• 5-Dimethylamino-3-(3-fluor-phenyl)-1-(4-methoxy-phenyl)-2,4-dimethyl-pent-1-en-3-ol
• 3-(3-Chlor-phenyl)-5-dimethylamino-1-(4-methoxy-phenyl)-2,4-dimethyl-pent-1-en-3-ol
• 3-(3,5-Dichlor-phenyl)-5-dimethylamino-1-(4-methoxy-phenyl)-2,4-dimethyl-pent-1-en-3-ol
• 3-(2-Chlor-benzyl)-5-dimethylamino-1-(4-methoxy-phenyl)-2,4-dimethyl-pent-1-en-3-ol
• 5-Dimethylamino-3-(4-fluor-benzyl)-1-(4-methoxy-phenyl)-2,4-dimethyl-pent-1-en-3-ol
• 5-Dimethylamino-3-(3-fluor-benzyl)-1-(4-methoxy-phenyl)-2,4-dimethyl-pent-1-en-3-ol
• 5-Dimethylamino-3-(2-methoxy-phenyl)-1-(4-methoxy-phenyl)-2,4-dimethyl-pent-1-en-3-ol
• 5-Dimethylam ino-1-(4-methoxy-phenyl)-2,4-d imethyl-3-(2-methyl-benzyl)-pent-1-en-3-ol
• 3-(3-Chlor-4-fluor-phenyl)-5-dimethylamino-1-(4-methoxy-phenyl)-2,4-dimethyl-pent-1-en-3-ol
• 5-Dimethylamino-1-(4-methoxy-phenyl)-2,4-dimethyl-3-(3-trifluormethylphenyl)-pent-1-en-3-ol
• 3-(4-Chlor-benzyl)-5-dimethylamino-1-(4-methoxy-phenyl)-2,4-dimethyl-pent-1-en-3-ol
• 3-(2-Chlor-6-fluor-benzyl)-5-dimethylamino-1-(4-methoxy-phenyl)-2,4-dimethyl-pent-1-en-3-ol
• 3-(3-Chlor-benzyl)-5-dimethylamino-1-(4-methoxy-phenyl)-2,4-dimethyl-pent-1-en-3-ol
• 2-Benzyliden-1-(4-tert-butyl-phenyl)-5-dimethylaminomethyl-cyclopentanol
• 2-Benzyliden-1-cyclohexyl-5-dimethylaminomethyl-cyclopentanol
• 2-Benzyliden-5-dimethylaminomethyl-1-phenethyl-cyclopentanol
• 2-Benzyliden-5-dimethylaminomethyl-1-(4-fluor-benzyl)-cyclopentanol
• 2-Benzyliden-5-dimethylaminomethyl-1-(3-fluor-benzyl)-cyclopentanol
• 2-Benzyliden-5-dimethylaminomethyl-1-(2-methoxy-phenyl)-cyclopentanol
• 2-Benzyliden-5-dimethylaminomethyl-1-(2-methyl-benzyl)-cyclopentanol
• 2-Benzyliden-5-dimethylaminomethyl-1-(3-methyl-benzyl)-cyclopentanol
• 2-Benzyliden-1-(4-chlor-benzyl)-5-dimethylaminomethyl-cyclopentanol
• 2-Benzyliden-1-(2-chlor-6-fluor-benzyl)-5-dimethylaminomethyl-cyclopentanol
• 2-Benzyliden-5-dimethylaminomethyl-1-(2,5-dimethyl-benzyl)-cyclopentanol
• 2-Benzyliden-1-(3-chlor-benzyl)-5-dimethylaminomethyl-cyclopentanol
• 2-Dimethylaminomethyl-5-(3-methoxy-benzyl)-bicyclopentyl-1-ol
• 2-Dimethylaminomethyl-5-(3-methoxy-benzyl)-1-m-tolyl-cyclopentanol
• 1-Cyclohexyl-2-dimethylaminomethyl-5-(3-methoxy-benzyl)-cyclopentanol
• 2-Dimethylaminomethyl-5-(3-methoxy-benzyl)-1-phenethyl-cyclopentanol
• 2-Dimethylaminomethyl-5-(3-methoxy-benzyl)-1-phenylethynyl-cyclopentanol
• 2-Dimethylaminomethyl-5-(3-methoxy-benzyl)-1-thiophen-2-yl-cyclopentanol
• 2-Dimethylaminomethyl-5-(3-methoxy-benzyl)-1-(3-methoxy-phenyl)-cyclopentanol
• 2-Dimethylaminomethyl-5-(3-methoxy-benzyl)-1-(3-phenyl-propyl)-cyclopentanol
• 2-Dimethylaminomethyl-5-(3-methoxy-benzyl)-1-(4-methoxy-phenyl)-cyclopentanol
• 2-Dimethylaminomethyl-1-(3-fluor-benzyl)-5-(3-methoxy-benzyliden)-cyclopentanol
• 2-Dimethylaminomethyl-5-(3-methoxy-benzyliden)-1-(2-methoxy-phenyl)-cyclopentanol
• 2-Dimethylaminomethyl-5-(3-methoxy-benzyliden)-1-(2-methyl-benzyl)-cyclopentanol
• 2-Dimethylaminomethyl-5-(3-methoxy-benzyliden)-1-(3-methyl-benzyl)-cyclopentanol
• 2-Dimethylaminomethyl-1 -(2,5-dimethyl-benzyl)-5-(3-methoxy-benzyliden)-cyclopentanol
• 1-(3-Chlor-benzyl)-2-dimethylaminomethyl-5-(3-methoxy-benzyliden)-cyclopentanol
• 1-Benzyl-2-dimethylaminomethyl-5-(3-methoxy-benzyliden)-cyclopentanol
• 2-Dimethylaminomethyl-5-(3-methoxy-benzyliden)-1-o-tolyl-cyclopentanol
• 2-Dimethylaminomethyl-5-(3-methoxy-benzyliden)-1-vinyl-cyclopentanol
• 1-(4-tert-Butyl-phenyl)-2-dimethylaminomethyl-5-(3-methoxy-benzyliden)-cyclopentanol
• 2-Dimethylaminomethyl-5-(3-methoxy-benzyliden)-bicyclopentyl-1-ol
• 1-Cyclohexyl-2-dimethylaminomethyl-5-(3-methoxy-benzyliden)-cyclopentanol
• 2-Dimethylaminomethyl-1-(4-fluor-phenyl)-5-(3-methoxy-benzyliden)-cyclopentanol
• 2-Dimethylaminomethyl-5-(3-methoxy-benzyliden)-1-phenethyl-cyclopentanol
• 2-Dimethylaminomethyl-5-(3-methoxy-benzyliden)-1-phenylethynyl-cyclopentanol
• 2-Dimethylaminomethyl-5-(3-methoxy-benzyliden)-1-thiophen-2-yl-cyclopentanol
• 2-Dimethylaminomethyl-5-(3-methoxy-benzyliden)-1-(3-methoxy-phenyl)-cyclopentanol
• 2-Dimethylaminomethyl-5-(3-methoxy-benzyliden)-1-(3-phenyl-propyl)-cyclopentanol
• 2-Dimethylaminomethyl-5-(3-methoxy-benzyliden)-1-p-tolyl-cyclopentanol
• 2-Dimethylaminomethyl-5-(3-methoxy-benzyliden)-1-(4-methoxy-phenyl)-cyclopentanol
• 1-(2-Chlor-benzyl)-2-dimethylaminomethyl-5-(4-methoxy-benzyliden)-cyclopentanol
• 2-Dimethylaminomethyl-1-(3-methoxy-benzyl)-5-(4-methoxy-benzyliden)-cyclopentanol
• 2-Dimethylaminomethyl-1-(3-fluor-benzyl)-5-(4-methoxy-benzyliden)-cyclopentanol
• 2-Dimethylaminomethyl-5-(4-methoxy-benzyliden)-1-(2-methoxy-phenyl)-cyclopentanol
• 2-Dimethylaminomethyl-5-(4-methoxy-benzyliden)-1-(3-methyl-benzyl)-cyclopentanol
• 1-(4-Chlor-benzyl)-2-dimethylaminomethyl-5-(4-methoxy-benzyliden)-cyclopentanol
• 1-(2-Chlor-6-fluor-benzyl)-2-dimethylaminomethyl-5-(4-methoxy-benzyliden)-cyclopentanol
• 1-(3-Chlor-benzyl)-2-dimethylaminomethyl-5-(4-methoxy-benzyliden)-cyclopentanol
• 1-(2,4-Dichlor-benzyl)-2-dimethylaminomethyl-5-(4-methoxy-benzyliden)-cyclopentanol
• 2-Benzyliden-7-dimethylaminomethyl-1-phenyl-cycloheptanol
• 2-Benzyliden-1-(4-chlor-phenyl)-7-dimethylaminomethyl-cycloheptanol
• 1-Benzyl-2-benzyliden-7-dimethylaminomethyl-cycloheptanol
• 2-Benzyliden-7-dimethylaminomethyl-1-(4-fluor-3-methyl-phenyl)-cycloheptanol
• 2-Benzyliden-7-dimethylaminomethyl-1-o-tolyl-cycloheptanol
• 2-Benzyliden-7-dimethylaminomethyl-1-vinyl-cycloheptanol
• 2-Benzyliden-1-(4-tert-butyl-phenyl)-7-dimethylaminomethyl-cycloheptanol
• 2-Benzyliden-1-cyclopentyl-7-dimethylaminomethyl-cycloheptanol
• 2-Benzyliden-7-dimethylaminomethyl-1-m-tolyl-cycloheptanol
• 2-Benzyliden-1-cyclohexyl-7-dimethylaminomethyl-cycloheptanol
• 2-Benzyliden-7-dimethylaminomethyl-1-(4-fluor-phenyl)-cycloheptanol
• 2-Benzyliden-7-dimethylaminomethyl-1-phenylethynyl-cycloheptanol
• 2-Benzyliden-7-dimethylaminomethyl-1-thiophen-2-yl-cycloheptanol
• 2-Benzyliden-7-dimethylaminomethyl-1-(3-methoxy-phenyl)-cycloheptanol
• 2-Benzyliden-1-cyclohexylmethyl-7-dimethylaminomethyl-cycloheptanol
• 2-Benzyliden-7-dimethylaminomethyl-1-(3-fluor-4-methoxy-phenyl)-cycloheptanol
• 2-Benzyliden-7-dimethylaminomethyl-1-(3-fluor-phenyl)-cycloheptanol
• 2-Benzyliden-1-(3-chlor-phenyl)-7-dimethylaminomethyl-cycloheptanol
• 2-Benzyliden-1-(3,5-dichlor-phenyl)-7-dimethylaminomethyl-cycloheptanol
• 2-Benzyliden-7-dimethylaminomethyl-1-(4-fluor-benzyl)-cycloheptanol
• 2-Benzyliden-7-dimethylaminomethyl-1-(4-methoxy-benzyl)-cycloheptanol
• 2-Benzyliden-7-dimethylaminomethyl-1-(3-fluor-benzyl)-cycloheptanol
• 2-Benzyliden-7-dimethylaminomethyl-1-(2-methoxy-phenyl)-cycloheptanol
• 2-Benzyliden-7-dimethylaminomethyl-1-(2-methyl-benzyl)-cycloheptanol
• 2-Benzyliden-1-(3-chlor-4-fluor-phenyl)-7-dimethylaminomethyl-cycloheptanol
• 2-Benzyliden-7-dimethylaminomethyl-1-(3-trifluormethyl-phenyl)-cycloheptanol
• 2-Benzyliden-7-dimethylaminomethyl-1-(3-methyl-benzyl)-cycloheptanol
• 2-Benzyliden-1-(4-chlor-benzyl)-7-dimethylaminomethyl-cycloheptanol
• 2-Benzyliden-1-(2-chlor-6-fluor-benzyl)-7-dimethylaminomethyl-cycloheptanol
• 2-Benzyliden-7-dimethylaminomethyl-1-(2,5-dimethyl-benzyl)-cycloheptanol
• 2-Benzyliden-1-(3-chlor-benzyl)-7-dimethylaminomethyl-cycloheptanol
• 2-Dimethylaminomethyl-7-(3-methoxy-benzyliden)-1-phenyl-cycloheptanol
• 1-(4-Chlor-phenyl)-2-dimethylaminomethyl-7-(3-methoxy-benzyliden)-cycloheptanol
• 1-Benzyl-2-d im ethylaminomethyl-7-(3-methoxy-benzyliden)-cycloheptanol
• 2-Dimethylaminomethyl-1-(4-fluor-3-methyl-phenyl)-7-(3-methoxy-benzyliden)-cycloheptanol
• 2-Dimethylaminomethyl-7-(3-methoxy-benzyliden)-1-o-tolyl-cycloheptanol
• 2-Dimethylaminomethyl-7-(3-methoxy-benzyliden)-1-vinyl-cycloheptanol
• 1-(4-tert-Butyl-phenyl)-2-dimethylaminomethyl-7-(3-methoxy-benzyliden)-cycloheptanol
• 1-Cyclopentyl-2-dimethylaminomethyl-7-(3-methoxy-benzyliden)-cycloheptanol
• 2-Dimethylaminomethyl-7-(3-methoxy-benzyliden)-1-m-tolyl-cycloheptanol
• 1-Cyclohexyl-2-dimethylaminomethyl-7-(3-methoxy-benzyliden)-cycloheptanol
• 2-Dimethylaminomethyl-1-(4-fluor-phenyl)-7-(3-methoxy-benzyliden)-cycloheptanol
• 2-Dimethylaminomethyl-7-(3-methoxy-benzyliden)-1-phenethyl-cycloheptanol
• 2-Dimethylaminomethyl-7-(3-methoxy-benzyliden)-1-phenylethynyl-cycloheptanol
• 2-Dimethylaminomethyl-7-(3-methoxy-benzyliden)-1-thiophen-2-yl-cycloheptanol
• 2-Dimethylaminomethyl-7-(3-methoxy-benzyliden)-1-(3-methoxy-phenyl)-cycloheptanol
• 2-Dimethylaminomethyl-7-(3-methoxy-benzyliden)-1-(3-phenyl-propyl)-cycloheptanol
• 2-Dimethylaminomethyl-7-(3-methoxy-benzyliden)-1-p-tolyl-cycloheptanol
• 2-Dimethylaminomethyl-7-(3-methoxy-benzyliden)-1-(4-methoxy-phenyl)-cycloheptanol
• 1-Cyclohexylmethyl-2-dimethylaminomethyl-7-(3-methoxy-benzyliden)-cycloheptanol
• 1-(3-Chlor-phenyl)-2-dimethylaminomethyl-7-(3-methoxy-benzyliden)-cycloheptanol
• 1-(3,5-Dichlor-phenyl)-2-dimethylaminomethyl-7-(3-methoxy-benzyliden)-cycloheptanol
• 1-(2-Chlor-benzyl)-2-dimethylaminomethyl-7-(3-methoxy-benzyliden)-cycloheptanol
• 1-(4-Chlor-3-trifluormethyl-phenyl)-2-dimethylaminomethyl-7-(3-methoxy-benzyliden)-cycloheptanol
• 2-Dimethylaminomethyl-1-(3-fluor-phenyl)-7-(3-methoxy-benzyliden)-cycloheptanol
• 2-Dimethylaminomethyl-7-(3-methoxy-benzyliden)-1-(2-methoxy-phenyl)-cycloheptanol
• 2-Dimethylaminomethyl-7-(3-methoxy-benzyliden)-1-(2-methyl-benzyl)-cycloheptanol
• 2-Dimethylaminomethyl-7-(3-methoxy-benzyliden)-1-(3-trifluormethyl-phenyl)-cycloheptanol
• 2-Dimethylaminomethyl-7-(3-methoxy-benzyliden)-1-(3-methyl-benzyl)-cycloheptanol
• 2-Dimethylaminomethyl-1-(2,5-dimethyl-benzyl)-7-(3-methoxy-benzyliden)-cycloheptanol
• 1-(3-Chlor-benzyl)-2-dimethylaminomethyl-7-(3-methoxy-benzyliden)-cycloheptanol
• 1-Benzyl-2-dimethylaminomethyl-7-(4-methoxy-benzyliden)-cycloheptanol
• 2-Dimethylaminomethyl-1-(4-fluor-3-methyl-phenyl)-7-(4-methoxy-benzyliden)-cycloheptanol
• 2-Dimethylaminomethyl-7-(4-methoxy-benzyliden)-1-o-tolyl-cycloheptanol
• 2-Dimethylaminomethyl-7-(4-methoxy-benzyliden)-1-vinyl-cycloheptanol
• 1-(4-tert-Butyl-phenyl)-2-dimethylaminomethyl-7-(4-methoxy-benzyliden)-cycloheptanol
• 1-Cyclopentyl-2-dimethylaminomethyl-7-(4-methoxy-benzyliden)-cycloheptanol
• 2-Dimethylaminomethyl-7-(4-methoxy-benzyliden)-1-m-tolyl-cycloheptanol
• 1-Cyclohexyl-2-dimethylaminomethyl-7-(4-methoxy-benzyliden)-cycloheptanol
• 2-Dimethylaminomethyl-1-(4-fluor-phenyl)-7-(4-methoxy-benzyliden)-cycloheptanol
• 2-Dimethylaminomethyl-7-(4-methoxy-benzyliden)-1-phenethyl-cycloheptanol
• 2-Dimethylaminomethyl-7-(4-methoxy-benzyliden)-1-phenylethynyl-cycloheptanol
• 2-Dimethylaminomethyl-7-(4-methoxy-benzyliden)-1-thiophen-2-yl-cycloheptanol
• 2-Dimethylaminomethyl-7-(4-methoxy-benzyliden)-1-(3-methoxy-phenyl)-cycloheptanol
• 2-Dimethylaminomethyl-7-(4-methoxy-benzyliden)-1-(3-phenyl-propyl)-cycloheptanol
• 2-Dimethylaminomethyl-7-(4-methoxy-benzyliden)-1-p-tolyl-cycloheptanol
• 2-Dimethylaminomethyl-7-(4-methoxy-benzyliden)-1-(4-methoxy-phenyl)-cycloheptanol
• 1-Cyclohexylmethyl-2-dimethylaminomethyl-7-(4-methoxy-benzyliden)-cycloheptanol
• 1-(3-Chlor-phenyl)-2-dimethylaminomethyl-7-(4-methoxy-benzyliden)-cycloheptanol
• 1-(2-Chlor-benzyl)-2-dimethylaminomethyl-7-(4-methoxy-benzyliden)-cycloheptanol
• 2-Dimethylaminomethyl-7-(4-methoxy-benzyliden)-1-(2-methoxy-phenyl)-cycloheptanol
• 2-Dimethylaminomethyl-7-(4-methoxy-benzyliden)-1-(2-methyl-benzyl)-cycloheptanol
• 2-Dimethylaminomethyl-7-(4-methoxy-benzyliden)-1-(3-trifluormethyl-phenyl)-cycloheptanol
• 2-Dimethylaminomethyl-7-(4-methoxy-benzyliden)-1-(3-methyl-benzyl)-cycloheptanol
• 1-(3-Chlor-benzyl)-2-dimethylaminomethyl-7-(4-methoxy-benzyliden)-cycloheptanol
• 1-(3,5-Dichlor-phenyl)-2-dimethylaminomethyl-7-(4-methoxy-benzyliden)-cycloheptanol
• 2-Benzyliden-7-dimethylaminomethyl-1-(3-hydroxy-phenyl)-cycloheptanol;
• 2-Dimethylaminomethyl-1-(3-hydroxy-phenyl)-7-(3-methoxy-benzyliden)-cycloheptanol;
• 2-Benzyliden-7-dimethylaminomethyl-1-(3-methoxy-phenyl)-cycloheptanol;
• 3-[1-(2-Dimethylamino-1-methyl-ethyl)-1-hydroxy-2-methyl-3-phenyl-allyl]-phenol;
• 3-(4-Chlor-benzyl)-5-dimethylamino-2,4-dimethyl-1-phenyl-pent-1-en-3-ol;
• 5-Dimethylamino-3-(3-methoxy-phenyl)-2,4-dimethyl-1-phenyl-pent-1-en-3-ol;
• 3-(2-Benzyliden-6-dimethylaminomethyl-1-hydroxy-cyclohexyl)-phenol;
• 1-Benzyl-2-benzyliden-7-(4-methyl-piperazin-1-ylmethyl)-cycloheptanol;
• 2-Benzyliden-1-(2-chlor-benzyl)-7-(4-methyl-piperazin-1-ylmethyl)-cycloheptanol;
• 2-Benzyliden-1-(3-chlor-benzyl)-7-(4-methyl-piperazin-1-ylmethyl)-cycloheptanol;
• 2-Benzyliden-1-(4-chlor-benzyl)-7-(4-methyl-piperazin-1-ylmethyl)-cycloheptanol;
• 2-Benzyliden-1-(2-fluor-benzyl)-7-(4-methyl-piperazin-1-ylmethyl)-cycloheptanol;
• 2-Benzyliden-1-(3-fluor-benzyl)-7-(4-methyl-piperazin-1-ylmethyl)-cycloheptanol;
• 2-Benzyliden-1-(4-fluor-benzyl)-7-(4-methyl-piperazin-1-ylmethyl)-cycloheptanol;
• 2-Benzyliden-1-(2-methyl-benzyl)-7-(4-methyl-piperazin-1-ylmethyl)-cycloheptanol;
• 2-Benzyliden-1-(3-methyl-benzyl)-7-(4-methyl-piperazin-1-ylmethyl)-cycloheptanol;
• 2-Benzyliden-1-(4-methyl-benzyl)-7-(4-methyl-piperazin-1-ylmethyl)-cycloheptanol;
• 2-Benzyliden-1-(2-methoxy-benzyl)-7-(4-methyl-piperazin-1-ylmethyl)-cycloheptanol;
• 2-Benzyliden-1-(3-methoxy-benzyl)-7-(4-methyl-piperazin-1-ylmethyl)-cycloheptanol;
• 2-Benzyliden-1-(4-methoxy-benzyl)-7-(4-methyl-piperazin-1-ylmethyl)-cycloheptanol;
• 2-Benzyliden-1-(2,6-dichlor-benzyl)-7-(4-methyl-piperazin-1-ylmethyl)-cycloheptanol;
• 2-Benzyliden-1-(2-chlor-6-fluor-benzyl)-7-(4-methyl-piperazin-1-ylmethyl)-cycloheptanol;
• 2-Benzyliden-1-(2,6-difluor-benzyl)-7-(4-methyl-piperazin-1-ylmethyl)-cycloheptanol;
• 1-Benzyl-2-(4-chlor-benzyliden)-7-(4-methyl-piperazin-1-ylmethyl)-cycloheptanol;
• 1-(2-Chlor-benzyl)-2-(4-chlor-benzyliden)-7-(4-methyl-piperazin-1-ylmethyl)-cycloheptanol;
• 1-(3-Chlor-benzyl)-2-(4-chlor-benzyliden)-7-(4-methyl-piperazin-1-ylmethyl)-cycloheptanol;
• 1-(4-Chlor-benzyl)-2-(4-chlor-benzyliden)-7-(4-methyl-piperazin-1-ylmethyl)-cycloheptanol;
• 2-(4-Chlor-benzyliden)-1-(2-fluor-benzyl)-7-(4-methyl-piperazin-1-ylmethyl)-cycloheptanol;
• 2-(4-Chlor-benzyliden)-1-(3-fluor-benzyl)-7-(4-methyl-piperazin-1-ylmethyl)-cycloheptanol;
• 2-(4-Chlor-benzyliden)-1-(4-fluor-benzyl)-7-(4-methyl-piperazin-1-ylmethyl)-cycloheptanol;
• 2-(4-Chlor-benzyliden)-1-(2-methyl-benzyl)-7-(4-methyl-piperazin-1-ylmethyl)-cycloheptanol;
• 2-(4-Chlor-benzyliden)-1-(3-methyl-benzyl)-7-(4-methyl-piperazin-1-ylmethyl)-cycloheptanol;
• 2-(4-Chlor-benzyliden)-1-(4-methyl-benzyl)-7-(4-methyl-piperazin-1-ylmethyl)-cycloheptanol;
• 2-(4-Chlor-benzyliden)-1-(2-methoxy-benzyl)-7-(4-methyl-piperazin-1-ylmethyl)-cycloheptanol;
• 2-(4-Chlor-benzyliden)-1-(3-methoxy-benzyl)-7-(4-methyl-piperazin-1-ylmethyl)-cycloheptanol;
• 2-(4-Chlor-benzyliden)-1-(4-methoxy-benzyl)-7-(4-methyl-piperazin-1-ylmethyl)-cycloheptanol;
• 2-(4-Chlor-benzyliden)-1-(2,6-dichlor-benzyl)-7-(4-methyl-piperazin-1-ylmethyl)-cycloheptanol;
• 2-(4-Chlor-benzyliden)-1-(2,6-difluor-benzyl)-7-(4-methyl-piperazin-1-ylmethyl)-cycloheptanol;
• 2-(4-Chlor-benzyliden)-1-(2-chlor-6-fluor-benzyl)-7-(4-methyl-piperazin-1-ylmethyl)-cycloheptanol;
• 1-Benzyl-2-(4-fluor-benzyliden)-7-(4-methyl-piperazin-1-ylmethyl)-cycloheptanol;
• 1-(2-Chlor-benzyl)-2-(4-fluor-benzyliden)-7-(4-methyl-piperazin-1-ylmethyl)-cycloheptanol;
• 1-(3-Chlor-benzyl)-2-(4-fluor-benzyliden)-7-(4-methyl-piperazin-1-ylmethyl)-cycloheptanol;
• 1-(4-Chlor-benzyl)-2-(4-fluor-benzyliden)-7-(4-methyl-piperazin-1-ylmethyl)-cycloheptanol;
• 1-(2-Fluor-benzyl)-2-(4-fluor-benzyliden)-7-(4-methyl-piperazin-1-ylmethyl)-cycloheptanol;
• 1-(3-Fluor-benzyl)-2-(4-fluor-benzyliden)-7-(4-methyl-piperazin-1-ylmethyl)-cycloheptanol;
• 1-(4-Fluor-benzyl)-2-(4-fluor-benzyliden)-7-(4-methyl-piperazin-1-ylmethyl)-cycloheptanol;
• 2-(4-Fluor-benzyliden)-1-(2-methyl-benzyl)-7-(4-methyl-piperazin-1-ylmethyl)-cycloheptanol;
• 2-(4-Fluor-benzyliden)-1-(3-methyl-benzyl)-7-(4-methyl-piperazin-1-ylmethyl)-cycloheptanol;
• 2-(4-Fluor-benzyliden)-1-(4-methyl-benzyl)-7-(4-methyl-piperazin-1-ylmethyl)-cycloheptanol;
• 2-(4-Fluor-benzyliden)-1-(2-methoxy-benzyl)-7-(4-methyl-piperazin-1-ylmethyl)-cycloheptanol;
• 2-(4-Fluor-benzyliden)-1-(3-methoxy-benzyl)-7-(4-methyl-piperazin-1-ylmethyl)-cycloheptanol;
• 2-(4-Fluor-benzyliden)-1-(4-methoxy-benzyl)-7-(4-methyl-piperazin-1-ylmethyl)-cycloheptanol;
• 1-(2,6-Dichlor-benzyl)-2-(4-fluor-benzyliden)-7-(4-methyl-piperazin-1-ylmethyl)-cycloheptanol;
• 1-(2,6-Difluor-benzyl)-2-(4-fluor-benzyliden)-7-(4-methyl-piperazin-1-ylmethyl)-cycloheptanol;
• 1-(2-Chlor-6-fluorbenzyl)-2-(4-fluor-benzyliden)-7-(4-methyl-piperazin-1-ylmethyl)-cycloheptanol;
• 1-Benzyl-2-furan-2-ylmethylen-7-(4-methyl-piperazin-1-ylmethyl)-cycloheptanol;
• 1-(2-Chlor-benzyl)-2-furan-2-ylmethylen-7-(4-methyl-piperazin-1-ylmethyl)-cycloheptanol;
• 1-(3-Chlor-benzyl)-2-furan-2-ylmethylen-7-(4-methyl-piperazin-1-ylmethyl)-cycloheptanol;
• 1-(4-Chlor-benzyl)-2-furan-2-ylmethylen-7-(4-methyl-piperazin-1-ylmethyl)-cycloheptanol;
• 1-(2-Fluor-benzyl)-2-furan-2-ylmethylen-7-(4-methyl-piperazin-1-ylmethyl)-cycloheptanol;
• 1-(3-Fluor-benzyl)-2-furan-2-ylmethylen-7-(4-methyl-piperazin-1-ylmethyl)-cycloheptanol;
• 1-(4-Fluor-benzyl)-2-furan-2-ylmethylen-7-(4-methyl-piperazin-1-ylmethyl)-cycloheptanol;
• 2-Furan-2-ylmethylen-1-(2-methyl-benzyl)-7-(4-methyl-piperazin-1-ylmethyl)-cycloheptanol;
• 2-Furan-2-ylmethylen-1-(3-methyl-benzyl)-7-(4-methyl-piperazin-1-ylmethyl)-cycloheptanol;
• 2-Furan-2-ylmethylen-1-(4-methyl-benzyl)-7-(4-methyl-piperazin-1-ylmethyl)-cycloheptanol;
• 2-Furan-2-ylmethylen-1-(2-methoxy-benzyl)-7-(4-methyl-piperazin-1-ylmethyl)-cycloheptanol;
• 2-Furan-2-ylmethylen-1-(3-methoxy-benzyl)-7-(4-methyl-piperazin-1-ylmethyl)-cycloheptanol;
• 2-Furan-2-ylmethylen-1-(4-methoxy-benzyl)-7-(4-methyl-piperazin-1-ylmethyl)-cycloheptanol;
• 1-(2,6-Dichlor-benzyl)-2-furan-2-ylmethylen-7-(4-methyl-piperazin-1-ylmethyl)-cycloheptanol;
• 1-(2,6-Difluor-benzyl)-2-furan-2-ylmethylen-7-(4-methyl-piperazin-1-ylmethyl)-cycloheptanol;
• 1-(2-Chlor-6-fluor-benzyl)-2-furan-2-ylmethylen-7-(4-methyl-piperazin-1-ylmethyl)-cycloheptanol;
• 1-Benzyl-3-furan-2-ylmethylen-7-(4-methyl-piperazin-1-ylmethyl)-cycloheptanol;
• 1-(2-Chlor-benzyl)-3-furan-2-ylmethylen-7-(4-methyl-piperazin-1-ylmethyl)-cycloheptanol;
• 1-(3-Chlor-benzyl)-3-furan-2-ylmethylen-7-(4-methyl-piperazin-1-ylmethyl)-cycloheptanol;
• 1-(4-Chlor-benzyl)-3-furan-2-ylmethylen-7-(4-methyl-piperazin-1-ylmethyl)-cycloheptanol;
• 1-(2-Fluor-benzyl)-3-furan-2-ylmethylen-7-(4-methyl-piperazin-1-ylmethyl)-cycloheptanol;
• 1-(3-Fluor-benzyl)-3-furan-2-ylmethylen-7-(4-methyl-piperazin-1-ylmethyl)-cycloheptanol;
• 1-(4-Fluor-benzyl)-3-furan-2-ylmethylen-7-(4-methyl-piperazin-1-ylmethyl)-cycloheptanol;
• 3-Furan-2-ylmethylen-1-(2-methyl-benzyl)-7-(4-methyl-piperazin-1-ylmethyl)-cycloheptanol;
• 3-Furan-2-ylmethylen-1-(3-methyl-benzyl)-7-(4-methyl-piperazin-1-ylmethyl)-cycloheptanol;
• 3-Furan-2-ylmethylen-1-(4-methyl-benzyl)-7-(4-methyl-piperazin-1-ylmethyl)-cycloheptanol;
• 3-Furan-2-ylmethylen-1-(2-methoxy-benzyl)-7-(4-methyl-piperazin-1-ylmethyl)-cycloheptanol;
• 3-Furan-2-ylmethylen-1-(3-methoxy-benzyl)-7-(4-methyl-piperazin-1-ylmethyl)-cycloheptanol;
• 3-Furan-2-ylmethylen-1-(4-methoxy-benzyl)-7-(4-methyl-piperazin-1-ylmethyl)-cycloheptanol;
• 1-(2,6-Dichlor-benzyl)-3-furan-2-ylmethylen-7-(4-methyl-piperazin-1-ylmethyl)-cycloheptanol;
• 1-(2,6-Difluor-benzyl)-3-furan-2-ylmethylen-7-(4-methyl-piperazin-1-ylmethyl)-cycloheptanol;
• 1-(2-Chlor-6-fluor-benzyl)-3-furan-2-ylmethylen-7-(4-methyl-piperazin-1-ylmethyl)-cycloheptanol
gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form ihrer Säuren oder ihrer Basen oder in Form ihrer Salze, insbesondere der physiologisch verträglichen Salze, oder in Form ihrer Solvate, insbesondere der Hydrate, vorzugsweise des Hydrochlorids oder Bishydrochlorids.

9. Substituierte 5-Amino-1-penten-3-ol-Derivate gemäß einem der Ansprüche 1 bis 8, **dadurch gekennzeichnet, daß** die E-Isomeren der Derivate gemäß Formel I bevorzugt sind wie in Formel I' gezeigt:

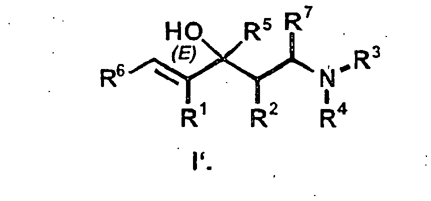

10. Substituierte 5-Amino-1-penten-3-ol-Derivate gemäß einem der Ansprüche 1 bis 9, **dadurch gekennzeichnet, daß**, wenn R7 Wasserstoff ist und R1 und R2 zusammen einen Ring bilden, die OH-Gruppe und die Aminomethylengruppe CHR⁷-NR³R⁴ gemäß Formel I cis zueinander stehen, wie in Formel I" gezeigt:

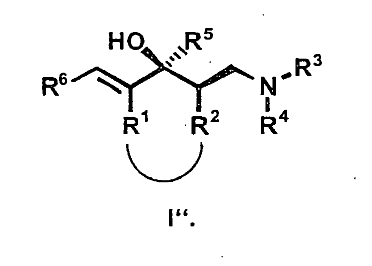

11. Arzneimittel enthaltend wenigstens ein substituiertes 5-Amino-1-penten-3-ol-Derivat gemäß einem der Ansprüche 1 bis 10, sowie gegebenenfalls geeignete Zusatz- und/oder Hilfsstoffe und/oder gegebenenfalls weitere Wirkstoffe.

12. Arzneimittel gemäß Anspruch 11, **dadurch gekennzeichnet, daß** ein enthaltenes substituiertes 5-Amino-1-penten-3-ol-Derivat gemäß einem der Ansprüche 1 bis 10, als reines Diastereomer und/oder Enantiomer, als Razemat oder als nicht-äquimolare oder äquimolare Mischung der Diastereomere und/oder Enantiomere vorliegt.

13. Verwendung eines substituierten 5-Amino-1-penten-3-ol-Derivats gemäß einem der Ansprüche 1 bis 10 zur Herstellung eines Arzneimittels zur Behandlung von Schmerz, insbesondere von akutem, neuropathischem oder chronischem Schmerz.

14. Verwendung eines substituierten 5-Amino-1-penten-3-ol-Derivats gemäß einem der Ansprüche 1 bis 10 zur Herstellung eines Arzneimittels zur Behandlung von Migräne, Hyperalgesie und Allodynie, insbesondere thermischer Hyperalgesie, mechanischer Hyperalgesie und Allodynie und Kälte-Allodynie, oder von inflammatorischem oder postoperativem Schmerz.

15. Verwendung eines substituierten 5-Amino-1-penten-3-ol-Derivats gemäß einem der Ansprüche 1 bis 10 zur Herstellung eines Arzneimittels zur Behandlung von Epilepsie, Hitzewallungen, Beschwerden in der Postmenopause, Amyotropischer Lateraler Sklerose (ALS), Reflex Sympasthetic Dystrophy (RSD), Spastischer Lähmung, Restless Leg Syndrom, erworbenem Nystagmus; psychatrischen bzw. neuropathologischen Störungen, wie bipolaren Störungen, Anxiety, Panikanfällen, Stimmungsschwankungen, manischem Verhalten, Depressionen, manisch-depressivem Verhalten; schmerzvoller diabetischer Neuropathie, Symptomen und Schmerzen aufgrund von Multipler Sklerose oder der Parkinsonschen Krankheit, neurodegenerativen Erkrankungen, wie Morbus Alzheimer. Morbus Huntington, Morbus Parkinson und Epilepsie; gastrointestinaler Schädigung; von erythromelalgischem oder postpoliomyelitischem Schmerz, trigeminaler oder postherpetischer Neuralgie; oder als Antikonvulsivum, Analgetikum oder Anxiolytikum.

16. Verwendung gemäß einem der Ansprüche 13 - 15, **dadurch gekennzeichnet, daß** ein verwendetes substituiertes 5-Amino-1-penten-3-ol-Derivat gemäß einem der Ansprüche 1 bis 10, als reines Diastereomer und/oder Enantiomer, als Razemat oder als nicht-äquimolare oder äquimolare Mischung der Diastereomere und/oder Enantiomere vorliegt.

17. Verfahren zur Herstellung eines substituierten 5-Amino-1-penten-3-ol-Derivats gemäß einem der Ansprüche 1 bis 10, in dem ein β-Aminoketon der Formel **IA**, in dem die Reste R¹ bis R⁴, R⁶ und R⁷ die in Anspruch 1 für Formel **I** beschriebene Bedeutung haben 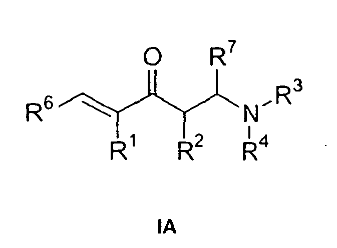 mit einer metallorganischen Verbindung der Formel **III**
R⁵-Z **III**
in der Z MgCl, MgBr, Mgl oder Li bedeutet und R⁵ die in Anspruch 1 für Formel **I** beschriebene Bedeutung hat, zu einer Verbindung der Formel **I** umsetzt.

## Claims

1. Substituted 5-amino-1-penten-3-ol derivatives of the general formula I, 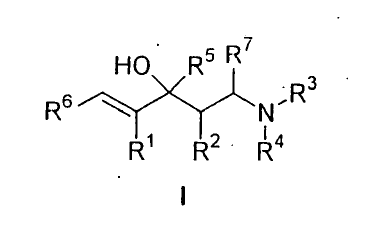 wherein
R¹ and R² in each case independently of one another are selected from C₁₋₆ alkyl, branched or unbranched, saturated or unsaturated, unsubstituted or singly or multiply substituted, or
R¹ and R² together form a (CH₂)₂₋₉ ring that may optionally be substituted by C₁₋₈ alkyl, branched or unbranched, saturated or unsaturated, unsubstituted or singly or multiply substituted, or by aryl, unsubstituted or singly or multiply substituted,
R³ and R⁴ in each case independently of one another are selected from C₁₋₆alkyl, branched or unbranched, saturated or unsaturated, unsubstituted or singly or multiply substituted, from C₃₋₆ cycloalkyl, saturated or unsaturated, unsubstituted or singly or multiply substituted, or from phenyl, benzyl or phenethyl, unsubstituted or singly or multiply substituted
or
the radicals R³ and R⁴ together form a ring and denote CH₂CH₂OCH₂CH₂, CH₂CH₂NR²²CH₂CH₂ or (CH₂)₃₋₆,
where R²² is selected from H; C₁-₁₀ alkyl or C₃₋₁₀ cycloalkyl that is in each case saturated or unsaturated, branched or unbranched, singly or multiply substituted or unsubstituted; aryl or heteroaryl, in each case singly or multiply substituted or unsubstituted; or aryl bound via • C₁₋₃ alkyl that is saturated or unsaturated, C₃₋₁₀ cycloalkyl or heteroaryl, in each case singly or multiply substituted or unsubstituted,
R⁵ is selected from C₁-₁₀ alkyl, saturated or unsaturated, branched or unbranched, singly or multiply substituted or unsubstituted, from C₃-₉ cycloalkyl, saturated or unsaturated, from aryl, heteroaryl, aryl bound via saturated or unsaturated C₁₋₃ alkyl, from C₃₋₁₀ cycloalkyl bound via saturated or unsaturated C₁₋₃ alkyl, or from heteroalkyl bound via saturated or unsaturated C₁₋₃ alkyl, wherein all aryl, heteroaryl and cycloalkyl radicals may in each case independently of one another be unsubstituted or may be singly or multiply substituted with radicals selected independently of one another from
F, Cl, Br, I, OR¹⁸, SR¹⁸, SO₂R¹⁸, SO₂OR¹⁸, CN, COOR¹⁸, NR¹⁹R²⁰; C₁-C₁₀ alkyl, saturated or unsaturated, branched or unbranched, singly or multiply substituted or unsubstituted; C₃₋₉ cycloalkyl, saturated or unsaturated, unsubstituted or singly or multiply substituted; aryl or heteroaryl, in each case unsubstituted or singly or multiply substituted; or from aryl, C₃-₉ cycloalkyl or heteroaryl bound via saturated or unsaturated C₁-₃ alkyl, and may in each case be unsubstituted or singly or multiply substituted,
where R¹⁸ is selected from H; C₁₋₁₀ alkyl, saturated or unsaturated, branched or unbranched, unsubstituted or singly or multiply substituted; C₃₋₉ cycloalkyl, saturated or unsaturated, unsubstituted or singly or multiply substituted; aryl or heteroaryl, unsubstituted or singly or multiply substituted; or aryl, C₃₋₉ cycloalkyl or heteroaryl bound via saturated or unsaturated C₁₋₃ alkyl and in each case unsubstituted or singly or multiply substituted;
R¹⁹ and R²⁰ independently of one another are selected from H; C₁₋₁₀ alkyl, saturated or unsaturated, branched or unbranched, unsubstituted or singly or multiply substituted; C₃₋₉ cycloalkyl, saturated or unsaturated, unsubstituted or singly or multiply substituted; aryl or heteroaryl, in each case unsubstituted or singly or multiply substituted; or from aryl, C₃₋₉ cycloalkyl or heteroaryl bound via saturated or unsaturated C₁₋₃ alkyl and in each case unsubstituted or singly or multiply substituted;
or R¹⁹ and R²⁰ together form CH₂CH₂OCH₂CH₂, CH₂CH₂NR²¹CH₂CH₂ or (CH₂)₃₋₆,
where R²¹ is selected from H; phenyl, substituted or unsubstituted; C₁₋₁₀ alkyl, saturated or unsaturated, branched or unbranched, unsubstituted or singly or multiply substituted;
R⁶ is selected from C₁₋₁₀ alkyl, saturated or unsaturated, branched or unbranched, singly or multiply substituted or unsubstituted; or from C₅-₇ cycloalkyl, aryl or heteroaryl, in each case unsubstituted or singly or multiply substituted;
R⁷ is selected from H; aryl or heteroaryl; in each case unsubstituted or singly or multiply substituted; optionally in the form of their racemates, their pure stereoisomers, in particular enantiomers or diastereomers, or in the form of mixtures of the stereoisomers, in particular of the enantiomers or diastereomers, in an arbitrary mixture ratio; in the specified form or in the form of their acids or their bases or in the form of their salts, in particular of physiologically acceptable salts or in the form of their solvates, in particular hydrates.

2. Substituted 5-amino-1-penten-3-ol derivatives according to claim 1, **characterised in that**
R⁷ is selected from H or heteroaryl or is a radical according to the formula II, 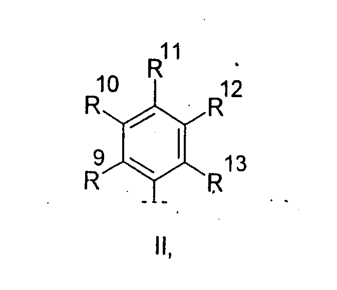 where R⁹ to R¹³ in each case independently of one another are selected from H, F, Cl, Br, I, CF₃, CHF₂, CH₂F, OH, OR¹⁴, OCF₃, OCHF₂, OCH₂F, SR¹⁴, SO₂CH₃, SO₂CF₃; C₁-₆ alkyl, branched or unbranched, saturated or unsaturated, unsubstituted or singly or multiply substituted; phenyl, unsubstituted or singly or multiply substituted; CN, COOR¹⁴, NO₂ or
R⁹ and R¹⁰ or R¹⁰ and R¹¹ together form an OCH₂O or OCH₂CH₂O ring, and
R¹⁴ is selected from C₁₋₆ alkyl, branched or unbranched, saturated or unsaturated, unsubstituted or singly or multiply substituted; or from phenyl, benzyl, phenethyl or thiophene, in each case unsubstituted or singly or multiply substituted,
and preferably R⁷ is hydrogen.

3. Substituted 5-amino-1-penten-3-ol derivatives according to one of claims 1 or 2, **characterised in that**
R¹ and R² together form a (CH₂)₂₋₅ ring that may optionally be substituted with C₁-₆ alkyl, branched or unbranched, saturated or unsaturated, unsubstituted or singly or multiply substituted; or phenyl, unsubstituted or singly or multiply substituted, though the said ring is preferably unsubstituted, and wherein R¹ and R² preferably together form an unsubstituted (CH₂)₂₋₄ ring
or
R¹ and R² independently of one another are selected from C₁₋₃ alkyl, unbranched, saturated and unsubstituted, preferably CH₃, and in particular R¹ and R² both denote CH₃.

4. Substituted 5-amino-1-penten-3-ol derivatives according to one of claims 1 to 3, **characterised in that**
R³ and R⁴ in each case independently of one another are selected from C₁₋₆ alkyl, branched or unbranched, saturated or unsaturated, unsubstituted or singly or multiply substituted, and preferably both are CH₃
or
R³ and R⁴ together form a ring and denote CH₂CH₂NR²²CH₂CH₂ or (CH₂)₃₋₆, in particular denote (CH₂)₄₋₅ or CH₂CH₂NR²²CH₂CH₂, where R²² is selected from H or C₁₋₆ alkyl that is saturated, branched or unbranched and unsubstituted; in particular H or CH₃.

5. Substituted 5-amino-1-penten-3-ol derivatives according to one of claims 1 to 4, **characterised in that**
R⁵ is selected from C₁₋₆ alkyl, saturated or unsaturated, branched or unbranched, singly or multiply substituted or unsubstituted, from C₅-₆ cycloalkyl, phenyl, thiophenyl, furyl, benzofuranyl, benzothiophenyl, pyrrolyl, pyridinyl, pyrimidinyl, quinolinyl, isoquinolinyl or quinazolinyl, preferably phenyl, furyl, thiophenyl or C₅₋₆ cycloalkyl; from phenyl, C₅₋₆ cycloalkyl, thiophenyl, furyl, benzofuranyl, benzothiophenyl, pyrrolyl, pyridinyl, pyrimidinyl, quinolinyl, isoquinolinyl, quinazolinyl bound via saturated or unsaturated C₁-₃ alkyl, preferably phenyl, furyl, thiophenyl or C₅-₆ cycloalkyl;
wherein all aryl, heteroaryl and cycloalkyl radicals in each case independently of one another may be unsubstituted or singly or multiply substituted, preferably unsubstituted, or are singly or multiply substituted by substituents selected independently of one another from F, Cl, Br, I, OH, O-C₁₋₄ alkyl, C₁₋₆ alkyl, CF₃, CHF₂, CH₂F, NH₂ and/or SH,
preferably
R⁵ is selected from C₁₋₃ alkyl, saturated or unsaturated, unsubstituted and/or unbranched; or from naphthyl, furyl, cyclohexyl, cyclopentyl, phenyl or thiophenyl, unsubstituted or singly or multiply substituted, preferably by F, Cl, Br, I, CF₃, CHF₂, CH₂F, OH, O-C₁₋₄ alkyl, C₁₋₆ alkyl, NH₂ and/or SH; or phenyl bound via saturated or unsaturated C₁₋₃ alkyl and unsubstituted or singly or multiply substituted preferably by F, Cl, Br, I, CF₃, CHF₂, CH₂F, OH, O-C₁₋₄ alkyl, C₁₋₆ alkyl, NH₂ and/or SH,
in particular
R⁵ is selected from -CH=CH₂, cyclohexyl, cyclopentyl, phenyl, phenethyl (phenyl bound via CH₂-CH₂), benzyl (phenyl bound via CH₂) or thiophenyl, unsubstituted or singly or multiply substituted, preferably by F, Cl, Br, I, OH, OCH₃, OC₂H₅, OC₃H₇, SH, CF₃, CHF₂, CH₂F, CH₃, C₂H₅, C₃H₇ and/or C₄H₉ or t-butyl.

6. Substituted 5-amino-1-penten-3-ol derivatives according to one of claims 1 to 5, **characterised in that**
R⁶ is selected from phenyl or furyl, in each case unsubstituted or singly or multiply substituted, preferably unsubstituted or singly or multiply substituted by substituents selected independently of one another from fluorine, chlorine, CH₃, OCH₃, CF₃ or tert.-butyl.

7. Substituted 5-amino-1-penten-3-ol derivatives according to one of claims 1 to 6, **characterised in that** if R⁵ is selected from aryl, C₃-₉ cycloalkyl or heteroaryl bound via saturated or unsaturated C₁-₃ alkyl, then the C₁-₃ alkyl, bound via the aryl, heteroaryl or cycloalkyl, is selected from:
-CH₂-, -C₂H₄-, -C₃H₆-, -C≡C-, -CH=CH-, -CH=CH-CH₂-, -CH₂-CH=CH-, -C≡C-CH₂- or -CH₂-C≡C-, preferably -CH₂-, -C₂H₄- or -C≡C-.

8. Substituted 5-amino-1-penten-3-ol derivatives according to one of claims 1 to 7, **characterised in that** they are selected from the following group:
• 2-benzylidene-1-(3-chlorobenzyl)-6-dimethylaminomethyl-cyclohexanol
• 2-benzylidene-6-dimethylaminomethyl-1-(2,5-dimethyl-benzyl)-cyclohexanol
• 2-benzylidene-1-(2-chloro-6-fluorobenzyl)-6-dimethylamino-methyl-cyclohexanol
• 2-benzylidene-1-(4-chlorobenzyl-6-dimethylaminomethyl)-cyclohexanol
• 2-benzylidene-6-dimethylaminomethyl-1-(3-methylbenzyl)-cyclohexanol
• 2-benzylidene-6-dimethylaminomethyl-1-(3-trifluoromethyl-phenyl)-cyclohexanol
• 2-benzylidene-1-(3-chloro-4-fluorophenyl)-6-dimethylamino-methyl-cyclohexanol
• 2-benzylidene-6-dimethylaminomethyl-1-(2-methylbenzyl)-cyclohexanol
• 2-benzylidene-6-dimethylaminomethyl-1-(2-methoxyphenyl)-cyclohexanol
• 2-benzylidene-6-dimethylaminomethyl-1-(3-fluorobenzyl)-cyclohexanol
• 2-benzylidene-1-(4-chloro-3-trifluoromethylphenyl)-6-dimethylamino-methyl-cyclohexanol
• 2-benzylidene-6-dimethylaminomethyl-1-(3-methoxybenzyl)-cyclohexanol
• 2-benzylidene-6-dimethylaminomethyl-1-(4-fluorobenzyl)-cyclohexanol
• 2-benzylidene-1-(2-chlorobenzyl)-6-dimethylaminomethyl-cyclohexanol
• 2-benzylidene-1-(3,5-dichlorophenyl)-6-dimethylamino-methyl-cyclohexanol
• 2-benzylidene-1-(3-chlorophenyl)-6-dimethylaminomethyl-cyclohexanol
• 2-benzylidene-6-dimethylaminomethyl-1-(3-fluorophenyl)-cyclohexanol
• 2-benzylidene-6-dimethylaminomethyl-1-(5-fluoro-2-methoxyphenyl)-cyclohexanol
• 2-benzylidene-1-cyclohexylmethyl-6-dimethylaminomethyl-cyclohexanol
• 2-benzylidene-6-dimethylaminomethyl-1-(4-methoxyphenyl)-cyclohexanol
• 2-benzylidene-6-dimethylaminomethyl-1-p-tolyl-cyclohexanol
• 2-benzylidene-6-dimethylaminomethyl-1-(3-phenylpropyl)-cyclohexanol
• 2-benzylidene-6-dimethylaminomethyl-1-(3-methoxyphenyl)-cyclohexanol
• 2-benzylidene-6-dimethylaminomethyl-1-thiophen-2-yl-cyclohexanol
• 2-benzylidene-6-dimethylaminomethyl-1-phenylethynyl-cyclohexanol
• 2-benzylidene-6-dimethylaminomethyl-1-phenethyl-cyclohexanol
• 2-benzylidene-6-dimethylaminomethyl-1-(4-fluorophenyl)-cyclohexanol
• 2-benzylidene-6-dimethylaminomethyl-bicyclohexyl-1-ol
• 2-benzylidene-6-dimethylaminomethyl-1-m-tolyl-cyclohexanol
• 2-benzylidene-1-cyclopentyl-6-dimethylaminomethyl-cyclohexanol
• 2-benzylidene-1-(4-tert.-butylphenyl)-6-dimethylaminomethyl-cyclohexanol
• 2-benzylidene-6-dimethylaminomethyl-1-vinyl-cyclohexanol
• 2-benzylidene-6-dimethylaminomethyl-1-o-tolyl-cyclohexanol
• 2-benzylidene-6-dimethylaminomethyl-1-(4-fluoro-3-methylphenyl)-cyclohexanol
• 1-benzyl-2-benzylidene-6-dimethylaminomethyl-cyclohexanol
• 2-benzylidene-1-(4-chlorophenyl)-6-dimethylaminomethyl-cyclohexanol
• 2-benzylidene-6-dimethylaminomethyl-1-phenyl-cyclohexanol
• 1-(3-chlorobenzyl)-2-(4-chlorobenzylidene)-6-dimethyl-aminomethyl-cyclohexanol
• 2-(4-chlorobenzylidene)-6-dimethylaminomethyl-1-(2,5-dimethylbenzyl)-cyclohexanol
• 2-(4-chlorobenzylidene)-1-(2-chloro-6-fluorobenzyl)-6-dimethylaminomethyl-cyclohexanol
• 1-(4-chlorobenzyl)-2-(4-chlorobenzylidene)-6-dimethyl-aminomethyl-cyclohexanol
• 2-(4-chlorobenzylidene)-6-dimethylaminomethyl-1-(3-trifluoromethylphenyl)-cyclohexanol
• 2-(4-chlorobenzylidene)-1-(3-chloro-4-fluorophenyl)-6-dimethylamino-methyl-cyclohexanol
• 2-(4-chlorobenzylidene)-6-dimethylaminomethyl-1-(2-methylbenzyl)-cyclohexanol
• 2-(4-chlorobenzylidene)-6-dimethylaminomethyl-1-(2-methoxyphenyl)-cyclohexanol
• 2-(4-chlorobenzylidene)-6-dimethylaminomethyl-1-(3-fluorobenzyl)-cyclohexanol
• 2-(4-chlorobenzylidene)-1-(4-chloro-3-trifluoromethyl-phenyl)-6-dimethylaminomethyl-cyclohexanol
• 2-(4-chlorobenzylidene)-6-dimethylaminomethyl-1-(3-methoxybenzyl)-cyclohexanol
• 2-(4-chlorobenzylidene)-6-dimethylaminomethyl-1-(4-fluorobenzyl)-cyclohexanol
• 1-(2-chlorobenzyl)-2-(4-chlorobenzylidene)-6-dimethyl-aminomethyl-cyclohexanol
• 2-(4-chlorobenzylidene)-1-(3,5-dichlorophenyl)-6-dimethylaminomethyl-cyclohexanol
• 2-(4-chlorobenzylidene)-1-(3-chlorophenyl)-6-dimethyl-aminomethyl-cyclohexanol
• 2-(4-chlorobenzylidene)-6-dimethylaminomethyl-1-(3-fluorophenyl)-cyclohexanol
• 2-(4-chlorobenzylidene)-6-dimethylaminomethyl-1-(5-fluoro-2-methoxy-phenyl)-cyclohexanol
• 2-(4-chlorobenzylidene)-1-cyclohexylmethyl-6-dimethyl-aminomethyl-cyclohexanol
• 2-(4-chlorobenzylidene)-6-dimethylaminomethyl-1-(4-methoxyphenyl)-cyclohexanol
• 2-(4-chlorobenzylidene)-6-dimethylaminomethyl-1-p-tolyl-cyclohexanol
• 2-(4-chlorobenzylidene)-6-dimethylaminomethyl-1-(3-phenylpropyl)-cyclohexanol
• 2-(4-chlorobenzylidene)-6-dimethylaminomethyl-1-(3-methoxyphenyl)-cyclohexanol
• 2-(4-chlorobenzylidene)-6-dimethylaminomethyl-1-thiophen-2-yl-cyclohexanol
• 2-(4-chlorobenzylidene)-6-dimethylaminomethyl-1-phenyl-ethynyl-cyclohexanol
• 2-(4-chlorobenzylidene)-6-dimethylaminomethyl-1-phenethyl-cyclohexanol
• 2-(4-chlorobenzylidene)-6-dimethylaminomethyl-1-(4-fluorophenyl)-cyclohexanol
• 2-(4-chlorobenzylidene)-6-dimethylaminomethyl-bicylcohexyl-1-ol
• 2-(4-chlorobenzylidene)-6-dimethylaminomethyl-1-m-tolyl-cyclohexanol
• 2-(4-chlorobenzylidene)-1-cyclopentyl-6-dimethylamino-methyl-cyclohexanol
• 1-(4-tert.-butylphenyl)-2-(4-chlorobenzylidene-6-dimethyl-aminomethyl-cyclohexanol
• 2-(4-chlorobenzylidene)-6-dimethylaminomethyl-1-vinyl-cyclohexanol
• 2-(4-chlorobenzylidene)-6-dimethylaminomethyl-1-o-tolyl-cyclohexanol
• 2-(4-chlorobenzylidene)-6-dimethylaminomethyl-1-(4-fluoro-3-methylphenyl)-cyclohexanol
• 1-benzyl-2-(4-chlorobenzylidene)-6-dimethylaminomethyl-cyclohexanol
• 2-(4-chlorobenzylidene)-1-(4-chlorophenyl)-6-dimethyl-aminomethyl-cyclohexanol
• 2-(4-chlorobenzylidene)-6-dimethylaminomethyl-1-phenyl-cyclohexanol
• 1-(3-chlorobenzyl)-2-dimethylaminomethyl-6-(4-methoxy-benzylidene)-cyclohexanol
• 2-dimethylaminomethyl-1-(2,5-dimethylbenzyl)-6-(4-methoxybenzylidene)-cyclohexanol
• 1-(2-chloro-6-fluorobenzyl)-2-dimethylaminomethyl-6-(4-methoxybenzyl-idene)-cyclohexanol
• 2-dimethylaminomethyl-6-(4-methoxybenzylidene)-1-(3-methylbenzyl)-cyclohexanol
• 2-dimethylaminomethyl-6-(4-methoxybenzylidene)-1-(3-trifluoromethylphenyl)-cyclohexanol
• 1-(3-chloro-4-fluorophenyl)-2-dimethylaminomethyl-6-(4-methoxybenzyl-idene)-cyclohexanol
• 2-dimethylaminomethyl-6-(4-methoxybenzylidene)-1-(2-methoxyphenyl)-cyclohexanol
• 1-(4-chloro-3-trifluoromethylphenyl)-2-dimethylamino-methyl-6-(4-methoxybenzylidene)-cyclohexanol
• 2-dimethylaminomethyl-1-(4-fluorobenzyl)-6-(4-methoxy-benzylidene)-cyclohexanol
• 1-(2-chlorobenzyl)-2-dimethylaminomethyl-6-(4-methoxy-benzylidene)-cyclohexanol
• 1-(3,5-dichlorophenyl)-2-dimethylaminomethyl-6-(4-methoxybenzylidene)-cyclohexanol
• 1-(3-chlorophenyl)-2-dimethylaminomethyl-6-(4-methoxy-benzylidene)-cyclohexanol
• 2-dimethylaminomethyl-1-(5-fluoro-2-methoxyphenyl)-6-(4-methoxybenzyl-idene)-cyclohexanol
• 1-cyclohexylmethyl-2-dimethylaminomethyl-6-(4-methoxy-benzylidene)-cyclohexanol
• 2-dimethylaminomethyl-6-(4-methoxybenzylidene)-1-(4-methoxyphenyl)-cyclohexanol
• 2-dimethylaminomethyl-6-(4-methoxybenzylidene)-1-p-tolyl-cyclohexanol
• 1-(2,3-dichlorophenyl)-2-dimethylaminomethyl-6-(4-methoxybenzylidene)-cyclohexanol
• 2-dimethylaminomethyl-6-(4-methoxybenzylidene)-1-(3-phenylpropyl)-cyclohexanol
• 2-dimethylaminomethyl-6-(4-methoxybenzylidene)-1-(3-methoxyphenyl)-cyclohexanol
• 2-dimethylaminomethyl-6-(4-methoxybenzylidene)-1-thiophen-2-yl-cyclohexanol
• 2-dimethylaminomethyl-6-(4-methoxybenzylidene)-1-phenylethynyl-cyclohexanol
• 2-dimethylaminomethyl-6-(4-methoxybenzylidene)-1-phenethyl-cyclohexanol
• 2-dimethylaminomethyl-1-(4-fluorophenyl)-6-(4-methoxy-benzylidene)-cyclohexanol
• 6-dimethylaminomethyl-2-(4-methoxybenzylidene)-bicyclohexyl-1-ol
• 2-dimethylaminomethyl-6-(4-methoxybenzylidene)-1-m-tolyl-cyclohexanol
• 1-cyclopentyl-2-dimethylaminomethyl-6-(4-methoxy-benzylidene)-cyclohexanol
• 1-(4-tert.-butylphenyl)-2-dimethylaminomethyl-6-(4-methoxybenzylidene)-cyclohexanol
• 2-dimethylaminomethyl-6-(4-methoxybenzylidene)-1-vinyl-cyclohexanol
• 2-dimethylaminomethyl-6-(4-methoxybenzylidene)-1-o-tolyl-cyclohexanol
• 2-dimethylaminomethyl-1-(4-fluoro-3-methylphenyl)-6-(4-methoxy-benzylidene)-cyclohexanol
• 1-benzyl-2-dimethylaminomethyl-6-(4-methoxybenzylidene)-cyclohexanol
• 1-(4-chlorophenyl)-2-dimethylaminomethyl-6-(4-methoxy-benzylidene)-cyclohexanol
• 2-dimethylaminomethyl-6-(4-methoxybenzylidene)-1-phenyl-cyclohexanol
• 1-(3-chlorobenzyl)-2-dimethylaminomethyl-6-(3-methoxy-benzylidene)-cyclohexanol
• 2-dimethylaminomethyl-1-(2,5-dimethylbenzyl)-6-(3-methoxybenzylidene)-cyclohexanol
• 2-dimethylaminomethyl-6-(3-methoxybenzylidene)-1-(2-methylbenzyl)-cyclohexanol
• 2-dimethylaminomethyl-6-(3-methoxybenzylidene)-1-(2-methoxyphenyl)-cyclohexanol
• 1-(4-chloro-3-trifluoromethylphenyl)-2-dimethylamino-methyl-6-(3-methoxybenzylidene)-cyclohexanol
• 1-(2-chlorobenzyl)-2-dimethylaminomethyl-6-(3-methoxybenzylidene)-cyclohexanol
• 6-dimethylaminomethyl-2-(3-methoxybenzylidene)-bicyclohexyl-1-ol
• 1-cyclopentyl-2-dimethylaminomethyl-6-(3-methoxybenzylidene)-cyclohexanol
• 2-dimethylaminomethyl-6-(3-methoxybenzylidene)-1-vinyl-cyclohexanol
• 2-dimethylaminomethyl-1-(4-fluoro-3-methylphenyl)-6-(3-methoxy-benzylidene)-cyclohexanol
• 1-benzyl-2-dimethylaminomethyl-6-(3-methoxybenzylidene)-cyclohexanol
• 2-dimethylaminomethyl-6-(3-methoxybenzylidene)-1-phenyl-cyclohexanol
• 1-benzyl-2-(2-chlorobenzylidene)-6-dimethylaminomethyl-cyclohexanol
• 5-dimethylaminomethyl-2,4-dimethyl-1,3-diphenyl-pent-1-en-3-ol
• 3-(4-chlorophenyl)-5-dimethylamino-2,4-dimethyl-1-phenyl-pent-1-en-3-ol
• 3-benzyl-5-dimethylamino-2,4-dimethyl-1-phenyl-pent-1-en-3-ol
• 5-dimethylamino-3-(4-fluoro-3-methylphenyl)-2,4-dimethyl-1-phenyl-pent-1-en-3-ol
• 5-dimethylamino-2,4-dimethyl-1-phenyl-3-o-tolyl-pent-1-en-3-ol
• 3-(2-d imethylamino-1-methylethyl)-2-methyl-1-phenylpenta-1,4-dien-3-ol
• 3-(4-tert.-butylphenyl)-5-dimethylamino-2,4-dimethyl-1-phenyl-pent-1-en-3-ol
• 3-cyclopentyl-5-dimethylamino-2,4-dimethyl-1-phenyl-pent-1-en-3-ol
• 5-dimethylamino-2,4-dimethyl-1-phenyl-3-m-tolyl-pent-1-en-3-ol
• 3-cyclohexyl-5-dimethylamino-2,4-dimethyl-1-phenyl-pent-1-en-3-ol
• 5-dimethylamino-3-(4-fluorophenyl)-2,4-dimethyl-1-phenyl-pent-1-en-3-ol
• 5-dimethylamino-2,4-dimethyl-3-phenethyl-1-phenyl-pent-1-en-3-ol
• 3-(2-dimethylamino-1-methylethyl)-2-methyl-1,5-d iphenyl-pent-1-en-4-yn-3-ol
• 5-dimethylamino-2,4-dimethyl-1-phenyl-3-thiophen-2-yl-pent-1-en-3-ol
• 3-(2,4-dichlorophenyl)-5-dimethylamino-2,4-dimethyl-1-phenyl-pent-1-en-3-ol
• 5-dimethylamino-3-(3-methoxyphenyl)-2,4-dimethyl-1-phenyl-pent-1-en-3-ol
• 3-(2-dimethylamino-1-methylethyl)-2-methyl-1,6-diphenylhex-1-en-3-ol
• 3-(2,3-dichlorophenyl)-5-dimethylamino-2,4-dimethyl-1-phenyl-pent-1-en-3-ol
• 5-dimethylamino-2,4-dimethyl-1-phenyl-3-p-tolyl-pent-1-en-3-ol
• 5-dimethylamino-3-(4-methoxyphenyl)-2,4-dimethyl-1-phenyl-pent-1-en-3-ol
• 3-cyclohexylmethyl-5-dimethylamino-2,4-d imethyl-1-phenyl-pent-1-en-3-ol
• 5-dimethylamino-3-(5-fluoro-2-methoxyphenyl)-2,4-dimethyl-1-phenyl-pent-1-en-3-ol
• 5-dimethylamino-3-(3-fluorophenyl)-2,4-dimethyl-1-phenyl-pent-1-en-3-ol
• 3-(3-chlorophenyl)-5-dimethylamino-2,4-dimethyl-1-phenyl-pent-1-en-3-ol
• 3-(3,5-chlorophenyl)-5-dimethylamino-2,4-dimethyl-1-phenyl-pent-1-en-3-ol
• 3-(2-chlorobenzyl)-5-dimethylamino-2,4-dimethyl-1-phenyl-pent-1-en-3-ol
• 5-dimethylamino-3-(4-fluorobenzyl)-2,4-dimethyl-1-phenyl-pent-1-en-3-ol
• 3-(4-chloro-3-trifluoromethylphenyl)-5-dimethylamino-2,4-dimethyl-1-phenyl-pent-1-en-3-ol
• 5-dimethylamino-3-(2-methoxyphenyl)-2,4-dimethyl-1-phenyl-pent-1-en-3-ol
• 5-dimethylamino-2,4-dimethyl-3-(2-methylbenzyl)-1-phenyl-pent-1-en-3-ol
• 3-(3-chloro-4-fluorophenyl)-5-dimethylamino-2,4-dimethyl-1-phenyl-pent-1-en-3-ol
• 5-dimethylamino-2,4-dimethyl-1-phenyl-3-(3-trifluoromethylphenyl)-pent-1-en-3-ol
• 5-dimethylamino-2,4-dimethyl-3-(3-methylbenzyl)-1-phenyl-pent-1-en-3-ol
• 3-(4-chlorobenzyl)-5-dimethylamino-2,4-dimethyl-1-phenyl-pent-1-en-3-ol
• 3-(2-chloro-6-fluorobenzyl)-5-dimethylamino-2,4-dimethyl-1-phenyl-pent-1-en-3-ol
• 5-dimethylamino-3-(2,5-dimethylbenzyl)-2,4-dimethyl-1-phenyl-pent-1-en-3-ol
• 3-(3-chlorobenzyl)-5-dimethylamino-2,4-dimethyl-1-phenyl-pent-1-en-3-ol
• 3-(2,4-dichlorobenzyl)-5-dimethylamino-2,4-dimethyl-1-phenyl-pent-1-en-3-ol
• 5-dimethylamino-1-(4-fluorophenyl)-2,4-dimethyl-3-phenyl-pent-1-en-3-ol
• 3-benzyl-5-dimethylamino-1-(4-fluorophenyl)-2,4-dimethyl-pent-1-en-3-ol
• 5-dimethylamino-3-(4-fluoro-3-methylphenyl)-1-(4-fluoro-phenyl)-2,4-dimethyl-pent-1-en-3-ol
• 5-dimethylamino-1-(4-fluorophenyl)-2,4-dimethyl-3-o-tolyl-pent-1-en-3-ol
• 3-(2-dimethylamino-1-methylethyl)-1-(4-fluorophenyl)-2-methyl-pent-1,4-dien-3-ol
• 3-(4-tert.-butylphenyl)-5-dimethylamino-1-(4-fluorophenyl)-2,4-dimethyl-pent-1-en-3-ol
• 3-cyclopentyl-5-dimethylamino-1-(4-fluorophenyl)-2,4-dimethyl-pent-1-en-3-ol
• 5-dimethylamino-1-(4-fluorophenyl)-2,4-dimethyl-3-m-tolyl-pent-1-en-3-ol
• 3-cyclohexyl-5-dimethylamino-1-(4-fluorophenyl)-2,4-dimethyl-pent-1-en-3-ol
• 5-dimethylamino-1,3-bis-(4-fluorophenyl)-2,4-dimethyl-pent-1-en-3-ol
• 5-dimethylamino-1-(4-fluorophenyl)-2,4-dimethyl-3-phenethyl-pent-1-en-3-ol
• 3-(2-dimethylamino-1-methylethyl)-1-(4-fluorophenyl)-2-methyl-5-phenyl-pent-1-en-4-yn-3-ol
• 5-dimethylamino-1-(4-fluorophenyl)-2,4-dimethyl-3-thiophen-2-yl-pent-1-en-3-ol
• 3-(2,4-dichlorophenyl)-5-dimethylamino-1-(4-fluorophenyl)-2,4-dimethyl-pent-1-en-3-ol
• 5-dimethylamino-1-(4-fluorophenyl)-3-(3-methoxyphenyl)-2,4-dimethyl-pent-1-en-3-ol
• 3-(2-dimethylamino-1-methylethyl)-1-(4-fluorophenyl)-2-methyl-6-phenyl-hex-1-en-3-ol
• 3-(2,3-dichlorophenyl)-5-dimethylamino-1-(4-fluorophenyl)-2,4-dimethyl-pent-1-en-3-ol
• 5-dimethylamino-1-(4-fluorophenyl)-2,4-dimethyl-3-p-tolyl-pent-1-en-3-ol
• 5-dimethylamino-1-(4-fluorophenyl)-3-(4-methoxyphenyl)-2,4-dimethyl-pent-1-en-3-ol
• 3-cyclohexylmethyl-5-dimethylamino-1-(4-fluorophenyl)-2,4-dimethyl-pent-1-en-3-ol
• 5-dimethylamino-3-(5-fluoro-2-methoxyphenyl)-1-(4-fluorophenyl)-2,4-dimethyl-pent-1-en-3-ol
• 5-dimethylamino-3-(3-fluorophenyl)-1-(4-fluorophenyl)-2,4-dimethyl-pent-1-en-3-ol
• 3-(3-chlorophenyl)-5-dimethylamino-1-(4-fluorophenyl)-2,4-dimethyl-pent-1-en-3-ol
• 3-(3,5-dichlorophenyl)-5-dimethylamino-1-(4-fluorophenyl)-2,4-dimethyl-pent-1-en-3-ol
• 3-(2-chlorobenzyl)-5-dimethylamino-1-(4-fluorophenyl)-2,4-dimethyl-pent-1-en-3-ol
• 5-dimethylamino-3-(4-fluorobenzyl)-1-(4-fluorophenyl)-2,4-dimethyl-pent-1-en-3-ol
• 5-dimethylamino-1-(4-fluorophenyl)-3-(3-methoxybenzyl)-2,4-dimethyl-pent-1-en-3-ol
• 3-(4-chloro-3-trifluoromethylphenyl)-5-dimethylamino-1-(4-fluorophenyl)-2,4-dimethyl-pent-1-en-3-ol
• 5-dimethylamino-3-(3-fluorobenzyl)-1-(4-fluorophenyl)-2,4-dimethyl-pent-1-en-3-ol
• 5-dimethylamino-1-(4-fluorophenyl)-3-(2-methoxyphenyl)-2,4-dimethyl-pent-1-en-3-ol
• 5-dimethylamino-1-(4-fluorophenyl)-2,4-dimethyl-3-(2-methylbenzyl)-pent-1-en-3-ol
• 3-(3-chloro-4-fluorophenyl)-5-dimethylamino-1-(4-fluorophenyl)-2,4-dimethyl-pent-1-en-3-ol
• 5-dimethylamino-1-(4-fluorophenyl)-2,4-dimethyl-3-(3-trifluoromethylphenyl)-pent-1-en-3-ol
• 5-dimethylamino-1-(4-fluorophenyl)-2,4-dimethyl-3-(3-methylbenzyl)-pent-1-en-3-ol
• 3-(4-chlorobenzyl)-5-dimethylamino-1-(4-fluorophenyl)-2,4-dimethyl-pent-1-en-3-ol
• 3-(2-chloro-6-fluorobenzyl)-5-dimethylamino-1-(4-fluoro-phenyl)-2,4-dimethyl-pent-1-en-3-ol
• 5-dimethylamino-3-(2,5-dimethylbenzyl)-1-(4-fluorophenyl)-2,4-dimethyl-pent-1-en-3-ol
• 3-(3-chlorobenzyl)-5-dimethylamino-1-(4-fluorophenyl)-2,4-dimethyl-pent-1-en-3-ol
• 3-(2,4-dichlorobenzyl)-5-dimethylamino-1-(4-fluorophenyl)-2,4-dimethyl-pent-1-en-3-ol
• 1-(4-chlorophenyl)-5-dimethylamino-2,4-dimethyl-3-phenyl-pent-1-en-3-ol
• 1,3-bis-(4-chlorophenyl)-5-dimethylamino-2,4-dimethyl-pent-1-en-3-ol
• 3-benzyl-1-(4-chlorophenyl)-5-dimethylamino-2,4-dimethyl-pent-1-en-3-ol
• 1-(4-chlorophenyl)-5-dimethylamino-3-(4-fluoro-3-methyl-phenyl)-2,4-dimethyl-pent-1-en-3-ol
• 1-(4-chlorophenyl)-5-dimethylamino-2,4-dimethyl-3-o-tolyl-pent-1-en-3-ol
• 1-(4-chlorophenyl)-3-(2-dimethylamino-1-methylethyl)-2-methyl-penta-1,4-dien-3-ol
• 3-(4-tert.-butylphenyl)-1-(4-chlorophenyl)-5-dimethylamino-2,4-dimethyl-pent-1-en-3-ol
• 1-(4-chlorophenyl)-3-cyclopentyl-5-dimethylamino-2,4-dimethyl-pent-1-en-3-ol
• 1-(4-chlorophenyl)-5-dimethylamino-2,4-dimethyl-3-m-tolyl-pent-1-en-3-ol
• 1-(4-chlorophenyl)-3-cyclohexyl-5-dimethylamino-2,4-dimethyl-pent-1-en-3-ol
• 1-(4-chlorophenyl)-5-dimethylamino-3-(4-fluorophenyl)-2,4-dimethyl-pent-1-en-3-ol
• 1-(4-chlorophenyl)-5-dimethylamino-2,4-dimethyl-3-phenethyl-pent-1-en-3-ol
• 1-(4-chlorophenyl)-5-dimethylamino-2,4-dimethyl-3-thiophen-2-yl-pent-1-en-3-ol
• 1-(4-chlorophenyl)-3-(2,4-dichlorophenyl)-5-dimethylamino-2,4-dimethyl-pent-1-en-3-ol
• 1-(4-chlorophenyl)-5-dimethylamino-3-(3-methoxyphenyl)-2,4-dimethyl-pent-1-en-3-ol
• 1-(4-chlorophenyl)-3-(2-dimethylamino-1-methylethyl)-2-methyl-6-phenyl-hex-1-en-3-ol
• 1-(4-chlorophenyl)-3-(2,3-dichlorophenyl)-5-dimethylamino-2,4-dimethyl-pent-1-en-3-ol
• 1-(4-chlorophenyl)-5-dimethylamino-2,4-dimethyl-3-p-tolyl-pent-1-en-3-ol
• 1-(4-chlorophenyl)-5-dimethylamino-3-(4-methoxyphenyl)-2,4-dimethyl-pent-1-en-3-ol
• 1-(4-chlorophenyl)-3-cyclohexylmethyl-5-dimethylamino-2,4-dimethyl-pent-1-en-3-ol
• 1-(4-chlorophenyl)-5-dimethylamino-3-(5-fluoro-2-methoxyphenyl)-2,4-dimethyl-pent-1-en-3-ol
• 1-(4-chlorophenyl)-5-dimethylamino-3-(3-fluorophenyl)-2,4-dimethyl-pent-1-en-3-ol
• 3-(3-chlorophenyl)-1-(4-chlorophenyl)-5-dimethylamino-2,4-dimethyl-pent-1-en-3-ol
• 1-(4-chlorophenyl)-3-(3,5-dichlorophenyl)-5-dimethylamino-2,4-dimethyl-pent-1-en-3-ol
• 3-(2-chlorobenzyl)-1-(4-chlorophenyl)-5-dimethylamino-2,4-dimethyl-pent-1-en-3-ol
• 1-(4-chlorophenyl)-5-dimethylamino-3-(4-fluorobenzyl)-2,4-dimethyl-pent-1-en-3-ol
• 1-(4-chlorophenyl)-5-dimethylamino-3-(3-methoxybenzyl)-2,4-dimethyl-pent-1-en-3-ol
• 1-(4-chlorophenyl)-3-(4-chloro-3-trifluoromethylphenyl)-5-dimethylamino-2,4-dimethyl-pent-1-en-3-ol
• 1-(4-chlorophenyl)-5-dimethylamino-3-[2-(3-fluorophenyl)-ethyl]-2,4-dimethyl-pent-1-en-3-ol
• 1-(4-chlorophenyl)-5-dimethylamino-3-(2-methoxyphenyl)-2,4-dimethyl-pent-1-en-3-ol
• 1-(4-chlorophenyl)-5-dimethylamino-2,4-dimethyl-3-(2-methylbenzyl)-pent-1-en-3-ol
• 3-(3-chloro-4-fluorophenyl)-1-(4-chlorophenyl)-5-dimethyl-amino-2,4-dimethyl-pent-1-en-3-ol
• 1-(4-chlorophenyl)-5-dimethylamino-2,4-dimethyl-3-(3-trifluoromethylphenyl)-pent-1-en-3-ol
• 1-(4-chlorophenyl)-5-dimethylamino-2,4-dimethyl-3-(3-methylbenzyl)-pent-1-en-3-ol
• 3-(4-chlorobenzyl)-1-(4-chlorophenyl)-5-dimethylamino-2,4-dimethyl-pent-1-en-3-ol
• 3-(2-chloro-6-fluorobenzyl)-1-(4-chlorophenyl)-5-dimethyl-amino-2,4-dimethyl-pent-1-en-3-ol
• 1-(4-chlorophenyl)-5-dimethylamino-3-(2,5-dimethylbenzyl)-2,4-dimethyl-pent-1-en-3-ol
• 3-(3-chlorobenzyl)-1-(4-chlorophenyl)-5-dimethylamino-2,4-dimethyl-pent-1-en-3-ol
• 1-(4-chlorophenyl)-3-(2,4-dichlorobenzyl)-5-dimethylamino-2,4-dimethyl-pent-1-en-3-ol
• 5-dimethylamino-1-(4-methoxyphenyl)-2,4-dimethyl-3-phenyl-pent-1-en-3-ol
• 3-(4-chlorophenyl)-5-dimethylamino-1-(4-methoxyphenyl)-2,4-dimethyl-pent-1-en-3-ol
• 3-benzyl-5-dimethylamino-1-(4-methoxyphenyl)-2,4-dimethyl-pent-1-en-3-ol
• 5-dimethylamino-3-(4-fluoro-3-methylphenyl)-1-(4-methoxyphenyl)-2,4-dimethyl-pent-1-en-3-ol
• 5-dimethylamino-1-(4-methoxyphenyl)-2,4-dimethyl-3-o-tolyl-pent-1-en-3-ol
• 3-(2-dimethylamino-1-methylethyl)-1-(4-methoxyphenyl)-2-methyl-penta-1,4-dien-3-ol
• 3-(4-tert.-butylphenyl)-5-dimethylamino-1-(4-methoxy-phenyl)-2,4-dimethyl-pent-1-en-3-ol
• 3-cyclopentyl-5-dimethylamino-1-(4-methoxyphenyl)-2,4-dimethyl-pent-1-en-3-ol
• 5-dimethylamino-1-(4-methoxyphenyl)-2,4-dimethyl-3-m-tolyl-pent-1-en-3-ol
• 3-cyclohexyl-5-dimethylamino-1-(4-methoxyphenyl)-2,4-dimethyl-pent-1-en-3-ol
• 5-dimethylamino-3-(4-fluorophenyl)-1-(4-methoxyphenyl)-2,4-dimethyl-pent-1-en-3-ol
• 5-dimethylamino-1-(4-methoxyphenyl)-2,4-dimethyl-3-phenethyl-pent-1-en-3-ol
• 3-(2-dimethylamino-1-methylethyl)-1-(4-methoxyphenyl)-2-methyl-5-phenyl-pent-1-en-4-yn-3-ol
• 5-dimethylamino-1-(4-methoxyphenyl)-2,4-dimethyl-3-thiophen-2-yl-pent-1-en-3-ol
• 3-(2,4-dichlorophenyl)-5-dimethylamino-1-(4-methoxy-phenyl)-2,4-dimethyl-pent-1-en-3-ol
• 5-dimethylamino-3-(3-methoxyphenyl)-1-(4-methoxyphenyl)-2,4-dimethyl-pent-1-en-3-ol
• 3-(2-dimethylamino-1-methylethyl)-1-(4-methoxyphenyl)-2-methyl-6-phenyl-hex-1-en-3-ol
• 3-(2,3-dichlorophenyl)-5-dimethylamino-1-(4-methoxy-phenyl)-2,4-dimethyl-pent-1-en-3-ol
• 5-dimethylamino-1-(4-methoxyphenyl)-2,4-dimethyl-3-p-tolyl-pent-1-en-3-ol
• 5-dimethylamino-1,3-bis-(4-methoxyphenyl)-2,4-dimethyl-pent-1-en-3-ol
• 3-cyclohexylmethyl-5-dimethylamino-1-(4-methoxyphenyl)-2,4-dimethyl-pent-1-en-3-ol
• 5-dimethylamino-3-(5-fluoro-2-methoxyphenyl)-1-(4-methoxyphenyl)-2,4-dimethyl-pent-1-en-3-ol
• 5-dimethylamino-3-(3-fluorophenyl)-1-(4-methoxyphenyl)-2,4-dimethyl-pent-1-en-3-ol
• 3-(3-chlorophenyl)-5-dimethylamino-1-(4-methoxyphenyl)-2,4-dimethyl-pent-1-en-3-ol
• 3-(3,5-dichlorophenyl)-5-dimethylamino-1-(4-methoxyphenyl)-2,4-dimethyl-pent-1-en-3-ol
• 3-(2-chlorobenzyl)-5-dimethylamino-1-(4-methoxyphenyl)-2,4-dimethyl-pent-1-en-3-ol
• 5-dimethylamino-3-(4-fluorobenzyl)-1-(4-methoxyphenyl)-2,4-dimethyl-pent-1-en-3-ol
• 5-dimethylamino-3-(3-fluorobenzyl)-1-(4-methoxyphenyl)-2,4-dimethyl-pent-1-en-3-ol
• 5-dimethylamino-3-(2-methoxyphenyl)-1-(4-methoxyphenyl)-2,4-dimethyl-pent-1-en-3-ol
• 5-dimethylamino-1-(4-methoxyphenyl)-2,4-dimethyl-3-(2-methylbenzyl)-pent-1-en-3-ol
• 3-(3-chloro-4-fluorophenyl)-5-dimethylamino-1-(4-methoxy-phenyl)-2,4-dimethyl-pent-1-en-3-ol
• 5-dimethylamino-1-(4-methoxyphenyl)-2,4-dimethyl-3-(3-trifluoromethylphenyl)-pent-1-en-3-ol
• 3-(4-chlorobenzyl)-5-dimethylamino-1-(4-methoxyphenyl)-2,4-dimethyl-pent-1-en-3-ol
• 3-(2-chloro-6-fluorobenzyl)-5-dimethylamino-1-(4-methoxy-phenyl)-2,4-dimethyl-pent-1-en-3-ol
• 3-(3-chlorobenzyl)-5-dimethylamino-1-(4-methoxyphenyl)-2,4-dimethyl-pent-1-en-3-ol
• 2-benzylidene-1-(4-tert.-butylphenyl)-5-dimethylaminomethyl-cyclopentanol
• 2-benzylidene-1-cyclohexyl-5-dimethylaminomethyl-cyclopentanol
• 2-benzylidene-5-dimethylaminomethyl-1-phenethyl-cyclopentanol
• 2-benzylidene-5-dimethylaminomethyl-1-(4-fluorobenzyl)-cyclopentanol
• 2-benzylidene-5-dimethylaminomethyl-1-(3-fluorobenzyl)-cyclopentanol
• 2-benzylidene-5-dimethylaminomethyl-1-(2-methoxyphenyl)-cyclopentanol
• 2-benzylidene-5-dimethylaminomethyl-1-(2-methylbenzyl)-cyclopentanol
• 2-benzylidene-5-dimethylaminomethyl-1-(3-methylbenzyl)-cyclopentanol
• 2-benzylidene-1-(4-chlorobenzyl)-5-dimethylaminomethyl-cyclopentanol
• 2-benzylidene-1-(2-chloro-6-fluorobenzyl)-5-dimethylamino-methyl-cyclopentanol
• 2-benzylidene-5-dimethylaminomethyl-1-(2,5-dimethyl-benzyl)-cyclo-pentanol
• 2-benzylidene-1-(3-chlorobenzyl)-5-dimethylaminomethyl-cyclopentanol
• 2-dimethylaminomethyl-5-(3-methoxybenzyl)-bicyclopentyl-1-ol
• 2-dimethylaminomethyl-5-(3-methoxybenzyl)-1-m-tolyl-cyclopentanol
• 1-cyclohexyl-2-dimethylaminomethyl-5-(3-methoxybenzyl)-cyclopentanol
• 2-dimethylaminomethyl-5-(3-methoxybenzyl)-1-phenethyl-cyclopentanol
• 2-dimethylaminomethyl-5-(3-methoxybenzyl)-1-phenyl-ethynyl-cyclo-pentanol
• 2-dimethylaminomethyl-5-(3-methoxybenzyl)-1-thiophen-2-yl-cyclo-pentanol
• 2-dimethylaminomethyl-5-(3-methoxybenzyl)-1-(3-methoxy-phenyl)-cyclopentanol
• 2-dimethylaminomethyl-5-(3-methoxybenzyl)-1-(3-phenyl-propyl)-cyclopentanol
• 2-dimethylaminomethyl-5-(3-methoxybenzyl)-1-(4-methoxy-phenyl)-cyclopentanol
• 2-dimethylaminomethyl-1-(3-fluorobenzyl)-5-(3-methoxy-benzylidene)-cyclopentanol
• 2-dimethylaminomethyl-5-(3-methoxybenzylidene)-1-(2-methoxyphenyl)-cyclopentanol
• 2-dimethylaminomethyl-5-(3-methoxybenzylidene)-1-(2-methylbenzyl)-cyclopentanol
• 2-dimethylaminomethyl-5-(3-methoxybenzylidene)-1-(3-methylbenzyl)-cyclopentanol
• 2-dimethylaminomethyl-1-(2,5-dimethylbenzyl)-5-(3-methoxybenzylidene)-cyclopentanol
• 1-(3-chlorobenzyl)-2-dimethylaminomethyl-5-(3-methoxybenzylidene)-cyclopentanol
• 1-benzyl-2-dimethylaminomethyl-5-(3-methoxybenzylidene)-cyclopentanol
• 2-dimethylaminomethyl-5-(3-methoxybenzylidene)-1-o-tolyl-cyclopentanol
• 2-dimethylaminomethyl-5-(3-methoxybenzylidene)-1-vinyl-cyclopentanol
• 1-(4-tert.-butylphenyl)-2-dimethylaminomethyl-5-(3-methoxybenzylidene)-cyclopentanol
• 2-dimethylaminomethyl-5-(3-methoxybenzylidene)-bicyclopentyl-1-ol
• 1-cyclohexyl-2-dimethylaminomethyl-5-(3-methoxy-benzylidene)-cyclopentanol
• 2-dimethylaminomethyl-1-(4-fluorophenyl)-5-(3-methoxy-benzylidene)-cyclopentanol
• 2-dimethylaminomethyl-5-(3-methoxybenzylidene)-1-phenethyl-cyclopentanol
• 2-dimethylaminomethyl-5-(3-methoxybenzylidene)-1-phenylethynyl-cyclopentanol
• 2-dimethylaminomethyl-5-(3-methoxybenzylidene)-1-thiophen-2-yl-cyclopentanol
• 2-dimethylaminomethyl-5-(3-methoxybenzylidene)-1-(3-methoxyphenyl)-cyclopentanol
• 2-dimethylaminomethyl-5-(3-methoxybenzylidene)-1-(3-phenylpropyl)-cyclopentanol
• 2-dimethylaminomethyl-5-(3-methoxybenzylidene)-1-p-tolyl-cyclopentanol
• 2-dimethylaminomethyl-5-(3-methoxybenzylidene)-1-(4-methoxyphenyl)-cyclopentanol
• 1-(2-chlorobenzyl)-2-dimethylaminomethyl-5-(4-methoxy-benzylidene)-cyclopentanol
• 2-dimethylaminomethyl-1-(3-methoxybenzyl)-5-(4-methoxy-benzylidene)-cyclopentanol
• 2-dimethylaminomethyl-1-(3-fluorobenzyl)-5-(4-methoxy-benzylidene)-cyclopentanol
• 2-dimethylaminomethyl-5-(4-methoxybenzylidene)-1-(2-methoxyphenyl)-cyclopentanol
• 2-dimethylaminomethyl-5-(4-methoxybenzylidene)-1-(3-methylbenzyl)-cyclopentanol
• 1-(4-chlorobenzyl)-2-dimethylaminomethyl-5-(4-methoxy-benzylidene)-cyclopentanol
• 1-(2-chloro-6-fluorobenzyl)-2-dimethylaminomethyl-5-(4-methoxy-benzylidene)-cyclopentanol
• 1-(3-chlorobenzyl)-2-dimethylaminomethyl-5-(4-methoxy-benzylidene)-cyclopentanol
• 1-(2,4-dichlorobenzyl)-2-dimethylaminomethyl-5-(4-methoxybenzylidene)-cyclopentanol
• 2-benzylidene-7-dimethylaminomethyl-1-phenyl-cycloheptanol
• 2-benzylidene-1-(4-chlorophenyl)-7-dimethylaminomethyl-cycloheptanol
• 1-benzyl-2-benzylidene-7-dimethylaminomethyl-cycloheptanol
• 2-benzylidene-7-dimethylaminomethyl-1-(4-fluoro-3-methylphenyl)-cycloheptanol
• 2-benzylidene-7-dimethylaminomethyl-1-o-tolyl-cycloheptanol
• 2-benzylidene-7-dimethylaminomethyl-1-vinyl-cycloheptanol
• 2-benzylidene-1-(4-tert.-butylphenyl)-7-dimethylamino-methyl-cycloheptanol
• 2-benzylidene-1-cyclopentyl-7-dimethylaminomethyl-cycloheptanol
• 2-benzylidene-7-dimethylaminomethyl-1-m-tolyl-cycloheptanol
• 2-benzylidene-1-cyclohexyl-7-dimethylaminomethyl-cycloheptanol
• 2-benzylidene-7-dimethylaminomethyl-1-(4-fluorophenyl)-cycloheptanol
• 2-benzylidene-7-dimethylaminomethyl-1-phenylethynyl-cycloheptanol
• 2-benzylidene-7-dimethylaminomethyl-1-thiophen-2-yl-cycloheptanol
• 2-benzylidene-7-dimethylaminomethyl-1-(3-methoxyphenyl)-cycloheptanol
• 2-benzylidene-1-cyclohexylmethyl-7-dimethylaminomethyl-cycloheptanol
• 2-benzylidene-7-dimethylaminomethyl-1-(3-fluoro-4-methoxyphenyl)-cycloheptanol
• 2-benzylidene-7-dimethylaminomethyl-1-(3-fluorophenyl)-cycloheptanol
• 2-benzylidene-1-(3-chlorophenyl)-7-dimethylaminomethyl-cycloheptanol
• 2-benzylidene-1-(3,5-dichlorophenyl)-7-dimethylamino-methyl-cyclo-heptanol
• 2-benzylidene-7-dimethylaminomethyl-1-(4-fluorobenzyl)-cycloheptanol
• 2-benzylidene-7-dimethylaminomethyl-1-(4-methoxybenzyl)-cycloheptanol
• 2-benzylidene-7-dimethylaminomethyl-1-(3-fluorobenzyl)-cycloheptanol
• 2-benzylidene-7-dimethylaminomethyl-1-(2-methoxyphenyl)-cycloheptanol
• 2-benzylidene-7-dimethylaminomethyl-1-(2-methylbenzyl)-cycloheptanol
• 2-benzylidene-1-(3-chloro-4-fluorophenyl)-7-dimethylamino-methyl-cyclo-heptanol
• 2-benzylidene-7-dimethylaminomethyl-1-(3-trifluoromethyl-phenyl)-cycloheptanol
• 2-benzylidene-7-dimethylaminomethyl-1-(3-methylbenzyl)-cycloheptanol
• 2-benzylidene-1-(4-chlorobenzyl)-7-dimethylaminomethyl-cycloheptanol
• 2-benzylidene-1-(2-chloro-6-fluorobenzyl)-7-dimethylamino-methyl-cyclo-heptanol
• 2-benzylidene-7-dimethylaminomethyl-1-(2,5-dimethylbenzyl)-cyclo-heptanol
• 2-benzylidene-1-(3-chlorobenzyl)-7-dimethylaminomethyl-cycloheptanol
• 2-dimethylaminomethyl-7-(3-methoxybenzylidene)-1-phenyl-cycloheptanol
• 1-(4-chlorophenyl)-2-dimethylaminomethyl-7-(3-methoxy-benzylidene)-cycloheptanol
• 1-benzyl-2-dimethylaminomethyl-7-(3-methoxybenzylidene)-cycloheptanol
• 2-dimethylaminomethyl-1-(4-fluoro-3-methylphenyl)-7-(3-methoxy-benzylidene)-cycloheptanol
• 2-dimethylaminomethyl-7-(3-methoxybenzylidene)-1-o-tolyl-cycloheptanol
• 2-dimethylaminomethyl-7-(3-methoxybenzylidene)-1-vinyl-cycloheptanol
• 1-(4-tert.-butylphenyl)-2-dimethylaminomethyl-7-(3-methoxybenzylidene)-cycloheptanol
• 1-cyclopentyl-2-dimethylaminomethyl-7-(3-methoxy-benzylidene)-cycloheptanol
• 2-dimethylaminomethyl-7-(3-methoxybenzylidene)-1-m-tolyl-cycloheptanol
• 1-cyclohexyl-2-dimethylaminomethyl-7-(3-methoxy-benzylidene)-cycloheptanol
• 2-dimethylaminomethyl-1-(4-fluorophenyl)-7-(3-methoxy-benzylidene)-cycloheptanol
• 2-dimethylaminomethyl-7-(3-methoxybenzylidene)-1-phenethyl-cycloheptanol
• 2-dimethylaminomethyl-7-(3-methoxybenzylidene)-1-phenylethynyl-cycloheptanol
• 2-dimethylaminomethyl-7-(3-methoxybenzylidene)-1-thiophen-2-yl-cycloheptanol
• 2-dimethylaminomethyl-7-(3-methoxybenzylidene)-1-(3-methoxyphenyl)-cycloheptanol
• 2-dimethylaminomethyl-7-(3-methoxybenzylidene)-1-(3-phenylpropyl)-cycloheptanol
• 2-dimethylaminomethyl-7-(3-methoxybenzylidene)-1-p-tolyl-cycloheptanol
• 2-dimethylaminomethyl-7-(3-methoxybenzylidene)-1-(4-methoxyphenyl)-cycloheptanol
• 1-cyclohexylmethyl-2-dimethylaminomethyl-7-(3-methoxy-benzylidene)-cycloheptanol
• 1-(3-chlorophenyl)-2-dimethylaminomethyl-7-(3-methoxy-benzylidene)-cycloheptanol
• 1-(3,5-dichlorophenyl)-2-dimethylaminomethyl-7-(3-methoxybenzylidene)-cycloheptanol
• 1-(2-chlorobenzyl)-2-dimethylaminomethyl-7-(3-methoxy-benzylidene)-cycloheptanol
• 1-(4-chloro-3-trifluoromethylphenyl)-2-dimethylamino-methyl-7-(3-methoxybenzylidene)-cycloheptanol
• 2-dimethylaminomethyl-1-(3-fluorophenyl)-7-(3-methoxybenzylidene)-cycloheptanol
• 2-dimethylaminomethyl-7-(3-methoxybenzylidene)-1-(2-methoxyphenyl)-cycloheptanol
• 2-dimethylaminomethyl-7-(3-methoxybenzylidene)-1-(2-methylbenzyl)-cycloheptanol
• 2-dimethylaminomethyl-7-(3-methoxybenzylidene)-1-(3-trifluoromethylphenyl)-cycloheptanol
• 2-dimethylaminomethyl-7-(3-methoxybenzylidene)-1-(3-methylbenzyl)-cycloheptanol
• 2-dimethylaminomethyl-1-(2,5-dimethylbenzyl)-7-(3-methoxybenzylidene)-cycloheptanol
• 1-(3-chlorobenzyl)-2-dimethylaminomethyl-7-(3-methoxy-benzylidene)-cycloheptanol
• 1-benzyl-2-dimethylaminomethyl-7-(4-methoxybenzylidene)-cycloheptanol
• 2-dimethylaminomethyl-1-(4-fluoro-3-methylphenyl)-7-(4-methoxybenzylidene)-cycloheptanol
• 2-dimethylaminomethyl-7-(4-methoxybenzylidene)-1-o-tolyl-cycloheptanol
• 2-dimethylaminomethyl-7-(4-methoxybenzylidene)-1-vinyl-cycloheptanol
• 1-(4-tert.-butylphenyl)-2-dimethylaminomethyl-7-(4-methoxybenzylidene)-cycloheptanol
• 1-cyclopentyl-2-dimethylaminomethyl-7-(4-methoxybenzylidene)-cycloheptanol
• 2-dimethylaminomethyl-7-(4-methoxybenzylidene)-1-m-tolyl-cycloheptanol
• 1-cyclohexyl-2-dimethylaminomethyl-7-(4-methoxybenzylidene)-cycloheptanol
• 2-dimethylaminomethyl-1-(4-fluorophenyl)-7-(4-methoxybenzylidene)-cycloheptanol
• 2-dimethylaminomethyl-7-(4-methoxybenzylidene)-1-phenethyl-cycloheptanol
• 2-dimethylaminomethyl-7-(4-methoxybenzylidene)-1-phenethynyl-cycloheptanol
• 2-dimethylaminomethyl-7-(4-methoxybenzylidene)-1-thiophen-2-yl-cycloheptanol
• 2-dimethylaminomethyl-7-(4-methoxybenzylidene)-1-(3-methoxyphenyl)-cycloheptanol
• 2-dimethylaminomethyl-7-(4-methoxybenzylidene)-1-(3-phenylpropyl)-cycloheptanol
• 2-dimethylaminomethyl-7-(4-methoxybenzylidene)-1-p-tolyl-cycloheptanol
• 2-dimethylaminomethyl-7-(4-methoxybenzylidene)-1-(4-methoxyphenyl)-cycloheptanol
• 1-cyclohexylmethyl-2-dimethylaminomethyl-7-(4-methoxy-benzylidene)-cycloheptanol
• 1-(3-chlorophenyl)-2-dimethylaminomethyl-7-(4-methoxy-benzylidene)-cycloheptanol
• 1-(2-chlorobenzyl)-2-dimethylaminomethyl-7-(4-methoxy-benzylidene)-cycloheptanol
• 2-dimethylaminomethyl-7-(4-methoxybenzylidene)-1-(2-methoxyphenyl)-cycloheptanol
• 2-dimethylaminomethyl-7-(4-methoxybenzylidene)-1-(2-methylbenzyl)-cycloheptanol
• 2-dimethylaminomethyl-7-(4-methoxybenzylidene)-1-(3-trifluoromethylphenyl)-cycloheptanol
• 2-dimethylaminomethyl-7-(4-methoxybenzylidene)-1-(3-methylbenzyl)-cycloheptanol
• 1-(3-chlorobenzyl)-2-dimethylaminomethyl-7-(4-methoxy-benzylidene)-cycloheptanol
• 1-(3,5-dichlorophenyl)-2-dimethylaminomethyl-7-(4-methoxybenzylidene)-cycloheptanol
• 2-benzylidene-7-dimethylaminomethyl-1-(3-hydroxyphenyl)-cycloheptanol
• 2-dimethylaminomethyl-1-(3-hydroxyphenyl)-7-(3-methoxy-benzylidene)-cycloheptanol
• 2-benzylidene-7-dimethylaminomethyl-1-(3-methoxyphenyl)-cycloheptanol
• 3-[1-(2-dimethylaminomethyl-1-methylethyl)-1-hydroxy-2-methyl-3-phenyl-allyl]-phenol
• 3-(4-chlorobenzyl)-5-dimethylamino-2,4-d imethyl-1-phenyl-pent-1-en-3-ol
• 5-dimethylamino-3-(3-methoxyphenyl)-2,4-dimethyl-1-phenyl-pent-1-en-3-ol
• 3-(2-benzylidene-6-dimethylaminomethyl-1-hydroxycyclo-hexyl)-phenol
• 1-benzyl-2-benzylidene-7-(4-methylpiperazin-1-ylmethyl)-cycloheptanol
• 2-benzylidene-1-(2-chlorobenzyl)-7-(4-methylpiperazin-1-ylmethyl)-cycloheptanol
• 2-benzylidene-1-(3-chlorobenzyl)-7-(4-methylpiperazin-1-ylmethyl)-cycloheptanol
• 2-benzylidene-1-(4-chlorobenzyl)-7-(4-methylpiperazin-1-ylmethyl)-cycloheptanol
• 2-benzylidene-1-(2-fluorobenzyl)-7-(4-methylpiperazin-1-ylmethyl)-cycloheptanol
• 2-benzylidene-1-(3-fluorobenzyl)-7-(4-methylpiperazin-1-ylmethyl)-cycloheptanol
• 2-benzylidene-1-(4-fluorobenzyl)-7-(4-methylpiperazin-1-ylmethyl)-cycloheptanol
• 2-benzylidene-1-(2-methylbenzyl)-7-(4-methylpiperazin-1-ylmethyl)-cycloheptanol
• 2-benzylidene-1-(3-methylbenzyl)-7-(4-methylpiperazin-1-ylmethyl)-cycloheptanol
• 2-benzylidene-1-(4-methylbenzyl)-7-(4-methylpiperazin-1-ylmethyl)-cycloheptanol
• 2-benzylidene-1-(2-methoxybenzyl)-7-(4-methylpiperazin-1-ylmethyl)-cycloheptanol
• 2-benzylidene-1-(3-methoxybenzyl)-7-(4-methylpiperazin-1-ylmethyl)-cycloheptanol
• 2-benzylidene-1-(4-methoxybenzyl)-7-(4-methylpiperazin-1-ylmethyl)-cycloheptanol
• 2-benzylidene-1-(2,6-dichlorobenzyl)-7-(4-methylpiperazin-1-ylmethyl)-cycloheptanol
• 2-benzylidene-1-(2-chloro-6-fluorobenzyl)-7-(4-methyl-piperazin-1-ylmethyl)-cycloheptanol
• 2-benzylidene-1-(2,6-difluorobenzyl)-7-(4-methylpiperazin-1-ylmethyl)-cycloheptanol
• 1-benzyl-2-(4-chlorobenzylidene)-7-(4-methylpiperazin-1-ylmethyl)-cycloheptanol
• 1-(2-chlorobenzyl)-2-(4-chlorobenzylidene)-7-(4-methyl-piperazin-1-ylmethyl)-cycloheptanol
• 1-(3-chlorobenzyl)-2-(4-chlorobenzylidene)-7-(4-methylpiperazin-1-ylmethyl)-cycloheptanol
• 1-(4-chlorobenzyl)-2-(4-chlorobenzylidene)-7-(4-methyl-piperazin-1-ylmethyl)-cycloheptanol
• 2-(4-chlorobenzylidene)-1-(2-fluorobenzyl)-7-(4-methyl-piperazin-1-ylmethyl)-cycloheptanol
• 2-(4-chlorobenzylidene)-1-(3-fluorobenzyl)-7-(4-methyl-piperazin-1-ylmethyl)-cycloheptanol
• 2-(4-chlorobenzylidene)-1-(4-fluorobenzyl)-7-(4-methyl-piperazin-1-ylmethyl)-cycloheptanol
• 2-(4-chlorobenzylidene)-1-(2-methylbenzyl)-7-(4-methyl-piperazin-1-ylmethyl)-cycloheptanol
• 2-(4-chlorobenzylidene)-1-(3-methylbenzyl)-7-(4-methyl-piperazin-1-ylmethyl)-cycloheptanol
• 2-(4-chlorobenzylidene)-1-(4-methylbenzyl)-7-(4-methyl-piperazin-1-ylmethyl)-cycloheptanol
• 2-(4-chlorobenzylidene)-1-(2-methoxybenzyl)-7-(4-methyl-piperazin-1-ylmethyl)-cycloheptanol
• 2-(4-chlorobenzylidene)-1-(3-methoxybenzyl)-7-(4-methyl-piperazin-1-ylmethyl)-cycloheptanol
• 2-(4-chlorobenzylidene)-1-(4-methoxybenzyl)-7-(4-methyl-piperazin-1-ylmethyl)-cycloheptanol
• 2-(4-chlorobenzylidene)-1-(2,6-dichlorobenzyl)-7-(4-methyl-piperazin-1-ylmethyl)-cycloheptanol
• 2-(4-chlorobenzylidene)-1-(2,6-difluorobenzyl)-7-(4-methyl-piperazin-1-ylmethyl)-cycloheptanol
• 2-(4-chlorobenzylidene)-1-(2-chloro-6-fluorobenzyl)-7-(4-methyl-piperazin-1-ylmethyl)-cycloheptanol
• 1-benzyl-2-(4-fluorobenzylidene)-7-(4-methylpiperazin-1-ylmethyl)-cycloheptanol
• 1-(2-chlorobenzyl)-2-(4-fluorobenzylidene)-7-(4-methyl-piperazin-1-ylmethyl)-cycloheptanol
• 1-(3-chlorobenzyl)-2-(4-fluorobenzylidene)-7-(4-methyl-piperazin-1-ylmethyl)-cycloheptanol
• 1-(4-chlorobenzyl)-2-(4-fluorobenzylidene)-7-(4-methyl-piperazin-1-ylmethyl)-cycloheptanol
• 1-(2-fluorobenzyl)-2-(4-fluorobenzylidene)-7-(4-methyl-piperazin-1-ylmethyl)-cycloheptanol
• 1-(3-fluorobenzyl)-2-(4-fluorobenzylidene)-7-(4-methyl-piperazin-1-ylmethyl)-cycloheptanol
• 1-(4-fluorobenzyl)-2-(4-fluorobenzylidene)-7-(4-methyl-piperazin-1-ylmethyl)-cycloheptanol
• 2-(4-fluorobenzylidene)-1-(2-methylbenzyl)-7-(4-methyl-piperazin-1-ylmethyl)-cycloheptanol
• 2-(4-fluorobenzylidene)-1-(3-methylbenzyl)-7-(4-methyl-piperazin-1-ylmethyl)-cycloheptanol
• 2-(4-fluorobenzylidene)-1-(4-methylbenzyl)-7-(4-methyl-piperazin-1-ylmethyl)-cycloheptanol
• 2-(4-fluorobenzylidene)-1-(2-methoxybenzyl)-7-(4-methyl-piperazin-1-ylmethyl)-cycloheptanol
• 2-(4-fluorobenzylidene)-1-(3-methoxybenzyl)-7-(4-methyl-piperazin-1-ylmethyl)-cycloheptanol
• 2-(4-fluorobenzylidene)-1-(4-methoxybenzyl)-7-(4-methyl-piperazin-1-ylmethyl)-cycloheptanol
• 1-(2,6-dichlorobenzyl)-2-(4-fluorobenzylidene)-7-(4-methyl-piperazin-1-ylmethyl)-cycloheptanol
• 1-(2,6-difluorobenzyl)-2-(4-fluorobenzylidene)-7-(4-methyl-piperazin-1-ylmethyl)-cycloheptanol
• 1-(2-chloro-6-fluorobenzyl)-2-(4-fluorobenzylidene)-7-(4-methylpiperazin-1-ylmethyl)-cycloheptanol
• 1-benzyl-2-furan-2-ylmethylene-7-(4-methylpiperazin-1-ylmethyl)-cycloheptanol
• 1-(2-chlorobenzyl)-2-furan-2-ylmethylene-7-(4-methyl-piperazin-1-ylmethyl)-cycloheptanol
• 1-(3-chlorobenzyl)-2-furan-2-ylmethylene-7-(4-methyl-piperazin-1-ylmethyl)-cycloheptanol
• 1-(4-chlorobenzyl)-2-fu ran-2-ylmethylene-7-(4-methyl-piperazin-1-ylmethyl)-cycloheptanol
• 1-(2-fluorobenzyl)-2-furan-2-ylmethylene-7-(4-methyl-piperazin-1-ylmethyl)-cycloheptanol
• 1-(3-fluorobenzyl)-2-furan-2-ylmethylene-7-(4-methyl-piperazin-1-ylmethyl)-cycloheptanol
• 1-(4-fluorobenzyl)-2-furan-2-ylmethylene-7-(4-methyl-piperazin-1-ylmethyl)-cycloheptanol
• 2-furan-2-ylmethylene-1-(2-methylbenzyl)-7-(4-methyl-piperazin-1-ylmethyl)-cycloheptanol
• 2-furan-2-ylmethylene-1-(3-methylbenzyl)-7-(4-methyl-piperazin-1-ylmethyl)-cycloheptanol
• 2-furan-2-ylmethylene-1-(4-methylbenzyl)-7-(4-methyl-piperazin-1-ylmethyl)-cycloheptanol
• 2-furan-2-ylmethylene-1-(2-methoxybenzyl)-7-(4-methyl-piperazin-1-ylmethyl)-cycloheptanol
• 2-furan-2-ylmethylene-1-(3-methoxybenzyl)-7-(4-methyl-piperazin-1-ylmethyl)-cycloheptanol
• 2-furan-2-ylmethylene-1-(4-methoxybenzyl)-7-(4-methyl-piperazin-1-ylmethyl)-cycloheptanol
• 1-(2,6-dichlorobenzyl)-2-furan-2-ylmethylene-7-(4-methyl-piperazin-1-ylmethyl)-cycloheptanol
• 1-(2,6-difluorobenzyl)-2-furan-2-ylmethylene-7-(4-methyl-piperazin-1-ylmethyl)-cycloheptanol
• 1-(2-chloro-6-fluorobenzyl)-2-furan-2-ylmethylene-7-(4-methylpiperazin-1-ylmethyl)-cycloheptanol
• 1-benzyl-3-furan-2-ylmethylene-7-(4-methylpiperazin-1-ylmethyl)-cycloheptanol
• 1-(2-chlorobenzyl)-3-furan-2-ylmethylene-7-(4-methyl-piperazin-1-ylmethyl)-cycloheptanol
• 1-(3-chlorobenzyl)-3-furan-2-ylmethylene-7-(4-methyl-piperazin-1-ylmethyl)-cycloheptanol
• 1-(4-chlorobenzyl)-3-furan-2-ylmethylene-7-(4-methyl-piperazin-1-ylmethyl)-cycloheptanol
• 1-(2-fluorobenzyl)-3-furan-2-ylmethylene-7-(4-methyl-piperazin-1-ylmethyl)-cycloheptanol
• 1-(3-fluorobenzyl)-3-furan-2-ylmethylene-7-(4-methyl-piperazin-1-ylmethyl)-cycloheptanol
• 1-(4-fluorobenzyl)-3-furan-2-ylmethylene-7-(4-methyl-piperazin-1-ylmethyl)-cycloheptanol
• 3-furan-2-ylmethylene-1-(2-methylbenzyl)-7-(4-methyl-piperazin-1-ylmethyl)-cycloheptanol
• 3-furan-2-ylmethylene-1-(3-methylbenzyl)-7-(4-methyl-piperazin-1-ylmethyl)-cycloheptanol
• 3-furan-2-ylmethylene-1-(4-methylbenzyl)-7-(4-methyl-piperazin-1-ylmethyl)-cycloheptanol
• 3-furan-2-ylmethylene-1-(2-methoxybenzyl)-7-(4-methyl-piperazin-1-ylmethyl)-cycloheptanol
• 3-furan-2-ylmethylene-1-(3-methoxybenzyl)-7-(4-methyl-piperazin-1-ylmethyl)-cycloheptanol
• 3-furan-2-ylmethylene-1-(4-methoxybenzyl)-7-(4-methyl-piperazin-1-ylmethyl)-cycloheptanol
• 1-(2,6-dichlorobenzyl)-3-furan-2-ylmethylene-7-(4-methyl-piperazin-1-ylmethyl)-cycloheptanol
• 1-(2,6-difluorobenzyl)-3-furan-2-ylmethylene-7-(4-methyl-piperazin-1-ylmethyl)-cycloheptanol
• 1-(2-chloro-6-fluorobenzyl)-3-furan-2-ylmethylene-7-(4-methyl-piperazin-1-ylmethyl)-cycloheptanol
optionally in the form of their racemates, their pure stereoisomers, in particular enantiomers or diastereomers, or in the form of mixtures of the stereoisomers, in particular of the enantiomers or diastereomers, in an arbitrary mixture ratio; in the specified form or in the form of their acids or their bases or in the form of their salts, in particular of physiologically acceptable salts or in the form of their solvates, in particular hydrates, preferably the hydrochloride or dihydrochloride.

9. Substituted 5-amino-1-penten-3-ol derivatives according to one of claims 1 to 8, **characterised in that** the E isomers of the derivatives according to formula I are preferably as shown in formula I':

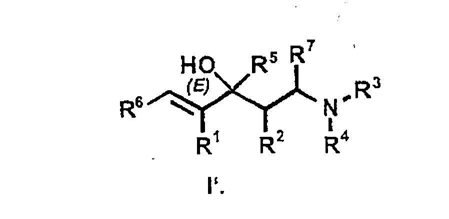

10. Substituted 5-amino-1-penten-3-ol derivatives according to one of claims 1 to 9, **characterised in that** if R⁷ is hydrogen and R¹ and R² together form a ring, then the OH group and the aminomethylene group CHR⁷-NR³R⁴ according to formula I are in the cis position relative to one another, as shown in formula I":

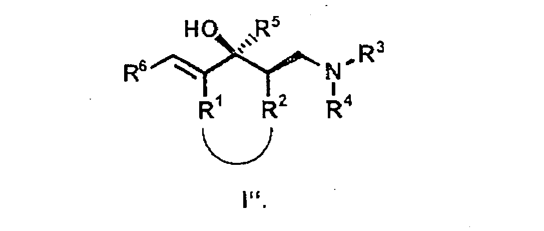

11. Medicament containing at least one substituted 5-amino-1-penten-3-ol derivative according to one of claims 1 to 10, as well as optionally suitable additives and/or auxiliary substances and/or optionally further active constituents;

12. Medicament according to claim 11, **characterised in that** a contained substituted 5-amino-1-penten-3-ol derivative according to one of claims 1 to 10 is present as a pure diastereomer and/or enantiomer, as a racemate or as a non-equimolar or equimolar mixture or the diastereomers and/or enantiomers.

13. Use of a substituted 5-amino-1-penten-3-ol derivative according to one of claims 1 to 10 for the production of a medicament for treating pain, in particular acute, neuropathic or chronic pain.

14. Use of a substituted 5-amino-1-penten-3-ol derivative according to one of claims 1 to 10 for the production of a medicament for treating migraine, hyperalgesia and allodynia, in particular thermal hyperalgesia, mechanical hyperalgesia and allodynia and cold-induced allodynia, or inflammatory or post-operative pain.

15. Use of a substituted 5-amino-1-penten-3-ol derivative according to one of claims 1 to 10 for the production of a medicament for treating epilepsy, hot flushes, post-menopausal discomfort, amyotropic lateral sclerosis (ALS), reflex sympathetic dystrophy (RSD), spastic palsy, restless leg syndrome, acquired nystagmus; psychiatric or neuropathological disorders such as bipolar disfunctions, anxiety, panic attacks, mood fluctuations, manic behaviour, depression, manic depressive behaviour; painful diabetic neuropathy, symptoms and pain caused by multiple sclerosis or Parkinson's disease, neurodegenerative diseases such as Alzheimer's disease, Huntington's disease, Parkinson's disease and epilepsy; gastrointestinal damage; erythromelalgic or post-poliomyelitic pain, trigeminal or post-herpes neuralgia; or as an anticonvulsive, analgesic or anxiolytic.

16. Use according to one of claims 13 to 15, **characterised in that** an employed substituted 5-amino-1-penten-3-ol derivative according to one of claims 1 to 10 is present as a pure diastereomer and/or enantiomer, as a racemate or as a non-equimolar or equimolar mixture of the diastereomers and/or enantiomers.

17. Process for the production of a substituted 5-amino-1-penten-3-ol derivative according to one of claims 1 to 10, in which a β-aminoketone of the formula **IA**, in which the radicals R¹ to R⁴, R⁶ and R⁷ have the meanings given in claim 1 for formula **I** 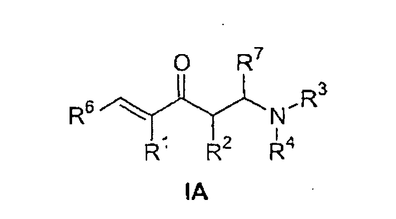 are reacted with an organometallic compound of the formula **III**
R⁵-Z **III**
in which Z denotes MgCl, MgBr, Mgl or Li and R⁵ has the meaning given in claim 1 for formula I, to form a compound of the formula I.

## Revendications

1. Dérivés substitués de 5-amino-1-pentène-3-ol de formule générale I : 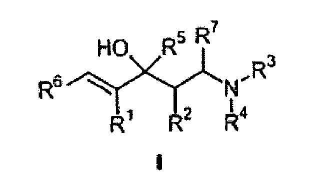 dans laquelle
R¹ et R² sont tous deux choisis, indépendamment l'un de l'autre, entre des restes alkyle en C₁ à C₆, ramifiés ou non ramifiés, saturés ou non saturés, non substitués ou substitués une ou plusieurs fois, ou bien
R¹ et R² forment ensemble un noyau (CH₂)₂₋₉, qui peut être substitué le cas échéant avec un reste alkyle en C₁ à C₈, ramifié ou non ramifié, saturé ou non saturé, non substitué ou substitué une ou plusieurs fois ; ou avec un reste aryle non substitué ou substitué une ou plusieurs fois,
R³ et R⁴ sont tous deux choisis, indépendamment l'un de l'autre, entre des restes alkyle en C₁ à C₆, ramifiés ou non ramifiés, saturés ou non saturés, non substitués ou substitués une ou plusieurs fois ; des restes cycloalkyle en C₃ à C₆, saturés ou non saturés, non substitués ou substitués une ou plusieurs fois ; un reste phényle, benzyle ou phénéthyle non substitué ou substitué une ou plusieurs fois, ou bien
les restes R³ et R⁴ forment ensemble un noyau et représentent CH₂CH₂OCH₂CH₂, CH₂CH₂NR²²CH₂CH₂ ou (CH₂)₃₋₆,
R²² étant choisi entre H ; un reste alkyle en C₁ à C₁₀ ou cycloalkyle en C₃ à C₁₀, chacun saturé ou non saturé, ramifié ou non ramifié, substitué une ou plusieurs fois ou non substitué ; un reste aryle ou hétéroaryle, chacun substitué une ou plusieurs fois ou non substitué ; ou un reste lié par l'intermédiaire d'un radical alkyle en C₁ à C₃ saturé ou non saturé, aryle, cycloalkyle en C₃ à C₁₀ ou hétéroaryle, chacun substitué une ou plusieurs fois ou non substitué ;
R⁵ est choisi entre des restes alkyle en C₁ à C₁₀, saturés ou non saturés, ramifiés ou non ramifiés, substitués une ou plusieurs fois ou non substitués ; des restes cycloalkyle en C₃ à C₉, saturés ou non saturés ; un reste aryle, hétéroaryle, aryle lié par l'intermédiaire d'un radical alkyle en C₁ à C₃ saturé ou non saturé, cycloalkyle en C₃ à C₁₀ lié par l'intermédiaire d'un radical alkyle en C₁ à C₃ saturé ou non saturé, ou hétéroaryle lié par l'intermédiaire d'un radical alkyle en C₁ à C₃ saturé ou non saturé, tous les restes aryle, hétéroaryle et cycloalkyle pouvant être dans chaque cas, indépendamment les uns des autres, non substitués ou substitués une ou plusieurs fois avec des radicaux choisis, indépendamment les uns des autres, entre des radicaux
F, Cl, Br, I, OR¹⁸, SR¹⁸, SO₂R¹⁸, SO₂OR¹⁸, CN, COOR¹⁸, NR¹⁹R²⁰ ; alkyle en C₁ à C₁₀, saturé ou non saturé, ramifié ou non ramifié, substitué une ou plusieurs fois ou non substitué ; cycloalkyle en C₃ à C₉, saturé ou non saturé, non substitué ou substitué une ou plusieurs fois ; aryle ou hétéroaryle, chacun non substitué ou substitué une ou plusieurs fois ; ou aryle, cycloalkyle en C₃ à C₉ ou hétéroaryle lié par l'intermédiaire d'un radical alkyle en C₁ à C₃ saturé ou non saturé, chacun non substitué ou substitué une ou plusieurs fois ;
R¹⁸ étant choisi entre H ; un reste alkyle en C₁ à C₁₀, saturé ou non saturé, ramifié ou non ramifié, non substitué ou substitué une ou plusieurs fois ; un reste cycloalkyle en C₃ à C₉, saturé ou non saturé, non substitué ou substitué une ou plusieurs fois ; un reste aryle ou hétéroaryle, non substitué ou substitué une ou plusieurs fois ; ou un reste, lié par l'intermédiaire d'un radical alkyle en C₁ à C₃ saturé ou non saturé, aryle, cycloalkyle en C₃ à C₉ ou hétéroaryle, chacun non substitué ou substitué une ou plusieurs fois ;
R¹⁹ et R²⁰ sont choisis indépendamment l'un de l'autre entre H ; un reste alkyle en C₁ à C₁₀, saturé ou non saturé, ramifié ou non ramifié, non substitué ou substitué une ou plusieurs fois ; un reste cycloalkyle en C₃ à C₉, saturé ou non saturé, non substitué ou substitué une ou plusieurs fois ; un reste aryle ou hétéroaryle, chacun non substitué ou substitué une ou plusieurs fois ; ou un reste, lié par l'intermédiaire d'un radical alkyle en C₁ à C₃ saturé ou non saturé, aryle, cycloalkyle en C₃ à C₉ ou hétéroaryle, chacun non substitué ou substitué une ou plusieurs fois ;
ou bien R¹⁹ et R²⁰ forment ensemble un groupement CH₂CH₂OCH₂CH₂, CH₂CH₂NR²¹CH₂CH₂ ou (CH₂)₃₋₆,
R²¹ étant choisi entre H ; un radical phényle, substitué ou non substitué ; un radical alkyle en C₁ à C₁₀ saturé ou non saturé, ramifié ou non ramifié, non substitué ou substitué une ou plusieurs fois ;
R⁶ est choisi entre un reste alkyle en C₁ à C₁₀, saturé ou non saturé, ramifié ou non ramifié, substitué une ou plusieurs fois ou non substitué ; un reste cycloalkyle en C₅ à C₇, aryle ou hétéroaryle, chacun non substitué ou substitué une ou plusieurs fois ;
R⁷ est choisi entre H ; un reste aryle ou hétéroaryle ; chacun non substitué ou substitué une ou plusieurs fois ;
le cas échéant sous forme de leurs racémates, de leurs stéréoisomères purs, en particulier des énantiomères ou des diastéréoisomères, ou sous forme de mélanges des stéréoisomères, en particulier des énantiomères ou des diastéréoisomères, dans un rapport de mélange quelconque ; sous la forme représentée ou sous forme de leurs acides ou de leurs bases ou sous forme de leurs sels, en particulier des sels physiologiquement acceptables, ou sous forme de leurs produits de solvatation, en particulier des hydrates.

2. Dérivés substitués de 5-amino-1-pentène-3-ol suivant la revendication 1, **caractérisés en ce que**
R⁷ est choisi entre H ou un reste hétéroaryle, ou bien R⁷ est un reste répondant à la formule II 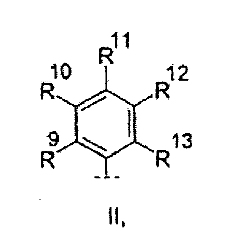 dans laquelle R⁹ à R¹³ sont choisis indépendamment les uns des autres entre H, F, Cl, Br, I, CF₃, CHF₂, CH₂F, OH, OR¹⁴, OCF₃, OCHF₂, OCH₂F, SR¹⁴, SO₂CH₃, SO₂CF₃ ; alkyle en C₁ à C₆, ramifié ou non ramifié, saturé ou non saturé, non substitué ou substitué une ou plusieurs fois ; phényle, non substitué ou substitué une ou plusieurs fois ; CN, COOR¹⁴, NO₂, ou bien
R⁹ et R¹⁰ ou R¹⁰ et R¹¹ forment ensemble un noyau OCH₂O-ou OCH₂CH₂O-, et
R¹⁴ est choisi entre un reste alkyle en C₁ à C₆, ramifié ou non ramifié, saturé ou non saturé, non substitué ou substitué une ou plusieurs fois ; un reste phényle, benzyle, phénéthyle ou thiophène, chacun non substitué ou substitué une ou plusieurs fois,
avantageusement R⁷ représente l'hydrogène.

3. Dérivés substitués de 5-amino-1-pentène-3-ol suivant l'une des revendications 1 ou 2, **caractérisés en ce que**
R¹ et R² forment ensemble un noyau (CH₂)₂₋₅ qui peut être substitué le cas échéant avec un reste alkyle en C₁ à C₆ ramifié ou non ramifié, saturé ou non saturé, non substitué ou substitué une ou plusieurs fois ; ou un reste phényle non substitué ou substitué une ou plusieurs fois, ce noyau étant avantageusement non substitué ; R¹ et R² formant alors avantageusement ensemble un noyau (CH₂)₂₋₄ non substitué,
ou bien
R¹ et R² sont choisis indépendamment l'un de l'autre entre un reste alkyle en C₁ à C₃ non ramifié, saturé et non substitué, avantageusement CH₃ ; en particulier, R¹ et R² correspondent tous deux à CH₃.

4. Dérivés substitués de 5-amino-1-pentène-3-ol suivant l'une des revendications 1 à 3, **caractérisés en ce que**
R³ et R⁴ sont tous deux choisis indépendamment l'un de l'autre entre des restes alkyle en C₁ à C₆, ramifiés ou non ramifiés, saturés ou non saturés, non substitués ou substitués une ou plusieurs fois ; ils représentent avantageusement tous deux des restes CH₃ ;
ou bien
R³ et R⁴ forment ensemble un noyau et représentent un groupe CH₂CH₂NR²²CH₂CH₂ ou (CH₂)₃₋₆, ils forment ensemble en particulier un groupe (CH₂)₄₋₅ ou CH₂CH₂NR²²CH₂CH₂, R²² étant choisi entre H ou un reste alkyle en C₁ à C₆, saturé, ramifié ou non ramifié et non substitué ; en particulier H ou CH₃.

5. Dérivés substitués de 5-amino-1-pentène-3-ol suivant l'une des revendications 1 à 4, **caractérisés en ce que**
R⁵ est choisi entre un reste alkyle en C₁ à C₆, saturé ou non saturé, ramifié ou non ramifié, substitué une ou plusieurs fois ou non substitué, un reste cycloalkyle en C₅ ou C₆, phényle, thiophényle, furyle, benzofurannyle, benzothiophényle, pyrrolyle, pyridinyle, pyrimidinyle, quinolinyle, isoquinolinyle ou quinazolinyle, avantageusement un reste phényle, furyle, thiophényle ou cycloalkyle en C₅ ou C₆ ; un reste, lié par l'intermédiaire d'un radical alkyle en C₁ à C₃ saturé ou non saturé, phényle, cycloalkyle en C₅ ou C₆, thiophényle, furyle, benzofurannyle, benzothiophényle, pyrrolyle, pyridinyle, pyrimidinyle, quinolinyle, isoquinolinyle, quinazolinyle, avantageusement phényle, furyle, thiophényle ou cycloalkyle en C₅ ou C₆ ;
tous les restes aryle, hétéroaryle et cycloalkyle pouvant chacun, indépendamment les uns des autres, être non substitués ou substitués une ou plusieurs fois, avantageusement non substitués ou substitués ou une plusieurs fois avec des substituants choisis indépendamment les uns des autres entre F, Cl, Br, I, OH, O-(alkyle en C₁ à C₄), alkyle en C₁ à C₆, CF₃, CHF₂, CH₂F, NH₂ et/ou SH,
avantageusement
R⁵ est choisi entre un reste alkyle en C₁ à C₃, saturé ou non saturé, non substitué et/ou non ramifié ; un reste naphtyle, furyle, cyclohexyle, cyclopentyle, phényle ou thiophényle, non substitué ou substitué une ou plusieurs fois, avantageusement avec F, Cl, Br, I, CF₃, CHF₂, CH₂F, OH, O-(alkyle en C₁ à C₄), alkyle en C₁ à C₆, NH₂ et/ou SH ; ou bien un reste, lié par l'intermédiaire d'un radical alkyle en C₁ à C₃ saturé ou non saturé, phényle non substitué ou substitué une ou plusieurs fois avantageusement avec F, Cl, Br, I, CF₃, CHF₂, CH₂F, OH, O-(alkyle en C₁ à C₄), alkyle en C₁ à C₆, NH₂ et/ou SH,
en particulier
R⁵ est choisi entre des groupes -CH=CH₂, cyclohexyle, cyclopentyle ; phényle, phénéthyle (phényle lié par l'intermédiaire de CH₂-CH₂), benzyle (phényle lié par l'intermédiaire de CH₂) ou thiophényle non substitués ou substitués une ou plusieurs fois, avantageusement avec F, Cl, Br, I, OH, OCH₃, OC₂H₅, OC₃H₇, SH, CF₃, CHF₂, CH₂F, CH₃, C₂H₅, C₃H₇ et/ou C₄H₉ ou tertiobutyle.

6. Dérivés substitués de 5-amino-1-pentène-3-ol suivant l'une des revendications 1 à 5, **caractérisés en ce que**
R⁶ est choisi entre un reste phényle ou furyle, chacun non substitué ou substitué une ou plusieurs fois, avantageusement non substitué ou substitué une ou plusieurs fois avec des substituants choisis indépendamment les uns des autres entre les substituants fluor, chlore, CH₃, OCH₃, CF₃ ou tertiobutyle.

7. Dérivés substitués de 5-amino-1-pentène-3-ol suivant l'une des revendications 1 à 6, **caractérisés en ce que**, lorsque R⁵ est choisi entre un reste, lié par l'intermédiaire d'un radical alkyle en C₁ à C₃ saturé ou non saturé, aryle, cycloalkyle en C₃ à C₉ ou hétéroaryle, le radical alkyle en C₁ à C₃, par l'intermédiaire duquel le reste aryle, hétéroaryle ou cycloalkyle est lié, est choisi entre :
-CH₂-, -C₂H₄-, C₃H₆-, -C≡C, -CH=CH-, -CH=CH-CH₂-, -CH₂-CH=CH-, -C≡C-CH₂- ou -CH₂-C≡C-, avantageusement -CH₂-, -C₂H₄- ou -C≡C.

8. Dérivés substitués de 5-amino-1-pentène-3-ol suivant l'une des revendications 1 à 7, **caractérisés en ce qu'**ils sont choisis dans le groupe suivant :
• 2-benzylidène-1-(3-chloro-benzyl)-6-diméthylamino-méthyl-cyclohexanol,
• 2-benzylidène-6-diméthylaminométhyl-1-(2,5-diméthyl-benzyl)-cyclohexanol,
• 2-benzylidène-1-(2-chloro-6-fluoro-benzyl)-6-diméthyl-aminométhyl-cyclohexanol,
• 2-benzylidène-1-(4-chloro-benzyl)-6-diméthylamino-méthyl-cyclohexanol,
• 2-benzylidène-6-diméthylaminométhyl-1-(3-méthyl-benzyl)-cyclohexanol,
• 2-benzylidène-6-diméthylaminométhyl-1-(3-trifluoro-méthyl-phényl)-cyclohexanol,
• 2-benzylidène-1-(3-chloro-4-fluoro-phényl)-6-diméthyl-aminométhyl-cyclohexanol,
• 2-benzylidène-6-diméthylaminométhyl-1-(2-méthyl-benzyl)-cyclohexanol,
• 2-benzylidène-6-diméthylaminométhyl-1-(2-méthoxy-phényl)-cyclohexanol,
• 2-benzylidène-6-diméthylaminométhyl-1-(3-fluoro-benzyl)-cyclohexanol,
• 2-benzylidène-1-(4-chloro-3-trifluorométhyl-phényl)-6-diméthylaminométhyl-cyclohexanol,
• 2-benzylidène-6-diméthylaminométhyl-1-(3-méthoxy-benzyl)-cyclohexanol,
• 2-benzylidène-6-diméthylaminométhyl-1-(4-fluoro-benzyl)-cyclohexanol,
• 2-benzylidène-1-(2-chloro-benzyl)-6-diméthylamino-méthyl-cyclohexanol,
• 2-benzylidène-1-(3,5-dichloro-phényl)-6-diméthyl-aminométhyl-cyclohexanol,
• 2-benzylidène-1-(3-chloro-phényl)-6-diméthylamino-méthyl-cyclohexanol,
• 2-benzylidène-6-diméthylaminométhyl-1-(3-fluoro-phényl)-cyclohexanol,
• 2-benzylidène-6-diméthylaminométhyl-1-(5-fluoro-2-méthoxy-phényl)-cyclohexanol,
• 2-benzylidène-1-cyclohexylméthyl-6-diméthylamino-méthyl-cyclohexanol,
• 2-benzylidène-6-diméthylaminométhyl-1-(4-méthoxy-phényl)-cyclohexanol,
• 2-benzylidène-6-diméthylaminométhyl-1-p-tolyl-cyclo-hexanol,
• 2-benzylidène-6-diméthylaminométhyl-1-(3-phényl-propyl)-cyclohexanol,
• 2-benzylidène-6-diméthylaminométhyl-1-(3-méthoxy-phényl)-cyclohexanol,
• 2-benzylidène-6-diméthylaminométhyl-1-thiophène-2-yl-cyclohexanol,
• 2-benzylidène-6-diméthylaminométhyl-1-phényléthynyl-cyclohexanol,
• 2-benzylidène-6-diméthylaminométhyl-1-phénéthyl-cyclohexanol,
• 2-benzylidène-6-diméthylaminométhyl-1-(4-fluoro-phényl)-cyclohexanol,
• 2-benzylidène-6-diméthylaminométhyl-bicyclohexyl-1-ol,
• 2-benzylidène-6-diméthylaminométhyl-1-m-tolyl-cyclo-hexanol,
• 2-benzylidène-1-cyclopentyl-6-diméthylaminométhyl-cyclohexanol,
• 2-benzylidène-1-(4-tertiobutyl-phényl)-6-diméthyl-aminométhyl-cyclohexanol,
• 2-benzylidène-6-diméthylaminométhyl-1-vinyl-cyclohexanol,
• 2-benzylidène-6-diméthylaminométhyl-1-o-tolyl-cyclo-hexanol,
• 2-benzylidène-6-diméthylaminométhyl-1-(4-fluoro-3-méthyl-phényl)-cyclohexanol,
• 1-benzyl-2-benzylidène-6-diméthylaminométhyl-cyclo-hexanol,
• 2-benzylidène-1-(4-chloro-phényl)-6-diméthylamino-méthyl-cyclohexanol,
• 2-benzylidène-6-diméthylaminométhyl-1-phényl-cyclo-hexanol,
• 1-(3-chloro-benzyl)-2-(4-chloro-benzylidène)-6-diméthyl-aminométhyl-cyclohexanol,
• 2-(4-chloro-benzylidène)-6-diméthylaminométhyl-1-(2,5-diméthyl-benzyl)-cyclohexanol,
• 2-(4-chloro-benzylidène)-1-(2-chloro-6-fluoro-benzyl)-6-diméthylaminométhyl-cyclohexanol,
• 1-(4-chloro-benzyl)-2-(4-chloro-benzylidène)-6-diméthyl-aminométhyl-cyclohexanol,
• 2-(4-chloro-benzylidène)-6-diméthylaminométhyl-1-(3-trifluorométhyl-phényl)-cyclohexanol,
• 2-(4-chloro-benzylidène)-1-(3-chloro-4-fluoro-phényl)-6-diméthylaminométhyl-cyclohexanol,
• 2-(4-chloro-benzylidène)-6-diméthylaminométhyl-1-(2-méthyl-benzyl)-cyclohexanol,
• 2-(4-chloro-benzylidène)-6-diméthylaminométhyl-1-(2-méthoxy-phényl)-cyclohexanol,
• 2-(4-chloro-benzylidène)-6-diméthylaminométhyl-1-(3-fluoro-benzyl)-cyclohexanol,
• 2-(4-chloro-benzylidène)-1-(4-chloro-3-trifluorométhyl-phényl)-6-diméthylaminométhyl-cyclohexanol,
• 2-(4-chloro-benzylidène)-6-diméthylaminométhyl-1-(3-méthoxy-benzyl)-cyclohexanol,
• 2-(4-chloro-benzylidène)-6-diméthylaminométhyl-1-(4-fluoro-benzyl)-cyclohexanol,
• 1-(2-chloro-benzyl)-2-(4-chloro-benzylidène)-6-diméthyl-aminométhyl-cyclohexanol,
• 2-(4-chloro-benzylidène)-1-(3,5-dichloro-phényl)-6-diméthylaminométhyl-cyclohexanol,
• 2-(4-chloro-benzylidène)-1-(3-chloro-phényl)-6-diméthyl-aminométhyl-cyclohexanol,
• 2-(4-chloro-benzylidène)-6-diméthylaminométhyl-1-(3-fluoro-phényl)-cyclohexanol,
• 2-(4-chloro-benzylidène)-6-diméthylaminométhyl-1-(5-fluoro-2-méthoxy-phényl)-cyclohexanol,
• 2-(4-chloro-benzylidène)-1-cyclohexylméthyl-6-diméthyl-aminométhyl-cyclohexanol,
• 2-(4-chloro-benzylidène)-6-diméthylaminométhyl-1-(4-méthoxy-phényl)-cyclohexanol,
• 2-(4-chloro-benzylidène)-6-diméthylaminométhyl-1-p-tolyl-cyclohexanol,
• 2-(4-chloro-benzylidène)-6-diméthylaminométhyl-1-(3-phényl-propyl)-cyclohexanol,
• 2-(4-chloro-benzylidène)-6-diméthylaminométhyl-1-(3-méthoxy-phényl)-cyclohexanol,
• 2-(4-chloro-benzylidène)-6-diméthylaminométhyl-1-thiophène-2-yl-cyclohexanol,
• 2-(4-chloro-benzylidène)-6-diméthylaminométhyl-1-phényléthynyl-cyclohexanol,
• 2-(4-chloro-benzylidène)-6-diméthylaminométhyl-1-phénéthyl-cyclohexanol,
• 2-(4-chloro-benzylidène)-6-diméthylaminométhyl-1-(4-fluoro-phényl)-cyclohexanol,
• 2-(4-chloro-benzylidène)-6-diméthylaminométhyl-bicyclohexyl-1-ol,
• 2-(4-chloro-benzylidène)-6-diméthylaminométhyl-1-m-tolyl-cyclohexanol,
• 2-(4-chloro-benzylidène)-1-cyclopentyl-6-diméthylamino-méthyl-cyclohexanol,
• 1-(4-tertiobutyl-phényl)-2-(4-chloro-benzylidène)-6-diméthylaminométhyl-cyclohexanol,
• 2-(4-chloro-benzylidène)-6-diméthylaminométhyl-1-vinyl-cyclohexanol,
• 2-(4-chloro-benzylidène)-6-diméthylaminométhyl-1-o-tolyl-cyclohexanol,
• 2-(4-chloro-benzylidène)-6-diméthylaminométhyl-1-(4-fluoro-3-méthyl-phényl)-cyclohexanol,
• 1-benzyl-2-(4-chloro-benzylidène)-6-diméthylamino-méthyl-cyclohexanol,
• 2-(4-chloro-benzylidène)-1-(4-chloro-phényl)-6-diméthyl-aminométhyl-cyclohexanol,
• 2-(4-chloro-benzylidène)-6-diméthylaminométhyl-1-phényl-cyclohexanol,
• 1-(3-chloro-benzyl)-2-diméthylaminométhyl-6-(4-méthoxy-benzylidène)-cyclohexanol,
• 2-diméthylaminométhyl-1-(2,5-diméthyl-benzyl)-6-(4-méthoxy-benzylidène)-cyclohexanol,
• 1-(2-chloro-6-fluoro-benzyl)-2-diméthylaminométhyl-6-(4-méthoxy-benzylidène)-cyclohexanol,
• 2-diméthylaminométhyl-6-(4-méthoxy-benzylidène)-1-(3-méthyl-benzyl)-cyclohexanol,
• 2-diméthylaminométhyl-6-(4-méthoxy-benzylidène)-1-(3-trifluorométhyl-phényl)-cyclohexanol,
• 1-(3-chloro-4-fluoro-phényl)-2-diméthylaminométhyl-6-(4-méthoxy-benzylidène)-cyclohexanol,
• 2-diméthylaminométhyl-6-(4-méthoxy-benzylidène)-1-(2-méthoxy-phényl)-cyclohexanol,
• 1-(4-chloro-3-trifluorométhyl-phényl)-2-diméthylamino-méthyl-6-(4-méthoxy-benzylidène)-cyclohexanol,
• 2-diméthylaminométhyl-1-(4-fluoro-benzyl)-6-(4-méthoxy-benzylidène)-cyclohexanol,
• 1-(2-chloro-benzyl)-2-diméthylaminométhyl-6-(4-méthoxy-benzylidène)-cyclohexanol,
• 1-(3,5-dichloro-phényl)-2-diméthylaminométhyl-6-(4-méthoxy-benzylidène)-cyclohexanol,
• 1-(3-chloro-phényl)-2-diméthylaminométhyl-6-(4-méthoxy-benzylidène)-cyclohexanol,
• 2-diméthylaminométhyl-1-(5-fluoro-2-méthoxy-phényl)-6-(4-méthoxy-benzylidène)-cyclohexanol,
• 1-cyclohexylméthyl-2-diméthylaminométhyl-6-(4-méthoxy-benzylidène)-cyclohexanol,
• 2-diméthylaminométhyl-6-(4-méthoxy-benzylidène)-1-(4-méthoxy-phényl)-cyclohexanol,
• 2-diméthylaminométhyl-6-(4-méthoxy-benzylidène)-1-p-tolyl-cyclohexanol,
• 1-(2,3-dichloro-phényl)-2-diméthylaminométhyl-6-(4-méthoxy-benzylidène)-cyclohexanol,
• 2-diméthylaminométhyl-6-(4-méthoxy-benzylidène)-1-(3-phényl-propyl)-cyclohexanol,
• 2-diméthylaminométhyl-6-(4-méthoxy-benzylidène)-1-(3-méthoxy-phényl)-cyclohexanol,
• 2-diméthylaminométhyl-6-(4-méthoxy-benzylidène)-1-thiophène-2-yl-cyclohexanol,
• 2-diméthylaminométhyl-6-(4-méthoxy-benzylidène)-1-phényléthynyl-cyclohexanol,
• 2-diméthylaminométhyl-6-(4-méthoxy-benzylidène)-1-phénéthyl-cyclohexanol,
• 2-diméthylaminométhyl-1-(4-fluoro-phényl)-6-(4-méthoxy-benzylidène)-cyclohexanol,
• 6-diméthylaminométhyl-2-(4-méthoxy-benzylidène)-bicyclo-hexyl-1-ol,
• 2-diméthylaminométhyl-6-(4-méthoxy-benzylidène)-1-m-tolyl-cyclohexanol,
• 1-cyclopentyl-2-diméthylaminométhyl-6-(4-méthoxy-benzylidène)-cyclohexanol,
• 1-(4-tertiobutyl-phényl)-2-diméthylaminométhyl-6-(4-méthoxy-benzylidène)-cyclohexanol,
• 2-diméthylaminométhyl-6-(4-méthoxy-benzylidène)-1-vinyl-cyclohexanol,
• 2-diméthylaminométhyl-6-(4-méthoxy-benzylidène)-1-o-tolyl-cyclohexanol,
• 2-diméthylaminométhyl-1-(4-fluoro-3-méthyl-phényl)-6-(4-méthoxy-benzylidène)-cyclohexanol,
• 1-benzyl-2-diméthylaminométhyl-6-(4-méthoxy-benzylidène)-cyclohexanol,
• 1-(4-chloro-phényl)-2-diméthylaminométhyl-6-(4-méthoxy-benzylidène)-cyclohexanol,
• 2-diméthylaminométhyl-6-(4-méthoxy-benzylidène)-1-phényl-cyclohexanol,
• 1-(3-chloro-benzyl)-2-diméthylaminométhyl-6-(3-méthoxy-benzylidène)-cyclohexanol,
• 2-diméthylaminométhyl-1-(2,5-diméthyl-benzyl)-6-(3-méthoxy-benzylidène)-cyclohexanol,
• 2-diméthylaminométhyl-6-(3-méthoxy-benzylidène)-1-(2-méthyl-benzyl)-cyclohexanol,
• 2-diméthylaminométhyl-6-(3-méthoxy-benzylidène)-1-(2-méthoxy-phényl)-cyclohexanol,
• 1-(4-chloro-3-trifluorométhyl-phényl)-2-diméthyl-aminométhyl-6-(3-méthoxy-benzylidène)-cyclohexanol,
• 1-(2-chloro-benzyl)-2-diméthylaminométhyl-6-(3-méthoxy-benzylidène)-cyclohexanol,
• 6-diméthylaminométhyl-2-(3-méthoxy-benzylidène)-bicyclo-hexyl-1-ol,
• 1-cyclopentyl-2-diméthylaminométhyl-6-(3-méthoxy-benzylidène)-cyclohexanol,
• 2-diméthylaminométhyl-6-(3-méthoxy-benzylidène)-1-vinyl-cyclohexanol,
• 2-diméthylaminométhyl-1-(4-fluoro-3-méthyl-phényl)-6-(3-méthoxy-benzylidène)-cyclohexanol,
• 1-benzyl-2-diméthylaminométhyl-6-(3-méthoxy-benzylidène)-cyclohexanol,
• 2-diméthylaminométhyl-6-(3-méthoxy-benzylidène)-1-phényl-cyclohexanol,
• 1-benzyl-(2-chloro-benzylidène)-6-diméthylaminométhyl-cyclohexanol,
• 5-diméthylamino-2,4-diméthyl-1,3-diphényl-pent-1-ène-3-ol,
• 3-(4-chloro-phényl)-5-diméthylamino-2,4-diméthyl-1-phényl-pent-1-ène-3-ol,
• 3-benzyl-5-diméthylamino-2,4-diméthyl-1-phényl-pent-1-ène-3-ol,
• 5-diméthylamino-3-(4-fluoro-3-méthyl-phényl)-2,4-diméthyl-1-phényl-pent-1-ène-3-ol,
• 5-diméthylamino-2,4-diméthyl-1-phényl-3-o-tolyl-pent-1-ène-3-ol,
• 3-(2-diméthylamino-1-méthyl-éthyl)-2-méthyl-1-phényl-penta-1,4-diène-3-ol,
• 3-(4-tertiobutyl-phényl)-5-diméthylamino-2,4-diméthyl-1-phényl-pent-1-ène-3-ol,
• 3-cyclopentyl-5-diméthylamino-2,4-diméthyl-1-phényl-pent-1-ène-3-ol,
• 5-diméthylamino-2,4-diméthyl-1-phényl-3-m-tolyl-pent-1-ène-3-ol,
• 3-cyclohexyl-5-diméthylamino-2,4-diméthyl-1-phényl-pent-1-ène-3-ol,
• 5-diméthylamino-3-(4-fluoro-phényl)-2,4-diméthyl-1-phényl-pent-1-ène-3-ol,
• 5-diméthylamino-2,4-diméthyl-3-phénéthyl-1-phényl-pent-1-ène-3-ol,
• 3-(2-diméthylamino-1-méthyl-éthyl)-2-méthyl-1,5-diphényl-pent-1-ène-4-yne-3-ol,
• 5-diméthylamino-2,4-diméthyl-1-phényl-3-thiophène-2-yl-pent-1-ène-3-ol,
• 3-(2,4-dichloro-phényl)-5-diméthylamino-2,4-diméthyl-1-phényl-pent-1-ène-3-ol,
• 5-diméthylamino-3-(3-méthoxy-phényl)-2,4-diméthyl-1-phényl-pent-1-ène-3-ol,
• 3-(2-diméthylamino-1-méthyl-éthyl)-2-méthyl-1,6-diphényl-hex-1-ène-3-ol,
• 3-(2,3-dichloro-phényl)-5-diméthylamino-2,4-diméthyl-1-phényl-pent-1-ène-3-ol,
• 5-diméthylamino-2,4-diméthyl-1-phényl-3-p-tolyl-pent-1-ène-3-ol,
• 5-diméthylamino-3-(4-méthoxy-phényl)-2,4-diméthyl-1-phényl-pent-1-ène-3-ol,
• 3-cyclohexylméthyl-5-diméthylamino-2,4-diméthyl-1-phényl-pent-1-ène-3-ol,
• 5-diméthylamino-3-(5-fluoro-2-méthoxy-phényl)-2,4-diméthyl-1-phényl-pent-1-ène-3-ol,
• 5-diméthylamino-3-(3-fluoro-phényl)-2,4-diméthyl-1-phényl-pent-1-ène-3-ol,
• 3-(3-chloro-phényl)-5-diméthylamino-2,4-diméthyl-1-phényl-pent-1-ène-3-ol,
• 3-(3,5-dichloro-phényl)-5-diméthylamino-2,4-diméthyl-1-phényl-pent-1-ène-3-ol,
• 3-(2-chloro-benzyl)-5-diméthylamino-2,4-diméthyl-1-phényl-pent-1-ène-3-ol,
• 5-diméthylamino-3-(4-fluoro-benzyl)-2,4-diméthyl-1-phényl-pent-1-ène-3-ol,
• 3-(4-chloro-3-trifluorométhyl-phényl)-5-diméthylamino-2,4-diméthyl-1-phényl-pent-1-ène-3-ol,
• 5-diméthylamino-3-(2-méthoxy-phényl)-2,4-diméthyl-1-phényl-pent-1-ène-3-ol,
• 5-diméthylamino-2,4-diméthyl-3-(2-méthyl-benzyl)-1-phényl-pent-1-ène-3-ol,
• 3-(3-chloro-4-fluoro-phényl)-5-diméthylamino-2,4-diméthyl-1-phényl-pent-1-ène-3-ol,
• 5-diméthylamino-2,4-diméthyl-1-phényl-3-(3-trifluoro-méthyl-phényl)-pent-1-ène-3-ol,
• 5-diméthylamino-2,4-diméthyl-3-(3-méthyl-benzyl)-1-phényl-pent-1-ène-3-ol,
• 3-(4-chloro-benzyl)-5-diméthylamino-2,4-diméthyl-1-phényl-pent-1-ène-3-ol,
• 3-(2-chloro-6-fluoro-benzyl)-5-diméthylamino-2,4-diméthyl-1-phényl-pent-1-ène-3-ol,
• 5-diméthylamino-3-(2,5-diméthyl-benzyl)-2,4-diméthyl-1-phényl-pent-1-ène-3-ol,
• 3-(3-chloro-benzyl)-5-diméthylamino-2,4-diméthyl-1-phényl-pent-1-ène-3-ol,
• 3-(2,4-dichloro-benzyl)-5-diméthylamino-2,4-diméthyl-1-phényl-pent-1-ène-3-ol,
• 5-diméthylamino-1-(4-fluoro-phényl)-2,4-diméthyl-3-phényl-pent-1-ène-3-ol,
• 3-benzyl-5-diméthylamino-1-(4-fluoro-phényl)-2,4-diméthyl-pent-1-ène-3-ol,
• 5-diméthylamino-3-(4-fluoro-3-méthyl-phényl)-1-(4-fluoro-phényl)-2,4-diméthyl-pent-1-ène-3-ol,
• 5-diméthylamino-1-(4-fluoro-phényl)-2,4-diméthyl-3-o-tolyl-pent-1-ène-3-ol,
• 3-(2-diméthylamino-1-méthyl-éthyl)-1-(4-fluoro-phényl)-2-méthyl-penta-1,4-diène-3-ol,
• 3-(4-tertiobutyl-phényl)-5-diméthylamino-1-(4-fluoro-phényl)-2,4-diméthyl-pent-1-ène-3-ol,
• 3-cyclopentyl-5-diméthylamino-1-(4-fluoro-phényl)-2,4-diméthyl-pent-1-ène-3-ol,
• 5-diméthylamino-1-(4-fluoro-phényl)-2,4-diméthyl-3-m-tolyl-pent-1-ène-3-ol,
• 3-cyclohexyl-5-diméthylamino-1-(4-fluoro-phényl)-2,4-diméthyl-pent-1-ène-3-ol,
• 5-diméthylamino-1,3-bis-(4-fluoro-phényl)-2,4-diméthyl-pent-1-ène-3-ol,
• 5-diméthylamino-1-(4-fluoro-phényl)-2,4-diméthyl-3-phénéthyl-pent-1-ène-3-ol,
• 3-(2-diméthylamino-1-méthyl-éthyl)-1-(4-fluoro-phényl)-2-méthyl-5-phényl-pent-1-ène-4-yne-3-ol,
• 5-diméthylamino-1-(4-fluoro-phényl)-2,4-diméthyl-3-thiophène-2-yl-pent-1-ène-3-ol,
• 3-(2,4-dichloro-phényl)-5-diméthylamino-1-(4-fluoro-phényl)-2,4-diméthyl-pent-1-ène-3-ol,
• 5-diméthylamino-1-(4-fluoro-phényl)-3-(3-méthoxy-phényl)-2,4-diméthyl-pent-1-ène-3-ol,
• 3-(2-diméthylamino-1-méthyl-éthyl)-1-(4-fluoro-phényl)-2-méthyl-6-phényl-hex-1-ène-3-ol,
• 3-(2,3-dichloro-phényl)-5-diméthylamino-1-(4-fluoro-phényl)-2,4-diméthyl-pent-1-ène-3-ol,
• 5-diméthylamino-1-(4-fluoro-phényl)-2,4-diméthyl-3-p-tolyl-pent-1-ène-3-ol,
• 5-diméthylamino-1-(4-fluoro-phényl)-3-(4-méthoxy-phényl)-2,4-diméthyl-pent-1-ène-3-ol,
• 3-cyclohexylméthyl-5-diméthylamino-1-(4-fluoro-phényl)-2,4-diméthyl-pent-1-ène-3-ol,
• 5-diméthylamino-3-(5-fluoro-2-méthoxy-phényl)-1-(4-fluoro-phényl)-2,4-diméthyl-pent-1-ène-3-ol,
• 5-diméthylamino-3-(3-fluoro-phényl)-1-(4-fluoro-phényl)-2,4-diméthyl-pent-1-ène-3-ol,
• 3-(3-chloro-phényl)-5-diméthylamino-1-(4-fluoro-phényl)-2,4-diméthyl-pent-1-ène-3-ol,
• 3-(3,5-dichloro-phényl)-5-diméthylamino-1-(4-fluoro-phényl)-2,4-diméthyl-pent-1-ène-3-ol,
• 3-(2-chloro-benzyl)-5-diméthylamino-1-(4-fluoro-phényl)-2,4-diméthyl-pent-1-ène-3-ol,
• 5-diméthylamino-3-(4-fluoro-benzyl)-1-(4-fluoro-phényl)-2,4-diméthyl-pent-1-ène-3-ol,
• 5-diméthylamino-1-(4-fluoro-phényl)-3-(3-méthoxy-benzyl)-2,4-diméthyl-pent-1-ène-3-ol,
• 3-(4-chloro-3-trifluorométhyl-phényl)-5-diméthylamino-1-(4-fluoro-phényl)-2,4-diméthyl-pent-1-ène-3-ol,
• 5-diméthylamino-3-(3-fluoro-benzyl)-1-(4-fluoro-phényl)-2,4-diméthyl-pent-1-ène-3-ol,
• 5-diméthylamino-1-(4-fluoro-phényl)-3-(2-méthoxy-phényl)-2,4-diméthyl-pent-1-ène-3-ol,
• 5-diméthylamino-1-(4-fluoro-phényl)-2,4-diméthyl-3-(2-méthyl-benzyl)-pent-1-ène-3-ol,
• 3-(3-chloro-4-fluoro-phényl)-5-diméthylamino-1-(4-fluoro-phényl)-2,4-diméthyl-pent-1-ène-3-ol,
• 5-diméthylamino-1-(4-fluoro-phényl)-2,4-diméthyl-3-(3-trifluorométhyl-phényl)-pent-1-ène-3-ol,
• 5-diméthylamino-1-(4-fluoro-phényl)-2,4-diméthyl-3-(3-méthyl-benzyl)-pent-1-ène-3-ol,
• 3-(4-chloro-benzyl)-5-diméthylamino-1-(4-fluoro-phényl)-2,4-diméthyl-pent-1-ène-3-ol,
• 3-(2-chloro-6-fluoro-benzyl)-5-diméthylamino-1-(4-fluoro-phényl)-2,4-diméthyl-pent-1-ène-3-ol,
• 5-diméthylamino-3-(2,5-diméthyl-benzyl)-1-(4-fluoro-phényl)-2,4-diméthyl-pent-1-ène-3-ol,
• 3-(3-chloro-benzyl)-5-diméthylamino-1-(4-fluoro-phényl)-2,4-diméthyl-pent-1-ène-3-ol,
• 3-(2,4-dichloro-benzyl)-5-diméthylamino-1-(4-fluoro-phényl)-2,4-diméthyl-pent-1-ène-3-ol,
• 1-(4-chloro-phényl)-5-diméthylamino-2,4-diméthyl-3-phényl-pent-1-ène-3-ol,
• 1,3-bis-(4-chloro-phényl)-5-diméthylamino-2,4-diméthyl-pent-1-ène-3-ol,
• 3-benzyl-1-(4-chloro-phényl)-5-diméthylamino-2,4-diméthyl-pent-1-ène-3-ol,
• 1-(4-chloro-phényl)-5-diméthylamino-3-(4-fluoro-3-méthyl-phényl)-2,4-diméthyl-pent-1-ène-3-ol,
• 1-(4-chloro-phényl)-5-diméthylamino-2,4-diméthyl-3-o-tolyl-pent-1-ène-3-ol,
• 1-(4-chloro-phényl)-3-(2-diméthylamino-1-méthyl-éthyl)-2-méthyl-penta-1,4-diène-3-ol,
• 3-(4-tertiobutyl-phényl)-1-(4-chloro-phényl)-5-diméthyl-amino-2,4-diméthyl-pent-1-ène-3-ol,
• 1-(4-chloro-phényl)-3-cyclopentyl-5-diméthylamino-2,4-diméthyl-pent-1-ène-3-ol,
• 1-(4-chloro-phényl)-5-diméthylamino-2,4-diméthyl-3-m-tolyl-pent-1-ène-3-ol,
• 1-(4-chloro-phényl)-3-cyclohexyl-5-diméthylamino-2,4-diméthyl-pent-1-ène-3-ol,
• 1-(4-chloro-phényl)-5-diméthylamino-3-(4-fluoro-phényl)-2,4-diméthyl-pent-1-ène-3-ol,
• 1-(4-chloro-phényl)-5-diméthylamino-2,4-diméthyl-3-phénéthyl-pent-1-ène-3-ol,
• 1-(4-chloro-phényl)-5-diméthylamino-2,4-diméthyl-3-thiophène-2-yl-pent-1-ène-3-ol,
• 1-(4-chloro-phényl)-3-(2,4-dichloro-phényl)-5-diméthyl-amino-2,4-diméthyl-pent-1-ène-3-ol,
• 1-(4-chloro-phényl)-5-diméthylamino-3-(3-méthoxy-phényl)-2,4-diméthyl-pent-1-ène-3-ol,
• 1-(4-chloro-phényl)-3-(2-diméthylamino-1-méthyl-éthyl)-2-méthyl-6-phényl-hex-1-ène-3-ol,
• 1-(4-chloro-phényl)-3-(2,3-dichloro-phényl)-5-diméthyl-amino-2,4-diméthyl-pent-1-ène-3-ol,
• 1-(4-chloro-phényl)-5-diméthylamino-2,4-diméthyl-3-p-tolyl-pent-1-ène-3-ol,
• 1-(4-chloro-phényl)-5-diméthylamino-3-(4-méthoxy-phényl)-2,4-diméthyl-pent-1-ène-3-ol,
• 1-(4-chloro-phényl)-3-cyclohexylméthyl-5-diméthyl-amino-2,4-diméthyl-pent-1-ène-3-ol,
• 1-(4-chloro-phényl)-5-diméthylamino-3-(5-fluoro-2-méthoxy-phényl)-2,4-diméthyl-pent-1-ène-3-ol,
• 1-(4-chloro-phényl)-5-diméthylamino-3-(3-fluoro-phényl)-2,4-diméthyl-pent-1-ène-3-ol,
• 3-(3-chloro-phényl)-1-(4-chloro-phényl)-5-diméthyl-amino-2,4-diméthyl-pent-1-ène-3-ol,
• 1-(4-chloro-phényl)-3-(3,5-dichloro-phényl)-5-diméthyl-amino-2,4-diméthyl-pent-1-ène-3-ol,
• 3-(2-chloro-benzyl)-1-(4-chloro-phényl)-5-diméthyl-amino-2,4-diméthyl-pent-1-ène-3-ol,
• 1-(4-chloro-phényl)-5-diméthylamino-3-(4-fluoro-benzyl)-2,4-diméthyl-pent-1-ène-3-ol,
• 1-(4-chloro-phényl)-5-diméthylamino-3-(3-méthoxy-benzyl)-2,4-diméthyl-pent-1-ène-3-ol,
• 1-(4-chloro-phényl)-3-(4-chloro-3-trifluorométhyl-phényl)-5-diméthylamino-2,4-diméthyl-pent-1-ène-3-ol,
• 1-(4-chloro-phényl)-5-diméthylamino-3-[2-(3-fluoro-phényl)-éthyl]-2,4-diméthyl-pent-1-ène-3-ol,
• 1-(4-chloro-phényl)-5-diméthylamino-3-(2-méthoxy-phényl)-2,4-diméthyl-pent-1-ène-3-ol,
• 1-(4-chloro-phényl)-5-diméthylamino-2,4-diméthyl-3-(2-méthyl-benzyl)-pent-1-ène-3-ol,
• 3-(3-chloro-4-fluoro-phényl)-1-(4-chloro-phényl)-5-diméthylamino-2,4-diméthyl-pent-1-ène-3-ol,
• 1-(4-chloro-phényl)-5-diméthylamino-2,4-diméthyl-3-(3-trifluorométhyl-phényl)-pent-1-ène-3-ol,
• 1-(4-chloro-phényl)-5-diméthylamino-2,4-diméthyl-3-(3-méthyl-benzyl)-pent-1-ène-3-ol,
• 3-(4-chloro-benzyl)-1-(4-chloro-phényl)-5-diméthyl-amino-2,4-diméthyl-pent-1-ène-3-ol,
• 3-(2-chloro-6-fluoro-benzyl)-1-(4-chloro-phényl)-5-diméthylamino-2,4-diméthyl-pent-1-ène-3-ol,
• 1-(4-chloro-phényl)-5-diméthylamino-3-(2,5-diméthyl-benzyl)-2,4-diméthyl-pent-1-ène-3-ol,
• 3-(3-chloro-benzyl)-1-(4-chloro-phényl)-5-diméthyl-amino-2,4-diméthyl-pent-1-ène-3-ol,
• 1-(4-chloro-phényl)-3-(2,4-dichloro-benzyl)-5-diméthyl-amino-2,4-diméthyl-pent-1-ène-3-ol,
• 5-diméthylamino-1-(4-méthoxy-phényl)-2,4-diméthyl-3-phényl-pent-1-ène-3-ol,
• 3-(4-chloro-phényl)-5-diméthylamino-1-(4-méthoxy-phényl)-2,4-diméthyl-pent-1-ène-3-ol,
• 3-benzyl-5-diméthylamino-1-(4-méthoxy-phényl)-2,4-diméthyl-pent-1-ène-3-ol,
• 5-diméthylamino-3-(4-fluoro-3-méthyl-phényl)-1-(4-méthoxy-phényl)-2,4-diméthyl-pent-1-ène-3-ol,
• 5-diméthylamino-1-(4-méthoxy-phényl)-2,4-diméthyl-3-o-tolyl-pent-1-ène-3-ol,
• 3-(2-diméthylamino-1-méthyl-éthyl)-1-(4-méthoxy-phényl)-2-méthyl-penta-1,4-diène-3-ol,
• 3-(4-tertiobutyl-phényl)-5-diméthylamino-1-(4-méthoxy-phényl)-2,4-diméthyl-pent-1-ène-3-ol,
• 3-cyclopentyl-5-diméthylamino-1-(4-méthoxy-phényl)-2,4-diméthyl-pent-1-ène-3-ol,
• 5-diméthylamino-1-(4-méthoxy-phényl)-2,4-diméthyl-3-m-tolyl-pent-1-ène-3-ol,
• 3-cyclohexyl-5-diméthylamino-1-(4-méthoxy-phényl)-2,4-diméthyl-pent-1-ène-3-ol,
• 5-diméthylamino-3-(4-fluoro-phényl)-1-(4-méthoxy-phényl)-2,4-diméthyl-pent-1-ène-3-ol,
• 5-diméthylamino-1-(4-méthoxy-phényl)-2,4-diméthyl-3-phénéthyl-pent-1-ène-3-ol,
• 3-(2-diméthylamino-1-méthyl-éthyl)-1-(4-méthoxy-phényl)-2-méthyl-5-phényl-pent-1-ène-4-yne-3-ol,
• 5-diméthylamino-1-(4-méthoxy-phényl)-2,4-diméthyl-3-thiophène-2-yl-pent-1-ène-3-ol,
• 3-(2,4-dichloro-phényl)-5-diméthylamino-1-(4-méthoxy-phényl)-2,4-diméthyl-pent-1-ène-3-ol,
• 5-diméthylamino-3-(3-méthoxy-phényl)-1-(4-méthoxy-phényl)-2,4-diméthyl-pent-1-ène-3-ol,
• 3-(2-diméthylamino-1-méthyl-éthyl)-1-(4-méthoxy-phényl)-2-méthyl-6-phényl-hex-1-ène-3-ol,
• 3-(2,3-dichloro-phényl)-5-diméthylamino-1-(4-méthoxy-phényl)-2,4-diméthyl-pent-1-ène-3-ol,
• 5-diméthylamino-1-(4-méthoxy-phényl)-2,4-diméthyl-3-p-tolyl-2-yl-pent-1-ène-3-ol,
• 5-diméthylamino-1,3-bis-(4-méthoxy-phényl)-2,4-diméthyl-pent-1-ène-3-ol,
• 3-cyclohexylméthyl-5-diméthylamino-1-(4-méthoxy-phényl)-2,4-diméthyl-pent-1-ène-3-ol,
• 5-diméthylamino-3-(5-fluoro-2-méthoxy-phényl)-1-(4-méthoxy-phényl)-2,4-diméthyl-pent-1-ène-3-ol,
• 5-diméthylamino-3-(3-fluoro-phényl)-1-(4-méthoxy-phényl)-2,4-diméthyl-pent-1-ène-3-ol,
• 3-(3-chloro-phényl)-5-diméthylamino-1-(4-méthoxy-phényl)-2,4-diméthyl-pent-1-ène-3-ol,
• 3-(3,5-dichloro-phényl)-5-diméthylamino-1-(4-méthoxy-phényl)-2,4-diméthyl-pent-1-ène-3-ol,
• 3-(2-chloro-benzyl)-5-diméthylamino-1-(4-méthoxy-phényl)-2,4-diméthyl-pent-1-ène-3-ol,
• 5-diméthylamino-3-(4-fluoro-benzyl)-1-(4-méthoxy-phényl)-2,4-diméthyl-pent-1-ène-3-ol,
• 5-diméthylamino-3-(3-fluoro-benzyl)-1-(4-méthoxy-phényl)-2,4-diméthyl-pent-1-ène-3-ol,
• 5-diméthylamino-3-(2-méthoxy-phényl)-1-(4-méthoxy-phényl)-2,4-diméthyl-pent-1-ène-3-ol,
• 5-diméthylamino-1-(4-méthoxy-phényl)-2,4-diméthyl-3-(2-méthyl-benzyl)-pent-1-ène-3-ol,
• 3-(3-chloro-4-fluoro-phényl)-5-diméthylamino-1-(4-méthoxy-phényl)-2,4-diméthyl-pent-1-ène-3-ol,
• 5-diméthylamino-1-(4-méthoxy-phényl)-2,4-diméthyl-3-(3-trifluorométhyl-phényl)-pent-1-ène-3-ol,
• 3-(4-chloro-benzyl)-5-diméthylamino-1-(4-méthoxy-phényl)-2,4-diméthyl-pent-1-ène-3-ol,
• 3-(2-chloro-6-fluoro-benzyl)-5-diméthylamino-1-(4-méthoxy-phényl)-2,4-diméthyl-pent-1-ène-3-ol,
• 3-(3-chloro-benzyl)-5-diméthylamino-1-(4-méthoxy-phényl)-2,4-diméthyl-pent-1-ène-3-ol,
• 2-benzylidène-1-(4-tertiobutyl-phényl)-5-diméthyl-aminométhyl-cyclopentanol,
• 2-benzylidène-1-cyclohexyl-5-diméthylaminométhyl-cyclopentanol,
• 2-benzylidène-5-diméthylaminométhyl-1-phénéthyl-cyclopentanol,
• 2-benzylidène-5-diméthylaminométhyl-1-(4-fluoro-benzyl)-cyclopentanol,
• 2-benzylidène-5-diméthylaminométhyl-1-(3-fluoro-benzyl)-cyclopentanol,
• 2-benzylidène-5-diméthylaminométhyl-1-(2-méthoxy-phényl)-cyclopentanol,
• 2-benzylidène-5-diméthylaminométhyl-1-(2-méthyl-benzyl)-cyclopentanol,
• 2-benzylidène-5-diméthylaminométhyl-1-(3-méthyl-benzyl)-cyclopentanol,
• 2-benzylidène-1-(4-chloro-benzyl)-5-diméthylamino-méthyl-cyclopentanol,
• 2-benzylidène-1-(2-chloro-6-fluoro-benzyl)-5-diméthyl-aminométhyl-cyclopentanol,
• 2-benzylidène-5-diméthylaminométhyl-1-(2,5-diméthyl-benzyl)-cyclopentanol,
• 2-benzylidène-1-(3-chloro-benzyl)-5-diméthylamino-méthyl-cyclopentanol,
• 2-diméthylaminométhyl-5-(3-méthoxy-benzyl)-bicyclo-pentyl-1-ol,
• 2-diméthylaminométhyl-5-(3-méthoxy-benzyl)-1-m-tolyl-cyclopentanol,
• 1-cyclohexyl-2-diméthylaminométhyl-5-(3-méthoxy-benzyl)-cyclopentanol,
• 2-diméthylaminométhyl-5-(3-méthoxy-benzyl)-1-phénéthyl-cyclopentanol,
• 2-diméthylaminométhyl-5-(3-méthoxy-benzyl)-1-phényl-éthynyl-cyclopentanol,
• 2-diméthylaminométhyl-5-(3-méthoxy-benzyl)-1-thiophène-2-yl-cyclopentanol,
• 2-diméthylaminométhyl-5-(3-méthoxy-benzyl)-1-(3-méthoxy-phényl)-cyclopentanol,
• 2-diméthylaminométhyl-5-(3-méthoxy-benzyl)-1-(3-phényl-propyl)-cyclopentanol,
• 2-diméthylaminométhyl-5-(3-méthoxy-benzyl)-1-(4-méthoxy-phényl)-cyclopentanol,
• 2-diméthylaminométhyl-1-(3-fluoro-benzyl)-5-(3-méthoxy-benzylidène)-cyclopentanol,
• 2-diméthylaminométhyl-5-(3-méthoxy-benzylidène)-1-(2-méthoxy-phényl)-cyclopentanol,
• 2-diméthylaminométhyl-5-(3-méthoxy-benzylidène)-1-(2-méthyl-benzyl)-cyclopentanol,
• 2-diméthylaminométhyl-5-(3-méthoxy-benzylidène)-1-(3-méthyl-benzyl)-cyclopentanol,
• 2-diméthylaminométhyl-1-(2,5-diméthyl-benzyl)-5-(3-méthoxy-benzylidène)-cyclopentanol,
• 1-(3-chloro-benzyl)-2-diméthylaminométhyl-5-(3-méthoxy-benzylidène)-cyclopentanol,
• 1-benzyl-2-diméthylaminométhyl-5-(3-méthoxy-benzylidène)-cyclopentanol,
• 2-diméthylaminométhyl-5-(3-méthoxy-benzylidène)-1-o-tolyl-cyclopentanol,
• 2-diméthylaminométhyl-5-(3-méthoxy-benzylidène)-1-vinyl-cyclopentanol,
• 1-(4-tertiobutyl-phényl)-2-diméthylaminométhyl-5-(3-méthoxy-benzylidène)-cyclopentanol,
• 2-diméthylaminométhyl-5-(3-méthoxy-benzylidène)-bicyclopentyl-1-ol,
• 1-cyclohexyl-2-diméthylaminométhyl-5-(3-méthoxy-benzylidène)-cyclopentanol,
• 2-diméthylaminométhyl-1-(4-fluoro-phényl)-5-(3-méthoxy-benzylidène)-cyclopentanol,
• 2-diméthylaminométhyl-5-(3-méthoxy-benzylidène)-1-phénéthyl-cyclopentanol,
• 2-diméthylaminométhyl-5-(3-méthoxy-benzylidène)-1-phényléthynyl-cyclopentanol,
• 2-diméthylaminométhyl-5-(3-méthoxy-benzylidène)-1-thiophène-2-yl-cyclopentanol,
• 2-diméthylaminométhyl-5-(3-méthoxy-benzylidène)-1-(3-méthoxy-phényl)-cyclopentanol,
• 2-diméthylaminométhyl-5-(3-méthoxy-benzylidène)-1-(3-phényl-propyl)-cyclopentanol,
• 2-diméthylaminométhyl-5-(3-méthoxy-benzylidène)-1-p-tolyl-cyclopentanol,
• 2-diméthylaminométhyl-5-(3-méthoxy-benzylidène)-1-(4-méthoxy-phényl)-cyclopentanol,
• 1-(2-chloro-benzyl)-2-diméthylaminométhyl-5-(4-méthoxy-benzylidène)-cyclopentanol,
• 2-diméthylaminométhyl-1-(3-méthoxy-benzyl)-5-(4-méthoxy-benzylidène)-cyclopentanol,
• 2-diméthylaminométhyl-1-(3-fluoro-benzyl)-5-(4-méthoxy-benzylidène)-cyclopentanol,
• 2-diméthylaminométhyl-5-(4-méthoxy-benzylidène)-1-(2-méthoxy-phényl)-cyclopentanol,
• 2-diméthylaminométhyl-5-(4-méthoxy-benzylidène)-1-(3-méthyl-benzyl)-cyclopentanol,
• 1-(4-chloro-benzyl)-2-diméthylaminométhyl-5-(4-méthoxy-benzylidène)-cyclopentanol,
• 1-(2-chloro-6-fluoro-benzyl)-2-diméthylaminométhyl-5-(4-méthoxy-benzylidène)-cyclopentanol,
• 1-(3-chloro-benzyl)-2-diméthylaminométhyl-5-(4-méthoxy-benzylidène)-cyclopentanol,
• 1-(2,4-dichloro-benzyl)-2-diméthylaminométhyl-5-(4-méthoxy-benzylidène)-cyclopentanol,
• 2-benzylidène-7-diméthylaminométhyl-1-phényl-cycloheptanol,
• 2-benzylidène-1-(4-chloro-phényl)-7-diméthylaminométhyl-cycloheptanol,
• 1-benzyl-2-benzylidène-7-diméthylaminométhyl-cyclo-heptanol,
• 2-benzylidène-7-diméthylaminométhyl-1-(4-fluoro-3-méthyl-phényl)-cycloheptanol,
• 2-benzylidène-7-diméthylaminométhyl-1-o-tolyl-cyclo-heptanol,
• 2-benzylidène-7-diméthylaminométhyl-1-vinyl-cyclo-heptanol,
• 2-benzylidène-1-(4-tertiobutyl-phényl)-7-diméthyl-aminométhyl-cycloheptanol,
• 2-benzylidène-1-cyclopentyl-7-diméthylaminométhyl-cycloheptanol,
• 2-benzylidène-7-diméthylaminométhyl-1-m-tolyl-cyclo-heptanol,
• 2-benzylidène-1-cyclohexyl-7-diméthylaminométhyl-cycloheptanol,
• 2-benzylidène-7-diméthylaminométhyl-1-(4-fluoro-phényl)-cycloheptanol,
• 2-benzylidène-7-diméthylaminométhyl-1-phényléthynyl-cycloheptanol,
• 2-benzylidène-7-diméthylaminométhyl-1-thiophène-2-yl-cycloheptanol,
• 2-benzylidène-7-diméthylaminométhyl-1-(3-méthoxy-phényl)-cycloheptanol,
• 2-benzylidène-1-cyclohexylméthyl-7-diméthylamino-méthyl-cycloheptanol,
• 2-benzylidène-7-diméthylaminométhyl-1-(3-fluoro-4-méthoxy-phényl)-cycloheptanol, '
• 2-benzylidène-7-diméthylaminométhyl-1-(3-fluoro-phényl)-cycloheptanol,
• 2-benzylidène-1-(3-chloro-phényl)-7-diméthylamino-méthyl-cycloheptanol,
• 2-benzylidène-1-(3,5-dichloro-phényl)-7-diméthyl-aminométhyl-cycloheptanol,
• 2-benzylidène-7-diméthylaminométhyl-1-(4-fluoro-benzyl)-cycloheptanol,
• 2-benzylidène-7-diméthylaminométhyl-1-(4-méthoxy-benzyl)-cycloheptanol,
• 2-benzylidène-7-diméthylaminométhyl-1-(3-fluoro-benzyl)-cycloheptanol,
• 2-benzylidène-7-diméthylaminométhyl-1-(2-méthoxy-phényl)-cycloheptanol,
• 2-benzylidène-7-diméthylaminométhyl-1-(2-méthyl-benzyl)-cycloheptanol,
• 2-benzylidène-1-(3-chloro-4-fluoro-phényl)-7-diméthyl-aminométhyl-cycloheptanol,
• 2-benzylidène-7-diméthylaminométhyl-1-(3-trifluoro-méthyl-phényl)-cycloheptanol,
• 2-benzylidène-7-diméthylaminométhyl-1-(3-méthyl-benzyl)-cycloheptanol,
• 2-benzylidène-1-(4-chloro-benzyl)-7-diméthylamino-méthyl-cycloheptanol,
• 2-benzylidène-1-(2-chloro-6-fluoro-benzyl)-7-diméthyl-aminométhyl-cycloheptanol,
• 2-benzylidène-7-diméthylaminométhyl-1-(2,5-diméthyl-benzyl)-cycloheptanol,
• 2-benzylidène-1-(3-chloro-benzyl)-7-diméthylamino-méthyl-cycloheptanol,
• 2-diméthylaminométhyl-7-(3-méthoxy-benzylidène)-1-phényl-cycloheptanol,
• 1-(4-chloro-phényl)-2-diméthylaminométhyl-7-(3-méthoxy-benzylidène)-cycloheptanol,
• 1-benzyl-2-diméthylaminométhyl-7-(3-méthoxy-benzylidène)-cycloheptanol,
• 2-diméthylaminométhyl-1-(4-fluoro-3-méthyl-phényl)-7-(3-méthoxy-benzylidène)-cycloheptanol,
• 2-diméthylaminométhyl-7-(3-méthoxy-benzylidène)-1-o-tolyl-cycloheptanol,
• 2-diméthylaminométhyl-7-(3-méthoxy-benzylidène)-1-vinyl-cycloheptanol,
• 1-(4-tertiobutyl-phényl)-2-diméthylaminométhyl-7-(3-méthoxy-benzylidène)-cycloheptanol,
• 1-cyclopentyl-2-diméthylaminométhyl-7-(3-méthoxy-benzylidène)-cycloheptanol,
• 2-diméthylaminométhyl-7-(3-méthoxy-benzylidène)-1-m-tolyl-cycloheptanol,
• 1-cyclohexyl-2-diméthylaminométhyl-7-(3-méthoxy-benzylidène)-cycloheptanol,
• 2-diméthylaminométhyl-1-(4-fluoro-phényl)-7-(3-méthoxy-benzylidène)-cycloheptanol,
• 2-diméthylaminométhyl-7-(3-méthoxy-benzylidène)-1-phénéthyl-cycloheptanol,
• 2-diméthylaminométhyl-7-(3-méthoxy-benzylidène)-1-phényléthynyl-cycloheptanol,
• 2-diméthylaminométhyl-7-(3-méthoxy-benzylidène)-1-thiophène-2-yl-cycloheptanol,
• 2-diméthylaminométhyl-7-(3-méthoxy-benzylidène)-1-(3-méthoxy-phényl)-cycloheptanol,
• 2-diméthylaminométhyl-7-(3-méthoxy-benzylidène)-1-(3-phényl-propyl)-cycloheptanol,
• 2-diméthylaminométhyl-7-(3-méthoxy-benzylidène)-1-p-tolyl-cycloheptanol,
• 2-diméthylaminométhyl-7-(3-méthoxy-benzylidène)-1-(4-méthoxy-phényl)-cycloheptanol,
• 1-cyclohexylméthyl-2-diméthylaminométhyl-7-(3-méthoxy-benzylidène)-cycloheptanol,
• 1-(3-chloro-phényl)-2-diméthylaminométhyl-7-(3-méthoxy-benzylidène)-cycloheptanol,
• 1-(3,5-dichloro-phényl)-2-diméthylaminométhyl-7-(3-méthoxy-benzylidène)-cycloheptanol,
• 1-(2-chloro-benzyl)-2-diméthylaminométhyl-7-(3-méthoxy-benzylidène)-cycloheptanol,
• 1-(4-chloro-3-trifluorométhyl-phényl)-2-diméthylamino-méthyl-7-(3-méthoxy-benzylidène)-cycloheptanol,
• 2-diméthylaminométhyl-1-(3-fluoro-phényl)-7-(3-méthoxy-benzylidène)-cycloheptanol,
• 2-diméthylaminométhyl-7-(3-méthoxy-benzylidène)-1-(2-méthoxy-phényl)-cycloheptanol,
• 2-diméthylaminométhyl-7-(3-méthoxy-benzylidène)-1-(2-méthyl-benzyl)-cycloheptanol,
• 2-diméthylaminométhyl-7-(3-méthoxy-benzylidène)-1-(3-trifluorométhyl-phényl)-cycloheptanol,
• 2-diméthylaminométhyl-7-(3-méthoxy-benzylidène)-1-(3-méthyl-benzyl)-cycloheptanol,
• 2-diméthylaminométhyl-1-(2,5-diméthyl-benzyl)-7-(3-méthoxy-benzylidène)-cycloheptanol,
• 1-(3-chloro-benzyl)-2-diméthylaminométhyl-7-(3-méthoxy-benzylidène)-cycloheptanol,
• 1-benzyl-2-diméthylaminométhyl-7-(4-méthoxy-benzylidène)-cycloheptanol,
• 2-diméthylaminométhyl-1-(4-fluoro-3-méthyl-phényl)-7-(4-méthoxy-benzylidène)-cycloheptanol,
• 2-diméthylaminométhyl-7-(4-méthoxy-benzylidène)-1-o-tolyl-cycloheptanol,
• 2-diméthylaminométhyl-7-(4-méthoxy-benzylidène)-1-vinyl-cycloheptanol,
• 1-(4-tertiobutyl-phényl)-2-diméthylaminométhyl-7-(4-méthoxy-benzylidène)-cycloheptanol,
• 1-cyclopentyl-2-diméthylaminométhyl-7-(4-méthoxy-benzylidène)-cycloheptanol,
• 2-diméthylaminométhyl-7-(4-méthoxy-benzylidène)-1-m-tolyl-cycloheptanol,
• 1-cyclohexyl-2-diméthylaminométhyl-7-(4-méthoxy-benzylidène)-cycloheptanol,
• 2-diméthylaminométhyl-1-(4-fluoro-phényl)-7-(4-méthoxy-benzylidène)-cycloheptanol,
• 2-diméthylaminométhyl-7-(4-méthoxy-benzylidène)-1-phénéthyl-cycloheptanol,
• 2-diméthylaminométhyl-7-(4-méthoxy-benzylidène)-1-phényléthynyl-cycloheptanol,
• 2-diméthylaminométhyl-7-(4-méthoxy-benzylidène)-1-thiophène-2-yl-cycloheptanol,
• 2-diméthylaminométhyl-7-(4-méthoxy-benzylidène)-1-(3-méthoxy-phényl)-cycloheptanol,
• 2-diméthylaminométhyl-7-(4-méthoxy-benzylidène)-1-(3-phényl-propyl)-cycloheptanol,
• 2-diméthylaminométhyl-7-(4-méthoxy-benzylidène)-1-p-tolyl-cycloheptanol,
• 2-diméthylaminométhyl-7-(4-méthoxy-benzylidène)-1-(4-méthoxy-phényl)-cycloheptanol,
• 1-cyclohexylméthyl-2-diméthylaminométhyl-7-(4-méthoxy-benzylidène)-cycloheptanol,
• 1-(3-chloro-phényl)-2-diméthylaminométhyl-7-(4-méthoxy-benzylidène)-cycloheptanol,
• 1-(2-chloro-benzyl)-2-diméthylaminométhyl-7-(4-méthoxy-benzylidène)-cycloheptanol,
• 2-diméthylaminométhyl-7-(4-méthoxy-benzylidène)-1-(2-méthoxy-phényl)-cycloheptanol,
• 2-diméthylaminométhyl-7-(4-méthoxy-benzylidène)-1-(2-méthyl-benzyl)-cycloheptanol,
• 2-diméthylaminométhyl-7-(4-méthoxy-benzylidène)-1-(3-trifluorométhyl-phényl)-cycloheptanol,
• 2-diméthylaminométhyl-7-(4-méthoxy-benzylidène)-1-(3-méthyl-benzyl)-cycloheptanol,
• 1-(3-chloro-benzyl)-2-diméthylaminométhyl-7-(4-méthoxy-benzylidène)-cycloheptanol,
• 1-(3,5-dichloro-phényl)-2-diméthylaminométhyl-7-(4-méthoxy-benzylidène)-cycloheptanol,
• 2-benzylidène-7-diméthylaminométhyl-1-(3-hydroxy-phényl)-cycloheptanol,
• 2-diméthylaminométhyl-1-(3-hydroxy-phényl)-7-(3-méthoxy-benzylidène)-cycloheptanol,
• 2-benzylidène-7-diméthylaminométhyl-1-(3-méthoxy-phényl)-cycloheptanol,
• 3-[1-(2-diméthylamino-1-méthyl-éthyl)-1-hydroxy-2-méthyl-3-phényl-allyl]-phénol,
• 3-(4-chloro-benzyl)-5-diméthylamino-2,4-diméthyl-1-phényl-pent-1-ène-3-ol,
• 5-diméthylamino-3-(3-méthoxy-phényl)-2,4-diméthyl-1-phényl-pent-1-ène-3-ol,
• 3-(2-benzylidène-6-diméthylaminométhyl-1-hydroxy-cyclohexyl)-phénol,
• 1-benzyl-2-benzylidène-7-(4-méthyl-pipérazine-1-ylméthyl)-cycloheptanol,
• 2-benzylidène-1-(2-chloro-benzyl)-7-(4-méthyl-pipérazine-1-ylméthyl)-cycloheptanol,
• 2-benzylidène-1-(3-chloro-benzyl)-7-(4-méthyl-pipérazine-1-ylméthyl)-cycloheptanol,
• 2-benzylidène-1-(4-chloro-benzyl)-7-(4-méthyl-pipérazine-1-ylméthyl)-cycloheptanol,
• 2-benzylidène-1-(2-fluoro-benzyl)-7-(4-méthyl-pipérazine-1-ylméthyl)-cycloheptanol,
• 2-benzylidène-1-(3-fluoro-benzyl)-7-(4-méthyl-pipérazine-1-ylméthyl)-cycloheptanol,
• 2-benzylidène-1-(4-fluoro-benzyl)-7-(4-méthyl-pipérazine-1-ylméthyl)-cycloheptanol,
• 2-benzylidène-1-(2-méthyl-benzyl)-7-(4-méthyl-pipérazine-1-ylméthyl)-cycloheptanol,
• 2-benzylidène-1-(3-méthyl-benzyl)-7-(4-méthyl-pipérazine-1-ylméthyl)-cycloheptanol,
• 2-benzylidène-1-(4-méthyl-benzyl)-7-(4-méthyl-pipérazine-1-ylméthyl)-cycloheptanol,
• 2-benzylidène-1-(2-méthoxy-benzyl)-7-(4-méthyl-pipérazine-1-ylméthyl)-cycloheptanol,
• 2-benzylidène-1-(3-méthoxy-benzyl)-7-(4-méthyl-pipérazine-1-ylméthyl)-cycloheptanol,
• 2-benzylidène-1-(4-méthoxy-benzyl)-7-(4-méthyl-pipérazine-1-ylméthyl)-cycloheptanol,
• 2-benzylidène-1-(2,6-dichloro-benzyl)-7-(4-méthyl-pipérazine-1-ylméthyl)-cycloheptanol,
• 2-benzylidène-1-(2-chloro-6-fluoro-benzyl)-7-(4-méthyl-pipérazine-1-ylméthyl)-cycloheptanol,
• 2-benzylidène-1-(2,6-difluoro-benzyl)-7-(4-méthyl-pipérazine-1-ylméthyl)-cycloheptanol,
• 1-benzyl-2-(4-chloro-benzylidène)-7-(4-méthyl-pipérazine-1-ylméthyl)-cycloheptanol,
• 1-(2-chloro-benzyl)-2-(4-chloro-benzylidène)-7-(4-méthyl-pipérazine-1-ylméthyl)-cycloheptanol,
• 1-(3-chloro-benzyl)-2-(4-chloro-benzylidène)-7-(4-méthyl-pipérazine-1-ylméthyl)-cycloheptanol,
• 1-(4-chloro-benzyl)-2-(4-chloro-benzylidène)-7-(4-méthyl-pipérazine-1-ylméthyl)-cycloheptanol,
• 2-(4-chloro-benzylidène)-1-(2-fluoro-benzyl)-7-(4-méthyl-pipérazine-1-ylméthyl)-cycloheptanol,
• 2-(4-chloro-benzylidène)-1-(3-fluoro-benzyl)-7-(4-méthyl-pipérazine-1-ylméthyl)-cycloheptanol,
• 2-(4-chloro-benzylidène)-1-(4-fluoro-benzyl)-7-(4-méthyl-pipérazine-1-ylméthyl)-cycloheptanol,
• 2-(4-chloro-benzylidène)-1-(2-méthyl-benzyl)-7-(4-méthyl-pipérazine-1-ylméthyl)-cycloheptanol,
• 2-(4-chloro-benzylidène)-1-(3-méthyl-benzyl)-7-(4-méthyl-pipérazine-1-ylméthyl)-cycloheptanol,
• 2-(4-chloro-benzylidène)-1-(4-méthyl-benzyl)-7-(4-méthyl-pipérazine-1-ylméthyl)-cycloheptanol,
• 2-(4-chloro-benzylidène)-1-(2-méthoxy-benzyl)-7-(4-méthyl-pipérazine-1-ylméthyl)-cycloheptanol,
• 2-(4-chloro-benzylidène)-1-(3-méthoxy-benzyl)-7-(4-méthyl-pipérazine-1-ylméthyl)-cycloheptanol,
• 2-(4-chloro-benzylidène)-1-(4-méthoxy-benzyl)-7-(4-méthyl-pipérazine-1-ylméthyl)-cycloheptanol,
• 2-(4-chloro-benzylidène)-1-(2,6-dichloro-benzyl)-7-(4-méthyl-pipérazine-1-ylméthyl)-cycloheptanol,
• 2-(4-chloro-benzylidène)-1-(2,6-difluoro-benzyl)-7-(4-méthyl-pipérazine-1-ylméthyl)-cycloheptanol,
• 2-(4-chloro-benzylidène)-1-(2-chloro-6-fluoro-benzyl)-7-(4-méthyl-pipérazine-1-ylméthyl)-cycloheptanol,
• 1-benzyl-2-(4-fluoro-benzylidène)-7-(4-méthyl-pipérazine-1-ylméthyl)-cycloheptanol,
• 1-(2-chloro-benzyl)-2-(4-fluoro-benzylidène)-7-(4-méthyl-pipérazine-1-ylméthyl)-cycloheptanol,
• 1-(3-chloro-benzyl)-2-(4-fluoro-benzylidène)-7-(4-méthyl-pipérazine-1-ylméthyl)-cycloheptanol,
• 1-(4-chloro-benzyl)-2-(4-fluoro-benzylidène)-7-(4-méthyl-pipérazine-1-ylméthyl)-cycloheptanol,
• 1-(2-fluoro-benzyl)-2-(4-fluoro-benzylidène)-7-(4-méthyl-pipérazine-1-ylméthyl)-cycloheptanol,
• 1-(3-fluoro-benzyl)-2-(4-fluoro-benzylidène)-7-(4-méthyl-pipérazine-1-ylméthyl)-cycloheptanol,
• 1-(4-fluoro-benzyl)-2-(4-fluoro-benzylidène)-7-(4-méthyl-pipérazine-1-ylméthyl)-cycloheptanol,
• 2-(4-fluoro-benzylidène)-1-(2-méthyl-benzyl)-7-(4-méthyl-pipérazine-1-ylméthyl)-cycloheptanol,
• 2-(4-fluoro-benzylidène)-1-(3-méthyl-benzyl)-7-(4-méthyl-pipérazine-1-ylméthyl)-cycloheptanol,
• 2-(4-fluoro-benzylidène)-1-(4-méthyl-benzyl)-7-(4-méthyl-pipérazine-1-ylméthyl)-cycloheptanol,
• 2-(4-fluoro-benzylidène)-1-(2-méthoxy-benzyl)-7-(4-méthyl-pipérazine-1-ylméthyl)-cycloheptanol,
• 2-(4-fluoro-benzylidène)-1-(3-méthoxy-benzyl)-7-(4-méthyl-pipérazine-1-ylméthyl)-cycloheptanol,
• 2-(4-fluoro-benzylidène)-1-(4-méthoxy-benzyl)-7-(4-méthyl-pipérazine-1-ylméthyl)-cycloheptanol,
• 1-(2,6-dichloro-benzyl)-2-(4-fluoro-benzylidène)-7-(4-méthyl-pipérazine-1-ylméthyl)-cycloheptanol,
• 1-(2,6-difluoro-benzyl)-2-(4-fluoro-benzylidène)-7-(4-méthyl-pipérazine-1-ylméthyl)-cycloheptanol,
• 1-(2-chloro-6-fluoro-benzyl)-2-(4-fluoro-benzylidène)-7-(4-méthyl-pipérazine-1-ylméthyl)-cycloheptanol,
• 1-benzyl-2-furanne-2-ylméthylène-7-(4-méthyl-pipérazine-1-ylméthyl)-cycloheptanol,
• 1-(2-chloro-benzyl)-2-furanne-2-ylméthylène-7-(4-méthyl-pipérazine-1-ylméthyl)-cycloheptanol,
• 1-(3-chloro-benzyl)-2-furanne-2-ylméthylène-7-(4-méthyl-pipérazine-1-ylméthyl)-cycloheptanol,
• 1-(4-chloro-benzyl)-2-furanne-2-ylméthylène-7-(4-méthyl-pipérazine-1-ylméthyl)-cycloheptanol,
• 1-(2-fluoro-benzyl)-2-furanne-2-ylméthylène-7-(4-méthyl-pipérazine-1-ylméthyl)-cycloheptanol,
• 1-(3-fluoro-benzyl)-2-furanne-2-ylméthylène-7-(4-méthyl-pipérazine-1-ylméthyl)-cycloheptanol,
• 1-(4-fluoro-benzyl)-2-furanne-2-ylméthylène-7-(4-méthyl-pipérazine-1-ylméthyl)-cycloheptanol,
• 2-furanne-2-ylméthylène-1-(2-méthyl-benzyl)-7-(4-méthyl-pipérazine-1-ylméthyl)-cycloheptanol,
• 2-furanne-2-ylméthylène-1-(3-méthyl-benzyl)-7-(4-méthyl-pipérazine-1-ylméthyl)-cycloheptanol,
• 2-furanne-2-ylméthylène-1-(4-méthyl-benzyl)-7-(4-méthyl-pipérazine-1-ylméthyl)-cycloheptanol,
• 2-furanne-2-ylméthylène-1-(2-méthoxy-benzyl)-7-(4-méthyl-pipérazine-1-ylméthyl)-cycloheptanol,
• 2-furanne-2-ylméthylène-1-(3-méthoxy-benzyl)-7-(4-méthyl-pipérazine-1-ylméthyl)-cycloheptanol,
• 2-furanne-2-ylméthylène-1-(4-méthoxy-benzyl)-7-(4-méthyl-pipérazine-1-ylméthyl)-cycloheptanol,
• 1-(2,6-dichloro-benzyl)-2-furanne-2-ylméthylène-7-(4-méthyl-pipérazine-1-ylméthyl)-cycloheptanol,
• 1-(2,6-difluoro-benzyl)-2-furanne-2-ylméthylène-7-(4-méthyl-pipérazine-1-ylméthyl)-cycloheptanol,
• 1-(2-chloro-6-fluoro-benzyl)-2-furanne-2-ylméthylène-7-(4-méthyl-pipérazine-1-ylméthyl)-cycloheptanol,
• 1-benzyl-3-furanne-2-ylméthylène-7-(4-méthyl-pipérazine-1-ylméthyl)-cycloheptanol,
• 1-(2-chloro-benzyl)-3-furanne-2-ylméthylène-7-(4-méthyl-pipérazine-1-ylméthyl)-cycloheptanol,
• 1-(3-chloro-benzyl)-3-furanne-2-ylméthylène-7-(4-méthyl-pipérazine-1-ylméthyl)-cycloheptanol,
• 1-(4-chloro-benzyl)-3-furanne-2-ylméthylène-7-(4-méthyl-pipérazine-1-ylméthyl)-cycloheptanol,
• 1-(2-fluoro-benzyl)-3-furanne-2-ylméthylène-7-(4-méthyl-pipérazine-1-ylméthyl)-cycloheptanol,
• 1-(3-fluoro-benzyl)-3-furanne-2-ylméthylène-7-(4-méthyl-pipérazine-1-ylméthyl)-cycloheptanol,
• 1-(4-fluoro-benzyl)-3-furanne-2-ylméthylène-7-(4-méthyl-pipérazine-1-ylméthyl)-cycloheptanol,
• 3-furanne-2-ylméthylène-1-(2-méthyl-benzyl)-7-(4-méthyl-pipérazine-1-ylméthyl)-cycloheptanol,
• 3-furanne-2-ylméthylène-1-(3-méthyl-benzyl)-7-(4-méthyl-pipérazine-1-ylméthyl)-cycloheptanol,
• 3-furanne-2-ylméthylène-1-(4-méthyl-benzyl)-7-(4-méthyl-pipérazine-1-ylméthyl)-cycloheptanol,
• 3-furanne-2-ylméthylène-1-(2-méthoxy-benzyl)-7-(4-méthyl-pipérazine-1-ylméthyl)-cycloheptanol,
• 3-furanne-2-ylméthylène-1-(3-méthoxy-benzyl)-7-(4-méthyl-pipérazine-1-ylméthyl)-cycloheptanol,
• 3-furanne-2-ylméthylène-1-(4-méthoxy-benzyl)-7-(4-méthyl-pipérazine-1-ylméthyl)-cycloheptanol,
• 1-(2,6-dichloro-benzyl)-3-furanne-2-ylméthylène-7-(4-méthyl-pipérazine-1-ylméthyl)-cycloheptanol,
• 1-(2,6-difluoro-benzyl)-3-furanne-2-ylméthylène-7-(4-méthyl-pipérazine-1-ylméthyl)-cycloheptanol,
• 1-(2-chloro-6-fluoro-benzyl)-3-furanne-2-ylméthylène-7-(4-méthyl-pipérazine-1-ylméthyl)-cycloheptanol,
éventuellement sous forme de leurs racémates, de leurs stéréoisomères purs, en particulier des énantiomères ou des diastéréoisomères, ou sous forme de mélanges des stéréoisomères, en particulier des énantiomères ou des diastéréoisomères, dans un rapport de mélange quelconque ; sous la forme représentée ou sous forme de leurs acides ou de leurs bases ou sous forme de leurs sels, en particulier des sels physiologiquement acceptables, ou sous forme de leurs produits de solvatation, en particulier des hydrates, avantageusement sous forme du chlorhydrate ou du dichlorhydrate.

9. Dérivés substitués de 5-amino-1-pentène-3-ol suivant l'une des revendications 1 à 8, **caractérisés en ce que** les isomères E des dérivés selon la formule I sont avantageusement tels que représentés par la formule I' :

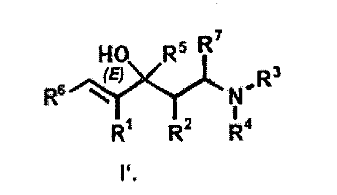

10. Dérivés substitués de 5-amino-1-pentène-3-ol suivant l'une des revendications 1 à 9, **caractérisés en ce que**, lorsque R⁷ représente l'hydrogène et R¹ et R² forment ensemble un noyau, le groupe OH et le groupe aminométhylène CHR⁷-NR³R⁴ selon la formule I sont en configuration cis l'un par rapport à l'autre, comme représenté par la formule I" :

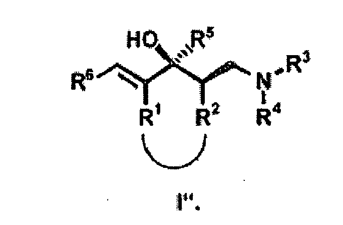

11. Médicament contenant au moins un dérivé substitué de 5-amino-1-pentène-3-ol suivant l'une des revendications 1 à 10, ainsi que, le cas échéant, des additifs et/ou des substances auxiliaires convenables et/ou, le cas échéant, d'autres substances actives.

12. Médicament suivant la revendication 11, **caractérisé en ce qu'**un dérivé substitué de 5-amino-1-pentène-ol qu'il contient selon l'une des revendications 1 à 10, est présent comme diastéréoisomère et/ou énantiomère pur, comme racémate ou comme mélange non équimolaire ou équimolaire des diastéréoisomères et/ou des énantiomères.

13. Utilisation d'un dérivé substitué de 5-amino-1-pentène-3-ol selon l'une des revendications 1 à 10, pour la préparation d'un médicament destiné au traitement de la douleur, en particulier d'une douleur aiguë, neuropathique ou chronique.

14. Utilisation d'un dérivé substitué de 5-amino-1-pentène-3-ol selon l'une des revendications 1 à 10, pour la préparation d'un médicament destiné au traitement de la migraine, de l'hyperalgésie et de l'allodynie, en particulier de l'hyperalgésie thermique, de l'hyperalgésie et de l'allodynie mécaniques et de l'allodynie en rapport avec le froid, ou d'une douleur inflammatoire ou post-opératoire.

15. Utilisation d'un dérivé substitué de 5-amino-1-pentène-3-ol selon l'une des revendications 1 à 10, pour la préparation d'un médicament destiné au traitement de l'épilepsie, de bouffées de chaleur, de problèmes survenant lors la ménopause, de la sclérose latérale amyotrophique (ALS), de la dystrophie sympathique réflexe (RSD), de la paralysie spastique, de restless leg syndrome, de nystagmus acquis ; de troubles psychiatriques ou neuropathologiques tels que des troubles bipolaires, l'anxiété, des crises de panique, des variations de l'humeur, un comportement maniaque, des dépressions, un comportement maniaco-dépressif ; une neuropathie diabétique douloureuse, des symptômes et des douleurs en rapport avec la sclérose en plaques ou la maladie de Parkinson, des maladies neurodégénératives telles que la maladie d'Alzheimer, la maladie de Huntington, la maladie de Parkinson et l'épilepsie ; une lésion gastro-intestinale ; d'une douleur érythromélalgique ou postpoliomyélitique, de la névralgie essentielle du trijumeau ou de la névralgie postherpétique ; ou comme anticonvulsif, analgésique ou anxiolytique.

16. Utilisation suivant l'une des revendications 13 à 15, **caractérisée en ce qu'**un dérivé substitué de 5-amino-1-pentène-3-ol, utilisé selon l'une des revendications 1 à 10, est présent comme diastéréoisomère et/ou énantiomère pur, comme racémate ou comme mélange non équimolaire ou équimolaire des diastéréoisomères et/ou des énantiomères.

17. Procédé de production d'un dérivé substitué de 5-amino-1-pentène-3-ol selon l'une des revendications 1 à 10, dans lequel une β-aminocétone de formule IA, dans laquelle les restes R¹ à R⁴, R⁶ et R⁷ ont la définition indiquée pour la formule I dans la revendication 1 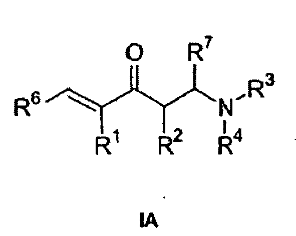 est amenée à réagir avec un composé organométallique de formule III
**R⁵-Z** **III**
dans laquelle Z représente MgCl, MgBr, MgI ou Li et R⁵ a la définition indiquée pour la formule I dans la revendication 1, pour obtenir un composé de formule I.
